# EUROPEAN PATENT SPECIFICATION

(11) **EP 2 144 909 B1**
(45) Date of publication and mention of the grant of the patent: **23.03.2011**
(21) Application number: 08735845.3
(22) Date of filing: 04.04.2008
(51) Int. Cl.: C07D 471/04, C07D 519/00, A61K 31/4375, A61P 35/00

(54) **[2,6]NAPHTHYRIDINES USEFUL AS PROTEIN KINASE INHIBITORS**
[2,6-]NAPHTHYRIDINE ALS PROTEINKINASEHEMMER
[2,6]NAPHTHYRIDINES UTILES EN TANT QU'INHIBITEURS DES PROTEINES KINASES

(30) Priority: 06.04.2007 US 910519 P
(43) Date of publication of application: 20.01.2010
(73) Proprietor: Novartis AG, 4056 Basel (CH)
(72) Inventor: DOBLER, Markus Rolf, Arlington, Massachusetts 02476 (US); JEWELL, JR., Charles Francis, Sudbury, Massachusetts 01776 (US); MEREDITH, Erik, Hudson, Massachusetts 01749 (US); MONOVICH, Lauren G., Belmont, Massachusetts 02478 (US); SISKA, Sarah, Cambridge, Massachusetts 02139 (US); VON MATT, Anette, CH-4105 Biel-benken (CH); VAN EIS, Maurice, F-68300 St. Louis (FR); YOON, Taeyoung, Newton, Massachusetts 02459 (US); GAUL, Christoph, CH-4147 Aesch (CH); CAPPARELLI, Michael Paul, Cambridge, Massachusetts 02139 (US)
(74) Representative: Vögeli-Lange, Regina
(86) International application number: PCT/EP2008/054105
(87) International publication number: WO 2008/122615

(56) References cited:
- WO-A-2006/032452

## Description

The present invention relates to novel compounds which may be inhibitors of a selective subset of kinases belonging to the AGC or calmodulin kinase family, such as for example MARK-1/2/3, PKD-1/2/3, PKN-1/2, CDK-9, CaMKII, ROCK-I/II, inhibitors of histone deacetylase (HDAC) phosphorylation, or inhibitors of other kinases. The selectivity of which would depend on the structural variation thereof, and for treatment of a disorder or disease mediated by those selected AGC or calmodulin family kinases.

The present invention provides a compound of formula (I): 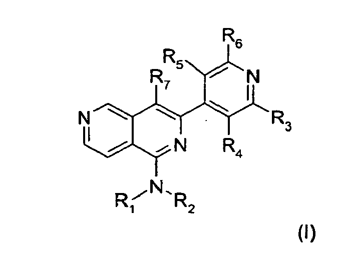 wherein
R₁ and R₂ are independently hydrogen, alkyl, cycloalkyl, heterocyclyl, each of which is optionally substituted by one to two R₈, wherein R₈ is hydrogen, halogen, alkyl, R₉-O--, (R₁₀)(R₁₁)N--, (R₁₂)(R₁₃)N-C(O)--, aryl, or heterocyclyl or heteroaryl, said heterocyclyl and heteroaryl are optionally substituted by one or two alkyl groups;
R₁ and R₂ taken together with the nitrogen atom to which they are attached to optionally form a 4-7 membered ring;
R₃ is (R₁₄)(R₁₅)N--, or halogen;
R₄, R₅, R₆ and R₇ are independently hydrogen, halogen, alkyl, (C₃-C₇) cycloalkyl, aryl-alkyl, aryl, or alkoxy;
R₉, R₁₀, R₁₁, R₁₂ and R₁₃ are independently hydrogen, alkyl-O-C(O)--, alkyl-NH-C(O)-, alkyl-C(O)-NH-C(O)-, cycloalkyl, cycloalkyl-alkyl--, R₁₆-SO₂--, R₁₇-C(O)--, heterocyclyl or alkyl, said heterocyclyl is further optionally substituted by one or two cycloalkyl-alkylgroups, and said alkyl is further optionally substituted by one or two groups selected from hydroxy, alkoxy, alkylamine, dialkylamine, or heteroaryl;
R₁₀ and R₁₁ taken together with the nitrogen atom to which they are attached to optionally form a 5-7 membered ring;
R₁₂ and R₁₃ taken together with the nitrogen atom to which they are attached to optionally form a 5-7 membered ring;
R₁₄ and R₁₅ are independently hydrogen, alkyl, aryl, cycloalkyl, aryl-alkyl--, heterocyclyl or heteroaryl, said alkyl, cycloalkyl, aryl and heteroaryl are further optionally substituted by one or two groups selected from alkyl, alkoxy, hydroxy, halogen, haloalkyl, cyano, or R₁₈-NH-C(O)--;
R₁₆ is aryl or heteroaryl;
R₁₇ is heterocyclyl, or alkyl optionally substituted by one or two groups selected from H₂N--, aryl-alkyl--, or alkyl-C(O)-NH-;
R₁₈ is heterocyclyl-alkyl--; or
a pharmaceutically acceptable salt thereof; or an optical isomer thereof; or a mixture of optical isomers.

The present invention provides the compound of formula (I), wherein R₁ and R₂ are independently hydrogen, (C₁-C₇) alkyl, (C₃-C₇) cycloalkyl, (4-7 membered)-heterocyclyl, each of which is optionally substituted by one to two R₈, wherein R₈ is hydrogen, (C₁-C₇) alkyl, R₉-O-, (R₁₀)(R₁₁)N--, (R₁₂)(R₁₃)N-C(O)--, (C₆-C₁₀) aryl, (5-7 membered)-heteroaryl, or (4-7 membered)-heterocyclyl;
R₃ is (R₁₄)(R₁₅)N--, or halogen;
R₄ and R₅ are independently hydrogen, halogen, (C₁-C₇) alkyl, (C₃-C₇) cycloalkyl, or (C₁-C₇) alkoxy;
R₉, R₁₀, R₁₁, R₁₂ and R₁₃ are independently hydrogen, (C₁-C₇) alkyl-O-C(O)--, (C₁-C₇) alkyl-NH-C(O)-, (C₁-C₇) alkyl-C(O)-NH-C(O)--, (C₃-C₇) cycloalkyl, (C₃-C₇) cycloalkyl-(C₁-C₇) alkyl, R₁₆-SO₂--, R₁₇-C(O)-, (4-7 membered)-heterocyclyl or (C₁-C₇) alkyl, said (4-7 membered)-heterocyclyl is further optionally substituted by one or two (C₃-C₇) cycloalkyl-(C₁-C₇) alkyl groups, and said (C₁-C₇) alkyl is further optionally substituted by one or two groups selected from hydroxy, (C₁-C₇) alkoxy, (C₁-C₇) dialkylamine, or (5-7 membered)-heteroaryl;
R₁₂ and R₁₃ taken together with the nitrogen atom to which they are attached to optionally form a 5-7 membered ring;
R₁₄ and R₁₅ are independently hydrogen, (C₁-C₇) alkyl, (C₆-C₁₀) aryl, (C₃-C₇) cycloalkyl, (C₆-C₁₀) aryl-(C₁-C₇) alkyl-, (4-7 membered)-heterocyclyl or (5-7 membered)-heteroaryl, said (C₁-C₇) alkyl, (C₃-C₇) cycloalkyl, (C₆-C₁₀) aryl and (5-7 membered)-heteroaryl are further optionally substituted by one or two groups selected from (C₁-C₇) alkyl, (C₁-C₇) alkoxy, hydroxy, halogen, (C₁-C₇) haloalkyl, or R₁₄-NH-C(O)--;
R₁₆ is (C₆-C₁₀) aryl or (5-7 membered)-heteroaryl;
R₁₇ is (4-7 membered)-heterocyclyl, or (C₁-C₇) alkyl optionally substituted by one or two groups selected from H₂N--, (C₆-C₁₀) aryl-(C₁-C₇) alkyl--, or (C₁-C₇) alkyl-C(O)-NH--;
R₁₈ is (4-7 membered)-heterocyclyl-(C₁-C₇) alkyl--; or a pharmaceutically acceptable salt thereof; or an optical isomer thereof; or a mixture of optical isomers.

For purposes of interpreting this specification, the following definitions will apply and whenever appropriate, terms used in the singular will also include the plural and vice versa.

As used herein, the term "alkyl" refers to a fully saturated branched or unbranched hydrocarbon moiety. In some embodiments the alkyl comprises 1 to 20 carbon atoms, more In some embodiments 1 to 16 carbon atoms, 1 to 10 carbon atoms, 1 to 7 carbon atoms, or 1 to 4 carbon atoms. Representative examples of alkyl include, but are not limited to, methyl, ethyl, *n*-propyl, *iso-*propyl, *n-*butyl, sec-butyl, *iso*-butyl, *tert-*butyl, *n-*pentyl, isopentyl, neopentyl, *n-*hexyl, 3-methylhexyl, 2,2- dimethylpentyl, 2,3-dimethylpentyl, *n-*heptyl, *n-*octyl, *n-*nonyl, *n-*decyl and the like.

As used herein, the term "haloalkyl" refers to an alkyl as defined herein, that is substituted by one or more halo groups as defined herein. The haloalkyl can be monohaloalkyl, dihaloalkyl or polyhaloalkyl including perhaloalkyl. A monohaloalkyl can have one iodo, bromo, chloro or fluoro within the alkyl group. Dihaloalky and polyhaloalkyl groups can have two or more of the same halo atoms or a combination of different halo groups within the alkyl. The polyhaloalkyl contains up to 12, or 10, or 8, or 6, or 4, or 3, or 2 halo groups. Non-limiting examples of haloalkyl include fluoromethyl, difluoromethyl, trifluoromethyl, chloromethyl, dichloromethyl, trichloromethyl, pentafluoroethyl, heptafluoropropyl, difluorochloromethyl, dichlorofluoromethyl, difluoroethyl, difluoropropyl, dichloroethyl and dichloropropyl. A perhaloalkyl refers to an alkyl having all hydrogen atoms replaced with halo atoms.

The term "aryl" refers to monocyclic or bicyclic aromatic hydrocarbon groups having 6-20 carbon atoms in the ring portion. In some embodiments, the aryl is a (C₆-C₁₀) aryl. Non-limiting examples include phenyl, biphenyl, naphthyl or tetrahydronaphthyl, each of which may optionally be substituted by 1-4 substituents, such as alkyl, trifluoromethyl, cycloalkyl, halogen, hydroxy, alkoxy, acyl, alkyl-C(O)-O--, aryl-O--, heteroaryl-O--, amino, thiol, alkyl-S--, aryl-S--, nitro, cyano, carboxy, alkyl-O-C(O)--, carbamoyl, alkyl-S(O)--, sulfonyl, sulfonamido, heterocyclyl and the like.

Furthermore, the term "aryl" as used herein, refers to an aromatic substituent which can be a single aromatic ring, or multiple aromatic rings that are fused together, linked covalently, or linked to a common group such as a methylene or ethylene moiety. The common linking group also can be a carbonyl as in benzophenone or oxygen as in diphenylether or nitrogen as in diphenylamine.

As used herein, the term "alkoxy" refers to alkyl-O-, wherein alkyl is defined herein above. Representative examples of alkoxy include, but are not limited to, methoxy, ethoxy, propoxy, 2-propoxy, butoxy, *tert-*butoxy, pentyloxy, hexyloxy, cyclopropyloxy-, cyclohexyloxy- and the like. In some embodiments, alkoxy groups have about 1-7, more In some embodiments about 1-4 carbons.

As used herein, the term "acyl" refers to a group R-C(O)- of from 1 to 10 carbon atoms of a straight, branched, or cyclic configuration or a combination thereof, attached to the parent structure through carbonyl functionality. Such group can be saturated or unsaturated, and aliphatic or aromatic. In some embodiments, R in the acyl residue is alkyl, or alkoxy, or aryl, or heteroaryl. Also in some embodiments, one or more carbons in the acyl residue may be replaced by nitrogen, oxygen or sulfur as long as the point of attachment to the parent remains at the carbonyl. Examples of acyl include but are not limited to, acetyl, benzoyl, propionyl, isobutyryl, t- butoxycarbonyl, benzyloxycarbonyl and the like. Lower acyl refers to acyl containing one to four carbons.

As used herein, the term "carbamoyl" refers to H₂NC(O)-, alkyl-NHC(O)-, (alkyl)₂NC(O)-, aryl-NHC(O)-, alkyl(aryl)-NC(O)-, heteroaryl-NHC(O)-, alkyl(heteroaryl)-NC(O)-, aryl-alkyl-NHC(O)-, alkyl(aryl-alkyl)-NC(O)- and the like.

As used herein, the term "sulfonyl" refers to R-SO₂--, wherein R is hydrogen, alkyl, aryl, hereoaryl, aryl-alkyl, heteroaryl-alkyl, alkoxy, aryloxy, cycloalkyl, or heterocyclyl.

As used herein, the term "sulfonamido" refers to alkyl-S(O)₂-NH-, aryl-S(O)₂-NH-, aryl-alkyl-S(O)₂-NH-, heteroaryl-S(O)₂-NH-, heteroaryl-alkyl-S(O)₂-NH-, alkyl-S(O)₂-N(alkyl)-, aryl-S(O)₂-N(alkyl)-, aryl-alkyl-S(O)₂-N(alkyl)-, heteroaryl-S(O)₂-N(alkyl)-, heteroaryl-alkyl-S(O)₂-N(alkyl)- and the like.

As used herein, the term "heterocyclyl" or "heterocyclo" refers to an optionally substituted, saturated or unsaturated non-aromatic ring or ring system, *e.g*., which is a 4-, 5- , 6-, or 7-membered monocyclic, 7-, 8-, 9-, 10-, 11-, or 12-membered bicyclic or 10-, 11-, 12- , 13-, 14- or 15-membered tricyclic ring system and contains at least one heteroatom selected from O, S and N, where the N and S can also optionally be oxidized to various oxidation states. The heterocyclic group can be attached at a heteroatom or a carbon atom. The heterocyclyl can include fused or bridged rings as well as spirocyclic rings. Examples of heterocycles include tetrahydrofuran (THF), dihydrofurari, 1, 4-dioxane, morpholine, 1,4-dithiane, piperazine, piperidine, 1,3-dioxolane, imidazolidine, imidazoline, pyrroline, pyrrolidine, tetrahydropyran, dihydropyran, oxathiolane, dithiolane, 1,3-dioxane, 1,3-dithiane, oxathiane, thiomorpholine, and the like.

The term "heterocyclyl" further refers to heterocyclic groups as defined herein substituted with 1, 2 or 3 substituents selected from the groups consisting of the following: (a) alkyl; (b) hydroxy (or protected hydroxy); (c) halo; (d) oxo, i.e., =O; (e) amino, alkylamino or dialkylamino; (f) alkoxy; (g) cycloalkyl; (h) carboxyl; (i) heterocyclooxy, wherein heterocyclooxy denotes a heterocyclic group bonded through an oxygen bridge; (j) alkyl-O-C(O)-; (k) mercapto; (I) nitro; (m) cyano; (n) sulfamoyl or sulfonamido; (o) aryl; (p) alkyl-C(O)-O--; (q) aryl-C(O)-O--; (r) aryl-S--; (s) aryloxy; (t) alkyl-S--; (u) formyl, i.e., HC(O)--; (v) carbamoyl; (w) aryl-alkyl--; and (x) aryl substituted with alkyl, cycloalkyl, alkoxy, hydroxy, amino, alkyl-C(O)-NH--, alkylamino, dialkylamino or halogen.

As used herein, the term "cycloalkyl" refers to saturated or unsaturated monocyclic, bicyclic or tricyclic hydrocarbon groups of 3-12 carbon atoms, in some embodiments 3-9, or 3-7 carbon atoms, each of which can be optionally substituted by one, or two, or three, or more substituents, such as alkyl, halo, oxo, hydroxy, alkoxy, alkyl-C(O)--, acylamino, carbamoyl, alkyl-NH--, (alkyl)₂N--, thiol, alkyl-S-, nitro, cyano, carboxy, alkyl-O-C(O)--, sulfonyl, sulfonamido, sulfamoyl, heterocyclyl and the like. Exemplary monocyclic hydrocarbon groups include, but are not limited to, cyclopropyl, cyclobutyl, cyctopentyl, cyclopentenyl, cyclohexyl and cyclohexenyl and the like. Exemplary bicyclic hydrocarbon groups include bornyl, indyl, hexahydroindyl, tetrahydronaphthyl, decahydronaphthyl, bicyclo[2.1.1]hexyl, bicyclo[2.2.1]heptyl, bicyclo[2.2.1]heptenyl, 6,6-dimethylbicyclo[3.1.1]heptyl, 2,6,6-trimethylbicyclo[3.1.1]heptyl, bicyclo[2.2.2]octyl and the like. Exemplary tricyclic hydrocarbon groups include adamantyl and the like.

As used herein, the term "sulfamoyl" refers to H₂NS(O)₂-, alkyl-NHS(O)₂-, (alkyl)₂NS(O)₂-, aryl-NHS(O)₂-, alkyl(aryl)-NS(O)₂-, (aryl)₂NS(O)₂-, heteroaryl-NHS(O)₂-, (aryl-alkyl)-NHS(O)₂-, (heteroaryl-alkyl)-NHS(O)₂- and the like.

As used herein, the term "aryloxy" refers to both an --O-aryl and an --O-heteroaryl group, wherein aryl and heteroaryl are defined herein.

As used herein, the term "heteroaryl" refers to a 5-14 membered monocyclic- or bicyclic- or polycyclic-aromatic ring system, having 1 to 8 heteroatoms selected from N, O or S. In some embodiments, the heteroaryl is a 5-10 or 5-7 membered ring system. Typical heteroaryl groups include 2- or 3-thienyl, 2- or 3-furyl, 2- or 3-pyrrolyl, 2-, 4-, or 5-imidazolyl, 3-, 4-, or 5- pyrazolyl, 2-, 4-, or 5-thiazolyl, 3-, 4-, or 5-isothiazolyl, 2-, 4-, or 5-oxazolyl, 3-, 4- , or 5-isoxazolyl, 3- or 5-1,2,4-triazolyl, 4- or 5-1,2, 3-triazolyl, tetrazolyl, 2-, 3-, or 4-pyridyl, 3- or 4-pyridazinyl, 3-, 4-, or 5-pyrazinyl, 2-pyrazinyl, 2-, 4-, or 5-pyrimidinyl.

The term "heteroaryl" also refers to a group in which a heteroaromatic ring is fused to one or more aryl, cycloaliphatic, or heterocyclyl rings, where the radical or point of attachment is on the heteroaromatic ring. Nonlimiting examples include but are not limited to 1-, 2-, 3-, 5-, 6-, 7-, or 8- indolizinyl, 1-, 3-, 4-, 5-, 6-, or 7-isoindolyl, 2-, 3-, 4-, 5-, 6-, or 7- indolyl, 2-, 3-, 4-, 5-, 6-, or 7-indazolyl, 2-, 4-, 5-, 6-, 7-, or 8- purinyl, 1-, 2-, 3-, 4-, 6-, 7-, 8-, or 9-quinolizinyl, 2-, 3-, 4-, 5-, 6-, 7-, or 8-quinoliyl, 1-, 3-, 4-, 5-, 6-, 7-, or 8-isoquinoliyl, 1-, 4-, 5-, 6-, 7-, or 8-phthalazinyl, 2-, 3-, 4-, 5-, or 6-naphthyridinyl, 2-, 3- , 5-, 6-, 7-, or 8-quinazolinyl, 3-, 4-, 5-, 6-, 7-, or 8-cinnolinyl, 2-, 4-, 6-, or 7-pteridinyl, 1-, 2-, 3-, 4-, 5-, 6-, 7-, or 8-4aH carbazolyl, 1-, 2-, 3-, 4-, 5-, 6-, 7-, or 8-carbzaolyl, 1-, 3-, 4-, 5-, 6-, 7-, 8-, or 9-carbolinyl, 1-, 2-, 3-, 4-, 6-, 7-, 8-, 9-, or 10-phenanthridinyl, 1- , 2-, 3-, 4-, 5-, 6-, 7-, 8-, or 9-acridinyl, 1-, 2-, 4-, 5-, 6-, 7-, 8-, or 9-perimidinyl, 2-, 3-, 4-, 5-, 6-, 8-, 9-, or 10-phenathrolinyl, 1-, 2-, 3-, 4-, 6-, 7-, 8-, or 9-phenazinyl, 1-, 2-, 3-, 4-, 6-, 7-, 8-, 9-, or 10-phenothiazinyl, 1-, 2-, 3-, 4-, 6-, 7-, 8-, 9-, or 10-phenoxazinyl, 2-, 3-, 4-, 5-, 6-, or 1-, 3-, 4-, 5-, 6-, 7-, 8-, 9-, or 10- benzisoqinolinyl, 2-, 3-, 4-, or thieno[2,3-b]furanyl, 2-, 3-, 5-, 6-, 7-, 8-, 9-, 10 -, or 11-7H-pyrazino[2,3-c]carbazolyl,2-, 3-, 5-, 6-, or 7-2H- furo[3,2-b]-pyranyl, 2-, 3-, 4-, 5-, 7-, or 8-5H-pyrido[2,3-d]-o-oxazinyl, 1-, 3-, or 5-1H-pyrazolo[4,3-d]-oxazolyl, 2-, 4-, or 54H-imidazo[4,5-d] thiazolyl, 3-, 5-, or 8-pyrazino[2,3-d]pyridazinyl, 2-, 3-, 5-, or 6-imidazo[2,1-b] thiazolyl, 1-, 3-, 6-, 7-, 8-, or 9-furo[3,4-c]cinnolinyl, 1-, 2-, 3-, 4-, 5-, 6-, 8-, 9-, 10, or 11-4H-pyrido[2,3-c]carbazolyl, 2-, 3-, 6-, or 7-imidazo[1,2-b][1,2,4]triazinyl, 7-benzo[b]thienyl, 2-, 4-, 5- , 6-, or 7-benzoxazolyl, 2-, 4-, 5-, 6-, or 7-benzimidazolyl, 2-, 4-, 4-, 5-, 6-, or 7-benzothiazolyl, 1-, 2-, 4-, 5-, 6-, 7-, 8-, or 9- benzoxapinyl, 2-, 4-, 5-, 6-, 7-, or 8-benzoxazinyl, 1-, 2-, 3-, 5-, 6-, 7-, 8-, 9-, 10-, or 11-1H-pyrrolo[1,2-b][2]benzazapinyl. Typical fused heteroary groups include, but are not limited to 2-, 3-, 4-, 5-, 6-, 7-, or 8-quinolinyl, 1-, 3-, 4-, 5-, 6-, 7-, or 8-isoquinolinyl, 2-, 3-, 4-, 5-, 6-, or 7-indolyl, 2-, 3-, 4-, 5-, 6-, or 7-benzo[b]thienyl, 2-, 4-, 5-, 6-, or 7-benzoxazolyl, 2-, 4-, 5-, 6-, or 7-benzimidazolyl, 2-, 4-, 5-, 6-, or 7-benzothiazolyl.

A heteroaryl group may be mono-, bi-, tri-, or polycyclic, In some embodiments mono-, bi-, or tricyclic, more In some embodiments mono- or bicyclic.

As used herein, the term "halogen" or "halo" refers to fluoro, chloro, bromo, and iodo.

As used herein, the term "isomers" refers to different compounds that have the same molecular formula but differ in arrangement and configuration of the atoms. Also as used herein, the term "an optical isomer" or "a stereoisomer" refers to any of the various stereo isomeric configurations which may exist for a given compound of the present invention and includes geometric isomers. It is understood that a substituent may be attached at a chiral center of a carbon atom. Therefore, the invention includes enantiomers, diastereomers or racemates of the compound. "Enantiomers" are a pair of stereoisomers that are non-superimposable mirror images of each other. A 1:1 mixture of a pair of enantiomers is a "racemic" mixture. The term is used to designate a racemic mixture where appropriate. "Diastereoisomers" are stereoisomers that have at least two asymmetric atoms, but which are not mirror-images of each other. The absolute stereochemistry is specified according to the Cahn- Ingold- Prelog R-S system. When a compound is a pure enantiomer the stereochemistry at each chiral carbon may be specified by either R or S. Resolved compounds whose absolute configuration is unknown can be designated (+) or (-) depending on the direction (dextro- or levorotatory) which they rotate plane polarized light at the wavelength of the sodium D line. Certain of the compounds described herein contain one or more asymmetric centers and may thus give rise to enantiomers, diastereomers, and other stereoisomeric forms that may be defined, in terms of absolute stereochemistry, as (R)- or (S)-. The present invention is meant to include all such possible isomers, including racemic mixtures, optically pure forms and intermediate mixtures. Optically active (R)- and (S)- isomers may be prepared using chiral synthons or chiral reagents, or resolved using conventional techniques. If the compound contains a double bond, the substituent may be E or Z configuration. If the compound contains a disubstituted cycloalkyl, the cycloalkyl substituent may have a cis- or trans-configuration. All tautomeric forms are also intended to be included.

As used herein, the term "pharmaceutically acceptable salts" refers to salts that retain the biological effectiveness and properties of the compounds of this invention and, which are not biologically or otherwise undesirable. In many cases, the compounds of the present invention are capable of forming acid and/or base salts by virtue of the presence of amino and/or carboxyl groups or groups similar thereto. Pharmaceutically acceptable acid addition salts can be formed with inorganic acids and organic acids. Inorganic acids from which salts can be derived include, for example, hydrochloric acid, hydrobromic acid, sulfuric acid, nitric acid, phosphoric acid, and the like. Organic acids from which salts can be derived include, for example, acetic acid, propionic acid, glycolic acid, pyruvic acid, oxalic acid, maleic acid, malonic acid, succinic acid, fumaric acid, tartaric acid, citric acid, benzoic acid, cinnamic acid, mandelic acid, methanesulfonic acid, ethanesulfonic acid, p-toluenesulfonic acid, salicylic acid, and the like. Pharmaceutically acceptable base addition salts can be formed with inorganic and organic bases. Inorganic bases from which salts can be derived include, for example, sodium, potassium, lithium, ammonium, calcium, magnesium, iron, zinc, copper, manganese, aluminum, and the like; particularly preferred are the ammonium, potassium, sodium, calcium and magnesium salts. Organic bases from which salts can be derived include, for example, primary, secondary, and tertiary amines, substituted amines including naturally occurring substituted amines, cyclic amines, basic ion exchange resins, and the like, specifically such as isopropylamine, trimethylamine, diethylamine, triethylamine, tripropylamine, and ethanolamine. The pharmaceutically acceptable salts of the present invention can be synthesized from a parent compound, a basic or acidic moiety, by conventional chemical methods. Generally, such salts can be prepared by reacting free acid forms of these compounds with a stoichiometric amount of the appropriate base (such as Na, Ca, Mg, or K hydroxide, carbonate, bicarbonate, or the like), or by reacting free base forms of these compounds with a stoichiometric amount of the appropriate acid. Such reactions are typically carried out in water or in an organic solvent, or in a mixture of the two. Generally, non-aqueous media like ether, ethyl acetate, ethanol, isopropanol, or acetonitrile are preferred, where practicable. Lists of additional suitable salts can be found, *e.g.,* in Remington's Pharmaceutical Sciences, 20th ed., Mack Publishing Company, Easton, Pa., (1985).

As used herein, the term "pharmaceutically acceptable carrier" includes any and all solvents, dispersion media, coatings, surfactants, antioxidants, preservatives (*e.g*., antibacterial agents, antifungal agents), isotonic agents, absorption delaying agents, salts, preservatives, drugs, drug stabilizers, binders, excipients, disintegration agents, lubricants, sweetening agents, flavoring agents, dyes, such like materials and combinations thereof, as would be known to one of ordinary skill in the art (see, for example, Remington's Pharmaceutical Sciences, 18th Ed. Mack Printing Company, 1990, pp. 1289- 1329, incorporated herein by reference). Except insofar as any conventional carrier is incompatible with the active ingredient, its use in the therapeutic or pharmaceutical compositions is contemplated.

The term "a therapeutically effective amount" of a compound of the present invention refers to an amount of the compound of the present invention that will elicit the biological or medical response of a subject, for example, reduction or inhibition of an enzyme or a protein activity, or ameliorate symptoms, alleviate conditions, slow or delay disease progression, or prevent a disease, etc. In one non-limiting embodiment, the term "a therapeutically effective amount" refers to the amount of the compound of the present invention that, when administered to a subject, is effective to (1) at least partially alleviating, inhibiting, preventing and/or ameliorating a condition, or a disorder or a disease (i) mediated by PKD, or (ii) associated with PKD activity, or (iii) characterized by abnormal activity of PKD; or (2) reducing or inhibiting the activity of PKD; or (3) reducing or inhibiting the expression of PKD. In another non-limiting embodiment, the term "a therapeutically effective amount" refers to the amount of the compound of the present invention that, when administered to a cell, or a tissue, or a non-cellular biological material, or a medium, is effective to at least partially reducing or inhibiting the activity of PKD; or at least partially reducing or inhibiting the expression of PKD. The meaning of the term "a therapeutically effective amount" as illustrated in the above embodiment for PKD also applies by the same means to any other relevant proteins/peptides/enzymes, such as MARK1/2/3, PKN-1/2, CDK-9, CaMKII, ROCK-I/II, histone deacetylase (HDAC), or other kinases, etc..

As used herein, the term "subject" refers to an animal. In some embodiments, the animal is a mammal. A subject also refers to for example, primates (*e.g*., humans), cows, sheep, goats, horses, dogs, cats, rabbits, rats, mice, fish, birds and the like.

As used herein, the term "a disorder" or " a disease" refers to any derangement or abnormality of function; a morbid physical or mental state. See Dorland's Illustrated Medical Dictionary, (W.B. Saunders Co. 27th ed. 1988).

As used herein, the term "inhibition" or "inhibiting" refers to the reduction or suppression of a given condition, symptom; or disorder, or disease, or a significant decrease in the baseline activity of a biological activity or process. In some embodiments, the condition or symptom or disorder or disease is mediated by PKD activity. More In some embodiments, the condition or symptom or disorder or disease is associated with the abnormal activity of PKD, or the condition or symptom or disorder or disease is associated with the abnormal expression of PKD. The term "inhibition" or "inhibiting" also applies by the same meaning to other enzymes/proteins/peptides, i.e., MARK1/2/3, PKN-1/2, CDK-9, CaMKII, ROCK-I/II, histone deacetylase (HDAC), or other kinases, etc..

As used herein, the term "treating" or "treatment" of any disease or disorder refers in one embodiment, to ameliorating the disease or disorder (i.e., slowing or arresting or reducing the development of the disease or at least one of the clinical symptoms thereof). In another embodiment "treating" or "treatment" refers to alleviating or ameliorating at least one physical parameter including those which may not be discernible by the patient. In yet another embodiment, "treating" or "treatment" refers to modulating the disease or disorder, either physically, (*e.g*., stabilization of a discernible symptom), physiologically, (*e.g*., stabilization of a physical parameter), or both. In yet another embodiment, "treating" or "treatment" refers to preventing or delaying the onset or development or progression of the disease or disorder.

As used herein, the term "abnormal" refers to an activity or feature which differs from a normal activity or feature.

As used herein, the term "abnormal activity" refers to an activity which differs from the activity of the wild-type or native gene or protein, or which differs from the activity of the gene or protein in a healthy subject. The abnormal activity can be stronger or weaker than the normal activity. In one embodiment, the "abnormal activity" includes the abnormal (either over- or under-) production of mRNA transcribed from a gene. In another embodiment, the "abnormal activity" includes the abnormal (either over- or under-) production of polypeptide from a gene. In another embodiment, the abnormal activity refers to a level of a mRNA or polypeptide that is different from a normal level of said mRNA or polypeptide by about 15%, about 25%, about 35%, about 50%, about 65%, about 85%, about 100% or greater. In some embodiments, the abnormal level of the mRNA or polypeptide can be either higher or lower than the normal level of said mRNA or polypeptide. Yet in another embodiment, the abnormal activity refers to functional activity of a protein that is different from a normal activity of the wild-type protein. In some embodiments, the abnormal activity can be stronger or weaker than the normal activity. In some embodiments, the abnormal activity is due to the mutations in the corresponding gene, and the mutations can be in the coding region of the gene or non-coding regions such as transcriptional promoter regions. The mutations can be substitutions, deletions, insertions.

As used herein, the term "a," "an," "the" and similar terms used in the context of the present invention (especially in the context of the claims) are to be construed to cover both the singular and plural unless otherwise indicated herein or clearly contradicted by the context. Recitation of ranges of values herein are merely intended to serve as a shorthand method of referring individually to each separate value falling within the range. Unless otherwise indicated herein, each individual value is incorporated into the specification as if it were individually recited herein. All methods described herein can be performed in any suitable order unless otherwise indicated herein or otherwise clearly contradicted by context. The use of any and all examples, or exemplary language (*e.g*. "such as") provided herein is intended merely to better illuminate the invention and does not pose a limitation on the scope of the invention otherwise claimed. No language in the specification should be construed as indicating any non-claimed element essential to the practice of the invention.

Any asymmetric carbon atom on the compounds of the present invention can be present in the (*R*)-, (S)- or (*R,S*)- configuration, In some embodiments in the (R)- or (*S*)-configuration. Substituents at atoms with unsaturated bonds may, if possible, be present in *cis-* (Z)- or *trans-* (*E*)- form. Therefore, the compounds of the present invention can be in the form of one of the possible isomers or mixtures thereof, for example, as substantially pure geometric (*cis* or *trans*) isomers, diastereomers, optical isomers (antipodes), racemates or mixtures thereof.

Any resulting mixtures of isomers can be separated on the basis of the physicochemical differences of the constituents, into the pure geometric or optical isomers, diastereomers, racemates, for example, by chromatography and/or fractional crystallization.

Any resulting racemates of final products or intermediates can be resolved into the optical antipodes by known methods, *e.g*., by separation of the diastereomeric salts thereof, obtained with an optically active acid or base, and liberating the optically active acidic or basic compound. In particular, the imidazolyl moiety may thus be employed to resolve the compounds of the present invention into their optical antipodes, *e.g*., by fractional crystallization of a salt formed with an optically active acid, *e.g*., tartaric acid, dibenzoyl tartaric acid, diacetyl tartaric acid, di-O,O'-p-toluoyl tartaric acid, mandelic acid, malic acid or camphor-10-sulfonic acid. Racemic products can also be resolved by chiral chromatography, *e.g*., high pressure liquid chromatography (HPLC) using a chiral adsorbent.

Finally, compounds of the present invention are either obtained in the free form, as a salt thereof, or as prodrug derivatives thereof.

When a basic group is present in the compounds of the present invention, the compounds can be converted into acid addition salts thereof, in particular, acid addition salts with the imidazolyl moiety of the structure, In some embodiments pharmaceutically acceptable salts thereof. These are formed, with inorganic acids or organic acids. Suitable inorganic acids include but are not limited to, hydrochloric acid, sulfuric acid, a phosphoric or hydrohalic acid. Suitable organic acids include but are not limited to, carboxylic acids, such as (C₁-C₄)alkanecarboxylic acids which, for example, are unsubstituted or substituted by halogen, *e*.*g*., acetic acid, such as saturated or unsaturated dicarboxylic acids, *e.g*., oxalic, succinic, maleic or fumaric acid, such as hydroxycarboxylic acids, *e.g*., glycolic, lactic, malic, tartaric or citric acid, such as amino acids, *e.g*., aspartic or glutamic acid, organic sulfonic acids, such as (C₁-C₄)alkylsulfonic acids, *e.g*., methanesulfonic acid; or arylsulfonic acids which are unsubstituted or substituted, *e*.*g*., by halogen. Preferred are salts formed with hydrochloric acid, methanesulfonic acid and maleic acid.

When an acidic group is present in the compounds of the present invention, the compounds can be converted into salts with pharmaceutically acceptable bases. Such salts include alkali metal salts, like sodium, lithium and potassium salts; alkaline earth metal salts, like calcium and magnesium salts; ammonium salts with organic bases, *e.g*., trimethylamine salts, diethylamine salts, tris(hydroxymethyl)methylamine salts, dicyclohexylamine salts and *N*-methyl-*D-*glucamine salts; salts with amino acids like arginine, lysine and the like. Salts may be formed using conventional methods, advantageously in the presence of an ethereal or alcoholic solvent, such as a lower alkanol. From the solutions of the latter, the salts may be precipitated with ethers, *e.g*., diethyl ether. Resulting salts may be converted into the free compounds by treatment with acids. These or other salts can also be used for purification of the compounds obtained.

When both a basic group and an acid group are present in the same molecule, the compounds of the present invention can also form internal salts.

Furthermore, the compounds of the present invention, including their salts, can also be obtained in the form of their hydrates, or include other solvents used for their crystallization.

The compounds of the present invention have valuable pharmacological properties. The compounds of the present invention are useful as PKD inhibitors. PKD is a family of serine/threonine protein kinases that is now classified as a subfamily of the Ca2+/calmodulin-dependent kinase (CaMK) superfamily. Currently the PKD family includes PKD1, 2 and 3. Recently, there have been reports demonstrating the biological functions of PKD. See Wang QJ, "PKD at the crossroads of DAG and PKC signaling," *TRENDS in Pharmacological Sciences,* 27(6): 3170323 (2006). For example, it has been found that activation of PKD regulates fission of transport carriers from the Golgi to the plasma membrane. See Liljedahl, M. et al., "protein kinase D regulates the fission of cell surface destined transport carriers from the trans-Golgi network," Cell, 104:409-420 (2001). PKD has a major role in cell motility, invasion, and adhesion. PKD has also been demonstrated to have pro-proliferative effect in many cellular systems, as well as promotes antiapoptotic responses in tumor cells. See Prigozhina, NL et al., "Protein kinase D-mediated anterograde membrane trafficking is required for fibroblast motility," Curr. Biol.,14:88-98 (2004), Rozengurt E. et al., "Protein kinase D signaling," JBC, 280(14): 13205-13208 (2005). PKD has also been found to regulate agonist-dependent cardiac hypertrophy through the nuclear export of class II histone deacetylase (HDAC5). See Vega, RB et al., "Protein kinase C and D mediate agonist-dependent cardiac hypertrophy through nuclear export of histone deacetylase 5," Mol. Cell. Biol., 24: 8374-8385 (2004). PKD is also involved in oxidative stress response by activating the transcription factor Nf-kB to protect the cell from oxidative-stress-induced cell death. See Storz, P. and Toker, A., "Protein kinase D mediates a stress-induced NF-kB activation and survival pathway," EMBO J., 22: 109-120 (2003). Sjoblom, T. et al. linked PKD to breast and colorectal cancers. See Sjoblom, T. et al., "The consensus coding sequences of human breast and colorectal cancers," Science, 314: 268-274 (2006). PKD has been found to regulate gene expression related to immune response and function of skin. See Matthews, SA et al., "Essential role-for protein kinase D family kinases in the regulation of class II histone deacetylases in B lymphocytes," Mol. Cell. Biol., 26(4): 1569-1577 (2006), Irie, A. et al., "Protein kinase D2 contributes to either IL-2 promoter regulation or induction of cell death upon TCR stimulation depending on its activity in Jurkat cells," Int. Immunology, 18(12): 1737-1747 (2006), Bollag, WB et al., "Protein kinase D and keratinocyte proliferation," Drug News Perspect, 17(2):117 (2004), etc. Given all the evidence for the PKD biological functions, PKD is implicated in diseases or disorders such as heart failure, colorectal cancer, regulation of cell growth, autoimmune disorders, or hyperproliferative skin disorders, etc.. Accordingly, the compounds of the present invention as PKD inhibitors, are also useful for treatment of a disorder or disease mediated by PKD or responsive to inhibition of PKD. In particular, the compounds of the present invention as PKD inhibitors are useful for treatment of a disorder or disease selected from heart failure, colorectal cancer, regulation of cell growth, autoimmune disorders, or hyperproliferative skin disorders, etc..

In addition, the compounds of the present invention are useful as CaMKII inhibitors. CaMKII is an intracellular enzyme found in the cytoplasm and nucleus, which can phosphorylate a number of substrates. Reports have linked or indicated CaMKII in hypertrophy, heart failure, cardia arrhythmia, opioid tolerance and dependence, and osteoporosis, etc. See, Ai X, Bers DM, Pogwizd SM (2005) Enhanced Ca2+/Calmodulin-dependent protein kinase activation in an arrhythmogenic rabbit model of heart failure. Biophysical Journal; 88 (1):322A, Ai X, Curran JW, Shannon TR, et al (2005) Ca2+/calmodulin-dependent protein kinase modulates cardiac ryanodine receptor phosphorylation and sarcoplasmic reticulum Ca2+ leak in heart failure. Circulation Research; 97 (12):1314-22. Anderson ME (2006) OT interval prolongation and arrhythmia: an unbreakable connection? Journal of Internal Medicine; 259 (1):81-90, Mills GD, Kubo H, Harris DM, et al (2006) Phosphorylation of phospholamban at threonine-17 reduces cardiac adrenergic contractile responsiveness in chronic pressure overload-induced hypertrophy. American Journal of Physiology-Heart and Circulatory Physiology, 291 (1): H61-H70, Seales EC, Micoli KJ, McDonald JM (2006) Calmodulin is a critical regulator of osteoclastic differentiation, function, and survival. Journal of Cellular Biochemistry; 97 (1):45-55, Tang L, Shukla PK, Wang LX, et al (2006) Reversal of morphine antinociceptive tolerance and dependence by the acute supraspinal inhibition of Ca2+/calmodulin-dependent protein kinase II. Journal of Pharmacology and Experimental Therapeutics; 317 (2):901-9, Wang ZJ, Tang L, Xin LL (2003) Reversal of morphine antinociceptive tolerance by acute spinal inhibition of Ca2+/calmodulin-dependent protein kinase II. European Journal of Pharmacology; 465 (1-2):199-200, Zhang R, Khoo MSC, Wu YJ, et al (2005) Calmodulin kinase II inhibition protects against structural heart disease. Nature Medicine; 11 (4):409-17, Zhang T, Dalton N, Maier LS, et al (2002) The delta(c) isoform of CaMKII is activated in cardiac hypertrophy and induces dilated cardiomyopathy and heart failure. Circulation; 106 (19):255.

Accordingly, the compounds of the present invention as CaMKII inhibitors, are also useful for treatment of a disorder or disease mediated by CaMKII or responsive to inhibition of CaMKII. In particular, the compounds of the present invention as CaMKII inhibitors are useful for treatment of a disorder or disease selected from hypertrophy, heart failure, cardiac arrhythmia, opioid tolerance and dependence, or osteoporosis, etc..

In addition, the compounds of the present invention are useful as MARK inhibitors. MARK inhibitors are linked to diseases such as including, but not limited to cancers, autoimmune diseases, tissue damage, central nervous system disorders, neurodegenerative disorders, ...fibrosis, bone disorders, polyglutamine-repeat disorders, anemias, thalassemias, inflammatory conditions, cardiovascular conditions, etc.. See Dequiedt, F. et al., Molecular and Cellular Biology (2006) 26, 7086-7102.

In addition, the compounds of the present invention are useful as PRK inhibitors. PRK has been implicated in a variety of processes, including regulation of cytoskeletal organization, apoptosis, and cell proliferation (reviewed in Mukai, 2003). PRKs reside in the cytosol but exhibit the capacity to kanslocate to the nucleus in a signal-dependent manner. As such, it has been proposed that PRK may play a role in transcriptional regulation of gene expression. PRK is linked to cardiac hypertrophy and heart failure. See WO 2005074941 and Morissette, M. et al., American Journal of Physiology, Heart Circulation Physiology (2000) H1769-1774.

In addition, the compounds of the present invention are useful as CDK9 inhibitors. CDK9 inhibitors are linked to cardiac hypertophy in the following literature references: Nature Medicine (2002) 8, 1310 and WO 200402226 and EMBO Journal (2004) 23, 3559

In addition, the compounds of the present invention are useful as ROCK inhibitors. ROCK inhibitors are linked to cardiac hypertophy in the following literature references: Journal of Hypertension (2005) 23, 87 and Journal of Molecular and Cellular Cardiology (2003) 35, 59.

In addition, the compounds of the present invention are useful as ClassIIa HDAC kinase inhibitors, which may include but are not limited to PKC, PKD, MARK, CaMKII, and PRK. The topic is recently reviewed in Cardiovascular Research (2007) 73, 667.

Compounds of the present invention are prepared from commonly available compounds using procedures known to those skilled in the art, including any one or more of the following conditions without limitation:

Within the scope of this text, only a readily removable group that is not a constituent of the particular desired end product of the compounds of the present invention is designated a "protecting group", unless the context indicates otherwise. The protection of functional groups by such protecting groups, the protecting groups themselves, and their cleavage reactions are described for example in standard reference works, such as J. F. W. McOmie, "Protective Groups in Organic Chemistry", Plenum Press, London and New York 1973, in T. W. Greene and P. G. M. Wuts, "Protective Groups in Organic Synthesis", Third edition, Wiley, New York 1999, in "The Peptides"; Volume 3 (editors: E. Gross and J. Meienhofer), Academic Press, London and New York 1981, in "Methoden der organischen Chemie" (Methods of Organic Chemistry), Houben Weyl, 4th edition, Volume 15/l, Georg Thieme Verlag, Stuttgart 1974, in H.-D. Jakubke and H. Jeschkeit, "Aminosäuren, Peptide, Proteine" (Amino acids, Peptides, Proteins), Verlag Chemie, Weinheim, Deerfield Beach, and Basel 1982, and in Jochen Lehmann, "Chemie der Kohlenhydrate: Monosaccharide und Derivate" (Chemistry of Carbohydrates: Monosaccharides and Derivatives), Georg Thieme Verlag, Stuttgart 1974.

Salts of compounds of the present invention having at least one salt-forming group may be prepared in a manner known per se. For example, salts of compounds of the present invention having acid groups may be formed, for example, by treating the compounds with metal compounds, such as alkali metal salts of suitable organic carboxylic acids, *e.g*. the sodium salt of 2-ethylhexanoic acid, with organic alkali metal or alkaline earth metal compounds, such as the corresponding hydroxides, carbonates or hydrogen carbonates, such as sodium or potassium hydroxide, carbonate or hydrogen carbonate, with corresponding calcium compounds or with ammonia or a suitable organic amine, stoichiometric amounts or only a small excess of the salt-forming agent In some embodiments being used. Acid addition salts of compounds of the present invention are obtained in customary manner, *e.g*. by treating the compounds with an acid or a suitable anion exchange reagent. Internal salts of compounds of the present invention containing acid and basic salt-forming groups, *e.g*. a free carboxy group and a free amino group, may be formed, *e.g*. by the neutralisation of salts, such as acid addition salts, to the isoelectric point, *e.g*. with weak bases, or by treatment with ion exchangers.

Salts can be converted in customary manner into the free compounds; metal and ammonium salts can be converted, for example, by treatment with suitable acids, and acid addition salts, for example, by treatment with a suitable basic agent.

Mixtures of isomers obtainable according to the invention can be separated in a manner known per se into the individual isomers; diastereoisomers can be separated, for example, by partitioning between polyphasic solvent mixtures, recrystallisation and/or chromatographic separation, for example over silica gel or by *e.g*. medium pressure liquid chromatography over a reversed phase column, and racemates can be separated, for example, by the formation of salts with optically pure salt-forming reagents and separation of the mixture of diastereoisomers so obtainable, for example by means of fractional crystallisation, or by chromatography over optically active column materials.

Intermediates and final products can be worked up and/or purified according to standard methods, *e.g*. using chromatographic methods, distribution methods, (re-) crystallization, and the like.

The following applies in general to all processes mentioned herein before and hereinafter.

All the above-mentioned process steps can be carried out under reaction conditions that are known per se, including those mentioned specifically, in the absence or, customarily, in the presence of solvents or diluents, including, for example, solvents or diluents that are inert towards the reagents used and dissolve them, in the absence or presence of catalysts, condensation or neutralizing agents, for example ion exchangers, such as cation exchangers, *e.g.* in the H+ form, depending on the nature of the reaction and/or of the reactants at reduced, normal or elevated temperature, for example in a temperature range of from about -100 °C to about 190 °C, including, for example, from approximately -80 °C to approximately 150 °C, for example at from -80 to -60 °C, at room temperature, at from -20 to 40 °C or at reflux temperature, under atmospheric pressure or in a closed vessel, where appropriate under pressure, and/or in an inert atmosphere, for example under an argon or nitrogen atmosphere.

At all stages of the reactions, mixtures of isomers that are formed can be separated into the individual isomers, for example diastereoisomers or enantiomers, or into any desired mixtures of isomers, for example racemates or mixtures of diastereoisomers, for example analogously to the methods described under "Additional process steps".

The solvents from which those solvents that are suitable for any particular reaction may be selected include those mentioned specifically or, for example, water, esters, such as lower alkyl-lower alkanoates, for example ethyl acetate, ethers, such as aliphatic ethers, for example diethyl ether, or cyclic ethers, for example tetrahydrofurane or dioxane, liquid aromatic hydrocarbons, such as benzene or toluene, alcohols, such as methanol, ethanol or 1- or 2-propanol, nitriles, such as acetonitrile, halogenated hydrocarbons, such as methylene chloride or chloroform, acid amides, such as dimethylformamide or dimethyl acetamide, bases, such as heterocyclic nitrogen bases, for example pyridine or N-methylpyrrolidin-2-one, carboxylic acid anhydrides, such as lower alkanoic acid anhydrides, for example acetic anhydride, cyclic, linear or branched hydrocarbons, such as cyclohexane, hexane or isopentane, or mixtures of those solvents, for example aqueous solutions, unless otherwise indicated in the description of the processes. Such solvent mixtures may also be used in working up, for example by chromatography or partitioning.

The compounds, including their salts, may also be obtained in the form of hydrates, or their crystals may, for example, include the solvent used for crystallization. Different crystalline forms may be present.

The invention relates also to those forms of the process in which a compound obtainable as an intermediate at any stage of the process is used as starting material and the remaining process steps are carried out, or in which a starting material is formed under the reaction conditions or is used in the form of a derivative, for example in a protected form or in the form of a salt, or a compound obtainable by the process according to the invention is produced under the process conditions and processed further in situ.

All starting materials, building blocks, reagents, acids, bases, dehydrating agents, solvents and catalysts utilized to synthesize the compounds of the present invention are either commercially available or can be produced by organic synthesis methods known to one of ordinary skill in the art (Houben-Weyl 4th Ed. 1952, Methods of Organic Synthesis, Thieme, Volume 21).

Generally, the compounds of formula (I) can be prepared according to Schemes 1-4. The first part of the synthesis is the preparation of the common dihalo intermediates **8** and **9** as shown in Scheme 1. Isonicotinamides **2** can be prepared starting from 3-methylisonicotinonitrile (1) as described in the literature (Y.G. Gu, et. al., Bioorg. Med. Chem. Lett 9 (10), 1999, 1341) or through coupling of 3-methylisonicotinic acid with and *tert-*butylamine in the presence of a common dehydrating reagent like oxalylchloride/*N,N-*dimethylformamide and a base (*e.g*., triethylamine) in an appropriate solvent (*e.g*., dichloromethane). Alternatively, a 3-pyridyl substituent may be introduced to *N-t-*butylisonicotinamide 3 by amide-directed deprotonation using a strong base (*e.g*., *n-*BuLi), followed by treatment with a suitable electrophile (*e.g*., methyl iodide). Picoline **2** can be further elaborated by treatment with a suitable base (e.g*.*, *n-*BuLi), followed by trapping with a suitable electrophile (e.g., methyl iodide) to give **4.** Base-initiated condensation of **2** or **4** with 2-chloroisonicotinic acid methyl ester furnishes **5**. Acid (*e.g*., acidic acid) mediated cyclization leads to lactone **6** and treatment thereof with a nucleophile (*e.g*., ammonia in ethanol) followed by acidification (*e.g*. AcOH) gives the desired lactam **7.** Subsequent reaction with a halogenating reagent (*e.g*., phosphorus oxychloride, POCl₃ or phosphorus oxybromide, POBr₃) yields the common intermediates **8** and **9.**

Dihalide **9** can be directly converted to compounds of formula 1 where R₁ = R₁₄ and R₂ = R₁₅ by treatment with a suitable amine nucleophile HNR₁R₂ (*e.g*., *n-*butylamine) in an autoclave at elevated temperature (*e.g*., 130 °C). At lower temperatures (*e.g*., 45 °C), the halogen of the naphthyridine is selectively displaced, to allow subsequent elaboration of the halopyridine and formation of compounds of formula 1, where R₁ ≠R₁₄ and R₂ ≠R₁₅.

Alternatively, on treatment of **8** with sodium methoxide in methanol **10** is obtained, allowing a subsequent functionalization of the chloropyridyl moiety to compounds of formula **1.** This nucleophilic displacement with a suitable amine can be achieved for example under either microwave reaction conditions or by applying a Buchwald-Hartwig protocol, (J. F. Hartwig, Angew. Chem. Int. Ed. 37, 1998, 2046), using a palladium source (*e.g*., Pd(OAc)₂), an appropriate solvent such as toluene or 1,4-dioxane, an appropriate ligand (*e.g*., BINAP) and a suitable base (*e.g*., *t*-BuOK). Either R₁ or R₂ of **11** can be further functionalized, for example, through an ester saponifaction step follow by an amide coupling reaction. Imidate hydrolysis of **11** applying *t*-BuOK in wet *t*-BuOH yields **12.** Under treatment of a suitable chlorinating agent (*e.g*., POCl₃) and subsequent nucleophilic displacement with a suitable amine, for example, by stirring in a In some embodiments polar protic solvent (*e.g*., ethanol) under In some embodiments elevated temperature until the reaction is completed, **13** is obtained. Any of the given residues R₁, R₂, R₁₄, or R₁₅ subsequently be object of further functionalization, as intermediate **11** or **12** for example through an saponifaction-amide formation protocol, and any of the residues R₁, R₂, R₁₄, or R₁₅ can be subjected to final deprotection (*e.g*., cleavage of a BOC group using a strong acid such as trifluoroacidic acid, TFA, in a suitable solvent such as dichloromethane, DCM). Depending on these steps the compounds of the present invention can be obtained as a neutral compound or any of its salt forms (*e.g*., hydrochloride, TFA salt)

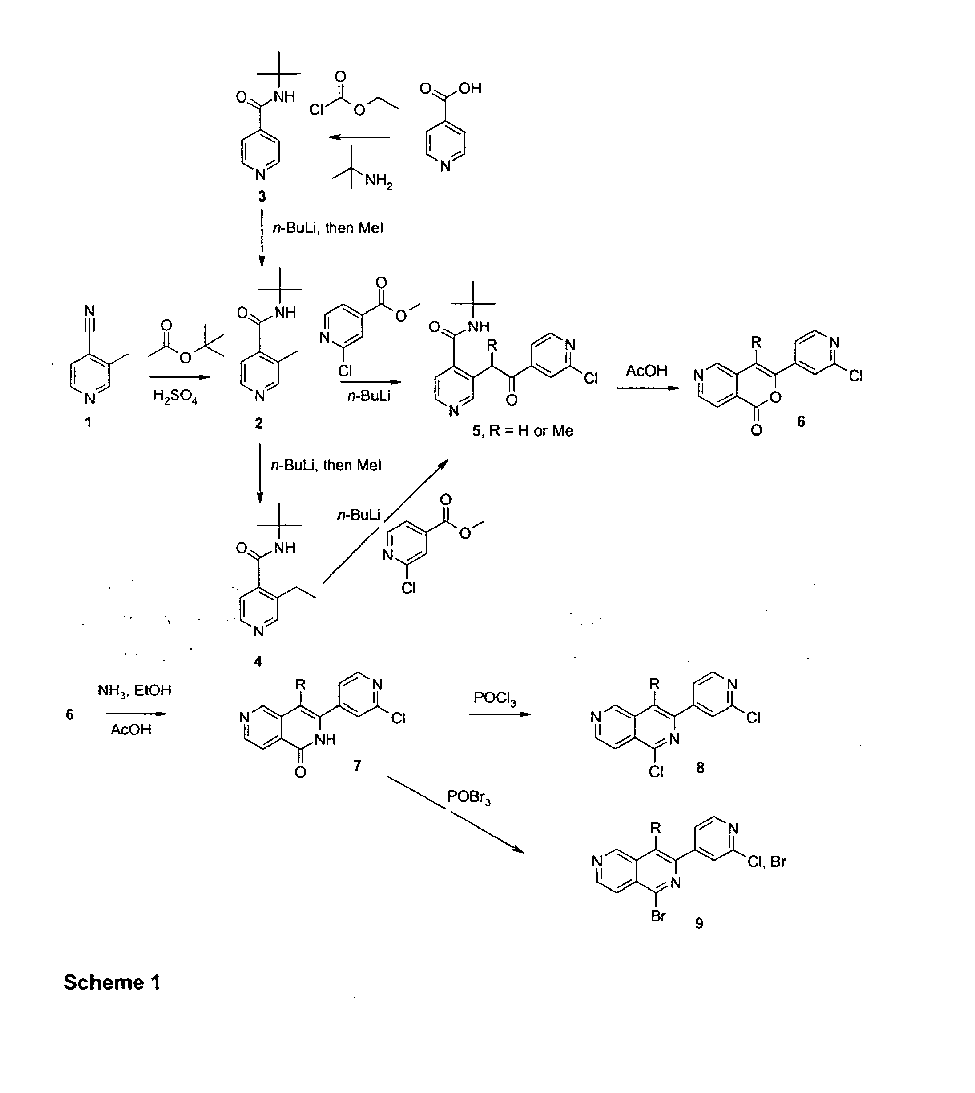

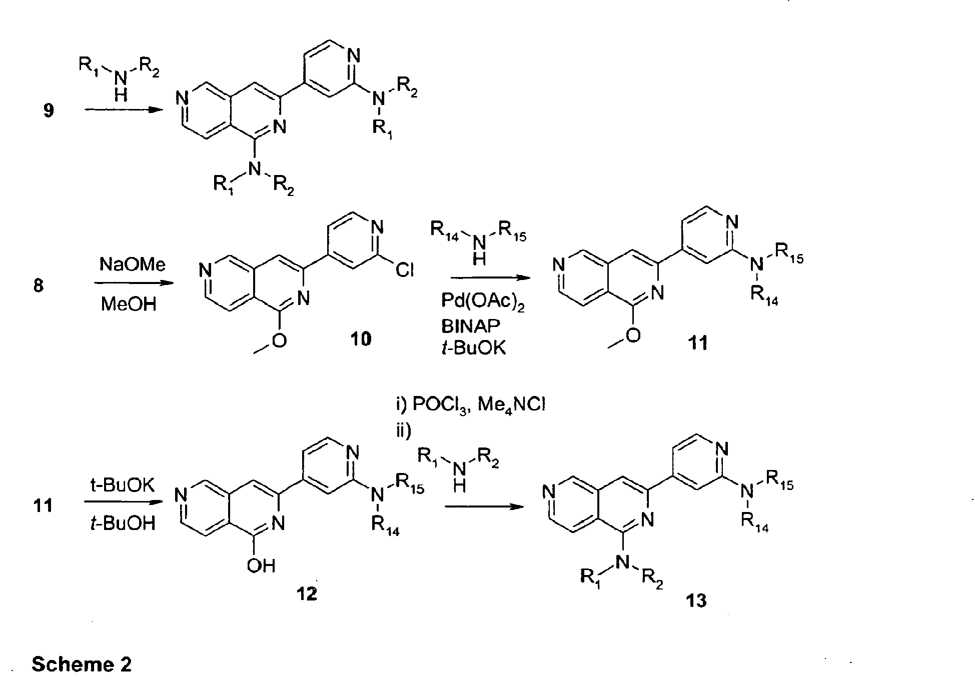

Alternatively to the route shown in Scheme 2, the common intermediate can undergo the following reaction sequence (Scheme 3). Nucleophilic displacement of chloride **8** with a suitable amine, for example under either microwave reaction conditions or by applying a Buchwald-Hartwig protocol, (J. F. Hartwig, Bioorg. Angew. Chem. Int. Ed. 37, 1998, 2046), using a palladium catalyst (*e.g*., Pd(OAc)₂), an appropriate solvent such as toluene or 1,4-dioxane, an appropriate ligand (*e.g*., BINAP) and a suitable base (*e.g*., *t*-BuOK) is yielding the chloropyridine **14.** Subsequent second nucleophilic displacement with a suitable amine can be achieved for example by stirring in a In some embodiments polar protic solvent (*e.g*., ethanol,) under In some embodiments elevated temperature until the reaction is completed, yielding **15.** Naphthyridine **14** may be further functinalized by the action of a suitable electrophile (*e.g*., bromine) to give compounds **16.** Bromide **16** undergoes Pd-catalyzed couplings, such as Suzuki couplings or Buchwald couplings and others known in the art, to yield compounds **17,** where R₇ ≠H. By analogy, compounds **17** may be converted to **15** by methods outlined above.

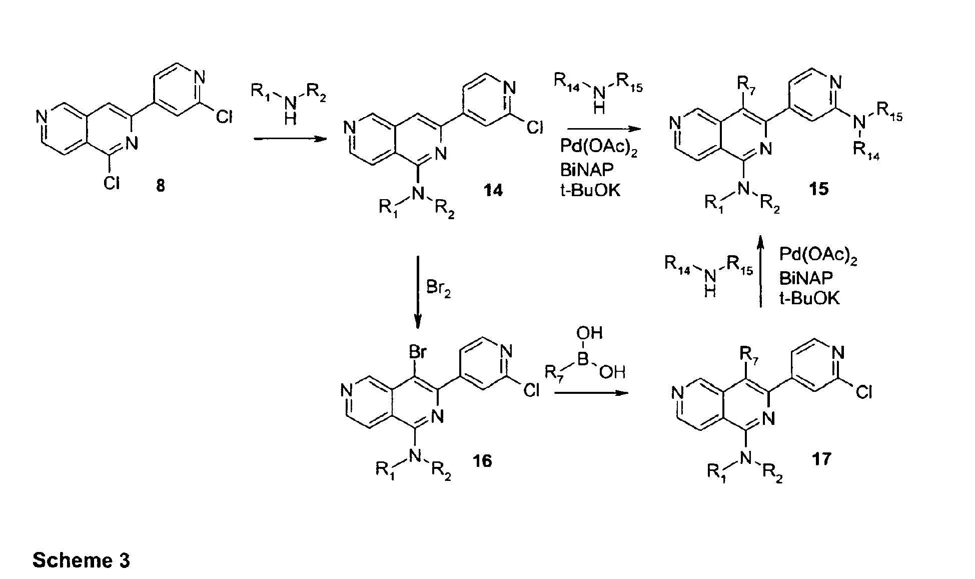

Alternatively, naphthyridines **14** may be accessed directly from nitrile **18** by treatment with a nucleophile HNR₁R₂ according to Scheme 4.

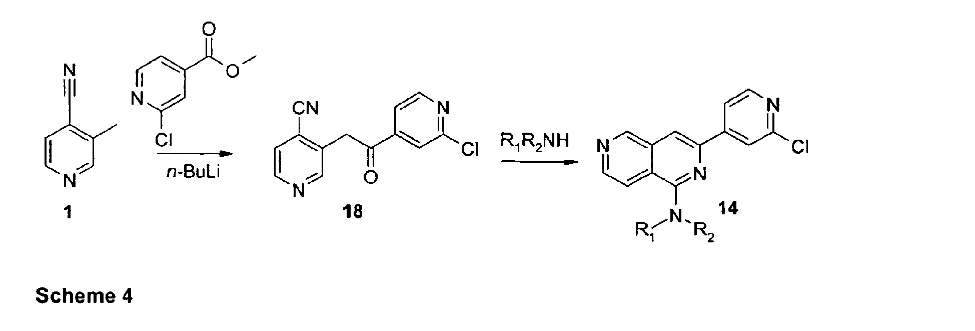

Alternatively, naphthyridines **13** may be accessed from **7** by Pd catalyzed coupling between the chloropyrine moeity an amine HR₁₄R₁₅, followed by chlorination with POCl₃ to give **19** and treatment with a nucleophile HNR₁R₂ according to Scheme 5. Compounds **19** may be further halogenated by electrophilic halogenating agents such as N-bromosuccinimide or the like to afford compounds **20.** Displacement of the 1-naphthyl chloride by suitable nucleophiles HR₁₄R₁₅ proceeds to compounds **21.** Any remaining halogens of **21** may be converted to groups R by methods known in the art, such as Suzuki couplings.

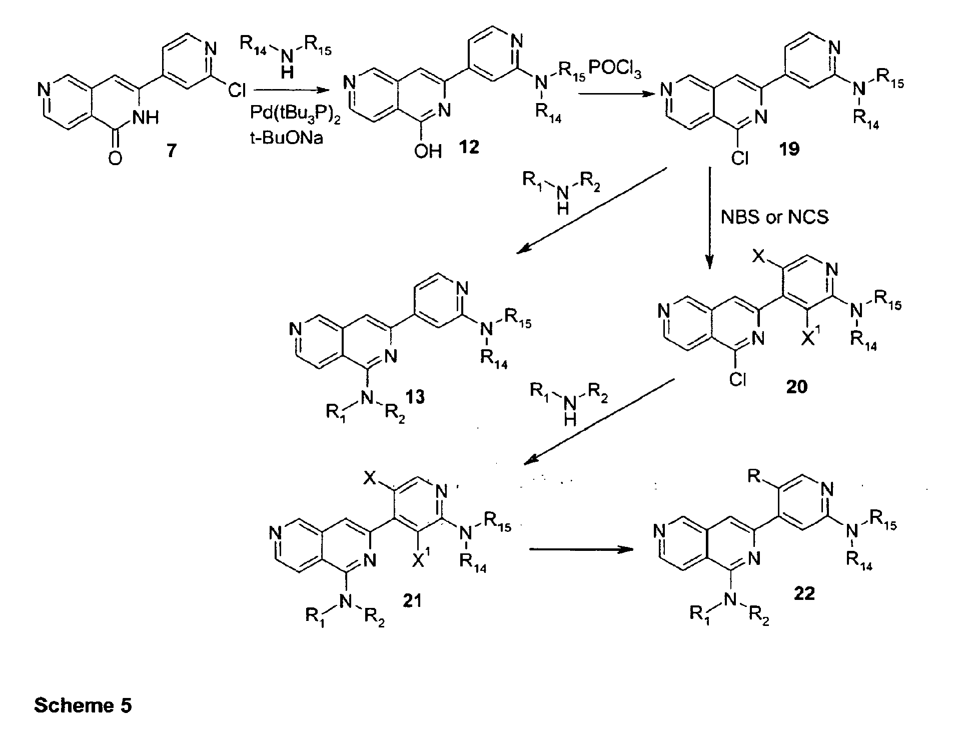

Any of the given residues R₁, R₂, R₁₄, or R₁₅ can subsequently be object of further functionalization, as intermediates **14 ,16,** or **17** for example through an saponifaction-amide formation protocol, and any of the residues R₁-R₄ can be subjected to final deprotection (*e.g*., cleavage of a BOC group using a strong acid such as trifluoroacidic acid, TFA, in a suitable solvent such as dichloromethane, DCM). Depending on these steps the compounds of the present invention can be obtained as a neutral compound or any of its salt forms (*e.g*., hydrochloride, TFA salt) acid such as trifluoroacidic acid, TFA, in a suitable solvent such as dichloromethane, DCM). Depending on these steps the compounds of the present invention can be obtained as a neutral compound or any of its salt forms (*e.g*., hydrochloride, TFA salt).

Generally, enantiomers of the compounds of the present invention can be prepared by methods known to those skilled in the art to resolve racemic mixtures, such as by formation and recrystallization of diastereomeric salts or by chiral chromotagraphy or HPLC separation utilizing chiral stationery phases.

In starting compounds and intermediates which are converted to the compounds of the invention in a manner described herein, functional groups present, such as amino, thiol, carboxyl and hydroxy groups, are optionally protected by conventional protecting groups that are common in preparative organic chemistry. Protected amino, thiol, carboxyl and hydroxyl groups are those that can be converted under mild conditions into free amino thiol, carboxyl and hydroxyl groups without the molecular framework being destroyed or other undesired side reactions taking place.

The purpose of introducing protecting groups is to protect the functional groups from undesired reactions with reaction components under the conditions used for carrying out a desired chemical transformation. The need and choice of protecting groups for a particular reaction is known to those skilled in the art and depends on the nature of the functional group to be protected (hydroxyl group, amino group, etc.), the structure and stability of the molecule of which the substituent is a part and the reaction conditions.

Well-known protecting groups that meet these conditions and their introduction and removal are described, *e.g*., in McOmie, "Protective Groups in Organic Chemistry", Plenum Press, London, NY (1973); and Greene and Wuts, "Protective Groups in Organic Synthesis", John Wiley and Sons, Inc., NY (1999).

The above-mentioned reactions are carried out according to standard methods, in the presence or absence of diluent, In some embodiments, such as are inert to the reagents and are solvents thereof, of catalysts, condensing or said other agents, respectively and/or inert atmospheres, at low temperatures, room temperature or elevated temperatures, In some embodiments at or near the boiling point of the solvents used, and at atmospheric or super-atmospheric pressure. The preferred solvents, catalysts and reaction conditions are set forth in the appended illustrative Examples.

The invention further includes any variant of the present processes, in which an intermediate product obtainable at any stage thereof is used as starting material and the remaining steps are carried out, or in which the starting materials are formed *in situ* under the reaction conditions, or in which the reaction components are used in the form of their salts or optically pure antipodes.

In another aspect, the present invention provides a pharmaceutical composition comprising a compound of the present invention and a pharmaceutically acceptable carrier. The pharmaceutical composition can be formulated for particular routes of administration such as oral administration, parenteral administration, and rectal administration, etc. In addition, the pharmaceutical compositions of the present invention can be made up in a solid form including capsules, tablets, pills, granules, powders or suppositories, or in a liquid form including solutions, suspensions or emulsions. The pharmaceutical compositions can be subjected to conventional pharmaceutical operations such as sterilization and/or can contain conventional inert diluents, lubricating agents, or buffering agents, as well as adjuvants, such as preservatives, stabilizers, wetting agents, emulsifers and buffers etc.

In some embodiments, the pharmaceutical compositions are tablets and gelatin capsules comprising the active ingredient together with
a) diluents, *e.g*., lactose, dextrose, sucrose, mannitol, sorbitol, cellulose and/or glycine;
b) lubricants, *e.g*., silica, talcum, stearic acid, its magnesium or calcium salt and/or polyethyleneglycol; for tablets also
c) binders, *e.g*., magnesium aluminum silicate, starch paste, gelatin, tragacanth, methylcellulose, sodium carboxymethylcellulose and/or polyvinylpyrrolidone; if desired
d) disintegrants, *e.g*., starches, agar, alginic acid or its sodium salt, or effervescent mixtures; and/or
e) absorbents, colorants, flavors and sweeteners.

Tablets may be either film coated or enteric coated according to methods known in the art.

Suitable compositions for oral administration include an effective amount of a compound of the invention in the form of tablets, lozenges, aqueous or oily suspensions, dispersible powders or granules, emulsion, hard or soft capsules, or syrups or elixirs. Compositions intended for oral use are prepared according to any method known in the art for the manufacture of pharmaceutical compositions and such compositions can contain one or more agents selected from the group consisting of sweetening agents, flavoring agents, coloring agents and preserving agents in order to provide pharmaceutically elegant and palatable preparations. Tablets contain the active ingredient in admixture with nontoxic pharmaceutically acceptable excipients which are suitable for the manufacture of tablets. These excipients are, for example, inert diluents, such as calcium carbonate, sodium carbonate, lactose, calcium phosphate or sodium phosphate; granulating and disintegrating agents, for example, corn starch, or alginic acid; binding agents, for example, starch, gelatin or acacia; and lubricating agents, for example magnesium stearate, stearic acid or talc. The tablets are uncoated or coated by known techniques to delay disintegration and absorption in the gastrointestinal tract and thereby provide a sustained action over a longer period. For example, a time delay material such as glyceryl monostearate or glyceryl distearate can be employed. Formulations for oral use can be presented as hard gelatin capsules wherein the active ingredient is mixed with an inert solid diluent, for example, calcium carbonate; calcium phosphate or kaolin, or as soft gelatin capsules wherein the active ingredient is mixed with water or an oil medium, for example, peanut oil, liquid paraffin or olive oil.

Injectable compositions are in some embodiments aqueous isotonic solutions or suspensions, and suppositories are advantageously prepared from fatty emulsions or suspensions. Said compositions may be sterilized and/or contain adjuvants, such as preserving, stabilizing, wetting or emulsifying agents, solution promoters, salts for regulating the osmotic pressure and/or buffers. In addition, they may also contain other therapeutically valuable substances. Said compositions are prepared according to conventional mixing, granulating or coating methods, respectively, and contain about 0.1-75%, In some embodiments about 1-50%, of the active ingredient.

Suitable compositions for transdermal application include an effective amount of a compound of the invention with carrier. Advantageous carriers include absorbable pharmacologically acceptable solvents to assist passage through the skin of the host. For example, transdermal devices are in the form of a bandage comprising a backing member, a reservoir containing the compound optionally with carriers, optionally a rate controlling barrier to deliver the compound of the skin of the host at a controlled and predetermined rate over a prolonged period of time, and means to secure the device to the skin.

Suitable compositions for topical application, *e.g*., to the skin and eyes, include aqueous solutions, suspensions, ointments, creams, gels or sprayable formulations, *e.g*., for delivery by aerosol or the like. Such topical delivery systems will in particular be appropriate for dermal application, *e.g*., for the treatment of skin cancer, *e.g*., for prophylactic use in sun creams, lotions, sprays and the like. They are thus particularly suited for use in topical, including cosmetic, formulations well-known in the art. Such may contain solubilizers, stabilizers, tonicity enhancing agents, buffers and preservatives.

The present invention further provides anhydrous pharmaceutical compositions and dosage forms comprising the compounds of the present invention as active ingredients, since water can facilitate the degradation of some compounds. For example, the addition of water (*e.g*., 5%) is widely accepted in the pharmaceutical arts as a means of simulating long-term storage in order to determine characteristics such as shelf-life or the stability of formulations over time. See, *e.g*., Jens T. Carstensen, Drug Stability: Principles & Practice, 2d. Ed., Marcel Dekker, NY, N.Y., 1995, pp. 379-80. In effect, water and heat accelerate the decomposition of some compounds. Thus, the effect of water on a formulation can be of great significance since moisture and/or humidity are commonly encountered during manufacture, handling, packaging, storage, shipment, and use of formulations.

Anhydrous pharmaceutical compositions and dosage forms of the invention can be prepared using anhydrous or low moisture containing ingredients and low moisture or low humidity conditions. Pharmaceutical compositions and dosage forms that comprise lactose and at least one active ingredient that comprises a primary or secondary amine are in some embodiments anhydrous if substantial contact with moisture and/or humidity during manufacturing, packaging, and/or storage is expected.

An anhydrous pharmaceutical composition should be prepared and stored such that its anhydrous nature is maintained. Accordingly, anhydrous compositions are In some embodiments packaged using materials known to prevent exposure to water such that they can be included in suitable formulary kits. Examples of suitable packaging include, but are not limited to, hermetically sealed foils, plastics, unit dose containers (e. g., vials), blister packs, and strip packs.

The invention further provides pharmaceutical compositions and dosage forms that comprise one or more agents that reduce the rate by which the compound of the present invention as an active ingredient will decompose. Such agents, which are referred to herein as "stabilizers," include, but are not limited to, antioxidants such as ascorbic acid, pH buffers, or salt buffers, etc.

The pharmaceutical compositions contain a therapeutically effective amount of a compound of the invention as defined above, either alone or in a combination with one or two or more therapeutic agents, *e.g*., each at an effective therapeutic dose as reported in the art. Such theraprutic agents include at least one or two or more selected from the following groups:
(i) angiotensin II receptor antagonist or a pharmaceutically acceptable salt thereof, (ii) HMG-Co-A reductase inhibitor or a pharmaceutically acceptable salt thereof, (iii) angiotensin converting enzyme (ACE) Inhibitor or a pharmaceutically acceptable salt thereof, (iv) calcium channel blocker (CCB) or a pharmaceutically acceptable salt thereof, (v) dual angiotensin converting enzyme/neutral endopeptidase (ACE/NEP) inhibitor or a pharmaceutically acceptable salt thereof, (vi) ndothelin antagonist or a pharmaceutically acceptable salt thereof, (vii) enin inhibitor or a pharmaceutically acceptable salt thereof, (viii) diuretic or a pharmaceutically acceptable salt thereof, (ix) an ApoA-I mimic; (x) an anti-diabetic agent; (xi) an obesity-reducing agent; (xii) an aldosterone receptor blocker; (xiii) an endothelin receptor blocker; (xiv) a CETP inhibitor; (xv) an inhibitor of Na-K-ATPase membrane pump; (xvi) a beta-adrenergic receptor blocker or an alpha-adrenergic receptor blocker; (xvii) a neutral endopeptidase (NEP) inhibitor; and (xviii) an inotropic agent.

Furthermore, the combinations as described above can be administered to a subject via simultaneous, separate or sequential administration (use). Simultaneous administration (use) can take place in the form of one fixed combination with two or three or more active ingredients , or by simultaneously administering two or three or more compounds that are formulated independently. Sequential administration(use) In some embodiments means administration of one (or more) compounds or active ingredients of a combination at one time point, other compounds or active ingredients at a different time point, that is, in a chronically staggered manner, In some embodiments such that the combination shows more efficiency than the single compounds administered independently (especially showing synergism). Separate administration (use) In some embodiments means administration of the compounds or active ingredients of the combination independently of each other at different time points, In some embodiments meaning that two, or three or more compounds are administered such that no overlap of measurable blood levels of both compounds are present in an overlapping manner (at the same time).

Also combinations of two or three or more of sequential, separate and simultaneous administrations are possible, In some embodiments such that the combination compound-drugs show a joint therapeutic effect that exceeds the effect found when the combination compound-drugs are used independently at time intervals so large that no mutual effect on their therapeutic efficiency can be found, a synergistic effect being especially preferred.

Alternatively, the pharmaceutical compositions contain a therapeutically effective amount of a compound of the invention as defined above, either alone or in a combination with one or more therapeutic agents, *e.g*., each at an effective therapeutic dose as reported in the art, selected from the group consisting of an antiestrogen; an anti-androgen; a gonadorelin agonist; a topoisomerase I inhibitor; a topoisomerase II inhibitor; a microtubule active agent; an alkylating agent; an anti-neoplastic anti-metabolite; a platin compound; a compound targeting/decreasing a protein or lipid kinase activity or a protein or lipid phosphatase activity, a anti-angiogenic compound; a compound which induces cell differentiation processes; monoclonal antibodies; a cyclooxygenase inhibitor; a bisphosphonate; a heparanase inhibitor; a biological response modifier; an inhibitor of Ras oncogenic isoforms; a telomerase inhibitor; a protease inhibitor, a matrix metalloproteinase inhibitor, a methionine aminopeptidase inhibitor; a proteasome inhibitor; agents which target, decrease or inhibit the activity of Flt-3; an HSP90 inhibitor; antiproliferative antibodies; an HDAC inhibitor; a compound which targets, decreases or inhibits the activity/function of serine/theronine mTOR kinase; a somatostatin receptor antagonist; an anti-leukemic compound; tumor cell damaging approaches; an EDG binder; a ribonucleotide reductase inhibitor; an S-adenosylmethionine decarboxylase inhibitor; a monoclonal antibody of VEGF or VEGFR; photodynamic therapy; an Angiostatic steroid; an implant containing corticosteroids; an AT1 receptor antagonist; and an ACE inhibitor.

Additionally, the present invention provides: a pharmaceutical composition or combination of the present invention for use as a medicament; the use of a pharmaceutical composition or combination of the present invention for the preparation of a pharmaceutical composition for the delay of progression and/or treatment of a disorder or disease mediated by PKD, or characterized by abnormal activity of PKD, or by abnormal expression of PKD; the use of a pharmaceutical composition or combination of the present invention for the preparation of a pharmaceutical composition for the delay of progression and/or treatment of a disorder or disease selected from heart failure, colorectal cancer, regulation of cell growth, autoimmune disorders, or hyperproliferative skin disorders, etc..

Additionally, the present invention provides: a pharmaceutical composition or combination of the present invention for use as a medicament; the use of a pharmaceutical composition or combination of the present invention for the preparation of a pharmaceutical composition for the delay of progression and/or treatment of a disorder or disease mediated by CaMKII, or characterized by abnormal activity of CaMKII, or by abnormal expression of CaMKII; the use of a pharmaceutical composition or combination of the present invention for the preparation of a pharmaceutical composition for the delay of progression and/or treatment of a disorder or disease selected from hypertrophy, heart failure, cardiac arrhythmia, opioid tolerance and dependence, or osteoporosis, etc..

Additionally, the present invention provides: a pharmaceutical composition or combination of the present invention for use as a medicament; the use of a pharmaceutical composition or combination of the present invention for the preparation of a pharmaceutical composition for the delay of progression and/or treatment of a disorder or disease mediated by MARK, or characterized by abnormal activity of MARK, or by abnormal expression of MARK; the use of a pharmaceutical composition or combination of the present invention for the preparation of a pharmaceutical composition for the delay of progression and/or treatment of a disorder or disease selected from cancers, autoimmune diseases, tissue damage, central nervous system disorders, neurodegenerative disorders, fibrosis, bone disorders, polyglutamine-repeat disorders, anemias, thalassemias, inflammatory conditions, cardiovascular conditions, etc..

Additionally, the present invention provides: a pharmaceutical composition or combination of the present invention for use as a medicament; the use of a pharmaceutical composition or combination of the present invention for the preparation of a pharmaceutical composition for the delay of progression and/or treatment of a disorder or disease mediated by PRK, or characterized by abnormal activity of MARK, or by abnormal expression of PRK; the use of a pharmaceutical composition or combination of the present invention for the preparation of a pharmaceutical composition for the delay of progression and/or treatment of a disorder or disease selected from cardiac hypertrophy and heart failure.

Additionally, the present invention provides: a pharmaceutical composition or combination of the present invention for use as a medicament; the use of a pharmaceutical composition or combination of the present invention for the preparation of a pharmaceutical composition for the delay of progression and/or treatment of a disorder or disease mediated by CDK9, or characterized by abnormal activity of MARK, or by abnormal expression of CDK9; the use of a pharmaceutical composition or combination of the present invention for the preparation of a pharmaceutical composition for the delay of progression and/or treatment of a disorder or disease selected from cardiac hypertrophy.

Additionally, the present invention provides: a pharmaceutical composition or combination of the present invention for use as a medicament; the use of a pharmaceutical composition or combination of the present invention for the preparation of a pharmaceutical composition for the delay of progression and/or treatment of a disorder or disease mediated by ROCK, or characterized by abnormal activity of MARK, or by abnormal expression of ROCK; the use of a pharmaceutical composition or combination of the present invention for the preparation of a pharmaceutical composition for the delay of progression and/or treatment of a disorder or disease selected from cardiac hypertrophy.

Additionally, the present invention provides the use of a pharmaceutical composition or combination of the present invention for the preparation of a pharmaceutical composition for the delay of progression and/or treatment of a disorder or disease selected from e.g. diseases or disorders mediated by T lymphocytes, B lymphocytes, mast cells, eosinophils or cardiomyocytes e.g. acute or chronic rejection of organ or tissue allo- or xenografts, graft-versus-host disease, host-versus-graft disease, atheriosclerosis, cerebral infarction, vascular occlusion due to vascular injury such as angioplasty, restenosis, fibrosis (especially pulmonary, but also other types of fibrosis, such as renal fibrosis), angiogenesis, hypertension, heart failure, chronic obstructive pulmonary disease, CNS disease such as Alzheimer disease or amyotrophic lateral sclerosis, cancer, infectious disease such as AIDS, septic shock or adult respiratory distress syndrome, ischemia/reperfusion injury e.g. myocardial infarction, stroke, gut ischemia, renal failure or hermorrhage shock, or traumatic shock. The compounds of the invention are also useful in the treatment and/or prevention of acute or chronic inflammatory diseases or disorders or autoimmune diseases e.g. sarcoidosis, fibroid lung, idiopathic interstitial pneumonia, obstructive airways disease, including conditions such as asthma, intrinsic asthma, extrinsic asthma, dust asthma, particularly chronic or inveterate asthma (for example late asthma and airway hyperreponsiveness), bronchitis, including bronchial asthma, infantile asthma, rheumatoid arthritis, osteoarthritis, systemic lupus erythematosus, nephrotic syndrome lupus, Hashimoto's thyroiditis, multiple sclerosis, myasthenia gravis, type I diabetes mellitus and complications associated therewith, type II adult onset diabetes mellitus, uveitis, nephrotic syndrome, steroid dependent and steroid-resistant nephrosis, palmoplantar pustulosis, allergic encephalomyelitis, glomerulonephritis, psoriasis, psoriatic arthritis, atopic eczema (atopic dermatitis), allergic contact dermatitis, irritant contact dermatitis and further eczematous dermatitises, seborrheic dermatitis, lichen planus, pemphigus, bullous pemphigoid, epidermolysis bullosa, urticaria, angioedemas, vasculitides, erythemas, cutaneous eosinophilias, acne, alopecia areata, eosinophilic fasciitis, atherosclerosis, conjunctivitis, keratoconjunctivitis, keratitis, vernal conjunctivitis, uveitis associated with Behcet's disease, herpetic keratitis, conical cornea, Sjoegren's syndrome,dystorphia epithelialis corneae, keratoleukoma, ocular pemphigus, Mooren's ulcer, scleritis, Graves' ophthalmopathy, severe intraocular inflammation, inflammation of mucosa or blood vessels such as leukotriene B4-mediated diseases, gastric ulcers, vascular damage caused by ischemic diseases and thrombosis, cardiac hypertrophy, ischemic bowel disease, inflammatory bowel disease (e.g. Crohn's disease or ulcerative colitis), necrotizing enterocolitis, renal diseases including interstitial nephritis, Goodpasture's syndrome hemolytic uremic syndrome and diabetic nephropathy, nervous diseases selected from multiple myositis, Guillain-Barre syndrome, Meniere's disease and radiculopathy, collagen disease including scleroderma, Wegener's granuloma and Sjogren' syndrome, chronic autoimmune liver diseases including autoimmune hepatitis, primary biliary cirrhosis and sclerosing cholangitis), partial liver resection, acute liver necrosis (e.g. necrosis caused by toxins, viral hepatitis, shock or anoxia), cirrhosis, fulminant hepatitis, pustular psoriasis, Behcet's disease, active chronic hepatitis, Evans syndrome, pollinosis, idiopathic hypoparathyroidism, Addison disease, autoimmune atrophic gastritis, lupoid hepatitis, tubulointerstitial nephritis, membranous nephritis, or rheumatic fever. The compounds of formula I are useful for treating tumors, e.g. breast cancer, genitourinary cancer, lung cancer, gastrointestinal cancer, epidermoid cancer, melanoma, ovarian cancer, pancreas cancer, neuroblastoma, head and/or neck cancer or bladder cancer, or in a broader sense renal, brain or gastric cancer; in particular (i) a breast tumor; an epidermoid tumor, such as an epidermoid head and/or neck tumor or a mouth tumor; a lung tumor, for example a small cell or non-small cell lung tumor; a gastrointestinal tumor, for example, a colorectal tumor; or a genitourinary tumor, for example, a prostate tumor (especially a hormone-refractory prostate tumor); or (ii) a proliferative disease that is refractory to the treatment with other chemothe-rapeutics; or (iii) a tumor that is refractory to treatment with other chemotherapeutics due to multidrug resistance. They are also useful for treating tumors of blood and lymphatic system (e.g. Hodgkin's disease, Non-Hodgkin's lymphoma, Burkitt's lymphoma, AIDS-related lymphomas, malignant immunoproliferative diseases, multiple myeloma and malignant plasma cell neoplasms, lymphoid leukemia, acute or chronic myeloid leukemia, acute or chronic lymphocytic leukemia, monocytic leukemia, other leukemias of specified cell type, leukemia of unspecified cell type, other and unspecified malignant neoplasms of lymphoid, haematopoietic and related tissues, for example diffuse large cell lymphoma, T-cell lymphoma or cutaneous T-cell lymphoma). Myeloid cancer includes e.g. acute or chronic myeloid leukaemia.

Where a tumor, a tumor disease, a carcinoma or a cancer are mentioned, also metastasis in the original organ or tissue and/or in any other location are implied alternatively or in addition, whatever the location of the tumor and/or metastasis.

The pharmaceutical composition or combination of the present invention can be in unit dosage of about 1-1000 mg of active ingredients for a subject of about 50-70 kg, In some embodiments about 1-500 mg or about 1-250 mg or about 1-150 mg or about 0.5-100 mg, or about 1-50 mg of active ingredients. The therapeutically effective dosage of a compound, the pharmaceutical composition, or the combinations thereof, is dependent on the species of the subject, the body weight, age and individual condition, the disorder or disease or the severity thereof being treated. A physician, clinician or veterinarian of ordinary skill can readily determine the effective amount of each of the active ingredients necessary to prevent, treat or inhibit the progress of the disorder or disease.

The above-cited dosage properties are demonstrable *in vitro* and *in vivo* tests using advantageously mammals, *e.g.*, mice, rats, dogs, monkeys or isolated organs, tissues and preparations thereof. The compounds of the present invention can be applied *in vitro* in the form of solutions, *e.g*., In some embodiments aqueous solutions, and *in vivo* either enterally, parenterally, advantageously intravenously, *e.g*., as a suspension or in aqueous solution. The dosage *in vitro* may range between about 10⁻³ molar and 10⁻⁹ molar concentrations. A therapeutically effective amount *in vivo* may range depending on the route of administration, between about 0.1-500 mg/kg, In some embodiments between about 1-100 mg/kg.

The activities of a compound according to the present invention can be assessed by the following *in vitro* & *in vivo* methods well-described in the art, such as the DSS rat model as described in Journal of Hypertension (2005) 23, 87, the mouse pressure overload model Circulation (1999) 84, 735, or by methods outlined in the current document, such as the GvH model or the peripheral lymphocyte reduction model.

The assay to measure protein kinase D1 (PKD1) activity is a time-resolved fluorescence resonance transfer (TR-FRET) assay using PerkinElmer's LANCE™ technology. In this case, a biotinylated syntide-2 peptide is used as the substrate in this reaction. Phosphorylation of the syntide-2 substrate is detected by a specific antibody that recognizes the phosphorylated peptide. A second flurophore, APC, is conjugated to streptavidin that binds the biotinylated syntide-2 peptide. For detection, the europium fluorophore can be excited by 340nM light which then emits at 615 nM. Therefore, when the europium labeled secondary antibody binds on the phosphorylated peptide, it is brought into close contact with the APC and excites this fluorophore. The APC emission is at 665 nM and the 665 nM:615 nM ratio is a readout of PKD1 activity.

This assay is performed with full length wild-type enzyme that is expressed and purified from Sf9 insect cells. The reaction buffer consists of 35mM Tris-HCl pH7.5, 5mM MgCl₂, 0.02% Tween-20, 20 µM ATP, 1 mM DTT and 0.2 µg/mL PKD1 enzyme. The enzyme reaction is initiated by the addition of 2 µM syntide-2 peptide substrate and the reaction carried out for 50 minutes at room temperature. The reaction is stopped by a stop/detection buffer consisting of 50 mM EDTA, 0.18 mg/mL rabbit polyclonal anti-phospho syntide-2 antibody, 0.5 nM europium labeled anti-rabbit IgG and 10 nM streptavidin conjugated APC. After a one hour incubation with the stop/detection buffer, the reaction is read on an Envision 2100 Reader using a LANCE™ Eu/APC dual protocol. As described above, a 665 nM:615 nM ratio is determined to measure substrate phosphorylation and enzyme activity. Compounds are typically tested in an 11 point dose response fashion in triplicate for each concentration used. IC₅₀ values are calculated using an Activity Base (IDBS) software program.

The assay to measure protein kinase D2 (PKD2) activity is a time-resolved fluorescence resonance transfer (TR-FRET) assay using PerkinElmer's LANCE™ technology. In this case, a biotinylated syntide-2 peptide is used as the substrate in this reaction. Phosphorylation of the syntide-2 substrate is detected by a specific antibody that recognizes the phosphorylated peptide. A second flurophore, APC, is conjugated to streptavidin that binds the biotinylated syntide-2 peptide. For detection, the europium fluorophore can be excited by 340nM light which then emits at 615 nM. Therefore, when the europium labeled secondary antibody binds on the phosphorylated peptide, it is brought into close contact with the APC and excites this fluorophore. The APC emission is at 665nM and the 665 nM:615 nM ratio is a readout of PKD2 activity.

This assay is performed with full length wild-type enzyme purchase from Invitrogen. The reaction buffer consists of 35 mM Tris-HCl pH7.5, 5 mM MgCl₂, 0.02% Tween-20, 20 µM ATP, 1 mM DTT and 0.2 µg/mL PKD2 enzyme. The enzyme reaction is initiated by the addition of 2 µM syntide-2 peptide substrate and the reaction carried out for 50 minutes at room temperature. The reaction is stopped by a stop/detection buffer consisting of 50 mM EDTA, 0.18 mg/mL rabbit polyclonal anti-phospho syntide-2 antibody, 0.5 nM europium labeled anti-rabbit IgG and 10 nM streptavidin conjugated APC. After a one hour incubation with the stop/detection buffer, the reaction is read on an Envision 2100 Reader using a LANCE™ Eu/APC dual protocol. As described above, a 665 nM:615 nM ratio is determined to measure substrate phosphorylation and enzyme activity. Compounds are typically tested in an 11 point dose response fashion in triplicate for each concentration used. IC₅₀ values are calculated using an Activity Base (IDBS) software program.

The assay to measure protein kinase D3 (PKD3) activity is a time-resolved fluorescence resonance transfer (TR-FRET) assay using PerkinElmer's LANCE™ technology. In this case, a biotinylated syntide-2 peptide is used as the substrate in this reaction. Phosphorylation of the syntide-2 substrate is detected by a specific antibody that recognizes the phosphorylated peptide. A second flurophore, APC, is conjugated to streptavidin that binds the biotinylated syntide-2 peptide. For detection, the europium fluorophore can be excited by 340nM light which then emits at 615 nM. Therefore, when the europium labeled secondary antibody binds on the phosphorylated peptide, it is brought into close contact with the APC and excites this fluorophore. The APC emission is at 665 nM and the 665 nM:615 nM ratio is a readout of PKD3 activity.

This assay is performed with full length wild-type enzyme that is purchased from Invitrogen. The reaction buffer consists of 35 mM Tris-HCl pH7.5, 5mM MgCl₂, 0.02% Tween-20, 20 µM ATP, 1 mM DTT and 0.2 µg/mL PKD3 enzyme. The enzyme reaction is initiated by the addition of 2 µM syntide-2 peptide substrate and the reaction carried out for 50 minutes at room temperature. The reaction is stopped by a stop/detection buffer consisting of 50 mM EDTA, 0.18 mg/mL rabbit polyclonal anti-phospho syntide-2 antibody, 0.5 nM europium labeled anti-rabbit IgG and 10 nM streptavidin conjugated APC. After a one hour incubation with the stop/detection buffer, the reaction is read on an Envision 2100 Reader using a LANCE™ Eu/APC dual protocol. As described above, a 665 nM:615 nM ratio is determined to measure substrate phosphorylation and enzyme activity. Compounds are typically tested in an 11 point dose response fashion in triplicate for each concentration used. IC₅₀ values are calculated using an Activity Base (IDBS) software program.

The calcium/calmodulin dependent kinase II (CaMKII) is activated by the binding of calcium-bound calmodulin. An *in vitro* biochemical assay has been established using the amplified luminescent proximity homogeneous or AlphaScreen™ technology (PerkinElmer). This assay utilizes "donor" and "acceptor" beads that when brought into close proximity and subsequently laser excited, produce an amplified light signal in the 520-620 nM range. In this assay, a biotinylated autocamtide-2 peptide substrate for CaMKII is bound to streptavidin-coated AlphaScreen donor beads. Phosphorylation of the substrate is recognized by an antibody specific for the phosphorylated autocamtide-2 peptide bound to the protein A coated acceptor beads. Therefore, phosphorylation of the autocamtide-2 by CaMKII will be recognized by the antibody, bring the acceptor and donor beads in close proximity and produce a strong signal.

The assay is performed with the human CaMKIIδ isoform and calmodulin (Millipore Corp.). The assay buffer consists of 20 mM HEPES pH 7.2, 10 mM MgCl₂, 1 mM CaCl₂, 0.1% BSA, 30 µM ATP and 1 mM DTT. Final concentration for CaMKIIδ and calmodulin is 0.78 ng/mL and 20 µg/mL, respectively. The reaction is incubated at room temperature for 30 minutes. The reaction is stopped by the addition of the quench/detection mix that is diluted to a final concentration when added to the reaction mix of 20 mM HEPES pH 7.2, 0.1% BSA, 45 mM EDTA, 3:1000 dilution of phosphoThr286 antibody and 30 µg/mL each of AlphaScreen™ streptavidin donor beads and protein A coated acceptor beads (PerkinElmer). After addition of the quench/detection mix the reaction is incubated at room temperature in the dark for 3 hours. The reaction is then read on an Envision reader (PerkinElmer). Test compounds are typically screened in a 10 point dose response at half log increments with a top concentration of 30µM.

The compounds of the invention are tested for their activity on different PKC isotypes according to the following method. Assay is performed in a white with clear bottom 384-well microtiterplate with non-binding surface. The reaction mixture (25 µl) contains 1.5 µM of a tridecapeptide acceptor substrate that mimics the pseudo substrate sequence of PKC □ with the Ala → Ser replacement, 10 µM ³³P-ATP, 10 mM Mg(NO₃)₂, 0.2 mM CaCl₂, PKC at a protein concentration varying from 25 to 400 ng/mL (depending on the isotype used), lipid vesicles (containing 30 mol% phosphatidylserine, 5 mol% DAG and 65 mol% phosphatidylcholine) at a final lipid concentration of 0.5 mM, in 20 mM Tris-HCl buffer pH 7.4 + 0.1% BSA. Incubation is performed for 60 min at room temperature. Reaction is stopped by adding 50 µl of stop mix (100 mM EDTA, 200 µM ATP, 0.1% Triton X-100, 0.375 mg/well streptavidin-coated SPA beads in phosphate buffered saline w/o Ca, Mg. After 10 min incubation at room temperature, the suspension is spun down for 10 min at 300g. Incorporated radioactivity is measured in a Trilux counter for 1 min. IC₅₀ measurement is performed on a routine basis by incubating a serial dilution of inhibitor at concentrations ranging between 1-1000 nM. IC₅₀ values are calculated from the graph by curve fitting with XL fit^{®} software. Human recombinant PKCθ is used under the assay conditions as described above. Human recombinant PKCα is obtained from Oxford Biomedical Research and is used under the assay conditions as described above. Human recombinant PKCβ is obtained from Oxford Biomedical Research and is used under the assay conditions as described above. Human recombinant PKCδ is obtained from Oxford Biomedical Research and is used under the assay conditions as described above. Human recombinant PKCε is obtained from Oxford Biomedical Research and is used under the assay conditions as described above. Human recombinant PKCη is obtained from PanVera and is used under the assay conditions as described above.

The PKN-2 assay is performed using the Upstate IC₅₀ Profiler Express™ service. In a final reaction volume of 25 mL, human recombinant PKN-2 (5-10 mU) is incubated with 50 mM Tris pH 7.5, 0.1 mM EGTA, 0.1% β-mercaptoethanol, 30 µM undecapeptide (AKRRRLSSLRA), 10 mM magnesium acetate and γ-³³P-ATP (specific activity approx. 500 cpm / pmol, concentration as required). The reaction is initiated by the addition of the Mg / ATP mix. After incubation for 40 minutes at room temperature, the reaction is stopped by the addition of 5 µL of a 3% phosphoric acid solution. 10 µL of the reaction is then spotted onto a P30 filtermat and washed three times for 5 minutes in 75 mM phosphoric acid and once in methanol prior to drying and scintillation counting.

The ROCK-II assay is performed using the Upstate IC₅₀ Profiler Express™ service. In a final reaction volume of 25 mL, human recombinant ROCK-II (5-10 mU) is incubated with 50 mM Tris pH 7.5, 0.1 mM EGTA, 30 µM KEAKEKRQEQIAKRRRLSSLRASTSKSGGSQK, 10 mM magnesium acetate and γ-³³P-ATP (specific activity approx. 500 cpm / pmol, concentration as required). The reaction is initiated by the addition of the Mg / ATP mix. After incubation for 40 minutes at room temperature, the reaction is stopped by the addition of 5 µL of a 3% phosphoric acid solution. 10 µL of the reaction is then spotted onto a P30 filtermat and washed three times for 5 minutes in 75 mM phosphoric acid and once in methanol prior to drying and scintillation counting.

The CDK9 assay is performed using the Upstate IC₅₀ Profiler Express™ service. In a final reaction volume of 25 µL, CDK9/cyclinT1 (h) (5-10 mU) is incubated with 8 mM MOPS pH 7.0, 0.2 mM EDTA, 100 µM KTFCGTPEYLAPEVRREPRILSEEEQEMFRDFDYIADWC, 10 mM MgAcetate and [©-33P-ATP] (specific activity approx. 500 cpm/pmol, concentration as required). The reaction is initiated by the addition of the MgATP mix. After incubation for 40 minutes at room temperature, the reaction is stopped by the addition of 5 µL of a 3% phosphoric acid solution. 10 µL of the reaction is then spotted onto a P30 filtermat and washed three times for 5 minutes in 75 mM phosphoric acid and once in methanol prior to drying and scintillation counting.

The MARK1 assay is performed using the Upstate IC₅₀ Profiler Express™ service. In a final reaction volume of 25 µL, MARK1 (h)(5-10 mU) is incubated with 8 mM MOPS pH 7.0, 0.2 mM EDTA, 100 µM KKKVSRSGLYRSPSMPENLNRPR, 10 mM MgAcetate and [γ-³³P-ATP] (specificactivity approx. 500 cpm/pmol, concentration as required). The reaction is initiated by the addition of the MgATP mix. After incubation for 40 minutes at room temperature, the reaction is stopped by the addition of 5 µL of a 3% phosphoric acid solution. 10 µL of the reaction is then spotted onto a P30 filtermat and washed three times for 5 minutes in 75 mM phosphoric acid and once in methanol prior to drying and scintillation counting.

The MARK2 assay is performed with enzyme purchased from Invitrogen. The following stock solutions are prepared for this assay. A 1x MARK2 assay buffer is prepared from 25 mM Tris-HCl, 5 mM MgCl₂, and 1 mM DTT. A 1.7x MARK2 enzyme dilution buffer is prepared from 42.5 mM Tris-HCl (pH 7.4), 1.7 mM DTT, 17% glycerol, 0.034% Triton X-100, and 1.7 mg/ml BSA. A 2x ATP solution is prepared from 400 µM ATP diluted in kinase reaction buffer. A 3x stop buffer is prepared from 25 mM EDTA in water. Assay compounds are resuspended in 100% DMSO and diluted to 5x the intended screening concentration in MilliQ water to give a final concentration of 2.44% DMSO. Assay reagent 2X ATP is prepared from 400 µM ATP in kinase reaction buffer (2X). A 3.33x MARK2 / CHKtide reagent is prepared by adding 3.33 pM MARK2 enzyme and 5.3µM CHKtide to 1.7X MARK2 enzyme dilution buffer. In 1X Lance buffer is diluted the detection reagents (18 µL per well) to the following concentrations:

| | |
|---|---|
| Kinase SA-APC | 27.5 nM |
| Eu-goat-anti-rabbit | 1.1 nM |
| Anti phospho-cdc25C | 1.1 nM |

The assay is performed from above reagents in the following steps: 1) add 6 µL MARK2 enzyme mix per well, 2) dose 4 µL from aqueous DMSO compound plate (2.44% DMSO) to MARK2 reaction mix and incubate for 10 minutes, 3) add 10 µL MARK2 reaction mix and incubate for 30 minutes, and 4) stop reaction by adding 10 µL reaction stop buffer. The Lance detection procedure is conducted as follows: 1) remove 2µL of stopped MARK2 assay to a Perkin Elmer Optiplate, 2) add 18 µL of the Lance Detection Reagents, 3) incubate in the dark at room temperature for 3 hours, and 4) read plate using the fusion

Compounds are evaluated in the HDAC5 nuclear exposrt assay, a 384-well plate-based assay that enables high throughput screening (HTS) to identify small molecules that block agonist-dependent nuclear export of HDAC5. This assay employs the Cellomics High Content Imaging platform (Giuliano & Taylor 1998) and adenovirus encoding green fluorescent protein (GFP) tagged HDAC5. Neonatal rat ventricular myocytes (NRVMs) are infected with GFP-HDAC5 encoding virus and plated on gelatin-coated 384-well dishes. Cells are exposed to compound and stimulated with an prostaglandin (PGF2α), which is a potent stimulus for HDAC5 nuclear export. Following two hours of stimulation, cells are fixed and GFP-HDAC5 localization quantified using the Cellomics system, which provides a read-out of relative fluorescence intensity in the cytoplasmic versus nuclear compartment.

A two-way allogenic mixed lymphocyte reaction (MLR) is performed according to standard procedures (J. Immunol. Methods, 1973, 2, 279 and Meo T. et al., Immunological Methods, New York, Academic Press, 1979, 227-39). Briefly, spleen cells from CBA and BALB/c mice (1.6 x 10⁵ cells from each strain per well in flat bottom tissue culture microtiter plates, 3.2 x 10⁵ in total) are incubated in RPMI medium containing 10% FCS, 100 U/ml penicillin, 100 µg/ml streptomycin (Gibco BRL, Basel, Switzerland), 50 µM 2-mercaptoethanol (Fluka, Buchs, Switzerland) and serially diluted compounds. Seven three-fold dilution steps in duplicates per test compound are performed. After four days of incubation 1 µCi ³H-thymidine is added. Cells are harvested after an additional five-hour incubation period, and incorporated ³H-thymidine is determined according to standard procedures. Background values (low control) of the MLR are the proliferation of BALB/c cells alone. Low controls are subtracted from all values. High controls without any sample are taken as 100% proliferation. Percent inhibition by the samples is calculated, and the concentrations required for 50% inhibition (IC₅₀ values) are determined.

A bone marrow cell proliferation assay Bone marrow cells from CBA mice (2.5 x 10⁴ cells per well in flat bottom tissue culture microtiter plates) are incubated in 100 µL RPMI medium containing 10% FCS, 100 U/mL penicillin, 100 µg/mL streptomycin (Gibco BRL, Baselm Switzerland), 50 µM 2-mercaptoethanol (Fluka, Buchs, Switzerland), WEHI-3 conditioned medium (7.5% v/v) and L929 conditioned medium (3% v/v) as a source of growth factors and serially diluted compounds. Seven three-fold dilution steps in duplicates per test compounds are performed. After four days of incubation 1 µCi ³H-thymidine is added. Cells are harvested after an additional five-hour incubation period, and incorporated ³H-thymidine is determined according to standard procedures. Conditioned media are prepared as follows. WEHI-3 cells (ATCC TIB68) and L929 cells (ATCC CCL 1) are grown in RPMI medium until confluence for 4 days and one week, respectively. Cells are harvested, resuspended in the same culture flasks in medium C containing 1% FCS (Schreier and Tess 1981) for WEHI-3 cells and RPMI medium for L929 cells and incubated for 2 days (WEHI-3) or one week (L929). The supernatant is collected, filtered through 0.2 □m and stored in aliquots at -80 °C. Cultures without test compounds and without WEHI-3 and L929 supernatants are used as low control values. Low control values are substracted from all values. High controls without any sample are taken as 100% proliferation. Percent inhibition by the samples is calculated and the concentrations required for 50% inhibition (IC₆₀ values) are determined.

Compounds can be evaluated in the peripheral lymphocyte reduction model. Rats are subjected to a single oral dose of either placebo (control) or compound at different doses. Sublingual blood for hematological monitoring is collected before compound administration (baseline) and 2, 6, 8, and 24 hours after compound application. To this end, rats are anaesthetized with isoflurane and whole blood (< 200 µl) is sampled from the sublingual vein in EDTA-coated Eppendorf tubes. Subsequently, whole blood is subjected to hematological analysis using an automated hematology analyzer for counting different blood cell types and measuring various blood components. This includes red blood cells, hemoglobin, hematocrit, platelets and white blood cells such as neutrophils, lymphocytes, monocytes, eosinophils and basophils.

Compounds can be evaluated in the localized graft-versus-host model (GvH). Spleen cells (2x10⁷) from Wistar/F rats are injected subcutaneously into the right hind footpad of (Wistar/F x Fischer 344)F₁ hybrid rats. The left footpad is left untreated. The animals are treated with the test compounds on 4 consecutive days (0-3). The popliteal lymph nodes are removed on day 7, and the weight differences between two corresponding lymph nodes are determined. The results are expressed as the inhibition of lymph node enlargement (given in percent) comparing the lymph node weight differences in the experimental groups to the weight difference between the corresponding lymph nodes from a group of animals left untreated with a test compound.

**Table 1 Inhibitory Activity of Compounds**

| **#** | **Compound** | **PKD1 (nM)** | **PKD2 (nM)** | **PKD3 (nM)** | **HDAC (nM)** |
|---|---|---|---|---|---|
| 1 | 1-(3-hydroxypropylamino-3-(2-methylaminopyridin-4-yl)-[2,6]-naphthyridine | 377 | 903 | 227 | |
| 2 | 1-Methylamino-3-(2-methylaminopyridin-4-yl)-[2,6]-naphthyridine | 833 | 753 | 450 | |
| 3 | Cyclohexyl-[4-(1-piperazin-1-yl-[2,6]naphthyridin-3-yl)pyridin-2-yl]amine | 0.6 | 0.3 | 0.3 | 32 |
| 4 | Phenyl-[4-(1-piperazin-1-yl-[2,6]naphthyridin-3-yl)pyridin-2-yl]amine | 11 | 40 | 8 | 2 |
| 5 | 2-[3-(2-Cyclohexylaminopyridin-4-yl)-[2,6]naphthyridin-1-ylamino]ethanol | 382 | 1372 | 339 | |
| 6 | 2-[3-(2-Isopropylaminopyridin-4-yl)-[2,6]naphthyridin-1-ylamino]ethanol | 156 | 497 | 120 | |
| 7 | (3-Methoxyphenyl)-[4-(2-hydroxyl)amine-1-yl-[2,6]naphthyridin-3-yl)pyridin-2-yl]amine | 55 | 195 | 62 | 86 |
| 8 | *N-*[3-(2-Cyclohexylaminopyridin-4-yl)-[2,6]naphthyridin-1-yl]-*N*',*N*'-dimethylethane-1,2-diamine | 55 | 259 | 58 | |
| 9 | *N*,*N-*Dimethyl-N'-[3-(2-phenylaminopyridin-4-yl)-[2,6]naphthyridin-1-yl]-ethane-1,2-diamine | 41 | 169 | 39 | 756 |
| 10 | 1-[3-(2-Cyclohexylaminopyridin-4-yl)-[2,6]naphthyridin-1-yl]piperidine-4-carboxylic acid amide | 83 | 414 | 83 | 203 |
| 11 | 1-{4-[3-(2-Cyclohexylaminopyridin-4-yl)-[2,6]naphthyridin-1-yl]piperazin-1-yl}ethanone | 186 | 137 | 23 | |
| 12 | Cyclohexyl-{4-[1-(4-dimethylaminomethylpiperidin-1-yl)-[2,6]naphthyridin-3-yl]pyridin-2-yl}amine | 207 | 63 | 21 | |
| 13 | Cyclohexyl-{4-[1-(4-methylpiperazin-1-yl)-[2,6]naphthyridin-3-yl]pyridin-2-yl}amine | 2 | 14 | 1 | 206 |
| 14 | [3-(2-Cyclohexylaminopyridin-4-yl)-[2,6]naphthyridin-1-yl]pyrrolidin-3-ylamine | 1 | 6 | 1 | 36 |
| 15 | [3-(2-Cyclohexylaminopyridin-4-yl)-[2,6]naphthyridin-1-yl]piperidin-3-ylamine | 14 | 102 | 19 | 605 |
| 16 | 1-{3-[2-(Tetrahydropyran-4-ylamino)pyridin-4-yl]-[2,6]naphthyridin-1-yl}piperidine-4-carboxylic acid (2-hydroxyethyl)amide | 2 | 11 | 2 | 60 |
| 17 | 1-{3-[2-(Tetrahydropyran-4-ylamino)pyridin-4-yl]-[2,6]naphthyridin-1-yl}piperidine-4-carboxylic acid cyclopropylamide | 2 | 14 | 3 | 88 |
| 18 | 4-{3-[2-(1-Methyl-1*H*-pyrazol-3-ylamino)pyridin-4-yl]-[2,6]naphthyridin-1-yl}piperazine-1-carboxylic acid ethylamide | 20 | 127 | 30 | 534 |
| 19 | 1-{3-[2-(1-Methyl-1H-pyrazol-3-ylamino)pyridin-4-yl]-[2,6]naphthyridin-1-yl}piperidine-4-carboxylic acid amide | 0.4 | 2 | 0.4 | 12 |
| 20 | 8-{3-[2-(1-Methyl-1*H*-pyrazol-3-ylamino)pyridin-4-yl]-[2,6]naphthyridin-1-yl}-2,8-diaza-spiro[4.5]decan-1-one | 17 | 59 | 9 | 361 |
| 21 | (2-Methoxyethyl)-[4-(1-piperazin-1-yl-[2,6]naphthyridin-3-yl)-pyridin-2-yl]amine | 14 | 120 | 10 | 371 |

### Abbreviations:

- app: apparent
- ATP: adenosine 5'-triphosphate
- BINAP: racemic 2,2'-bis(diphenylphosphino)-1,1'-binaphthyl
- BOC: tertiary butyl carboxy
- br: broad
- BSA: bovine serum albumin
- d: doublet
- dd: doublet of doublets
- DCM: dichloromethane
- DIEA: diethylisopropylamine
- DME: 1,4-dimethoxyethane
- DMF: *N*,*N-*dimethylformamide
- DMSO: dimethylsulfoxide
- DTT: dithiothreitol
- EDTA: ethylenediamine tetraacetic acid
- ESI: electrospray ionization
- EtOAc: ethyl acetate
- FCC: flash column chromatography
- h: hour(s)
- HBTU: 1-[bis(dimethylamino)methylene]-1H-benzotriazoliumhexafluorophosphate(1-) 3-oxide
- HOBt: 1-hydroxy-7-azabenzotriazole
- HPLC: high pressure liquid chromatography
- IR: infrared spectroscopy
- LCMS: liquid chromatography and mass spectrometry
- MeOH: methanol
- MS: mass spectrometry
- MW: microwave
- m: multiplet
- min: minutes
- mL: milliliter(s)
- m/z: mass to charge ratio
- NMR: nuclear magnetic resonance
- ppm: parts per million
- PyBOP: benzotriazol-1-yloxytripyrrolidinophosphonium hexafluorophosphate
- rac: racemic
- rt: room temperature
- s: singlet
- t: triplet
- TFA: trifluoroacetic acid
- THF: tetrahydrofuran
- Tris·HCl: aminotris(hydroxymethyl)methane hydrochloride

### EXAMPLES

The following examples are intended to illustrate the invention and are not to be construed as being limitations thereon. Temperatures are given in degrees centrigrade. If not mentioned otherwise, all evaporations are performed under reduced pressure, In some embodiments between about 15 mm Hg and 100 mm Hg (= 20-133 mbar). The structure of final products, intermediates and starting materials is confirmed by standard analytical methods, *e.g*., microanalysis and spectroscopic characteristics, *e.g*., MS, IR, NMR. Abbreviations used are those conventional in the art. The compounds in the following examples have been found to have IC₅₀ values in the range of about 0.1 nM to about 1000.00 nM for PKD and other enzymes. The compounds in the following examples have been found to have IC₅₀ values in the range of about 1 nM to about 1000.00 nM for CaMKII. The compounds in the following examples have been found to have IC₅₀ values in the range of about 1 nM to about 1000.00 nM for PKN. The compounds in the following examples have been found to have IC₅₀ values in the range of about 1 nM to about 1000.00 nM for ROCK. The compounds in the following examples have been found to have IC₅₀ values in the range of about 1 nM to about 1000.00 nM for CDK9.

### Example 1

### A. 3-Methyl-isonicotinonitrile.

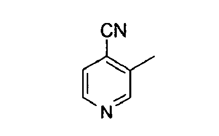

To 3-methyl-pyridine 1-oxide (15.90 g, 150 mmol) is added at 0 °C during 30 min. dimethylsulfate (15.60 mL). The resulting reaction mixture is stirred overnight at 40 °C. A solution of KCN (10.75 g, 165 mmol) in a mixture of EtOH/water 1:1 (120 mL) is added and the reaction mixture is stirred overnight at 40 °C. The reaction mixture is concentrated *in vacuo* and the residue is partitioned between EtOAc and water. The aqueous phase is extracted with EtOAc and the combined organic layers are dried over Na₂SO₄, filtered and concentrated at reduced pressure. Purification by flash column chromatography (silica gel, cyclohexane / EtOAc 85 : 15) affords the title compound as orange crystals (6.00 g, 50.80 mmol, 34%). ¹H NMR (400 MHz, DMSO-d₆) δ ppm 8.76 (s, 1H), 8.64 (d, *J* = 4.9 Hz, 1H), 7.80 (d, *J* = 4.9 Hz, 1H).

### B. N-t-Butylisonicotinamide.

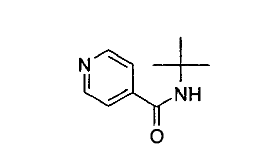

Isonicotinic acid (10 g, 80.4 mmol) is added to a 750 mL 5-necked flask equipped with an overhead stirrer, internal thermometer and nitrogen supply. Dichloromethane (300 mL) is added and the suspension is cooled to 0 °C. Triethylamine (17.6 mL, 121 mmol) is added maintaining a temperature under 0 °C to at which time the starting material dissolves. To the clear solution, ethyl chloroformate (9.5 mL, 98.1 mmol) is added dropwise over 25 min maintaining a temperature under 0 °C. The reaction mixture is stirred at 0 °C for 30 min. *tert-*Butylamine (10.4 mL, 96.5 mmol) is added slowly to the reaction mixture at 0 °C and the solution is allowed to warm to rt and stirred for 3.5 h. The reaction mixture is diluted with water (100 mL) and the dichloromethane layer is separated. The organic phase is washed with 1 M HCl (100 mL) and the aqueous phase, containing the product, neutralized to pH 9 with NaOH solution. The aqueous phase is washed twice with ethyl acetate (2 x 100 mL) and the combined organic phases, dried over Na₂SO₄, filtered and concentrated *in vacuo* to give a pale yellow solid (9.8 g, 68.4 %). MS (ESI) m/z 179 (M+1); ¹H-NMR (400 MHz, DMSO-*d₆*) δ ppm 8.66 (s, 2 H), 8.02 (br s, 1H), 7.70 (s, 2 H), 1.37 (s, 9 H).

### C. N-t-Butyl-3-methylisonicotinamide.

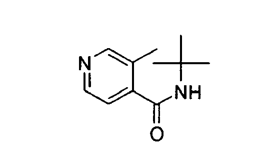

To the solution of the 3-methyl-isonicotinonitrile (18.90 g, 159.92 mmol) in DCM (50 mL) is added *t-*BuOAc (72.63 mL, 538.84 mmol), followed by concentrated H₂SO₄ (12.32 mL, 874.46 mmol). The reaction is stirred for 8 h at rt, then diluted with a solution of saturated aqueous NaHCO₃ and DCM (50 mL). The organic layer is washed with H₂O, brine, dried over anhydrous Na₂SO₄ and then evaporated under reduced pressure to provide a white solid (29.30 g, 152.44 mmol, 95%).

Alternatively, the title compound is prepared from *N-t-*butylisonicotinamide. *N-t-*butylisonicotinamide (9 g, 50.5 mmol) is added to a 750 mL 5-necked flask equipped with an overhead stirrer, internal thermometer and nitrogen supply. Tetrahydrofuran (225 mL) is added and the clear solution is cooled to -75 °C. *n-*Butyllithium solution 1.6 M in hexane (69 mL, 110 mmol) is added dropwise over 40 min maintaining a temperature under -70 °C. The reaction mixture is stirred at -70 °C for 1 h. Methyliodide (3.5 mL, 55 mmol) is added maintaining a temperature under -70 °C. The solution is stirred at -75 °C for 30 min then allowed to warm to rt and stirred overnight. The reaction mixture is cooled to 0 °C and a saturated aqueous solution of ammonium chloride (50 mL) is added. The reaction mixture is diluted with water (150 mL) and ethyl acetate (150 mL) and the organic layer separated. The aqueous phase is washed with ethyl acetate (150 mL). The combined organic phases are washed with brine (100 mL), dried over Na₂SO₄, filtered and concentrated *in vacuo* to give a pale yellow solid. The yellow solid is first triturated with hexane (30 mL) and then recrystallized with *tert-*butylmethyl ether (20 mL) to give a pale yellow solid (5.1 g, 53.1 %). MS (ESI) m/z 193 (M+1); ¹H-NMR (400 MHz, CDCl₃) δ ppm 8.45 (dd, *J* = 8, 4 Hz, 2 H), 7.17 (d, *J* = 8 Hz, 1H), 5.60 (br s, 1H), 2.39 (s, 3 H), 1.46 (s, 9 H).

### D. N-t-Butyl-3-ethylisonicotinamide.

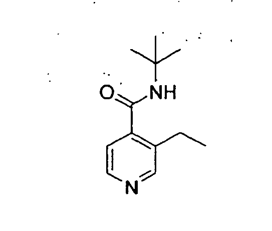

From *N-t-*butyl-3-methylisonicotinamide **Example 1C** above the title compound is prepared. *n-*Butyl lithium (1.6 M solution in hexanes, 14.96 mL, 23.94 mmol) is added with vigorous magnetic stirring to a solution of *N-tert*-butyl-3-methyl-isonicotinamide (2.19 g, 11.4 mmol) in tetrahydrofuran (30 mL) cooled in a dry ice/acetone bath to give a reddish solution with white precipitate. Additional tetrahydrofuran (10 mL) is added over 30 min to promote stirring. After an additional 75 min, methyl iodide (782 µL, 12.54 mmol) is added to the stirred reaction mixture. The red color changes to a dull yellow in this heterogeneous reaction mixture. The reaction is quenched with ammonium chloride (1.92 g, 35.91 mmol) in water (125 mL). The resulting mixture is extracted with ethylacetate (4 X 75 mL). The combined ethylacetate extracts are dried over sodium sulfate, filtered, and evaporated to give 2.76 g of yellow oil. The oil is chromatographed twice on a 120 g RediSep silica gel column with a 20% acetone/methylene chloride to 40% gradient over 20 min to give 646.3 mg (27% yield) of the title compound: ¹H NMR (400 MHz, CDCl₃) δ ppm 8.50 (s, 1H), 8.46

(d, *J* = 5.05 Hz, 1H), 7.16 (d, *J* = 4.80 Hz, 1H), 5.61 (br s, 1H), 2.80 (q, *J* = 7.58 Hz, 2 H), 1.48 (s, 9 H), 1.27 (t, *J* = 7.58 Hz, 3 H).

### E. N-t-Butyl-3-(2-(2-chloropyridin-4-yl)-2-oxoethyl)isonicotinamide.

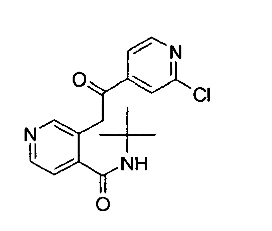

To a solution of *N-t-*butyl-3-methylisonicotinamide **Example 1C** above (10.40 g, 51.40 mmol) in THF (220 mL) is added *n-*BuLi (69.0 mL, 110 mmol, 1.6 *M* in hexanes) at -78 °C under inert atmosphere. The reaction mixture is stirred for 30 min at -78 °C, a dark red suspension is obtained, and then 2-chloroisonicotinic acid methyl ester (9.5 g, 54.8 mmol) is added drop by drop as a solution in THF (20 mL). Stirring of the clear yellow solution is continued for 2 h at -78 °C. The reaction is quenched at -78 °C through addition of 15G mL a saturated aqueous NH₄Cl solution. The pale white suspension is extracted twice with EtOAc and washed with saturated aqueous NaCl solution: MS (ESI) m/z 332.8 (M+1).

### F. N-t-Butyl-3-[2-(2-chloropyridin-4-yl)-1-methyl-2-oxoethyl]isonicotinamide.

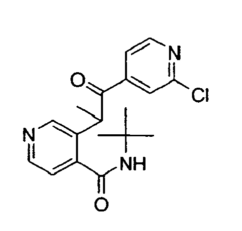

Prepared from **Example 1D** by analogy to **Example 1E**. MS (ESI) *m*/*z* 346.1 (M+1).

### G. 3-(2-Chloropyridin-4-yl)-1H-pyrano[4,3-c]pyridin-1-one.

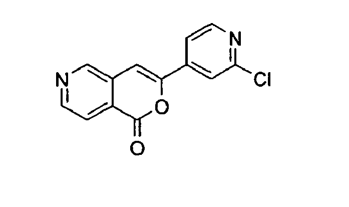

The crude *N-t-*butyl-3-(2-(2-chloropyridin-4-yl)-2-oxoethyl)isonicotinamide **Example 1E** above (6.83 g, 20.46 mmol) is suspended in acetic acid and heated at 100 °C overnight. The mixture is cooled to rt and evaporated under reduced pressure. To the residue is added H₂O and the product extracted with EtOAc. The combined organic layers are washed with brine, dried over anhydrous Na₂SO₄ and then concentrated. The solid is triturated with Et₂O to give an off-white solid (60%): MS (ESI) m/z 259.7 (M+1); ¹H NMR (400 MHz, CDCl₃) δ 9.12 (s, 1H), 8.86 (d, *J* = 5.5 Hz, 1H), 8.56 (d, *J* = 5.5 Hz, 1H), 8.18 (d, J = 5.1 Hz, 1H), 8.03 (s, 1H), 7.93 (d, *J* = 5.1 Hz, 1H), 7.77 (s, 1H).

### H. 3-(2-Chloropyridin-4-yl)-1H-4-methylpyrano[4,3-c]pyridin-1-one.

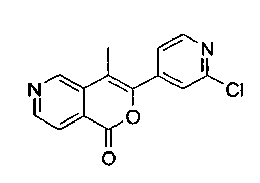

Prepared from **Example 1F** by analogy to **Example 1G.** MS (ESI) *m*/*z* 273.2 (M+1).

### I. 3-(2-Chloropyridin-4-yl)-2,6-naphthyridin-1-ol.

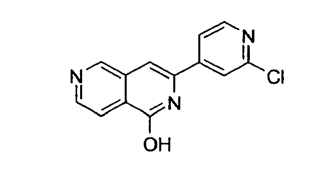

The 3-(2-chloropyridin-4-yl)-1*H*-pyrano[4,3-c]pyridin-1-one (1.66 g, 6.41 mmol) is suspended in EtOH (35 mL) and added 28.5% NH₄OH (26 mL) at rt and stirred overnight. The solvent is then evaporated and the residue is dried under reduced pressure at 45 °C for 30 min to provide a white solid. The crude product is suspended in EtOH (35 mL), added 4N HCl (8.7 ml) and stirred overnight at rt. The reaction is filtered and the solid obtained is dried under high vacuum (1.50 g, 91%): MS (ESI) *m*/*z* 257.7 (M+1); ¹H NMR (400 MHz, CDCl₃) δ 9.51 (s, 1H), 8.84 (d, J = 6.1 Hz, 1H), 8.72 (d, J = 6.1 Hz, 1H), 8.62 (d, J = 5.0 Hz, 1H), 7.95 (s, 1H), 7.80 (d, J = 5.0 Hz, 1H), 7.38 (s, 1H).

### J. 3-(2-Chloropyridin-4-yl)-4-methyl-2,6-naphthyridin-1-ol.

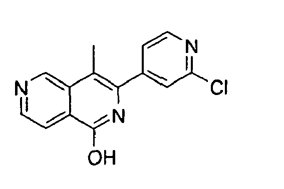

Prepared from **Example 1H** by analogy to **Example 1I**: MS (ESI) *m*/*z* 272.2 (M+1).

### K. 1-Chloro-3-(2-chloropyridin-4-yl)-2,6-naphthyridine.

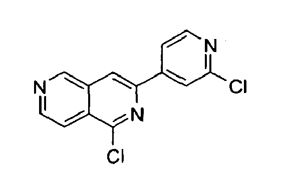

A mixture of 3-(2-chloropyridin-4-yl)-2,6-naphthyridin-1-ol (1.29 g, 5.02 mmol), POCl₃ (35 mL) and tetramethyl ammonium chloride (2.6 g, 23.72 mmol) is refluxed at 110 °C for 36 h. Then POCl₃ is removed by distillation and ice cold 10% K₂CO₃ is added carefully This mixture is then extracted with EtOAc, washed with water, brine and then dried over anhydrous Na₂SO₄ and concentrated under reduced pressure. The crude residue is washed with cold methanol (5 mL) to give a greenish brown solid (1.05 g, 3.80 mmol, 76%): MS (ESI) *m*/*z* 277.1 (M+1); ¹H NMR (400 MHz, CDCl₃) δ 9.55 (s, 1H), 8.88 (d, *J* = 6.1 Hz, 1H), 8.75 (s, 1H), 8.55 (d, *J* = 5.6 Hz, 1H), 8.27 (s, 1H), 8.25 (d, *J* = 6.1 Hz, 1H), 8.18 (d, *J* = 5.5 Hz, 1H).

### L. 1-Bromo-3-(2-chloropyridin-4-yl)-2,6-naphthyridine and 1-Bromo-3-(2-bromopyridin-4-yl)-2,6-naphthyridine.

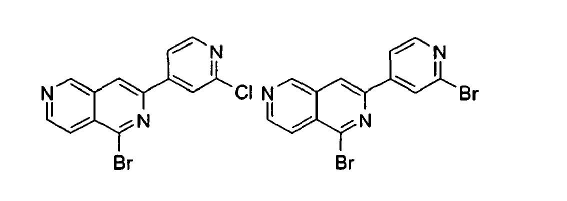

By analogy to **Example 1K** above, the product of **Example 1I** can be treated with POBr₃ to yield a mixture of the title compounds.

### M. 1-Chloro-3-(2-chtoropyri*din-4-yl)-4-methyl-[2,6]-naphthyridine.

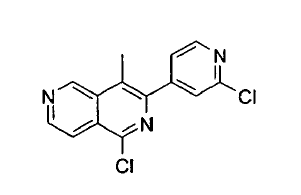

Prepared from **Example 1J** by analogy to **Example 1 K:** MS (ESI) *m*/*z* 290.2 (M+1).

### N. 3-(2-Chloropyridin-4-yl)-1-methoxy-[2,6]naphthyridine.

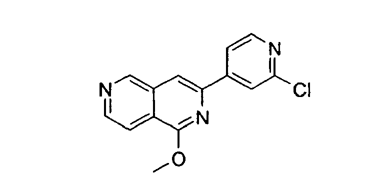

To a solution of 1-chloro-3-(2-chloropyridin-4-yl)-[2,6]naphthyridine (0.91 g, 3.3 mmol) in methanol (20 mL) is added sodium methoxide (0.27 g, 5 mmol). The mixture is heated reflux for 3 h and cooled to rt. Volatiles are removed by rotary evaporation and the residue is partitioned between water and DCM. Aqueous phase separated is extracted twice with DCM and combined organics are washed with water and brine, before it is dried (Na₂SO₄), filtered, concentrated *in vacuo,* and chromatographed on silica gel to give light yellow solid (0.80g, 89%): MS (ESI) *m*/*z* 272.1 (M+1); ¹H NMR (400 MHz, CDCl₃) δ ppm 9.3 (d, 1H), 8.75 (d, 1H), 8.5 (s, 1H), 8.1 (s, 1H), 8.0 (d, 1H), 7.95 (d, 1H), 7.9 (s, 1H), 4.28 (s, 3 H).

### Example 2

### A. 1-Methylamino-3-(2-methylaminopyridin-4-yl)-[2,6]-naphthyridine.

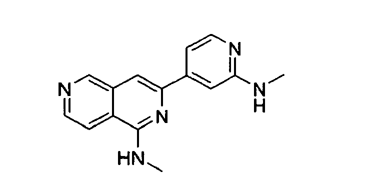

Dihalogen **Example 1 L** above is treated with a 33% solution methylamine in EtOH in an autoclave at 130 °C overnight to give the title compound: MS (ESI) *m*/*z* 266.4 (M+1).

### B. 1-n-Butylamino-3-(2-n-butylaminopyridin-4-yl)-[2,6]-naphthyridine.

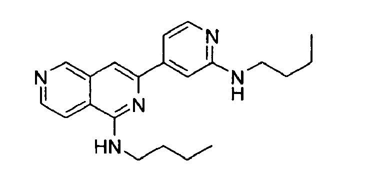

By analogy to the method desribed in **Example 2A,** dihalogen **Example 1L** above is treated with neat n-butylamine in an autoclave at 130 °C overnight to give the title compound: MS (ESI) *m*/*z* 350.5 (M+1).

### C. 1-(3-Hydroxypropylamino-3-(2-methylaminopyridin-4-yl)-[2,6]-naphthyridine.

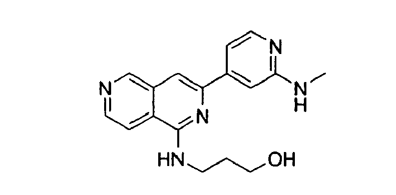

Treatment of dihalogen **Example 1L** with 3-hydroxypropylamine in THF at 45 °C for 3 h followed by treatment with methylamine in an autoclave generates the title compound: MS (ESI) *m*/*z* 310.4 (M+1).

### D. (3-Imidazol-1-yl-propyl)-[3-(2-methylamino-pyridin-4-yl)-[2,6]naphthyridin-1-yl]-amine.

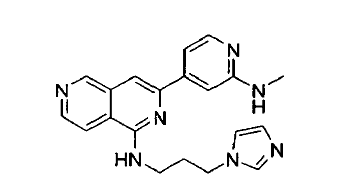

By analogy to **Example 2C,** treatment of dihalogen **Example 1 L** with 3-imidazol-1-ylpropylamine in THF at 45 °C for 3 h followed by treatment with methylamine in an autoclave generates the title compound: MS (ESI) *m*/*z* 360.5 (M+1).

### E. (3-Imidazol-1-yl-propyl)-[3-(2-n-butylamino-pyridin-4-yl)-[2,6]naphthyridin-1-yl)-amine.

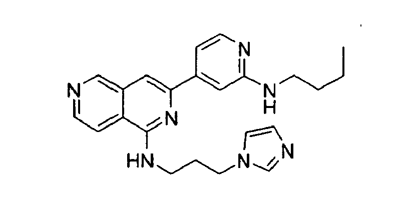

By analogy to **Example 2C,** treatment of dihalogen **Example 1L** with 3-imidazol-1-ylpropylamine in THF at 45 °C for 3 h followed by treatment of the product with ***n-*butylamine** in an autoclave generates the title compound: MS (ESI) *m*/*z* 402.5 (M+1).

### Example 3

### A. Cyclohexyl-[4-(1-methoxy-[2,6]naphthyridin-3-yl)-pyridin-2-yl]amine.

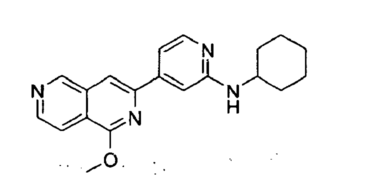

A mixture of the chloropyridine **Example 1N** (0.72 g, 2.70 mmol), cyclohexylamine (0.61 mL, 5.30 mmol), palladium(II)acetate (30 mg, 5 mol%), and BINAP (83 mg, 5 mol%) in toluene (20 mL) is degassed by N₂ bubbling. A solution of potassium *t-*butoxide (1M in THF, 6.6 mL, 6.6 mmol) is added and the mixture is heated at 100 °C overnight, then cooled to rt and quenched by aqueous NH₄Cl. The reaction mixture is extracted three times with EtOAc. The combined organics are washed with water and brine, dried (Na₂SO₄), filtered, and concentrated. The residue is chromatographed on silica gel to give the title compound (0.52 g, 59 %): MS (ESI) *m*/*z* 335.3 (M+1); ¹H NMR (400 MHz, CDCl₃) δ ppm 9.3 (s, 1H), 8.7 (d, 1H), 8.2 (d, 1H), 8.0 (d, 1H), 7.8 (s, 1H), 7.2 (dd, 1H), 7.15 (s, 1H), 4.6 (br s, 1H), 4.3 (s, 3 H), 3.7 (m, 1H), 2.2 (m, 2 H), 1.8 (m, 2 H), 1.7 (m, 1H), 1.4 (m, 2 H), 1.3 (m, 3 H).

### B. 3-(2-Cyclohexylaminopyridin-4-yl)-[2,6]naphthyridin-1-ol.

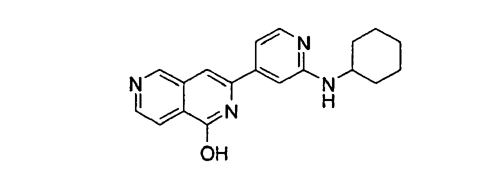

To a solution of the methoxynaphthyridine **Example 3A** (0.43 g, 1.29 mmol) in wet ***t-*butyl** alcohol (10 mL) is added potassium *t-*butoxide (1 M in THF, 6.4 mL, 6.4 mmol) and the mixture is heated at 100 °C overnight. After volatiles are removed by evaporation, the residue is suspended in water, pH is adjusted to 7, and the resulting solid is filtered and air-dried. The crude solid (406 mg, 94%) is pure in LCMS and used directly in the next step without further purification: MS (ESI) *m*/*z* 321.3 (M+1).

### C. [4-(1-Chloro-[2,6]naphthyridin-3-yl)-pyridin-2-yl]cyclohexylamine.

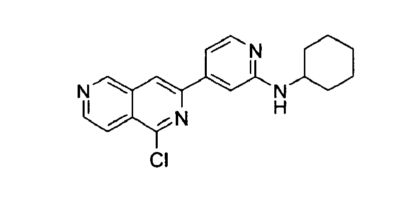

A mixture of the naphthyridinone **Example 3B** above (360 mg, 1.1 mmol) and tetramethylammonium chloride (100 mg) in POCl₃ (15 mL) is heated at 110 °C overnight. Volatiles are removed by evaporation and the residue is treated with ice, basified with 1N NaOH, and extracted with DCM (3 x 20 mL). Combined organics are dried over Na₂SO₄, filtered, and concentrated. The residue is chromatographed on silica gel to give the product (260 mg, 68%): MS (ESI) *m*/*z* 339.2 (M+1).

### D. Cyclohexyl-{4-[1-(4-methylpiperazin-1-yl)-[2,6]naphthyridin-3-yl]pyridin-2-yl}amine.

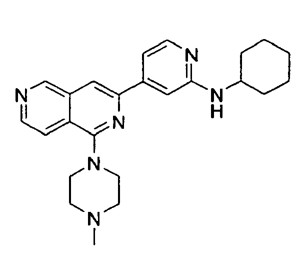

A solution of chloronaphthyridine **Example 3C** (34 mg, 0.1 mmol) and 1-methylpiperazine (0.033 mL, 0.3 mmol)) in 2-methoxyethanol (4 mL) is heated by microwave at 200 °C for 30 min. The reaction is then poured onto a solid phase extraction (SPE) cartridge containing strong cation exchanger (SCX) as the media (2 g). After washing with MeOH (10 mL), the product is eluted with 20:2:1 EtOAc-MeOH-Et₃N. The eluent is concentrated *in vacuo* and the residue is chromatographed on silica gel to give the product (40 mg, 99%): MS (ESI) *m*/*z* 403.4 (M+1); ¹H NMR (400 MHz, CDCl₃) δ ppm 9.3 (s, 1H), 8.6 (d, 1H), 8.2 (d, 1H), 7.8 (m, 2 H), 7.2 (m, 2 H), 4.8 (br s, 1H), 3.6 (br s, 5 H), 2.8 (br s, 4 H), 2.4 (s, 3 H), 2.1 (m, 2 H), 1.8 (m, 2 H), 1.7 (m, 1H), 1.5 (m, 2 H), 1.3 (m, 3 H).

The following compounds are prepared from **Example 3C** by a similar method.

### E. Cyclohexyl-{4-[1-((R)-3-ethylpiperazin-1-yl)-[2,6]naphthyridin-3-yl]-pyridin-2-yl}amine.

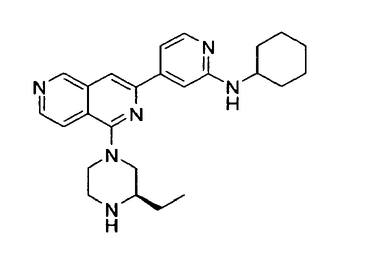

MS (ESI) *m*/*z* 417.4 (M+1); ¹H NMR (400 MHz, CDCl₃) δ ppm 9.3 (s, 1H), 8.6 (d, 1H), 8.2 (d, 1H), 7.8 (m, 2 H), 7.2 (m, 2 H), 4.8 (d, 1H), 3.9 (t, 2 H), 3.7 (br s, 1H), 3.2 (m, 2 H), 3.0 (m, 1H), 2.8 (dd, 1H), 2.1 (m, 2 H), 1.8 (m, 2 H), 1.7 (m, 1H), 1.5 (m, 2 H), 1.4 (q, 2 H), 1.3 (m, 3 H), 1.0 (t, 3 H).

### F. {4-[1-(3-Aminopiperidin-1-yl)-[2,6]naphthyridin-3-yl]-pyridin-2-yl}cyclohexylamine.

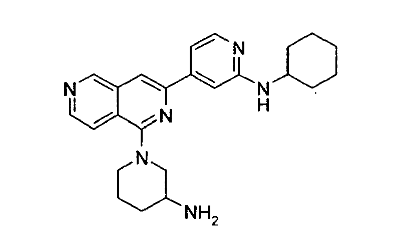

MS (ESI) *m*/*z* 403.4 (M+1); ¹H NMR (400 MHz, CDCl₃) δ ppm 9.3 (s, 1H), 8.6 (d, 1H), 8.2 (d, 1H), 7.8 (m, 2 H), 7.2 (m, 2 H), 5.0 (br s, 1H), 3.9 - 3.6 (m, 3 H), 3.2 (m, 2 H), 3.1 (dd, 1 H), 2.1 -1.2 (m, 14 H).

### G. {4-[1-((R)-3-Aminopiperidin-1-yl)-[2,6]naphthyridin-3-yl]-pyridin-2-yl}cyclohexylamine.

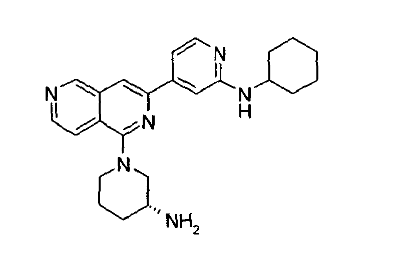

MS (ESI) *m*/*z* 403.4 (M+1); ¹H NMR (400 MHz, CDCl₃) δ ppm 9.3 (s, 1H), 8.6 (d, 1H), 8.2 (d, 1H), 7.8 (d, 1H), 7.7 (s, 1H), 7.2 (m, 2 H), 4.7 (br s, 1H), 3.9 - 3.6 (m, 3 H), 3.2 (m, 2 H), 3.0 (dd, 1H), 2.1 (m, 3 H), 1.9 (m, 2 H), 1.7 (m, 2 H), 1.5 (m, 3 H), 1.3 (m, 4 H).

### H. {4-[1-((S)-3-Aminopiperidin-1-yl)-[2,6]naphthyridin-3-yl]-pyridin-2-yl}cyclohexylamine.

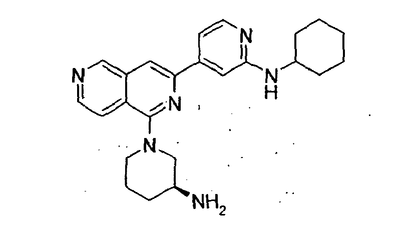

MS (ESI) m/z 403.4 (M+1); ¹H NMR (400 MHz, CDCl₃) δ ppm 9.3 (s, 1H), 8.6 (d, 1H), 8.2 (d, 1H), 7.8 (d, 1H), 7.7 (s, 1H), 7.2 (m, 2 H), 4.7 (br s, 1H), 3.9 - 3.6 (m, 3 H), 3.2 (m, 2 H), 3.0 (dd, 1H), 2.1 (m, 3 H), 1.9 (m, 2 H), 1.7 (m, 2 H), 1.5 (m, 3 H), 1.3 (m, 4 H).

### I. {4-[1-(3-Aminopyrrolidin-1-yl)-[2,6]naphthyridin-3-yl]-pyridin-2-yl}-cyclohexylamine.

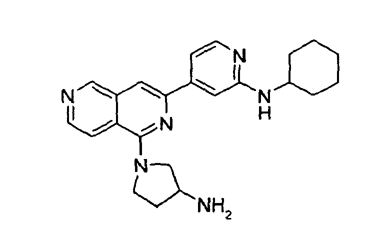

MS (ESI) *m*/*z* 389.4 (M+1); ¹H NMR (400 MHz, CDCl₃) δ ppm 9.2 (s, 1H), 8.5 (d, 1H), 8.1 (d, 1H), 7.9 (d, 1H), 7.5 (s, 1H), 7.2 (m, 2 H), 4.8 (br s, 1H), 4.2 (m, 2 H), 4.0 (m, 1H), 3.8 (m, 1H), 3.6 (m, 2 H), 2.2 -1.2 (m, 12 H).

### J. 1-[3-(2-Cyclohexylaminopyridin-4-yl)-[2,6]naphthyridin-1-yl]-piperidin-4-ol.

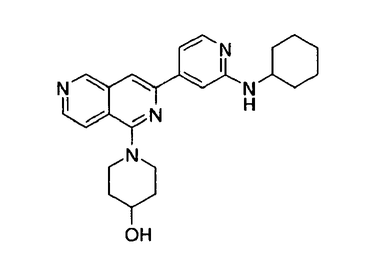

MS (ESI) *m*/*z* 404.4 (M+1); ¹H NMR (400 MHz, CDCl₃) δ ppm 9.3 (s, 1H), 8.6 (d, 1H), 8.1 (d, 1H), 7.8 (m, 2 H), 7.2 (m, 2 H), 5.6 (br s, 1H), 4.0 (m, 1H), 3.9 (m, 2 H), 3.6 (m, 1H), 3.4 (m, 2 H), 2.1-1.2(m, 14 H).

### K. {1-[3-(2-Cyclohexylaminopyridin-4-yl)-[2,6]naphthyridin-1-yl]-piperidin-4-yl}methanol.

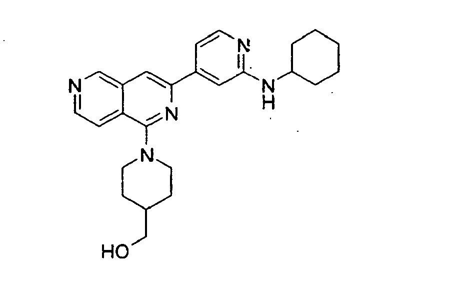

MS (ESI) *m*/*z* 418.4 (M+1).

### L. Cyclohexyl-{4-[1-((cis-3,5-dimethyl-piperazin-1-yl)-[2,6]naphthyridin-3-yl]-pyridin-2-yl}amine.

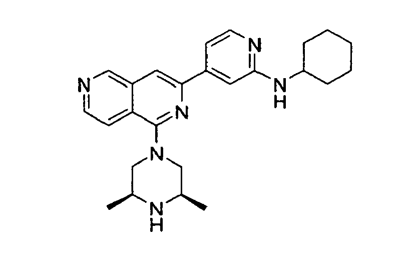

MS (ESI) *m*/*z* 417.4 (M+1); ¹H NMR (400 MHz, CDCl₃) ppm 9.3 (s, 1H), 8.6 (d, 1H), 8.2 (d, 1H), 7.8 (d, 1H), 7.7 (s, 1H), 7.2 (m, 2 H), 4.7 (br s, 1H), 3.9 (d, 2 H), 3.7 (br s, 5 H), 3.3 (br s, 2 H), 2.7 (t, 2 H), 2.1 (m, 2 H), 1.8 - 1.1 (m, 8 H), 1.2 (d, 6 H).

### M. 1-[3.(2-Cyclohexylaminopyridin-4.yl)-[2,6]naphthyridin-1-yl]piperidine-3-carboxylic acid amide.

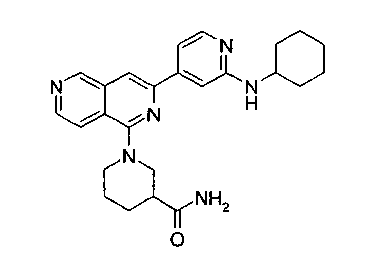

MS (ESI) *m*/*z* 431.5 (M+1).

### N. [3-(2-Cyclohexylaminopyridin-4-yl)-[2,6]naphthyridin-1-yl]pyrrolidin-3-ylamine.

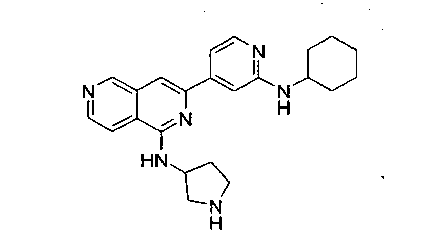

MS (ESI) *m*/*z* 389.4 (M+1); ¹H NMR (400 MHz, CDCl₃) δ ppm 9.2 (s, 1H), 8.6 (d, 1H), 8.2 (d, 1H), 7.8 (d, 1H), 7.5 (s, 1H), 7.2 (d, 1H), 7.15 (s, 1H), 6.3 (d, 1H), 5.0 (br s, 1H), 4.7 (d, 1H), 3.7 (br s, 1H), 3.4 (m, 2 H), 3.2 (m, 1H), 2.4 (m, 2 H), 2.2 (m, 1H), 1.8 (m, 2 H), 1.7 (m, 1H), 1.5 (m, 2 H), 1.3 (m, 3 H).

### O. [3-(2-Cyclohexylaminopyridin-4-yl)-[2,6]naphthyridin-1-yl]piperidin-3-ylamine.

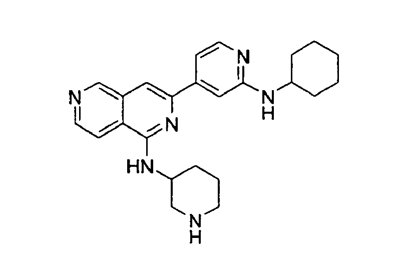

MS (ESI) *m*/*z* 403.4 (M+1); ¹H NMR (400 MHz, CDCl₃) δ ppm 9.2 (s, 1H), 8.6 (d, 1H), 8.2 (d, 1H), 7.8 (d, 1H), 7.5 (s, 1H), 7.2 (s, 1H), 7.1 (d, 1H), 6.2 (br s, 1H), 5.0 (br s, 1H), 4.7 (br s, 1H), 3.7 (br s, 1H), 3.4 (m, 1H), 3.0 (m, 2 H), 2.2 - 1.2 (m, 14 H).

### Example 4

### A. 1-[3-(2-Chloropyridin-4-yl)-[2,6]naphthyridin-1-yl]piperidine-4-carboxylic acid amide.

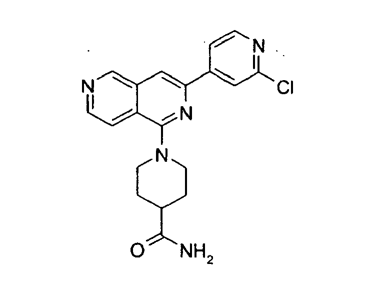

To a suspension of 1-chloro-3-(2-chloropyridin-4-yl)-2,6-naphthyridine **Example 1K** (200 mg, 0.72 mmol) in anhydrous ethanol (2.4 mL) in a sealable vial is added triethylamine (0.32 mL, 2.3 mmol) followed by isonipecotamide (120 mg, 0.90 mmol). The vial is flushed with nitrogen and then sealed. The reaction is heated in a 100 °C oil bath for 16 h. The heterogeneous mixture is cooled to rt, then filtered. The filtrate is washed with ethanol and dried under high vacuum to give the title compound as a yellowish brown solid (230 mg, 87%): MS (ESI) m/z 368.1 (M+1); ¹H NMR (400 MHz, DMSO-*d₆*) δ ppm 9.36 (s, 1H), 8.68 (d, *J* = 5.8 Hz, 1H), 8.55 (d, *J* = 5.8 Hz, 1H), 8.38 (s, 1H), 8.23 (s, 1H), 8.18 (dd, *J* = 5.3, 1.5 Hz, 1H), 7.89 (d, *J* = 5.8 Hz, 1H), 7.34 (br s, 1H), 6.82 (br s, 1H), 4.05 (d, *J* = 12.6 Hz, 2 H), 3.09 (m, 2 H), 2.43 (m, 1H), 1.91 (br m, 4 H).

### B. 1-[3-(2-Chloropyridin-4-yl)-[2,6]naphthyridin-1-yl]piperidine-4-carboxylic acid methylamide.

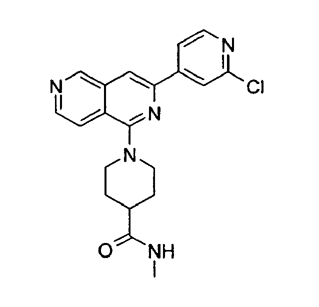

The title compound is prepared from **Example 1 K** and ***N-*methylisonipecotamide:** MS (ESI) *m*/*z* 382.1 (M+1).

### C. 1-{4-[3-(2-Chloropyridin-4-yl)-[2,6]naphthyridin-1-yl]-piperazin-1-yl}ethanone.

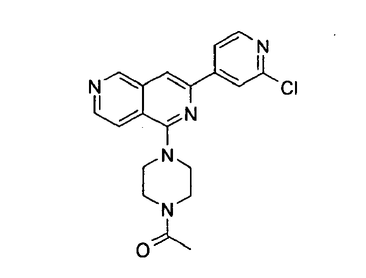

The title compound is prepared from **Example 1 K** and *N-*acetylpiperazine: MS (ESI) *m*/*z* 368.1 (M+1).

### D. 8-[3-(2-Chloropyridin-4-yl)-[2,6]naphthyridin-1-yl]-2,8-diaza-spiro[4.5]decan-1-one.

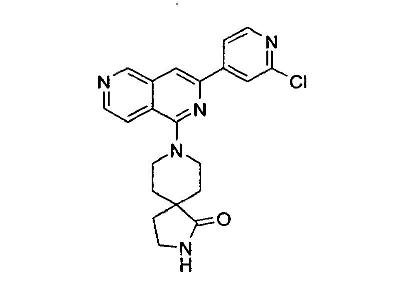

The title compound is prepared from **Example 1K** and 2,8-diaza-spiro[4.5]decan-1-one: MS (ESI) m/z 394.0 (M+1); ¹H NMR (400 MHz, CDCl₃) δ ppm 9.29 (s, 1H), 8.67 (d, *J* = 5.8 Hz, 1H), 8.49 (d, *J* = 5.1 Hz, 1H), 8.08 (s, 1H), 7.93 (dd, *J* = 5.3, 1.5 Hz, 1H), 7.85 - 7.80 (m, 2 H), 5.54 (br. s., 1H), 4.08 - 3.99 (m, 2 H), 3.44 (t, *J* = 6.8 Hz, 2 H), 3.34 - 3.23 (m, 2 H), 2.36 - 2.26 (m, 2 H), 2.22 (t, *J* = 6.8 Hz, 2 H), 1.72 (d, *J* = 13.4 Hz, 2 H).

### E. 4-[3-(2-Chloropyridin-4-yl)-[2,6]naphthyridin-1-yl]piperazin-2-one.

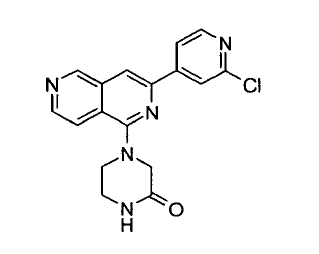

The title compound is prepared from **Example 1K** and 2,8-diaza-spiro[4.5]decan-1-one: and piperazin-2-one: MS (ESI) m/z 340.1 (M+1).

### F. 4-[3-(2-Chloropyridin-4-yl)-[2,6]naphthyridin-1-yl]piperazine-1-carboxylic acid methylamide.

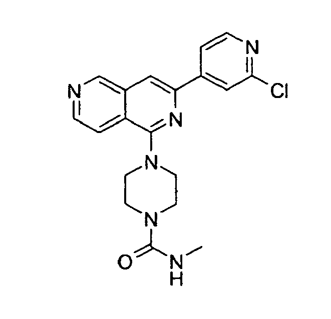

Piperazine-1-carboxylic acid methyl amide used in this synthesis is prepared as described in the following reference: Zhao, Matthew; Yin, Jingjun; Huffman, Mark A.; McNamara, James M.; Tetrahedron (2006), 62(6), 1110-1115. The title compound is prepared from **Example 1K** and piperazine-1-carboxylic acid methyl amide by analogy to the method outlined in **Example 4A:** MS (ESI) *m*/*z* 382.9 (M+1).

### G. 2-[3-(2-Chloropyridin-4-yl)-[2,6]naphthyridin-1-ylamino]ethanol.

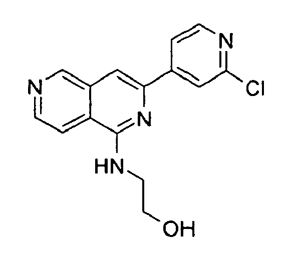

The title compound is prepared from **Example 1 K** and ethanolamine: MS (ESI) m/z 301.1 (M+1).

### H. 3-(2-Chloropyridin-4-yl)-1-(4-ethoxypiperidin-1-yl)-[2,6]-naphthyridine.

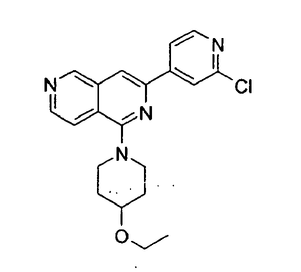

The title compound is prepared from **Example 1K** and 4-ethoxypiperadine: MS (ESI) m/z 369.2 (M+1).

### I. N'-[3-(2-Chloropyridin-4-yl)-[2,6]naphthyridin-1-yl]-N,N-dimethylethane-1,2-diamine.

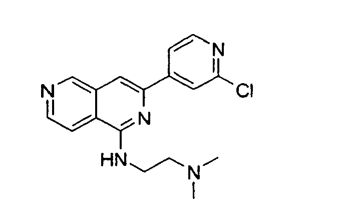

The title compound is prepared from **Example 1K** and *N*,*N-*dimethylethane-1,2-diamine by analogy to the method outlined in **Example 4A:** MS (ESI) *m*/*z* 328.2 (M+1).

### J. {1-[3-(2-Chloropyridin-4-yl)-[2,6]naphthyridin-1-yl]-piperidin-4-ylmethyl}-dimethylamine.

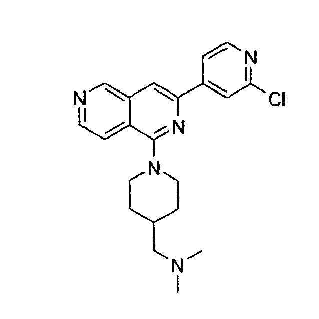

The title compound is prepared from **Example 1K** and dimethylpiperidin-4-ylmethylamine: MS (ESI) m/z 482.1 (M+1).

### K. 3-(2-Chloropyridin-4-yl)-l-((S)-3-methylpiperazin-1-yl)-[2,6]naphthyridine.

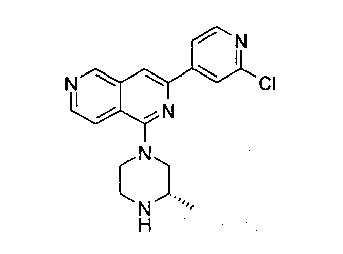

The title compound is prepared from **Example 1K** and *(S)*-2-methylpiperazine by analogy to the method outlined in **Example 4A:** MS (ESI) *m*/*z* 340.1 (M+1).

### L. 3-(2-Chloropyridin-4-yl)-1-(4-cyclopropylmethylpiperazin-1-yl)-[2,6]naphthyridine.

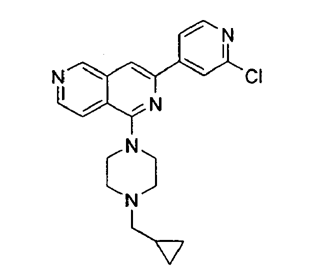

The title compound is prepared from **Example** 1**K** and cyclopropylmethylpiperazine by analogy to the method outlined in **Example 4A:** MS (ESI) m/z 380.3 (M+1).

### M. 3-(2-Chloropyridin-4-yl)-1-(4-cyclopropylpiperazin-1-yl)-[2,6]naphthyridine.

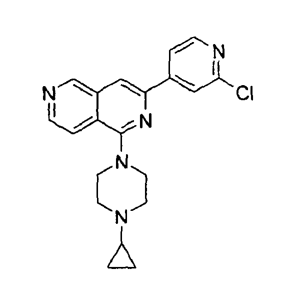

The title compound is prepared from **Example 1K** and cyclopropylpiperazine by analogy to the method outlined in **Example 4A:** MS (ESI) *m*/*z* 366.3 (M+1).

### N. [3-(2-Chloropyridin-4-yl)-[2,6]naphthyridin-1-yl]-(3-imidazol-1-ylpropy)amine.

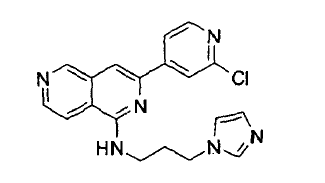

The title compound is prepared from **Example 1K** and 3-imidazol-1-ylpropylamine by analogy to the method outlined in **Example 4A:** MS (ESI) *m*/*z* 365.2 (M+1).

### O. 4-[3-(-Chloropyridin-4-yl)-[2,6]naphthyridin-1-yl]-piperazine-1-carboxylic acid tert-butyl ester.

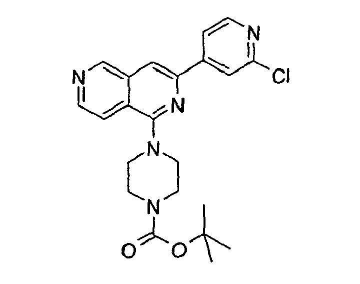

To a suspension of 1-chloro-3-(2-chloropyridin-4-yl)-[2,6]naphthyridine (2.00 g, 7.20 mmol) in anhydrous ethanol (24 mL) in a dried pressure vessel is added triethylamine (3.20 mL, 23 mmol) followed by piperazine-1-carboxylic acid *t-*butyl ester (1.70 g, 9.10 mmol). The vessel is flushed with nitrogen and then sealed. The suspension is heated in a 100 °C oil bath for 20 h. The dark brown, nearly homogeneous reaction mixture is cooled to room temperature, then concentrated *in vacuo.* The residue is diluted with dichloromethane and water. The layers are agitated and separated, and the aqueous layer is extracted twice with DCM. The combined organic layers are dried over sodium sulfate, filtered and concentrated. The material is purified by silica gel chromatography (120 g SiO₂, gradient 0 → 2.5% methanol/dichloromethane) to afford a brownish yellow solid, which is refluxed in 60 mL of diethyl ether. The mixture is cooled to room temperature and filtered to provide the title compound as a white solid (2.2 g, 71 %). MS (ESI) *m*/*z* 426.2 (M+1); ¹H NMR (400 MHz, DMSO-*d*₆) δ 9.39 (d, *J* = 0.76 Hz, 1H), 8.70 (d, *J* = 5.8 Hz, 1H), 8.55 (dd, *J* = 5.3, 0.51 Hz, 1H), 8.24 (dd, *J* = 1.5, 0.76 Hz, 1H), 8.18 (dd, *J* = 5.3, 1.5 Hz, 1H), 7.96 (d, *J* = 5.8 Hz, 1 H), 3.64 (br m, 4 H), 3.52 (m, 4 H), 1.45 (s, 9 H).

### P.(R)-3-[3-(2-Chloropyridin-4-yl)-[2,6]naphthyridin-1-ylamino]pyrrolidine-1-carboxylicacid tert-butyl ester.

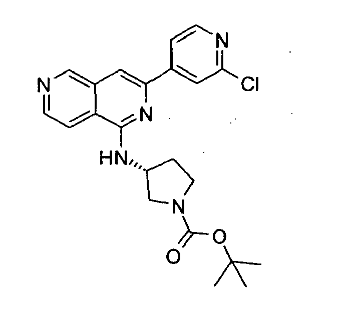

The title compound is prepared from **Example 1K** and *(R)*-3-aminopyrrolidine-1-carboxylic acid *tert*-butyl ester by analogy to the method outlined in **Example 40:** MS (ESI) *m*/*z* 426.3 (M+1).

### Q.(S)-3-[3-(2-Chloropyridin-4-yl)-[2,6]naphthyridin-1-ylamino]pyrrolidine-1-carboxylicacid tert-butyl ester.

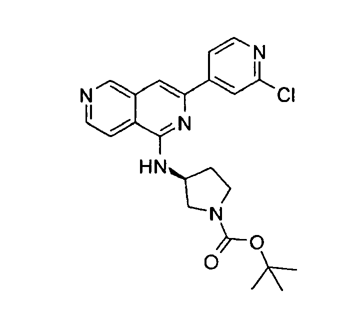

The title compound is prepared from **Example 1K** and *(S)*-3-aminopyrrolidine-1-carboxylic acid *tert*-butyl ester by analogy to the method outlined in **Example 40:** MS (ESI) *m*/*z* 426.2 (M+1).

### R. {1-[3-(2-Chloropyridin-4-yl)-[2,6]naphthyridin-1-yl]-piperidin-4-ylmethyl}carbamic acid tert-butyl ester.

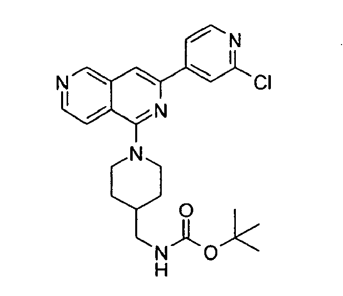

The title compound is prepared from **Example 1K** and piperidin-4-ylmethyl-carbamic acid *tert*-butyl ester by analogy to the method outlined in **Example 40:** MS (ESI) *m*/*z* 454.3 (M+1).

### S. 1-[3-(2-Chloropyridin-4-yl)-[2,6]naphthyridin-1-yl]piperidine-4-carboxylic acid methyl ester.

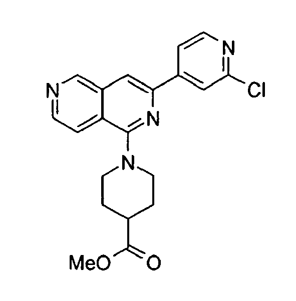

A solution of 1-chloro-3-(2-chloropyridin-4-yl)-[2,6]naphthyridine **Example 1K** (1.0 g, 3.62 mmol), Et₃N (1.50 mL, 10.9 mmol), methyl isonipecotate (0.74 mL, 5.43 mmol) and DMSO (4 mL) is heated to 80 °C for 3 h. At that time the solution is allowed to cool to rt and is slurried with 15 mL of water. The mixture is then poured into 200 mL of ice water. After 10 min the solid is collected via filtration. The solid is then dried under vacuum to give (1.25 g) 1-[3-(2-chloropyridin-4-yl)-[2,6]naphthyridin-1-yl]piperidine-4-carboxylic acid methyl ester as a brown solid: MS (ESI) m/z 383.1 (M+1); ¹H NMR (400 MHz, CDCl₃) δ ppm 9.29 (s, 1H), 8.67 (d, ***J*** = 5.8 Hz, 1H), 8.49 (d, *J* = 4.8 Hz, 1H), 8.07 (s, 1H), 7.95 - 7.91 (m, 1H), 7.83 (s, 1H), 7.79 (d, *J* = 5.8 Hz, 1H), 4.07 - 3.96 (m, 2 H), 3.76 (s, 3 H), 3.27 - 3.14 (m, 2 H), 2.73 - 2.61 (m, 1H), 2.23 - 2.01 (m, 4 H).

### Example 5

### A. 1-[3-(2-Chloropyridin-4-yl)-[2,6]naphthyridin-1-yl]piperidine-4-carboxylic acid isopropylamide.

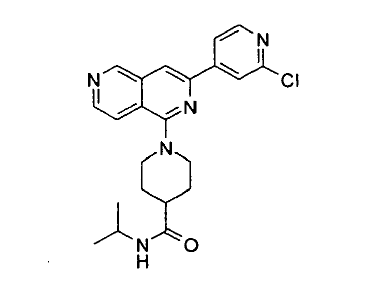

A solution of 1-[3-(2-chloropyridin-4-yl)-[2,6]naphthyridin-1-yl]-piperidine-4-carboxylic acid methyl ester **Example 4S** (2.65 g, 6.93 mmol), THF (30 mL), and H₂O (10 mL) is added LiOH·H₂O (1.45 g, 34.6 mmol). After 20 min, an additional 30 mL of THF is added. After 5 h, the reaction is complete as judged by LCMS. At that point, 1 M HCl in Et₂O (35 mL) is added. The mixture is stirred for 10 min and then concentrated *in vacuo.* The residue is then azeotroped with toluene (3 X 150 mL) to give 1-[3-(2-chloropyridin-4-yl)-[2,6]naphthyridin-1-yl]piperidine-4-carboxylic acid. The crude 1-[3-(2-chloropyridin-4-yl)-[2,6]naphthyridin-1-yl]piperidine-4-carboxylic acid is taken up in DMF (60 mL) and DIEA (5.75 mL, 34.60 mmol), *i*PrNH₂ (2.9 mL, 34.6 mmol), PyBOP (10.80 g, 20.80 mmol), and HOBt (0.94 g, 6.93 mmol) are added in sequence. The mixture is stirred at rt for 24 h at which point it is concentrated *in vacuo.* The residue is then taken up in DCM (500 mL) and H₂O (500 mL). The layers are mixed and then separated. The aqueous layer is extracted further with DCM (2 X 500 mL) and each organic layer is washed with and aliquot of H₂O (500 mL). The combined organic layers are then dried over Na₂SO₄, filtered and concentrated. The residue is stirred with hot EtOAc (50 mL) and then filtered. The filtrate is then washed several times with cold EtOAc to give 1-[3-(2-chloropyridin-4-yl)-[2,6]naphthyridin-1-yl]piperidine-4-carboxylic acid isopropylamide as a white solid: MS (ESI) *m*/*z* 410.2 (M+1); ¹H NMR (400 MHz, DMSO-*d₆*) δ ppm 9.36 (s, 1H), 8.68 (d, *J* = 5.8 Hz, 1 H), 8.56 (d, *J* = 5.3 Hz, 1H), 8.38 (s, 1H), 8.23 (s, 1H), 8.20 - 8.15 (m, 1H), 7.90 (d, *J* = 5.8 Hz, 1H), 7.71 (d, *J* = 7.6 Hz, 1H), 4.06 (d, *J* = 13.1 Hz, 2 H), 3.94 - 3.79 (m, 1H), 3.13 - 3.02 (m, 2 H), 2.47 - 2.34 (m, 1H), 2.00 - 1.81 (m, 4 H), 1.07 (d, *J* = 6.6 Hz, 6 H).

The following compounds can be prepared by a similar method.

### B. 1-[3-(2-Chloropyridin-4-yl)-[2,6]naphthyridin-1-yl]piperidine-4-carboxylic acid ethylamide.

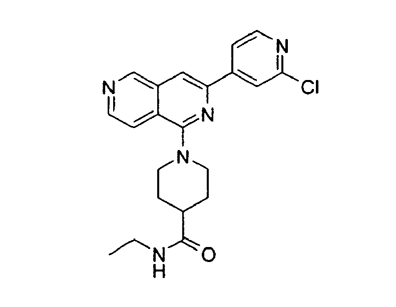

The title compound is prepared from ester **Example 4S** by analogy to the method outlined in **Example 5A:** MS (ESI) *m*/*z* 396.0 (M+1); ¹H NMR (400 MHz, CDCl₃) δ ppm 9.35 (s, 1H), 8.65 (d, *J*=5.8 Hz, 1H), 8.49 (d, *J* = 5.8 Hz, 1H), 8.29 - 8.26 (m, 1H), 8.21 (s, 1H), 8.20 - 8.15 (m, 1H), 8.00 (d, *J* = 5.8 Hz, 1H), 4.22 - 4.12 (m, 2 H), 3.30 - 3.22 (m, 2 H), 3.22 - 3.11 (m, 2 H), 2.59 - 2.45 (m, 1H), 2.18 - 2.04 (m, 2 H), 2.02 - 1.93 (m, 2 H), 1.17 (t, *J* = 7.3 Hz, 3 H).

### C. 1-[3-(2-Chloropyridin-4-yl)-[2,6]naphthyridin-1-yl]piperidine-4-carboxylic acid isobutylamide.

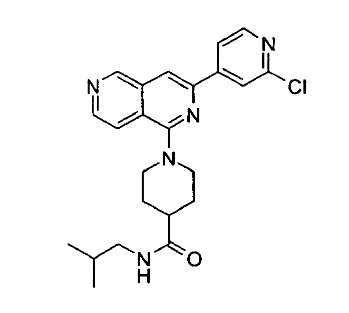

The title compound is prepared from ester **Example 4S** by analogy to the method outlined in **Example 5A:** MS (ESI) *m*/*z* 424.3 (M+1); ¹H NMR (400 MHz, CDCl₃) δ ppm 9.31 (s, 1H), 8.66 (d, *J* = 5.8 Hz, 1H), 8.50 (d, *J* = 5.3 Hz, 1H), 8.07 (s, 1H), 7.92 (dd, *J* = 5.3, 1.5 Hz, 1 H), 7.85 (d, *J* = 6.1 Hz, 1H), 7.84 (s, 1H), 5.63 - 5.48 (m, 1H), 4.19 - 4.04 (m, 2 H), 3.22 - 3.08 (m, 4 H), 2.48 - 2.34 (m, 1H), 2.24 - 1.99 (m, 4 H), 1.90 - 1.75 (m, 1H), 0.95 (d, *J* = 6.8 Hz, 6 H).

### D. 1-[3-(2-Chloropyridin-4-yl)-[2,6]naphthyridin-1-yl]piperidine-4-carboxylic acid cyclopropylamide.

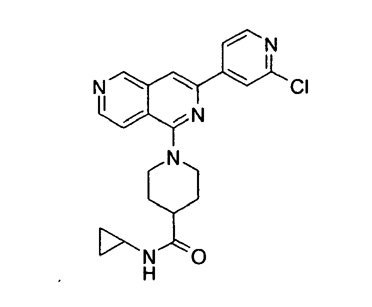

The title compound is prepared from ester **Example 4S** by analogy to the method outlined in **Example 5A:** MS (ESI) *m*/*z* 408.0 (M+1); ¹H NMR (400 MHz, CDCl₃) δ ppm 9.29 (s, 1H), 8.67 (d, *J* = 5.8 Hz, 1H), 8.49 (d, *J* = 5.3 Hz, 1H), 8.07 (s, 1H), 7.96 - 7.88 (m, 1H), 7.83 (s, 1H), 7.80 (d, *J* =5.8 Hz, 1H), 5.65 (br s, 1H), 4.19 - 4.03 (m, 2 H), 3.18 - 3.04 (m, 2 H), 2.83 - 2.72 (m, 1 H), 2.42 - 2.29 (m, 1 H), 2.18 - 1.97 (m, 4 H), 0.87 - 0.77 (m, 2 H), 0.56 - 0.47 (m, 2 H).

### E. 1-[3-(2-Chloropyridin-4-yl)-[2,6]naphthyridin-1-yl]piperidine-4-carboxylic acid diethylamide.

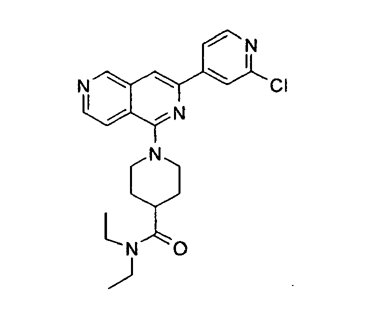

The title compound is prepared from ester **Example 4S** by analogy to the method outlined in **Example 5A:** MS (ESI) *m*/*z* 424.0 (M+1); ¹H NMR (400 MHz, CDCl₃) δ ppm 8.67 (d, *J* = 5.8 Hz, 1 H), 8.49 (d, *J* = 5.3 Hz, 1 H), 8.11 - 8.07 (m, 1 H), 7.93 (dd, *J* = 5.3, 1.5 Hz, 1 H), 7.85 - 7.81 (m, 1 H), 7.82 (s, 1H), 4.16 - 4.06 (m, 2 H), 3.80 - 3.65 (m, 1 H), 3.48 - 3.37 (m, . 4 H), 3.23 - 3.09 (m, 3 H), 2.81 - 2.70 (m, 1 H), 2.30 - 2.16 (m, 2 H), 1.96 - 1.87 (m, 2 H), 1.26 (t, *J*=7.2Hz, 2H), 1.16 (t, *J*=7.1 Hz, 3 H).

### F. {1-[3-(2-Chloropyridin-4-yl)-[2,6]naphthyridin-1-yl]-piperidin-4-yl}pyrrolidin-1-ylmethanone.

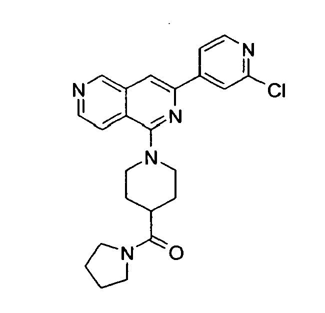

The title compound is prepared from ester **Example 4S** by analogy to the method outlined in **Example 5A:** MS (ESI) *m*/*z* 442.0 (M+1); ¹H NMR (400 MHz, CDCl₃) δ ppm 9.29 (s, 1 H), 8.67 (d, *J* = 5.6 Hz, 1 H), 8.49 (d, *J* = 5.8 Hz, 1 H), 8.09 (s, 1 H), 7.92 (dd, *J* = 5.3, 1.5 Hz, 1 H), 7.84 - 7.81 (m, 2 H), 4.17 - 4.08 (m, 2 H), 3.61 - 3.49 (m, 4 H), 3.19 - 3.08 (m, 2 H), 2.74 - 2.61 (m, 1 H), 2.28 - 2.14 (m, 2 H), 2.08 - 1.85 (m, 6 H).

### G. 1-[3-(2-Chloropyridin-4-yl)-[2,6]naphthyridin-1-yl]piperidine-4-carboxylic acid (2-methoxyethyl)amide.

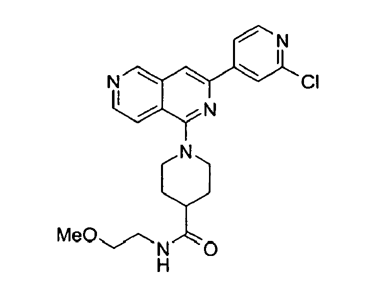

The title compound is prepared from ester **Example 4S** by analogy to the method outlined in **Example 5A:** MS (ESI) *m*/*z* 426.0 (M+1); ¹H NMR (400 MHz, CDCl₃) δ ppm 9.29 (s, 1 H), 8.67 (d, *J* = 5.8 Hz, 1 H), 8.49 (d, *J* = 5.1 Hz, 1 H), 8.08 (s, 1 H), 7.93 (d, *J* = 5.1 Hz, 1 H). 7.83 (s, 1 H), 7.80 (d, *J* = 5.8 Hz, 1 H), 5.95 (br s, 1 H), 4.17 - 4.04 (m, 2 H), 3.51 (br s, 4 H), 3.39 (s, 3 H), 3.13 (t, *J* = 13.1 Hz, 2 H), 2.51 - 2.36 (m, 1 H), 2.20 - 2.02 (m, 4 H).

### H. 1-[3-(2-Chloropyridin-4-yl)-[2,6]naphthyridin-1-yl]piperidine-4-carboxylic acid (2-tert-butoxyethyl)amide.

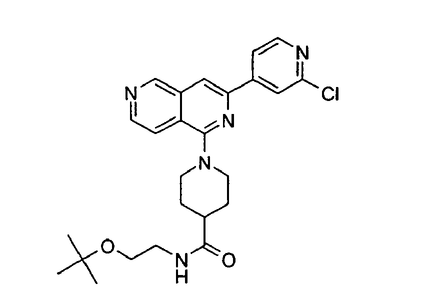

The title compound is prepared from ester **Example 45** by analogy to the method outlined in **Example 5A:** MS (ESI) *m*/*z* 468.3 (M+1); ¹H NMR (400 MHz, CDCl₃) δ ppm 9.29 (s, 1 H), 8.67 (d, *J* = 5.8 Hz, 1 H), 8.49 (d, *J* = 4.8 Hz, 1 H), 8.08 (d, *J* = 0.8 Hz, 1 H), 7.93 (dd, *J* = 5.3, 1.5 Hz, 1 H), 7.83 (s, 1 H), 7.80 (d, *J* = 5.8 Hz, 1 H), 6.00 (br s, 1 H), 4.16 - 4.07 (m, 2 H), 3.51 - 3.44 (m, 4 H), 3.20 - 3.10 (m, 2 H), 2.50 - 2.35 (m, 1 H), 2.19 - 2.03 (m, 4 H), 1.21 (s, 9 H).

### I. (3'R)-1-[3-(2-Chloropyridin-4-yl)-[2,6]naphthyridin-1-yl]-piperidine-4-carboxylic acid (tetrahydrofuran-3-yl)amide.

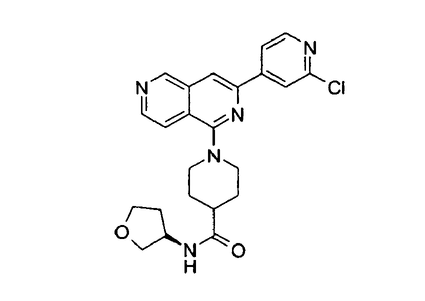

The title compound is prepared from ester **Example 4S** by analogy to the method outlined in **Example 5A:** MS (ESI) *m*/*z* 438.0 (M+1); ¹H NMR (400 MHz, CDCl₃) δ ppm 9.29 (s, 1 H), 8.67 (d, *J* = 5.8 Hz, 1 H), 8.49 (d, *J* = 5.1 Hz, 1 H), 8.07 (s, 1 H), 7.92 (dd, *J* = 5.3, 1.5 Hz, 1 H), 7.83 (s, 1 H), 7.80 (d, *J* = 5.8 Hz, 1 H), 5.76 - 5.68 (m, 1 H), 4.65 - 4.48 (m, 1 H), 4.15 - 4.05 (m, 2 H), 4.03 - 3.92 (m, 1 H), 3.88 - 3.76 (m, 2 H), 3.71 (dd, *J* = 9.3, 2.3 Hz, 1 H), 3.13 (d, *J* = 27.0 Hz, 2 H), 2.45 - 2.24 (m, 2 H), 2.18 - 2.00 (m, 4 H), 1.82 (d, *J* = 29.6 Hz, 1 H).

### J. 1-[3-(2-Chloropyridin-4-yl)-[2,6]naphthyridin-1-yl]-piperidine-4-carboxylic acid (2-aminoethyl)amide.

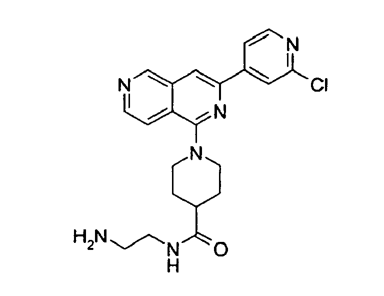

[2-({1-[3-(2-Chloropyridin-4-yl)-[2,6]naphthyridin-1-yl]-piperidine-4-carbonyl}-amino)ethyl]-carbamic acid *tert*-butyl ester is prepared from ester **Example 4S** and (2-aminoethyl)carbamic acid *tert*-butyl ester by analogy to the method outlined in **Example 5A:** MS (ESI) *m*/*z* 511.1 (M+1).

To a solution of [2-({1-[3-(2-chloropyridin-4-yl)-[2,6]naphthyridin-1-yl]-piperidine-4-carbonyl}-amino)ethyl]-carbamic acid *tert*-butyl ester (0.088 g, 0.082 mmol) in CH₂Cl₂ (1.5 mL) is added TFA (1.5 mL). After the reaction is complete, as judged by LCMS, the solution is concentrated. The residue is then separated via semi-prep HPLC to give 1-[3-(2-chloropyridin-4-yl)-[2,6]naphthyridin-1-yl]-piperidine-4-carboxylic acid (2-aminoethyl)amide: MS (ESI) *m*/*z* 411.0 (M+1); ¹H NMR (400 MHz, MeOD) (TFA salt) δ ppm 9.37 (s, 1 H), 8.64 (d, *J* = 5.8 Hz, 1 H), 8.47 (d, *J* = 5.3 Hz, 1 H), 8.27 (s, 1 H), 8.22 (s, 1 H), 8.16 (dd, *J* = 5.3, 1.5Hz, 1 H), 8.01 (d, *J*=6.1 Hz, 1 H), 4.17(d, *J*=12.6 Hz, 2H), 3.49 (t, *J*=6.2 Hz, 2H), 3.26 - 3.14 (m, 2 H), 3.08 (t, *J* = 6.1 Hz, 2 H), 2.66 - 2.47 (m, 1 H), 2.17 - 1.97 (m, 4 H).

### K. 1-[3-(2-Chloropyridin-4-yl)-[2,6]naphthyridin-1-yl]piperidine-4-carboxylic acid (2-dimethylaminoethyl)amide.

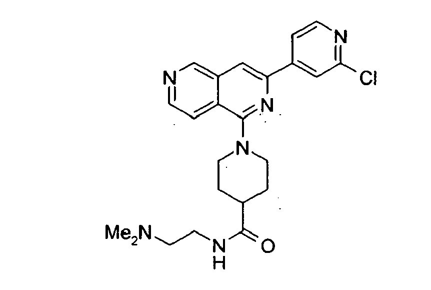

The title compound is prepared from ester **Example 4S** by analogy to the method outlined in **Example 5A:** MS (ESI) *m*/*z* 439.0 (M+1); ¹H NMR (400 MHz, MeOD) δ ppm 9.34 (s, 1 H), 8.63 (d, *J* = 5.8 Hz, 1 H), 8.47 (d, *J* = 5.3 Hz, 1 H), 8.26 (s, 1 H), 8.21 (s, 1 H), 8.16 (dd, *J* = 5.3, 1.5 Hz, 1 H), 7.98 (d, *J* = 5.8 Hz, 1 H), 4.17 (d, *J* = 13.1 Hz, 1 H), 3.59 (app t, *J* = 6.1 Hz, 2 H), 3.30 -3.27 (m, 1 H), 3.25 - 3.11 (m, 3 H), 2.97 (s, 6 H), 2.65 - 2.51 (m, 1 H), 2.15 - 1.98 (m, 4 H), 1.89 - 1.81 (m, 1 H).

### L. 1-[3-(2-Chloro-pyridin-4-yl)-[2,6]naphthyridin-1-yl]-piperidine-4-carboxylic acid (2-pyrrolidin-1-yl-ethyl)-amide.

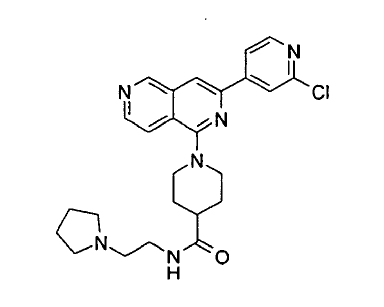

The title compound is prepared from ester **Example 4S** by analogy to the method outlined in **Example 5A:** MS (ESI) ***m*/*z* 465.2** (M+1).

### M. 1-[3-(2-Chloro-pyridin-4-yl)-[2,6]naphthyridin-1-yl]-piperidine-4-carboxylic acid methyl-(2-pyrrolidin-1-yl-ethyl)-amide.

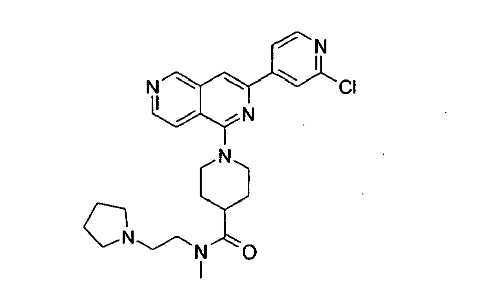

The title compound is prepared from ester **Example 4S** by analogy to the method outlined in **Example 5A:** MS (ESI) *m*/*z* 479.3 (M+1).

### N. 1-[3-(2-Chloro-pyridin-4-yl)-[2,6]naphthyridin-1-yl]-piperidine-4-carboxylic acid (2-morpholin-4-yl-ethyl)-amide.

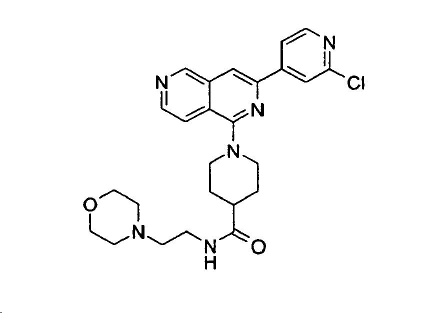

The title compound is prepared from ester **Example 4S** by analogy to the method outlined in **Example 5A:** MS (ESI) *m*/*z* 481.2 (M+1).

### O. 1-[3-(2-Chloro-pyridin-4-yl)-[2,6]naphthyridin-1-yl]-piperidine-4-carboxylic acid (2-methyl-2-piperidin-1-yl-propyl)-amide.

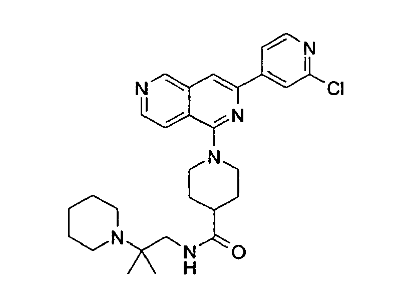

The title compound is prepared from ester **Example 4S** by analogy to the method outlined in **Example 5A:** MS (ESI) *m*/*z* 507.3 (M+1).

### P. {1-[3-(2-Chloro-pyridin-4-yl)-[2,6]naphthyridin-1-yl]-piperidin-4-yl}-(4-pyrrolidin-1-yl-piperidin-1-yl)-methanone.

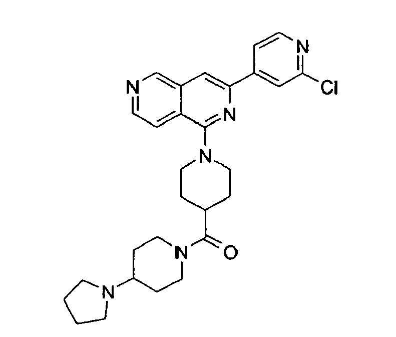

The title compound is prepared from ester **Example 4S** by analogy to the method outlined in **Example 5A:** MS (ESI) *m*/*z* 505.3 (M+1).

### Q. 1-[3-(2-Chloro-pyridin-4-yl)-[2,6]naphthyridin-1-yl]-piperidine-4-carboxylic acid (3-pyrrolidin-1-yl-propyl)-amide.

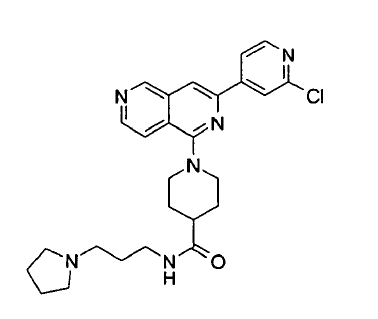

The title compound is prepared from ester **Example 4S** by analogy to the method outlined in **Example 5A:** MS (ESI) *m*/*z* 479.2 (M+1).

### R. 1-[3-(2-Chloropyridin-4-yl)-[2,6]naphthyridin-1-yl]-piperidine-4-carboxylic acid (3-imidazol-1-ylpropyl)amide.

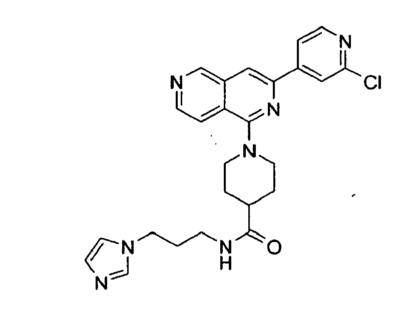

The title compound is prepared from ester **Example 4S** by analogy to the method outlined in **Example 5A:** MS (ESI) *m*/*z* 476.1 (M+1); ¹H NMR (400 MHz, MeOD) δ ppm 9.34 (s, 1 H), 8.63 (d, *J* = 5.8 Hz, 1 H), 8.57 (s, 1 H), 8.47 (d, *J* = 5.3 Hz, 1 H), 8.26 (s, 1 H), 8.20 (s, 1 H), 8.16 (dd, *J* = 5.3, 1.5 Hz, 1 H), 7.98 (d, *J* = 5.8 Hz, 1 H), 7.54 (s, 1 H), 7.40 (s, 1 H), 4.23 (t, *J* = 6.8 Hz, 2 H), 4.20 - 4.09 (m, 2 H), 3.26 (t, *J* = 6.7 Hz, 2 H), 3.22 - 3.11 (m, 2 H), 2.60 - 2.49 (m, 1 H), 2.16 - 2.03 (m, 4 H), 2.03 - 1.95 (m, 2 H).

### S. 1-[3-(2-Chloropyridin-4-yl)-[2,6]naphthyridin-1-yl]piperidine-4-carboxylic acid (2-fluoroethyl)amide.

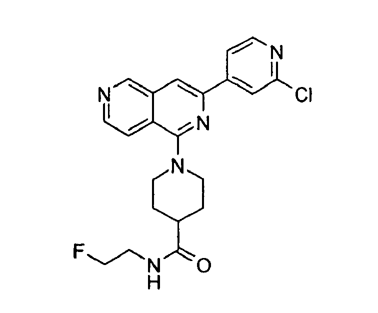

The title compound is prepared from ester **Example 4S** by analogy to the method outlined in **Example 5A:** MS (ESI) *m*/*z*414.0 (M+1); ¹H NMR (400 MHz, CDCl₃) δ ppm 9.30 (s, 1 H), 8.67 (d, *J* = 5.8 Hz, 1 H), 8.50 (d, *J* = 5.8 Hz, 1 H), 8.09 - 8.05 (m, 1 H), 7.93 (dd, *J* = 5.1, 1.5 Hz, 1 H), 7.83 (s, 1 H), 7.80 (d, *J* = 5.8 Hz, 1 H), 5.97 - 5.88 (m, 1 H), 4.60 (t, *J* = 4.7 Hz, 1 H), 4.49 (t, *J* = 4.7 Hz, 1 H), 4.12 (d, *J* = 13.6 Hz, 2 H), 3.72 - 3.65 (m, 1 H), 3.64 - 3.58 (m, 1 H), 3.20 - 3.09 (m, 2 H), 2.51 - 2.39 (m, 1 H), 2.12 (d, *J* = 51.8 Hz, 4 H).

### T. 1-[3-(2-Chloropyridin-4-yl)-[2,6]naphthyridin-1-yl]piperidine-4-carboxylic acid (2,2-difluoroethyl)amide.

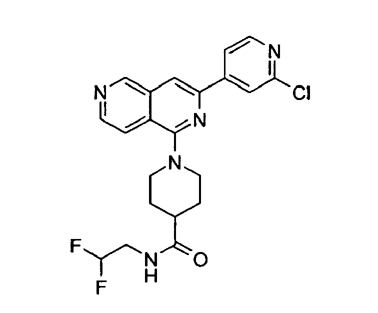

The title compound is prepared from ester **Example 4S** by analogy to the method outlined in **Example 5A:** MS (ESI) *m*/*z* 432.0 (M+1); ¹H NMR (400 MHz, CDCl₃) δ ppm 9.30 (s, 1 H), 8.67 (d, *J* = 5.6 Hz, 1 H), 8.50 (d, *J* = 5.3 Hz, 1 H), 8.07 (s, 1 H), 7.92 (dd, *J* = 5.3, 1.5 Hz, 1 H), 7.84 (s, 1 H), 7.80 (d, *J* = 5.8 Hz, 1 H), 6.07 - 5.74 (m, 1 H), 5.81 - 5.79 (m, 1 H), 4.11 (d, *J* = 13.1 Hz, 2 H), 3.79 - 3.64 (m, 2 H), 3.20 - 3.08 (m, 2 H), 2.55 - 2.39 (m, 1 H), 2.22 - 2.03 (m, 4 H).

### U. 1-[3-(2-Chloropyridin-4-yl)-[2,6]naphthyridin-1-yl]piperidine-4-carboxylic acid (2,2,2-trifluoroethyl)amide.

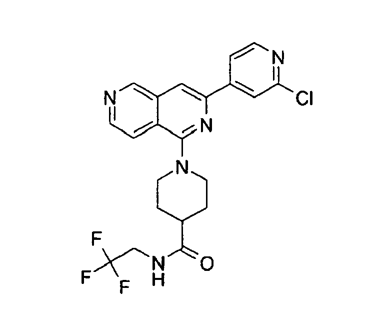

The title compound is prepared from ester **Example 4S** by analogy to the method outlined in **Example 5A:** MS (ESI) ***m*/*z*** 449.9 (M+1); ¹H NMR (400 MHz, CDCl₃) δ ppm 9.30 (s, 1 H), 8.68 (d, *J* = 5.8 Hz, 1 H), 8.50 (d, *J* = 5.8 Hz, 1 H), 8.09 - 8.04 (m, 1 H), 7.92 (dd, *J* = 5.2, 1.5 Hz, 1 H), 7.84 (s, 1 H), 7.79 (d, *J* = 5.8 Hz, 1 H), 5.84 - 5.77 (m, 1 H), 4.17 - 4.07 (m, 2 H), 4.05 - 3.94 (m, 2 H), 3.20 - 3.10 (m, 2 H), 2.56 - 2.44 (m, 1 H), 2.22 - 2.04 (m, 4 H).

### V. (S)-3-({1-[3-(2-Chloro-pyridin-4-yl)-[2,6]naphthyridin-1-yl]-piperidine-4-carbonyl}-amino)-pyrrolidine-1-carboxylic acid tert-butyl ester.

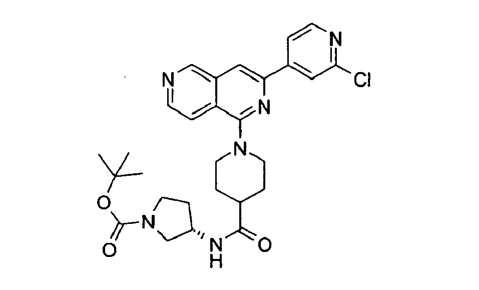

The title compound is prepared from ester **Example 4S** by analogy to the method outlined in **Example 5A:** MS (ESI) *m*/*z* 537.2 (M+1).

### W. (R)-3-({1-[3-(2-Chloro-pyridin-4-yl)-[2,6]naphthyridin-1-yl]-piperidine-4-carbonyl}-amino)-pyrrolidine-1-carboxylic acid tert-butyl ester.

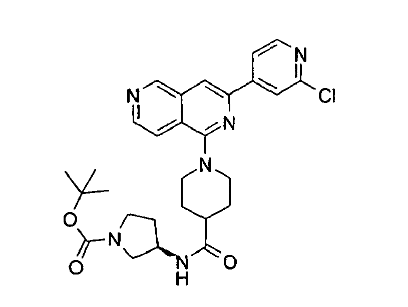

The title compound is prepared from ester **Example 4S** by analogy to the method outlined in **Example 5A:** MS (ESI) *m*/*z* 537.3 (M+1).

### X. 4-({1-[3-(2-Chloro-pyridin-4-yl)-[2,6]naphthyridin-1-yl]-piperidine-4-carbonyl}-amino)-piperidine-1-carboxylic acid tert-butyl ester.

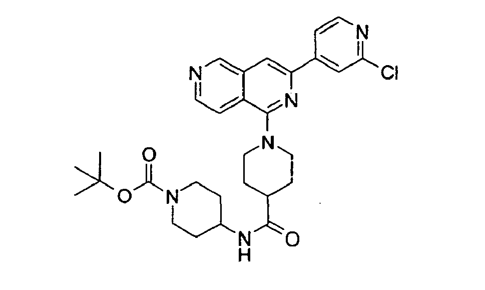

The title compound is prepared from ester **Example 4S** by analogy to the method outlined in **Example 5A:** MS (ESI) *m*/*z* 551.3 (M+1).

### Y. ((S)-1-{1-[3-(2-Chloro-pyridin-4-yl)-[2,6]naphthyridin-1-yl]-piperidine-4-carbonyl}-piperidin-3-yl)-carbamic acid tert-butyl ester.

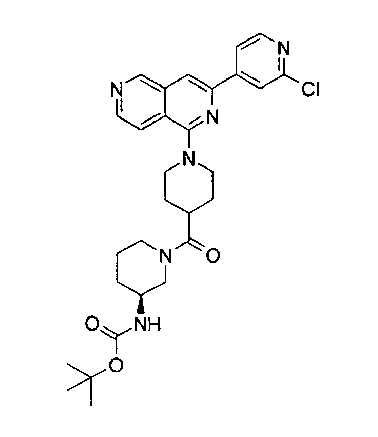

The title compound is prepared from ester **Example 4S** by analogy to the method outlined in **Example 5A:** MS (ESI) *m*/*z* 551.3 (M+1).

### Example 6

### 1-[3-(2-Chloro-pyridin-4-yl)-[2,6]naphthyridin-1-yl]-azetidine-3-carboxylic acid isopropylamide.

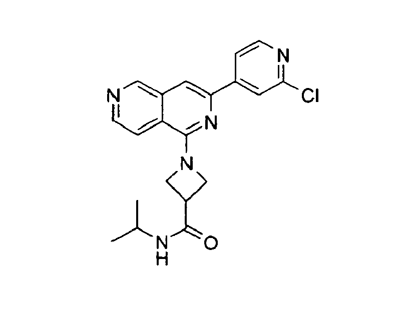

A solution of 1-chloro-3-(2-chloropyridin-4-yl)-[2,6]naphthyridine, **Example 1K,** (0.250 g, 0.900 mmol), azetidine-3-carboxylic acid hydrochloride salt (0.138 g, 0.990 mmol), Et₃N (0.4 mL, 2.70 mmol) and DMSO (3 mL) are heated at 80 °C for 2 h. After cooling to room temperature the contents of the flask are diluted with water (50 mL) and the precipitate is then collected via filtration. The solid residue is washed with water (50 mL) and then dried to give the crude acid 1-[3-(2-chloropyridin-4-yl)-[2,6]naphthyridin-1-yl]-azetidine-3-carboxylic acid. The resulting carboxylic acid is then coupled to isopropyl amine by analogy to the method outlined in **Example 5A** to afford the title compound: MS (ESI) *m*/*z* 382.1 (M+1).

### Example 7

### A. 1-[3-(2-Chloropyridin-4-yl)-[2,6]naphthyridin-1-yl]-piperidine-4-carboxylic acid (2,2-dimethyl-propyl)-amide.

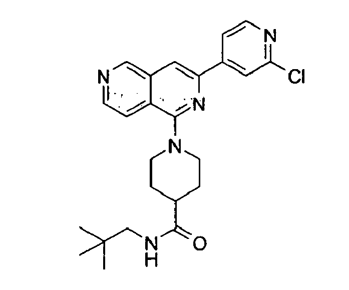

A solution of AlMe₃ (1.30 mL, 2.0 M heptane) is added under an argon atmosphere to a flask containing toluene (10 mL). After 5 min 2,2-dimethyl-propylamine (0.23 mL, 2.62 mmol) is added. After an additional 5 min ester, **Example 4S,** (0.500 g, 1.31 mmol) is added in portions. The contents of the flask are then heated to 110 °C. After 2 h the flask is allowed to cool to room temperature. The contents are then slurried with methanol (100 mL) for 5 min and filtered. Following concentration of the filtrate the resulting solid is triturated with Et₂O/heptane (1:1) to give the title compound 1-[3-(2-chloropyridin-4-yl)-[2,6]naphthyridin-1-yl]-piperidine-4-carboxylic acid (2,2-dimethyl-propyl)amide: MS (ESI) *m*/*z* 438.2 (M+1).

Similarly prepared are:

### B. 1-[3-(2-Chloropyridin-4-yl)-[2,6]naphthyridin-1-yl]-piperidine-4-carboxylic acid cyclohexylamide.

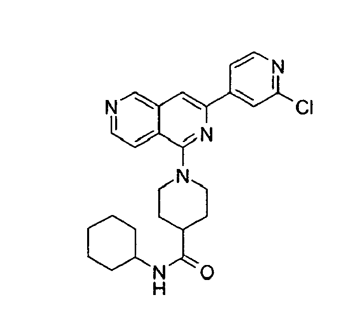

The title compound is prepared from ester **Example 4S:** MS (ESI) *m*/*z* 450.3 (M+1).

### C. 1-[3-(2-Chloro-pyridin-4-yl)-[2,6]naphthyridin-1-yl]-piperidine-4-carboxylic acid phenylamide.

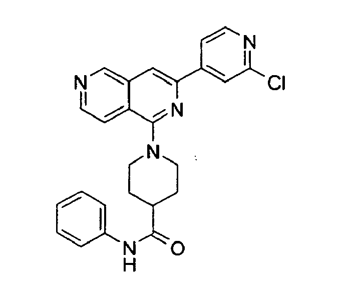

The title compound is prepared from ester **Example 4S:** MS (ESI) *m*/*z* 444.1 (M+1).

### D. 1-[3-(2-Chloro-pyridin-4-yl)-[2,6]naphthyridin-1-yl]-piperidine-4-carboxylic acid (tetrahydro-pyran-4-yl)-amide.

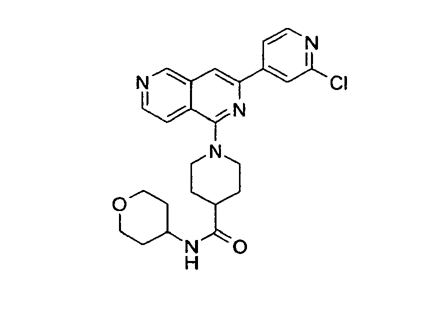

The title compound is prepared from ester **Example 4S:** MS (ESI) *m*/*z* 452.3 (M+1).

### E. {1-[3-(2-Chloro-pyridin-4-yl)-[2,6]naphthyridin-1-yl]-piperidin-4-yl}-morpholin-4-yl-methanone.

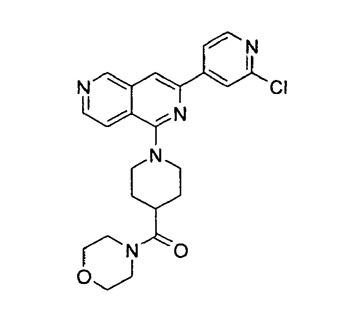

The title compound is prepared from ester **Example 4S:** MS (ESI) *m*/*z* 438.2 (M+1).

### F. {1-[3-(2-Chloro-pyridin-4-yl)-[2,6]naphthyridin-1-yl]-piperidin-4-yl}-(4-hydroxy-piperidin-1-yl)-methanone.

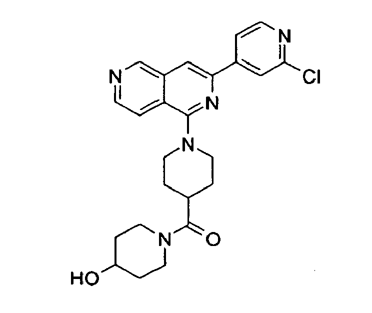

The title compound is prepared from ester **Example 4S:** MS (ESI) *m*/*z* 452.1 (M+1).

### G. 1-[3-(2-Chloro-pyridin-4-yl)-[2,6]naphthyridin-1-yl]-piperidine-4-carboxylic acid (2-methyl-2-pyrrolidin-1-yl-propyl)-amide.

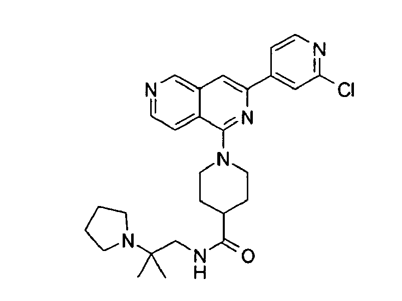

The title compound is prepared from ester **Example 4S:** MS (ESI) *m*/*z* 493.3 (M+1).

### H. 4-{1-[3-(2-Chloro-pyridin-4-yl)-[2,6]naphthyridin-1-yl]-piperidine-4-carbonyl}-piperazine-1-carboxylic acid tert-butyl ester.

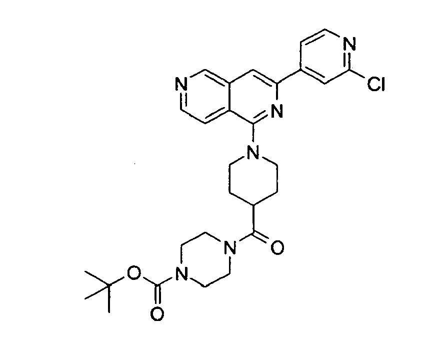

The title compound is prepared from ester **Example 4S:** MS (ESI) *m*/*z* 537.3 (M+1).

### Example 8

### A. 1-{3-[2-(1-Methyl-1H-pyrazol-3-ylamino)pyridin-4-yl]-[2,6]naphthyridin-1-yl}piperidine-4-carboxylic acid amide.

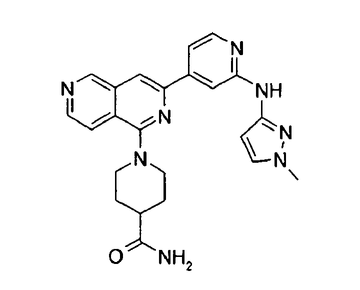

1-[3-(2-Chloropyridin-4-yl)-[2,6]naphthyridin-1-yl]-piperidine-4-carboxylic acid amide **Example 4A** (200 mg, 0.54 mmol), 1-methyl-1*H-*pyrazol-3-ylamine (110 mg, 1.10 mmol), and cesium carbonate (1.1 g, 3.3 mmol) are dissolved in anhydrous *N-*methylpyrrolidinone (8.00 mL) in a dried pressure vessel under argon. The mixture is sparged with argon for 5 min, then palladium(O) tris(tri-*t-*butylphosphine) (28 mg, 0.05 mmol) is added. The vessel is flushed with argon and sealed, and then heated in a 120 °C oil bath for 5 h. The resulting dark red solution is cooled to rt, then diluted with MeOH and filtered. The filtrate is acidified with several drops of TFA, then purified by preparative reverse-phase HPLC (X-Bridge C₁₈ column, flow rate = 30 mL/min, gradient 10%→80% acetonitrile/5 mM aqueous trifluoroacetic acid over 30 min). The isolated TFA salt of the product is dissolved in water and basified with 28% aqueous ammonium hydroxide. The aqueous layer is extracted three times with dichloromethane. The combined organic layers are washed with brine, dried over sodium sulfate, filtered, and concentrated to give the free base. Further purification by preparative reverse-phase HPLC (X-Bridge C₁₈ column, flow rate = 40 mL/min, gradient 10% → 80% acetonitrile/5 mM aqueous ammonium hydroxide over 20 min) afforded the title compound as a white solid (40 mg, 17%): MS (ESI) *m*/*z* 429.4 (M+1); ¹H NMR (400 MHz, DMSO-*d*₆) δ ppm 9.38 (d, *J* = 0.76 Hz, 1 H), 9.31 (br s, 1 H), 8.64 (d, *J* = 5.8 Hz, 1 H), 8.24 (s, 1 H), 8.22 (d, *J* = 5.3 Hz, 1 H), 8.1 (s, 1 H), 7.87 (d, *J* = 5.8 Hz, 1 H), 7.53 (d, *J* = 2.3 Hz, 1 H), 7.42 (dd, *J* = 5.4, 1.6 Hz, 1 H), 7.34 (br s, 1 H), 6.83 (br s, 1 H), 6.30 (d, *J* = 2.0 Hz, 1 H), 4.07 (br d, *J* = 13.4 Hz, 2 H), 3.77 (s, 3 H), 3.10 (m, 2 H), 2.43 (m, 1 H), 1.92 (br m, 4 H). The following compound are prepared by a similar method.

### B. 1-[3-(2-Cyclohexylaminopyridin-4-yl)-[2,6]naphthyridin-1-yl]piperidine-4-carboxylic acid amide.

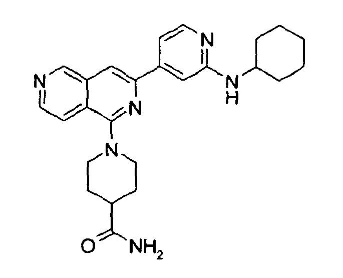

The title compound is prepared from 1-[3-(2-chloropyridin-4-yl)-[2,6]naphthyridin-1-yl]-piperidine-4-carboxylic acid amide **Example 4A:** MS (ESI) *m*/*z* 431.2 (M+1); ¹H NMR (400 MHz, CD₃OD) δ ppm 9.22 (d, 1 H), 8.56 (d, 1 H), 7.98 (d, 1 H), 7.90 (br m, 2 H), 7.31 (d, 1 H), 7.18 (d, 1 H), 4.01 (br d, 2 H), 3.61 (m, 1 H), 3.08 (dd, 2 H), 2.58 (m, 1 H), 2.01-1.92 (br m, 6 H), 1.71 (br d, 2 H), 1.60 (d, 1H), 1.37 (m, 2 H), 1.19 (m, 3 H).

### C. 1-{3-[2-(trans-4-Hydroxy-cyclohexylamino)-pyridin-4-yl]-[2,6]naphthyridin-1-yl}-piperidine-4-carboxylic acid amide.

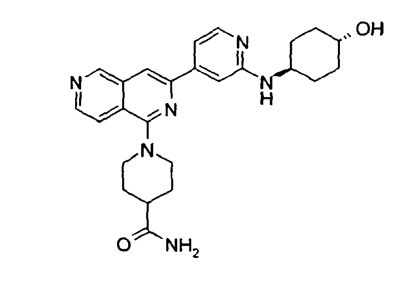

The title compound is prepared from 1-[3-(2-chloropyridin-4-yl)-[2,6]naphthyridin-1-yl]-piperidine-4-carboxylic acid amide **Example 4A:** MS (ESI) *m*/*z* 447.4 (M+1); ¹H NMR (400 MHz, DMSO-*d*₆) δ 9.36 (s, 1 H), 8.62 (d, *J* = 5.8 Hz, 1 H), 8.06 (d, *J* = 5.3 Hz, 1 H), 8.03 (s, 1 H), 7.85 (d, *J* = 5.8 Hz, 1 H), 7.34 (br s, 1 H), 7.28 (s, 1 H), 7.15 (dd, *J* = 5.6, 1.5 Hz, 1 H), 6.82 (br s, 1 H), 6.49 (d, *J* = 7.6 Hz, 1 H), 4.53 (d, *J* = 4.3 Hz, 1 H), 4.01 (br d, *J* = 12 Hz, 2 H), 3.68 (br s, 1 H), 3.44 (br s, 1 H), 3.06 (m, 2 H), 2.42 (m, 1 H), 1.98 - 1.84 (m, 8 H), 1.33 - 1.18(m,4H).

### D. 1-{3-[2-(Tetrahydropyran-4-ylamino)-pyridin-4-yl]-[2,6]naphthyridin-1-yl}-piperidine-4-carboxylic acid amide.

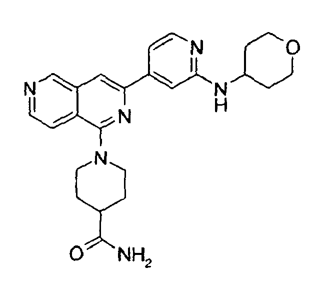

The title compound is prepared from 1-[3-(2-chloropyridin-4-yl)-[2,6]naphthyridin-1-yl]-piperidine-4-carboxylic acid amide **Example 4A:** MS (ESI) ***m*/*z*** 433.3 (M+1); ¹H NMR (400 MHz, DMSO-*d*₆) δ 9.36 (s, 1 H), 8.63 (d, *J* = 6.1 Hz, 1 H), 8.07 (m, 2 H), 7.86 (d, *J* = 5.6 Hz, 1 H), 7.34 (s, 2 H), 7.19 (d, *J* = 4.0 Hz, 1 H), 6.83 (s, 1 H), 6.70 (br s, 1 H), 4.00 (m, 3 H), 3.89 (br dt, *J* = 8.0, 3.2 Hz, 2 H), 3.43 (td, *J* = 11, 2.0 Hz, 2 H), 3.40 (m, 1 H), 3.06 (m, 2 H), 2.42 (m, 1 H), 1.91 (br m, 5 H), 1.47 (m, 2 H).

### E. 1-[3-(2-Cyclohexylaminopyridin-4-yl)-[2,6]naphthyridin-1-yl]piperidine-4-carboxylic acid methylamide.

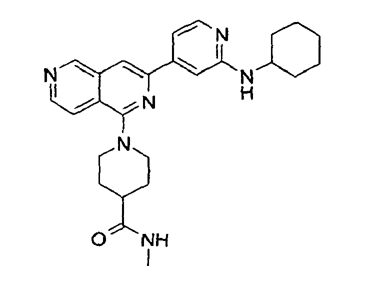

The title compound is prepared from **Example 4B:** MS (ESI) *m*/*z* 445.4 (M+1); ¹H NMR (400 MHz, CD₃OD) δ ppm 9.28 (s, 1 H), 8.57 (d, 1 H), 8.00 (m, 2 H), 7.99 (d, 1 H), 7.38 (s, 1 H), 7.26 (d, 1 H), 4.13 (br d, 2 H), 3.68 (m, 1 H), 3.14 (dd, 2 H), 2.77 (s, 3 H), 2.50 (m, 1 H), 2.07 (br m, 4 H), 1.95 (m, 2 H), 1.80, (m, 2 H), 1.69 (br d, 1 H), 1.46 (m, 2 H), 1.29 (m, 3 H).

### F. 1-{4-[3-(2-Cyclohexylaminopyridin-4-yl)-[2,6]naphthyridin-1-yl]piperazin-1-yl}ethanone.

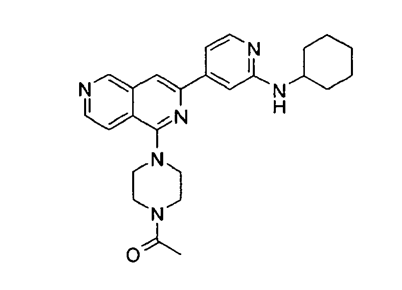

The title compound is prepared from **Example 4C:** MS (ESI) *m*/*z* 431.4 (M+1); ¹H NMR (400 MHz, CD₃OD) δ ppm 9.31 (d, 1 H), 8.60 (d, 1 H), 8.04 (s, 1 H), 8.00 (m, 2 H), 7.37 (d, 1 H), 7.22 (d, 1 H), 3.88 (br d, 4 H), 3.70 (m, 1 H), 3.61 (br d, 4 H), 2.19 (s, 3 H), 2.08 (br m, 2 H), 1.80 (m, 2 H), 1.69 (br d, 1 H), 1.47 (m, 2 H), 1.28 (m, 3 H).

### G. 8-{3-[2-(1-Methyl-1H-pyrazol-3-ylamino)pyridin-4-yl]-[2,6]naphthyridin-1-yl}-2,8-diaza-spiro[4.5]decan-1-one.

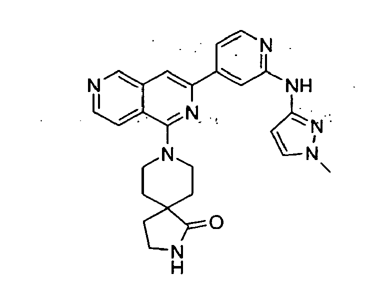

The title compound is prepared from **Example 4D:** MS (ESI) *m*/*z* 455.1 (M+1); ¹H NMR (400 MHz, CDCl₃) δ ppm 9.28 (s, 1 H), 8.62 (d, *J*=5.7 Hz, 1 H), 8.30 (d, *J* = 5.3 Hz, 1 H), 7.99 (s, 1 H), 7.84 - 7.80 (m, 1 H), 7.81 (s, 1 H), 7.46 (dd, *J* = 5.3, 1.5 Hz, 1 H), 7.29 (d, *J* = 2.3 Hz, 1 H), 6.88 (s, 1 H), 6.29 (d, *J* = 2.3 Hz, 1 H), 5.47 (s, 1 H), 4.09-3.97 (m, 2 H), 3.86 (s, 3 H), 3.49 (s, 1 H), 3.42 (t, *J* = 6.7 Hz, 2 H), 3.33 - 3.22 (m, 1 H), 2.36 - 2.26 (m, 2 H), 2.21 (t, *J* = 6.9 Hz, 2 H), 1.70 (d, *J* = 13.4 Hz, 2 H).

### H. 4-[3-(2-Cyclohexylaminopyridin-4-yl)-[2,6]naphthyridin-1-yl]piperazin-2-one.

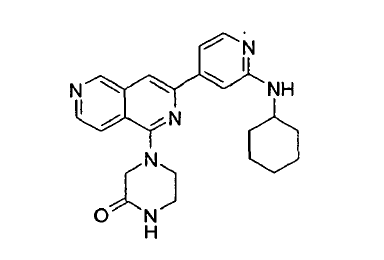

The title compound is prepared from **Example 4E:** MS (ESI) ***m*/*z* 403.3** (M+1).

### I. 4-{3-[2-(3-Methoxyphenylamino)pyridin-4-yl]-[2,6]naphthyridin-1-yl}piperazin-2-one.

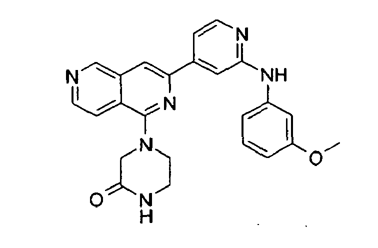

The title compound is prepared from **Example 4E:** MS (ESI) *m*/*z* 427.3 (M+1); ¹H NMR (400 MHz, CD₃OD) δ 9.29 (s, 1 H), 8.58 (d, 1 H), 8.13 (d, 1 H), 8.07 (d, 1 H), 7.95 (s, 1 H), 7.67 (d, 1 H), 7.39 (s, 1 H), 7.20 (d, 1 H), 7.11 (s, 1 H), 6.99 (t, 1 H), 6.44 (d, 1 H), 4.24 (s, 2H), 3.82 (m, 2H), 3.72 (s, 3H), 3.37 (m, 2H).

### J. 4-{3-[2-(Tetrahydropyran-4-ylamino)pyridin-4-yl]-[2,6]naphthyridin-1-yl}piperazine-1-carboxylic acid methylamide.

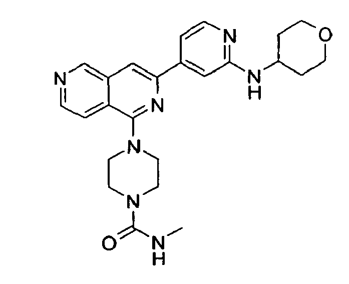

The title compound is prepared from **Example 4F:** HRMS (ESI) *m*/*z* 448.2462 (M+1); ¹H NMR (400 MHz, CDCl₃) δ ppm 9.30 (s, 1 H), 8.65 (d, *J* = 5.56 Hz, 1 H), 8.20 (d, *J* = 5.31 Hz, 1 H), 7.93 - 7.67 (m, 2 H), 7.23 (d, *J* = 5.31 Hz, 1 H), 7.18 (s, 1 H), 4.76 - 4.45 (m, 2 H), 4.03

(d, *J* = 11.37 Hz, 2 H), 3.76 - 3.64 (m, 3 H), 3.64 - 3.53 (m, 5 H), 2.88 (d, *J* = 4.29 Hz, 2 H), 2.12 (d, *J* = 12.13 Hz, 2 H), 1.77 (br s, 3 H), 1.68 - 1.49 (m, 2 H), 1.39 (d, *J* = 12.38 Hz, 1 H).

### K. 4-{3-[2-(Cyclohexylamino)pyridin-4-yl]-[2,6]naphthyridin-1-yl}piperazine-1-carboxylic acid methylamide.

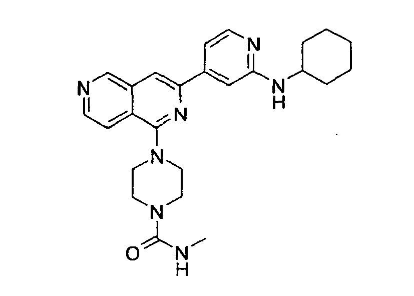

The title compound is prepared from **Example 4F:** HRMS (ESI) *m*/*z* 446.2661 (M+1); ¹H NMR (400 MHz, MeOD) δ ppm 9.31 (s, 1 H), 8.58 (d, *J* = 5.81 Hz, 1 H), 8.04 (s, 1 H), 8.01 (d, *J* = 5.56 Hz, 1 H), 7.97 (d, *J* = 6.06 Hz, 1 H), 7.37 (s, 1 H), 7.25 (d, *J* = 1.52 Hz, 1 H), 4.05 - 3.87 (m, 2 H), 3.78 - 3.62 (m, 1 H), 3.36 - 3.11 (m, 7 H), 2.91 (dd, *J* = 13.01, 10.48 Hz, 1 H), 2.15 - 2.00 (m, 2 H), 1.90 - 1.75 (m, 2 H), 1.70 (dd, *J* = 9.47, 3.66 Hz, 1 H), 1.57 - 1.38 (m, 2 H), 1.37 - 1.24 (m, 4 H), 1.24 (d, *J* = 6.32 Hz, 2 H).

### L. (3-Methoxyphenyl)-[4-(2-hydroxyl)amine-1-yl-[2,6]naphthyridin-3-yl)pyridin-2-yl]amine.

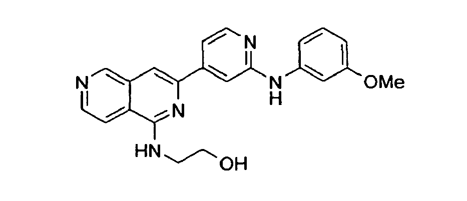

The title compound is prepared from **Example 4G:** MS (ESI) m/z 388.4 (M+1); ¹H NMR (400 MHz, CD₃OD) δ 9.13 (s, 1 H), 8.51 (d, *J* = 5.5 Hz, 1 H), 8.18 (d, *J* = 5.6 Hz, 1 H), 7.99 (d, *J* = 5.6 Hz, 1 H), 7.72 (s, 1 H), 7.61 (s, 1 H), 7.43 (d, *J* = 5.6 Hz, 1 H), 7.25 (s, 1 H), 7.08 (d, *J* = 8.1 Hz, 1 H), 6.59 (dd, *J* = 8.1, 2.6 Hz, 1 H), 3.95 - 3.90 (m, 2 H), 3.89 - 3.82 (m, 6 H).

### M. 2-[3-(2-Cyclohexylaminopyridin-4-yl)-[2,6]naphthyridin-1-ylamino]ethanol.

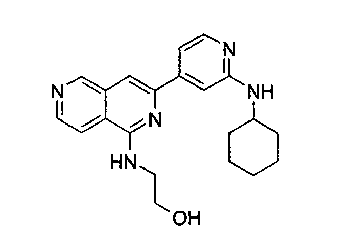

The title compound is prepared from **Example 4G:** MS (ESI) *m*/*z* 363.4 (M+1); ¹H NMR (400 MHz, CD₃OD) δ 9.21 (s, 1 H), 8.56 (d, 1 H), 8.04 (m, 2 H), 7.66 (s, 1 H), 7.22 (m, 2 H), 3.93 (m, 4 H), 3.74 (m, 2 H), 2.08 (m, 2 H), 1.84 (m, 2 H), 1.73 (m, 1 H), 1.51 (m, 2 H), 1.32 (m, 4 H).

### N. 2-[3-(2-Isopropylaminopyridin-4-yl)-[2,6]naphthyridin-1-ylamino]ethanol.

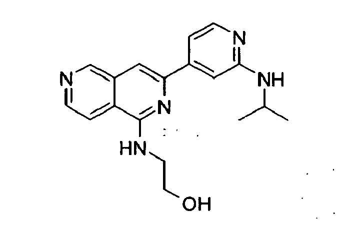

The title compound is prepared from **Example 4G:** MS (ESI) *m*/*z* 324.4 (M+1); ¹H NMR (400 MHz, CD₃OD) δ 9.19 (s, 1 H), 8.55 (d, 1 H), 8.04 (m, 2 H), 7.65 (s, 1 H), 7.23 (m, 2 H), 4.08 (m, 2 H), 3.92 (m, 4 H), 1.30 (d, 6 H).

### O. 2-[3-(2-Phenylaminopyridin-4-yl)-[2,6]naphthyridin-1-ylamino]ethanol.

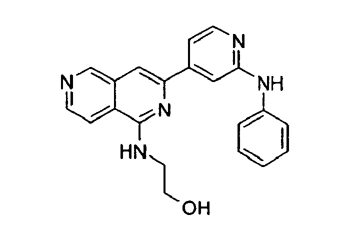

The title compound is prepared from **Example 4G:** MS (ESI) *m*/*z* 358.3 (M+1); ¹H NMR (400 MHz, CD₃OD) δ 9.22 (s, 1 H), 8.57 (d, 1 H), 8.75 (d, 1 H), 8.06 (d, 1 H), 7.70 (s, 1 H), 7.60 (s, 1 H), 7.55 (m, 2 H), 7.44 (d, 1 H), 7.35 (dd, 1 H), 7.03 (t, 1 H), 3.92 (m, 4 H).

### P. {4-[1-(4-Ethoxypiperidin-1-yl)-[2,6]naphthyridin-3-yl]pyridin-2-yl}-(tetrahydropyran-4-yl)amine.

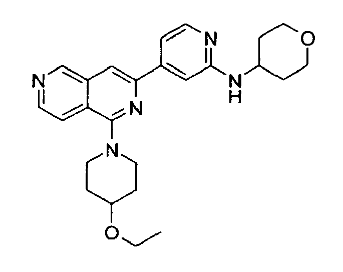

The title compound is prepared from **Example 4H:** MS (ESI) *m*/*z* 434.3 (M+1); ¹H NMR (400 MHz, DMSO-d₆) δ ppm 9.36 (s, 1 H), 8.62 (d, *J* = 5.8 Hz, 1 H), 8.07 (d, *J* = 5.3 Hz, 1 H), 8.05 (s, 1 H), 7.86 (d, *J* = 5.8 Hz, 1 H), 7.33 (s, 1 H), 7.18 (dd, *J* = 5.4, 1.4 Hz, 1 H), 6.67 (d, *J* = 7.8 Hz, 1 H), 4.05 - 3.93 (m, 1 H), 3.89 (dt, *J* = 11.3, 3.4, 3.2 Hz, 2 H), 3.84 - 3.75 (m, 2 H), 3.65 - 3.57 (m, 1 H), 3.54 (q, *J* = 7.1 Hz, 2 H), 3.42 (td, *J* = 11.5, 2.0 Hz, 2 H), 3.29 - 3.21 (m, 2 H), 2.16 - 2.00 (m, 2 H), 1.91 (dd, *J* = 12.6, 2.0 Hz, 2 H), 1.82 - 1.68 (m, 2 H), 1.54 - 1.38 (m, 2 H), 1.15 (t, *J* = 7.1 Hz, 3 H).

### Q. N-[3-(2-Cyclohexylaminopyridin-4-yl)-[2,6]naphthyridin-1-yl]-N',N'-dimethylethane-1,2-diamine.

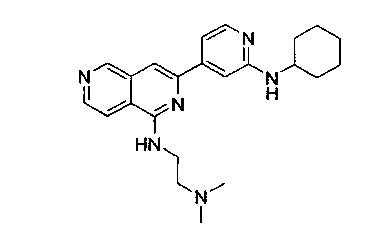

The title compound is prepared from **Example 4I:** MS (ESI) m/z 391.4 (M+1); ¹H NMR (400 MHz, CD₃OD δ 9.06 (s, 1 H), 8.45 (d, *J* = 5.6 Hz, 1 H), 7.95 (d, *J* = 5.6 Hz, 1 H), 7.91 (d, *J* = 6.0 Hz, 1 H), 7.52 (s, 1 H), 8.03 (s, 1 H), 7.18 (d, *J* = 6.0 Hz, 1 H), 3.83 (t, *J* = 6.5 Hz, 2 H), 3.72 - 3.63 (m, 1 H), 2.72 (t, *J* = 6.5 Hz, 2 H), 2.35 (s, 6 H), 2.08 - 2.01 (m, 2 H), 1.83 - 1.76 (m, 2 H), 1.71 - 1.64 (m, 1 H), 1.51 - 1.39 (m, 2 H), 1.34 - 1.19 (m, 3 H).

### R. N-[3-(2-Isopropylaminopyridin-4-yl)-[2,6]naphthyridin-1-yl]-N',N'-dimethylethane-1,2-diamine.

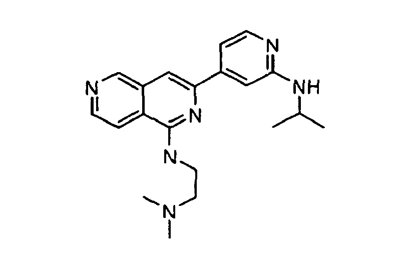

The title compound is prepared from **Example 4I:** MS (ESI) *m*/*z* 351.3 (M+1); ¹H NMR (400 MHz, CD₃OD δ 9.02 (s, 1 H), 8.43 (d, 1 H), 7.96 (d, 1 H), 7.87 (d, 1 H), 7.47 (s, 1 H), 7.31 (s, 1 H), 7.16 (d, 1 H), 4.02 (m, 1 H), 3.80 (t, 2 H), 2.70 (t, 2 H), 2.34 (s, 6 H), 1.25 (d, 6 H).

### S. N,N-Dimethyl-N'-[3-(2-phenylaminopyridin-4-yl)-[2,6]naphthyridin-1-yl]-ethane-1,2-diamine.

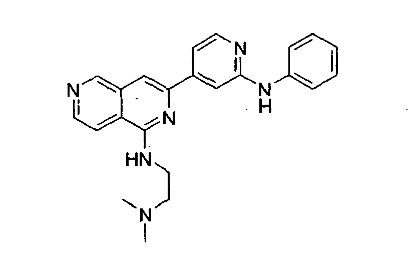

The title compound is prepared from **Example 4I:** MS (ESI) *m*/*z* 385.3 (M+1); ¹H NMR (400 MHz, CD₃OD) δ 9.15 (s, 1 H), 8.53 (d, 1 H), 8.18 (d, 1 H), 7.99 (d, 1 H), 7.78 (s, 1 H), 7.64 (s, 1 H), 7.54 (d, 2 H), 7.46 (d, 1 H), 7.34 (t, 2 H), 7.02 (t, 1 H), 3.87 (t, 2 H), 6.97 (s, 1 H), 2.76 (t, 2 H), 2.42 (s, 1 H), 2.37 (s, 6 H).

### T. N-{3-[2-(3-Methoxyphenylamino)pyridin-4-yl]-[2,6]naphthyridin-1-yl}-N',N'-dimethylethane-1,2-diamine.

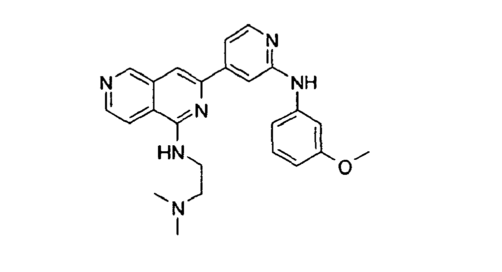

The title compound is prepared from **Example 4I**: MS (ESI) *m*/*z* 415.3 (M+1); ¹H NMR (400 MHz, CD₃OD δ 9.14 (s, 1 H), 8.52 (d, 1 H), 8.19 (d, 1 H), 7.98 (d, 1 H), 7.79 (s, 1 H), 7.63 (s, 1 H), 7.46 (d, 1 H), 7.46 (d, 1 H), 7.23 (m, 2 H), 7.08 (dd, 1 H), 6.59 (dd, 2 H), 3.87 (t, 2 H), 3.84 (s, 3 H), 2.76 (t, 2 H), 2.42 (s, 1 H), 2.37 (s, 6 H).

### U. {4-[1-(4-Dimethylaminomethylpiperidin-1-yl)-[2,6]naphthyridin-3-yl]-pyridin-2-yl}isopropylamine.

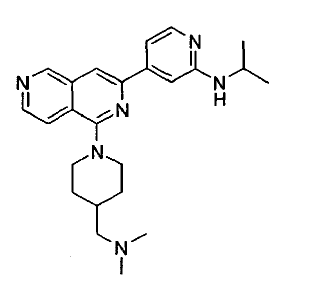

The title compound is prepared from **Example 4J**: MS (ESI) m/z 405.3 (M+1); ¹H NMR (400 MHz, CD₃OD) δ 9.21 (s, 1 H), 8.52 (d, *J* = 5.6 Hz, 1 H), 8.01 (d, *J* = 5.5 Hz, 1H), 7.86 (s, 1 H), 7.85 (d, *J* = 5.5 Hz, 1 H), 7.35 (s, 1 H), 7.22 (d, *J* = 5.5 Hz, 1 H), 4.12 - 3.99 (m, 3 H), 3.09 - 3.03 (t, *J* = 12.2 Hz, 2 H), 2.29 (d, *J* = 7.1 Hz, 2 H), 2.28 (s, 6 H), 1.97 - 1.90 (m, 2 H), 1.89 - 1.75 (m, 1 H), 1.62 - 1.48 (m, 3 H), 1.30 (s, 3 H), 1.28 (s, 3 H).

### V. Cyclohexyl-{4-[1-(4-dimethylaminomethylpiperidin-1-yl)-[2,6]naphthyridin-3-yl]pyridin-2-yl}amine.

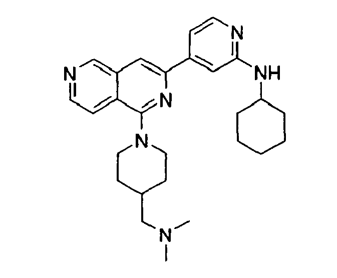

The title compound is prepared from **Example 4J**: MS (ESI) *m*/*z* 445.3 (M+1); ¹H NMR (400 MHz, CD₃OD) δ ppm 9.25 (s, 1 H), 8.56 (d, 1 H), 8.01 (d, 1 H), 7.92 (s, 1 H), 7.90 (d, 1 H), 7.39 (s, 1 H), 7.24 (d, 1 H), 4.07 (d, 2 H), 3.72 (m, 1 H), 3.11 (t, 2 H), 2.10 (m, 2 H), 1.93 (m, 2 H), 1.84 (m, 3 H), 1.73 (m, 1 H), 1.50 (m, 4 H), 1.32 (m, 3 H).

### W. Cyclohexyl-{4-[1-((S)-3-methylpiperazin-1-yl)-[2,6]naphthyridin-3-yl]-pyridin-2-yl}amine.

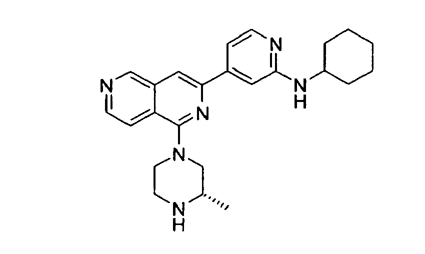

The title compound is prepared from **Example 4K**: HRMS (ESI) *m*/*z* 403.2690 (M+1); ¹H NMR (400 MHz, MeOD) δ ppm 9.21 (s, 1 H), 8.53 (d, *J* = 5.81 Hz, 1 H), 7.97 (d, *J* = 5.56 Hz, 1 H), 7.89 (s, 1 H), 7.88 (d, *J* = 6.06 Hz, 1 H), 7.31 (s, 1 H), 7.17 (dd, *J* = 5.56, 1.52 Hz, 1 H), 3.87 (t, *J*=12.13 Hz, 2 H), 3.71 - 3.60 (m, 1 H), 3.26 - 3.06 (m, 4 H), 2.82 (dd, *J* = 12.76, 10.48 Hz, 1 H), 2.12 - 2.00 (m, 2 H), 1.86 - 1.75 (m, 2 H), 1.73 - 1.63 (m, 1 H), 1.53-1.38 (m, 2 H), 1.34 - 1.22 (m, 3 H), 1.20 (d, *J* = 6.44 Hz, 3 H).

### X. Cyclohexyl-{4-[1-(4-cyclopropylmethylpiperazin-1-yl)-[2,6]naphthyridin-3-yl]-pyridin-2-yl}amine.

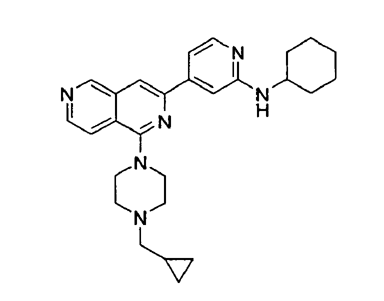

The title compound is prepared from **Example 4L**: HRMS (ESI) *m*/*z* 443.2924 (M+1); ¹H NMR (400 MHz, CDCl₃) δ ppm 9.27 (s, 1 H), 8.61 (d, *J* = 5.81 Hz, 1 H), 8.18 (d, *J* = 5.31 Hz, 1 H), 7.80 (d, *J* = 5.81 Hz, 1 H), 7.76 (s, 1 H), 7.25 - 7.16 (m, 2 H), 4.59 (d, *J* =8.08 Hz, 1 H), 3.79 - 3.57 (m, 5 H), 2.84 (t, *J* = 4.55 Hz, 4 H), 2.40 (d, *J* = 6.57 Hz, 2 H), 2.19 - 2.06 (m, 2 H), 1.88 - 1.73 (m, 2 H), 1.73 - 1.61 (m, 1 H), 1.55 - 1.37 (m, 2 H), 1.36 - 1.19 (m, 3 H), 1.03 - 0.87 (m, 1 H), 0.64 - 0.51 (m, 2 H), 0.18 (q, *J* = 4.88 Hz, 2 H).

### Y. {4-[1-(4-Cyclopropylmethylpiperazin-1-yl)-[2,6]naphthyridin-3-yl]-pyridin-2-yl}-(tetrahydropyran-4-yl)amine.

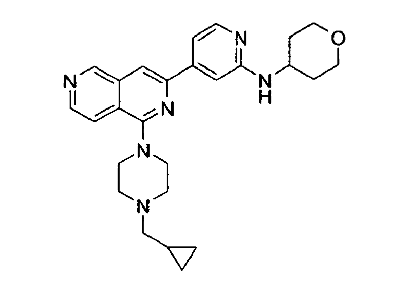

The title compound is prepared from **Example 4L**: HRMS (ESI) *m*/*z* 445.2717 (M+1); ¹H NMR (400 MHz, MeOD) δ ppm 9.30 (s, 1 H), 8.58 (d, *J* = 6.06 Hz, 1 H), 8.06 - 8.00 (m, 2 H), 7.97 (d, *J* = 5.81 Hz, 1 H), 7.42 (s, 1 H), 7.29 (dd, *J* = 5.56, 1.52 Hz, 1 H), 4.05 - 3.92 (m, 3 H), 3.72 - 3.63 (m, 4 H), 3.63 - 3.52 (m, 2 H), 2.97 - 2.82 (m, 4 H), 2.43 (d, *J* = 6.57 Hz, 2 H), 2.03 (d, *J* = 14.65 Hz, 2 H), 1.67 - 1.50 (m, 2 H), 1.06 - 0.89 (m, 1 H), 0.67 - 0.54 (m, 2 H), 0.23 (q, *J* = 5.05 Hz, 2 H).

### Z. {4-[1-(4-Cyclopropylpiperazin-1-yl)-[2,6]naphthyridin-3-yl]-pyridin-2-yl}-(tetrahydropyran-4-yl)amine.

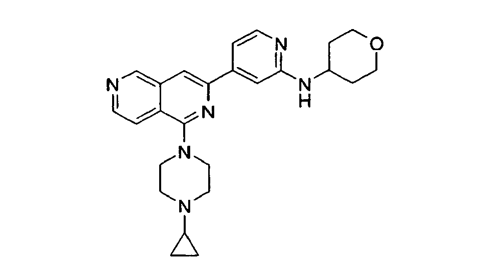

The title compound is prepared from **Example 4M**: HRMS (ESI) *m*/*z*431.2564 (M+1); ¹H NMR (400 MHz, CDCl₃) δ ppm 9.27 (s, 1 H), 8.63 (d, *J* = 5.81 Hz, 1 H), 8.19 (d, *J*=6.06 Hz, 1 H), 7.82 (d, *J* = 5.81 Hz, 1 H), 7.76 (s, 1 H), 7.26 - 7.21 (m, 2 H), 4.58 (d, *J* = 7.33 Hz, 1 H),4.10-3.95(m,3H),3.66-3.53(m,5H),2.92(m,4H),2.12(dd,*J*=12.51,2.02Hz,2 H), 1.87 (br s, 1 H), 1.80 - 1.72 (m, 1 H), 1.66 - 1.52 (m, 2 H), 0.60 - 0.46 (m, 4 H).

### AA. Cyclohexyl-{4-[1-(4-cyclopropylpiperazin-1-yl)-[2,6]naphthyridin-3-yl]-pyridin-2-yl}amine.

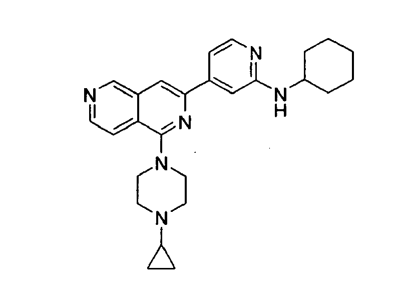

The title compound is prepared from **Example 4M**: HRMS (ESI) *m*/*z*429.2768 (M+1); ¹H NMR (400 MHz, CDCl₃) δ ppm 9.27 (s, 1 H), 8.62 (d, *J* = 5.81 Hz, 1 H), 8.18 (d, *J* = 5.43 Hz, 1 H), 7.81 (d, *J* = 5.81 Hz, 1 H), 7.76 (s, 1 H), 7.24 - 7.17 (m, 2 H), 4.60 (d, *J* = 7.96 Hz, 1 H), 3.76 - 3.63 (m, 1 H), 3.63 - 3.52 (m, 3 H), 2.98 - 2.85 (m, 4 H), 2.19 - 2.07 (m, 2 H), 1.90 - 1.73 (m, 4 H), 1.70 (d, *J* = 3.41 Hz, 1H), 1.55 - 1.38 (m, 2 H), 1.35 - 1.19 (m, 3 H), 0.60 - 0.46 (m, 4 H).

### AB. [3-(2-Cyclohexylaminopyridin-4-yl)-[2,6]naphthyridin-1-yl]-(3-imidazol-1-yl-propyl)amine.

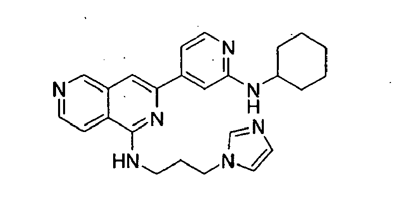

The title compound is prepared from **Example 4N:** MS (ESI) m/z 428.4 (M+1); ¹H NMR (400 MHz, CD₃OD δ 9.13 (s, 1 H), 8.50 (d, *J* = 5.6 Hz, 1 H), 8.01 - 7.95 (m, 2 H), 7.68 (s, 1 H), 7.61 (s, 1 H), 7.28 (s, 1 H), 7.19 (d, *J* = 5.6 Hz, 1 H), 7.17 (s, 1 H), 6.96 (s, 1 H), 4.21 (t, *J* = 7.1 Hz, 2 H), 3.75 (t, *J* = 7.1 Hz, 2 H), 3.72 - 3.63 (m, 1 H), 2.36 - 2.25 (m, 2 H), 2.10 - 2.03 (m, 2H), 1.84 - 1.77 (m, 2 H), 1.74 - 1.65 (m, 1 H), 1.53-1.38 (m, 2 H), 1.36 - 1.22 (m, 3 H).

### AC. [3-(2-i-Propylaminopyridin-4-yl)-[2,6]naphthyridin-1-yl]-(3-imidazol-1-yl-propyl)amine.

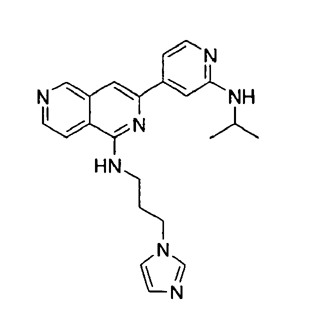

The title compound is prepared from **Example 4N**: MS (ESI) *m*/*z* 388.3 (M+1); ¹H NMR (400 MHz, CD₃OD δ 9.14 (s, 1 H), 8.52 (d, 1 H), 8.01 (m, 3 H), 7.71 (s, 1 H), 7.62 (s, 1 H), 7.30 (s, 1 H), 7.21 (d, 1 H), 6.99 (s, 1 H), 4.24 (t, 2 H), 4.06 (m, 1 H), 3.77 (t, 2 H), 2.33 (m, 2 H), 1.30 (d, 6 H).

### AD. [3-(2-Phenylaminopyridin-4-yl)-[2,6]naphthyridin-1-yl]-(3-imidazol-1-yl-propyl)amine.

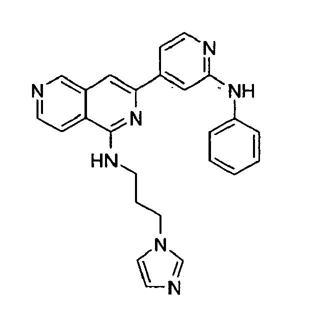

The title compound is prepared from **Example 4N**: MS (ESI) *m*/*z* 422.3 (M+1); ¹H NMR (400 MHz, CD₃OD δ 9.12 (s, 1 H), 8.51 (d, 1 H), 8.17 (d, 1 H), 7.96 (d, 1 H), 7.71 (s, 1 H), 7.66 (s, 1 H), 7.61 (s, 1 H), 7.53 (d, 2 H), 7.41 (d, 1 H), 7.32 (t, 2 H), 7.14 (s, 1 H), 7.02 (t, 1 H), 6.98 (s, 1 H), 4.16 (t, 2 H), 3.17 (t, 2 H), 2.26 (m, 2 H).

### AE. (3-Imidazol-1-yl-propyl)-{3-[2-(3-methoxyphenylamino)pyridin-4-yl]-[2,6]naphthyridin-1-yl}amine.

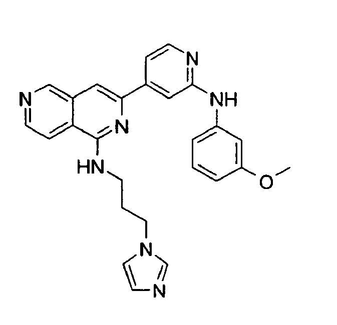

The title compound is prepared from **Example 4N:** MS (ESI) *m*/*z* 452.3 (M+1); ¹H NMR (400 MHz, CD₃OD) δ 9.10 (s, 1 H), 8.50 (d, 1 H), 8.17 (d, 1 H), 7.94 (d, 1 H), 7.71 (s, 1 H), 7.65 (s, 1 H), 7.58 (s, 1 H), 7.39 (d, 1 H), 7.21 (m, 2 H), 7.13 (s, 1 H), 7.07 (d, 1 H), 6.97 (s, 1 H), 6.58 (dd, 1 H), 4.14 (t, 2 H), 3.69 (t, 2H), 2.25 (m, 2 H).

### AF. 4-{3-[2-(Tetrahydropyran-4-ylamino)pyridin-4-yl]-[2,6]naphthyridin-1-yl}piperazine-1-carboxylic acid tert-butyl ester.

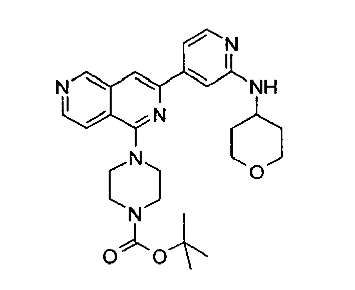

To a dried sealable vial is added 4-[3-(2-chloropyridin-4-yl)-[2,6]naphthyridin-1-yl]-piperazine-1-carboxylic acid *t*-butyl ester **Example 40** (180 mg, 0.43 mmol), sodium *t-*butoxide (120 mg, 1.30 mmol), and dioxane (6.10 mL). The dark red solution is sparged with argon for 10 min. 4-Aminotetrahydropyran (0.16 mL, 1.30 mmol) is added via syringe, followed by palladium(0)tris(tri-*t-*butylphosphine) (44 mg, 0.09 mmol). The vial is flushed with argon, then sealed and heated in a 120 °C oil bath for 14 h. The dark brown solution is cooled to rt, then diluted with water and DCM. The layers are agitated and separated. The aqueous layer is extracted twice with DCM. The combined organic layers are dried over sodium sulfate, filtered, and concentrated to give a brown residue. Purification via silica gel chromatography (40 g Si0₂, gradient 70 → 100% ethyl acetate/hexanes followed by 0 → 10% MeOH/DCM) afforded the title compound as a brown solid (120 mg, 80% pure as judged by ¹H NMR: MS (ESI) *m*/*z* 491.5 (M+1). The crude is used as it is for the subsequent deprotecting step.

The following compounds are prepared by a similar method.

### AG. 4-{3-[2-(2-Methoxyethylamino)pyridin-4-yl]-[2,6]naphthyridin-1-yl}piperazine-1-carboxylic acid tert-butyl ester.

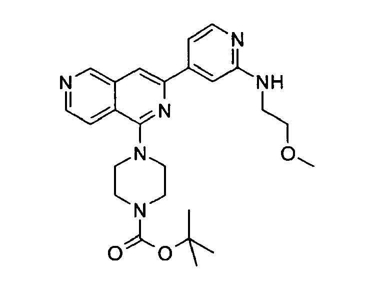

The title compound is prepared from **Example 4O**: MS (ESI) *m*/*z* 465.2 (M+1).

### AH. 4-[3-(2-Isopropylaminopyridin-4-yl)-[2,6]naphthyridin-1-yl]piperazine-1-carboxylic acid tert-butyl ester.

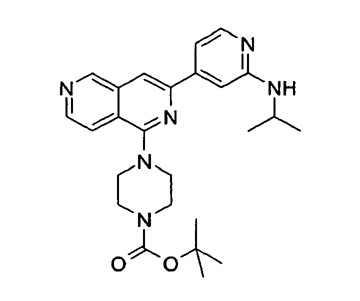

The title compound is prepared from **Example 4O**: MS (ESI) *m*/*z* 448.2 (M+1).

### AI. 4-[3-(2-Phenylaminopyridin-4-yl)-[2,6]naphthyridin-1-yl]piperazine-1-carboxylic acid tert-butyl ester.

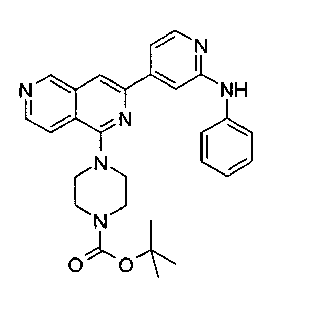

The title compound is prepared from **Example 4O**: MS (ESI) *m*/*z* 482.2 (M+1).

### AJ. 4-{3-[2-(1-Methyl-1H-pyrazol-3-ylamino)pyridin-4-yl]-[2,6]naphthyridin-1-yl}piperazine-1-carboxylic acid tert-butyl ester.

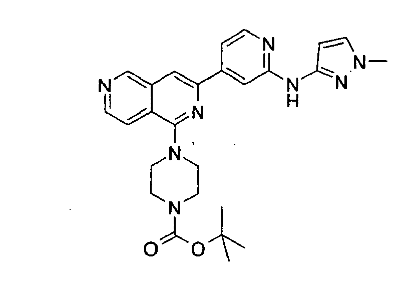

The title compound is prepared from **Example 4O**: MS (ESI) *m*/*z* 487.2 (M+1).

### AK. 4-[3-(2-Cyclopentylaminopyridin-4-yl)-[2,6]naphthyridin-1-yl]piperazine-1-carboxylic acid tert-butyl ester.

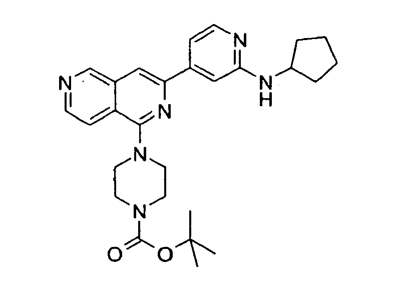

The title compound is prepared from **Example 4O**: MS (ESI) *m*/*z* 475.2 (M+1).

### AL. 4-[3-(2-Cyclohexylaminopyridin-4-yl)-[2,6]naphthyridin-1-yl]piperazine-1-carboxylic acid tert-butyl ester.

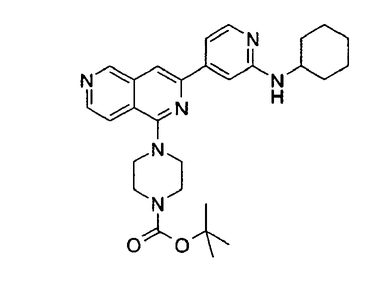

The title compound is prepared from **Example 40**: MS (ESI) *m*/*z* 489.2 (M+1).

### AM. 4-{3-[2-(4-Methylcyclohexylamino)pyridin-4-yl]-[2,6]naphthyridin-1-yl}piperazine-1-carboxylic acid tert-butyl ester.

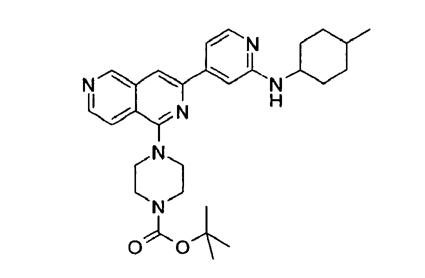

The title compound is prepared from **Example 4O**: MS (ESI) *m*/*z* 503.2 (M+1).

### AN. 4-{3-[2-(2-Methylcyclohexylamino)pyridin-4-yl]-[2,6]naphthyridin-1-yl}piperazine-1-carboxylic acid tert-butyl ester.

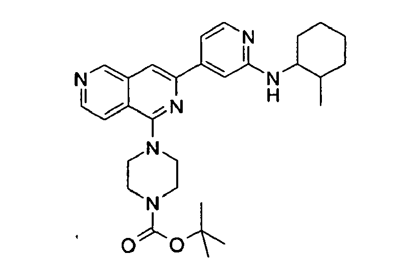

The title compound is prepared from **Example 4O**: MS (ESI) *m*/*z* 503.2 (M+1).

### AO. 4-[3-(2-tert-Butylamino-pyridin-4-yl)-[2,6]naphthyridin-1-yl]-piperazine-1-carboxylic acid tert-butyl ester.

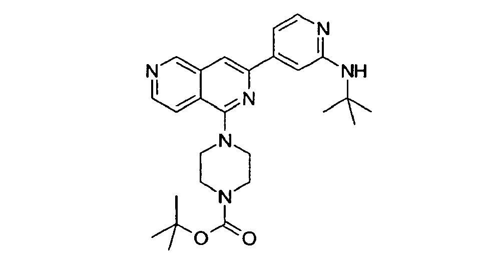

The title compound is prepared from **Example 4O**: MS (ESI) *m*/*z* 463.3 (M+1).

### AP. (R)-3-[3-(2-Cyclohexylaminopyridin-4-yl)-[2,6]naphthyridin-1-ylamino]pyrrolidine-1-carboxylic acid tert-butyl ester.

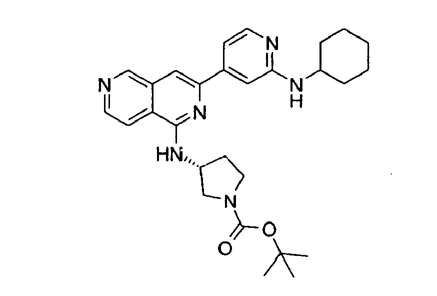

The title compound is prepared from **Example 4P**: MS (ESI) *m*/*z* 489.4 (M+1).

### AQ. (S)-3-[3-(2-Cyclohexylaminopyridin-4-yl)-[2,6]naphthyridin-1-ylamino]pyrrolidine-1-carboxylic acid tert-butyl ester.

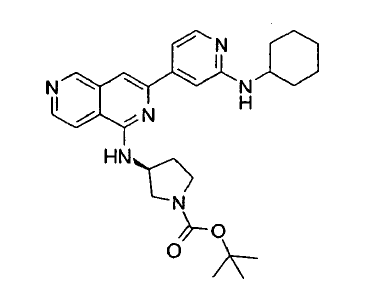

The title compound is prepared from **Example 4Q**: MS (ESI) *m*/*z* 489.4 (M+1).

### AR. {1-[3-(2-Cyclohexylaminopyridin-4-yl)-[2,6]naphthyridin-1-yl]piperidin-4-ylmethyl}carbamic acid tert-butyl ester.

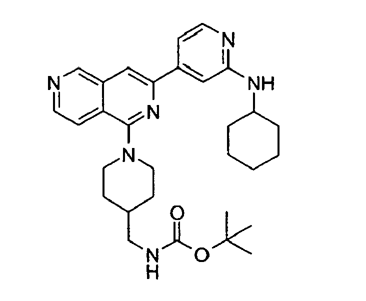

The title compound is prepared from **Example 4R**: MS (ESI) *m*/*z* 417.2 (M+1).

### AS. 1-{3-[2-(Tetrahydropyran-4-ylamino)pyridin-4-yl]-[2,6]naphthyridin-1-yl}-piperidine-4-carboxylic acid isopropylamide.

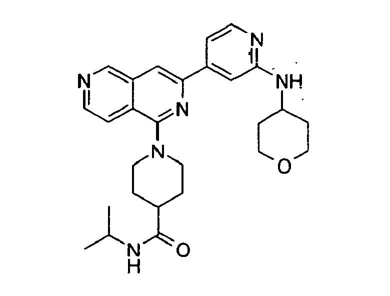

A pressure reaction vessel is charged with 1-[3-(2-chloropyridin-4-yl)-[2,6]naphthyridin-1-yl]-piperidine-4-carboxylic acid isopropylamide **Example 5A** (0.50 g, 1.22 mmol), 4-aminotetrahydropyran (0.25 g, 2.44 mmol), Pd(*t*Bu₃P)₂ (0.06 g, 0.12 mmol), *t*-BuONa (0.35 g, 3.66 mmol), and 1,4-dioxane. The mixture is sparged with argon for 10 min and the vessel is then sealed and heated to 130 °C for 2.5 h. The contents of the vessel are allowed to cool to rt before being diluted with DCM (150 mL) and brine (150 mL). The layers are mixed and then separated. The aqueous layer is extracted further with DCM (3 X 150 mL) and the combined organic layers are then dried over Na₂SO₄, filtered and concentrated. The residue is then separated via reversed phase prep HPLC (5-60% CH₃CN/H₂O/0.1%NH₄OH gradient) to give 1-{3-[2-(tetrahydro-pyran-4-ylamino)-pyridin-4-yl]-[2,6]naphthyridin-1-yl}-piperidine-4-carboxylic acid isopropylamide: MS (ESI) 475.1 *m*/*z* (M+1); ¹H NMR (400 MHz, DMSO-*d*₆) δ ppm 9.36 (s, 1 H), 8.62 (d, *J* = 5.6 Hz, 1 H), 8.08 (d, *J* = 5.3 Hz, 1 H), 8.04 (s, 1 H), 7.86 (d, *J* = 5.8 Hz, 1 H), 7.70 (d, *J* = 7.6 Hz, 1 H), 7.31 (s, 1 H), 7.22 - 7.14 (m, 1 H), 6.66 (d, *J* = 7.6 Hz, 1 H), 4.09 - 3.93 (m, 3 H), 3.94 - 3.77 (m, 3 H), 3.49 - 3.36 (m, 2 H), 3.05 (t, *J* = 12.0 Hz, 2 H), 2.46 - 2.31 (m, 1) H, 2.01 1.78 (m, 6 H), 1.56 - 1.38 (m, 2 H), 1.07 (d, *J* = 6.6 Hz, 6 H).

The following compounds can be prepared with similar method.

### AT. 1-[3-(2-Cyclohexylaminopyridin-4-yl)-[2,6]naphthyridin-1-yl]-piperidine-4-carboxylic acid isopropylamide.

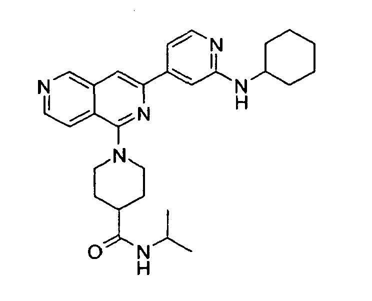

The title compound can be prepared from amide **Example 5A**: MS (ESI) m/z 473.2 (M+1); ¹H NMR (400 MHz, *MeOD*) δ ppm 9.29 (s, 1 H), 8.58 (d, *J*=5.8 Hz, 1 H), 7.92 - 8.05 (m, 3 H), 7.39 (s, 1 H), 7.26 (d, *J*=5.8 Hz, 1 H), 4.08 - 4.22 (m, 2 H), 3.95 - 4.07 (m, 1 H), 3.62 - 3.74 (m, 1 H), 3.07 - 3.21 (m, 2 H), 2.42 - 2.58 (m, 1 H), 2.02 - 2.19 (m, 4 H), 1.89 - 1.99 (m, 2 H), 1.77 - 1.88 (m, 2 H), 1.65 - 1.75 (m, 1 H), 1.44 - 1.57 (m, 1 H), 1.24 - 1.37 (m, 4 H), 1.12-1.23(m,6H).

### AU. 1-{3-[2-(Tetrahydropyran-4-ylamino)pyridin-4-yl]-[2,6]naphthyridin-1-yl}piperidine-4-carboxylic acid ethylamide.

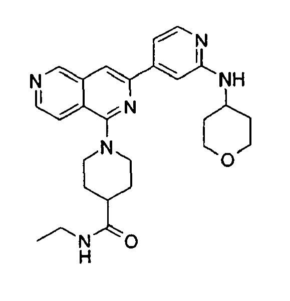

The title compound is prepared from **Example 5B:** MS (ESI) 461.2 *m*/*z* (M+1); ¹H NMR (400 MHz, MeOD) (TFA salt) δ ppm 8.68 (d, *J* = 5.8 Hz, 1 H), 8.22 (s, 1 H), 8.02 (d, *J* = 6.1 Hz, 1 H), 8.00 - 7.96 (m, 1 H), 7.95 - 7.86 (m, 1 H), 7.69 - 7.61 (m, 1 H), 4.26 - 4.16 (m, 2 H), 4.09 - 3.99 (m, 2 H), 3.96 - 3.84 (m, 1 H), 3.63 - 3.53 (m, 2 H), 3.29 - 3.14 (m, 4 H), 2.58 - 2.45 (m, 1 H), 2.15 - 2.01 (m, 4 H), 1.98 - 1.88 (m, 2 H), 1.79 - 1.64 (m, 2 H), 1.14 (t, *J* = 7.3 Hz, 3 H).

### AV. 1-{3-[2-(Tetrahydropyran-4-ylamino)pyridin-4-yl]-[2,6]naphthyridin-1-yl}piperidine-4-carboxylic acid isobutylamide.

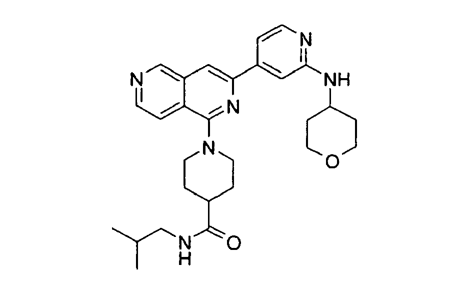

The title compound is prepared from **Example 5C**: MS (ESI) *m*/*z* 489.2 (M+1); ¹H NMR (400 MHz, CDCl₃) δ ppm 9.27 (s, 1 H), 8.62 (d, *J* = 5.8 Hz, 1 H), 8.20 (d, *J* = 5.6 Hz, 1 H), 7.79 (d, *J* = 5.8 Hz, 1 H), 7.76 (s, 1 H), 7.26 - 7.22 (m, 1 H), 7.20 (s, 1 H), 5.58 (t, *J* = 5.6 Hz, 1 H), 4.50 (d, *J* = 8.1 Hz, 1 H), 4.14 - 3.98 (m, 5 H), 3.65 - 3.54 (m, 2 H), 3.19 - 3.07 (m, 4 H), 2.46 - 2.33 (m, 1 H), 2.22 - 2.01 (m, 6 H), 1.87 - 1.75 (m, 1 H), 0.95 (d, *J* = 6.6 Hz, 6 H).

### AW. 1-{3-[2-(1-Methyl-1H-pyrazol-3-ylamino)pyridin-4-yl]-[2,6]naphthyridin-1-yl}piperidine-4-carboxylic acid isobutylamide.

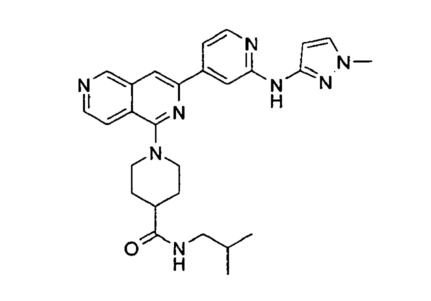

The title compound is prepared from **Example 5C:** MS (ESI) *m*/*z* 485.3 (M+1); ¹H NMR (400 MHz, MeOD) δ ppm 9.30 (d, *J* = 0.8 Hz, 1 H), 8.58 (d, *J* = 5.9 Hz, 1 H), 8.18 (dd, *J* = 5.5, 0.7 Hz, 1 H), 8.10 (d, *J* = 0.8 Hz, 1 H), 8.04 (s, 1 H), 7.96 (d, *J* = 5.9 Hz, 1 H), 7.51 (dd, *J* = 5.5, 1.6 Hz, 1 H), 7.49 (d, *J* = 2.0 Hz, 1 H), 6.28 (d, *J* = 2.4 Hz, 1 H), 4.15 (br d, *J* = 13.0 Hz, 2 H), 3.85 (s, 3 H), 3.16 (td, *J* = 12.5, 2.1 Hz, 2 H), 3.04 (d, *J* = 6.9 Hz, 2 H), 2.54 (tt, *J* = 11.6, 3.9 Hz, 1 H), 2.19 - 2.04 (m, 2 H), 2.00 - 1.91 (m, 2 H), 1.88 - 1.72 (m, 1 H), 0.94 (d, *J* = 6.7 Hz, 6 H).

### AX. 1-{3-[2-(2-Methyl-2H-pyrazol-3-ylamino)-pyridin-4-yl]-[2,6]naphthyridin-1-yl}-piperidine-4-carboxylic acid isobutylamide.

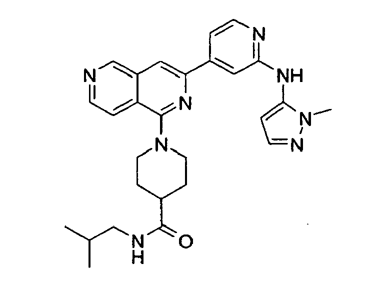

The title compound can be prepared from amide Example 5C by analogy to the method outlined in **Example 6AS:** HRMS (ESI) *m*/*z* 485.2787 (M+1); ¹H NMR (400 MHz, DMSO-*d*₆) δ ppm 9.38 (s, 1 H), 8.91 (s, 1H), 8.64 (d, *J* = 5.8 Hz, 1 H), 8.23 (d, *J* = 5.3 Hz, 1 H), 8.12 (s, 1 H), 7.92 - 7.80 (m, 2 H), 7.62 (s, 1 H), 7.50 (dd, *J* = 5.6, 1.5 Hz, 1 H), 7.35 (d, *J* = 2.0 Hz, 1 H), 6.26 (t, *J* = 1.8 Hz, 1 H), 4.04 (br d, *J* = 13.4 Hz, 2 H), 3.70 (s, 3 H), 3.07 (br t, *J* = 11.0 Hz, 2 H), 2.94 - 2.88 (m, 2 H), 2.49 - 2.41 (m, 1 H), 2.01 - 1.81 (m, 4 H), 1.76 - 1.65 (m, 1 H), 0.86 (d, *J* = 6.8 Hz, 6 H).

### AY. 1-{3-[2-((R)-1,2-Dimethyl-propylamino)-pyridin-4-yl]-[2,6]naphthyridin-1-yl}-piperidine-4-carboxylic acid isobutylamide.

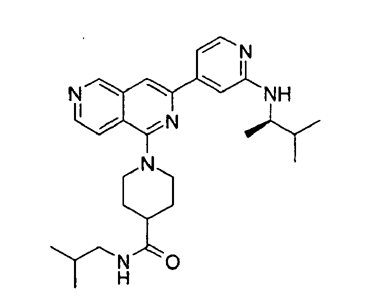

The title compound can be prepared from amide **Example 5C** by analogy to the method outlined in **Example 6AS:** HRMS (ESI) *m*/*z* 475.3191 (M+1); ¹H NMR (400 MHz, DMSO-*d*₆) δ ppm 9.36 (s, 1 H), 8.63 (d, *J* = 5.8 Hz, 1 H), 8.10 - 8.01 (m, 2 H), 7.91 - 7.81 (m, 2 H), 7.36 (br s, 1 H), 7.17 (br s, 1 H), 4.03 (br d, *J* = 12.1 Hz, 2 H), 3.96 - 3.81 (m, 2 H), 3.06 (br t, *J* = 12.3 Hz, 2 H), 2.95 - 2.86 (m, 2 H), 2.48 - 2.40 (m, 1 H), 2.03 - 1.77 (m, 5 H), 1.77 - 1.64 (m, 1 H), 1.08 (d, *J* = 6.6 Hz, 3 H), 0.94 (d, *J* = 6.8 Hz, 3 H), 0.90 (d, *J* = 6.8 Hz, 3 H), 0.85 (d, *J* = 6.6 Hz, 6 H).

### AZ. 1-{3-[2-((R)-sec-Butylamino)-pyridin-4-yl]-[2,6]naphthyridin-1-yl}-piperidine-4-carboxylic acid isobutylamide.

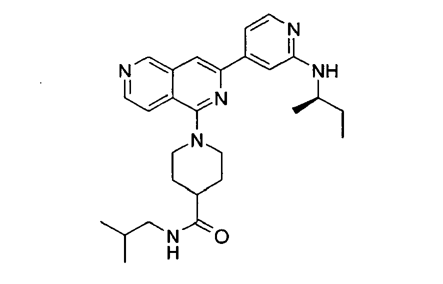

The title compound can be prepared from amide **Example 5C** by analogy to the method outlined in **Example 6AS:** HRMS (ESI) *m*/*z* 461.3041 (M+1); ¹H NMR (400 MHz, CD₃OD δ ppm 9.29 (s, 1 H), 8.58 (d, *J* = 5.8 Hz, 1 H), 8.00 (m, 2 H), 7.96 (d, *J* = 6.1 Hz, 1 H), 7.39 (s, 1 H), 7.27 (dd, *J* = 5.7, 1.6 Hz, 1 H), 4.14 (br d, *J* = 13.4 Hz, 2 H), 3.86 (q, *J* = 6.6 Hz, 1 H), 3.33 (m, 1 H), 3.22 - 3.09 (m, 2 H), 3.04 (d, *J* = 6.8 Hz, 2 H), 2.54 (m, 1 H), 2.20 - 2.04 (m, 2 H), 2.01 - 1.90 (m, 2 H), 1.87 - 1.75 (m, 1 H), 1.71 - 1.51 (m, 2 H), 1.23 (d, *J* = 6.6 Hz, 3 H), 1.05 - 0.97 (m, 3 H), 0.94 (d, *J* = 6.8 Hz, 6 H).

### BA. 1-{3-[2-Isopropylaminopyridin-4-yl]-[2,6]naphthyridin-1-yl}-piperidine-4-carboxylic acid isobutylamide.

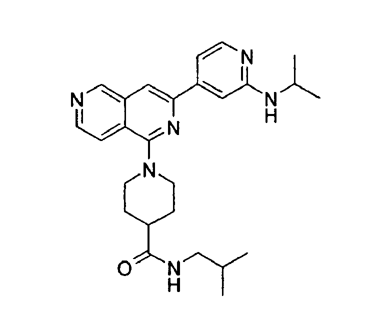

The title compound can be prepared from amide **Example 5C** by analogy to the method outlined in **Example 6AS:** MS (ESI) *m*/*z* 447.3 (M+1); ¹H NMR (400 MHz, MeOD) δ ppm 9.29 (s, 1 H), 8.57 (d, *J* = 5.9 Hz, 1 H), 8.02 (d, *J* = 5.6 Hz, 1 H), 8.00 (s, 1 H), 7.96 (d, *J* = 5.8 Hz, 1 H), 7.37 (s, 1 H), 7.28 (dd, *J=* 5.7, 1.5 Hz, 1 H), 4.13 (br d, *J* = 13.3 Hz, 2 H), 4.09 -4.00(m, 1 H), 3.21 - 3.09 (m, 2 H), 3.04 (d, *J* = 6.9 Hz, 2 H), 2.54 (tt, *J* = 11.7, 4.4, 4.1 Hz, 1 H), 2.11 (qd, *J* = 12.5, 12.4, 3.7 Hz, 2 H), 1.95 (dd, *J* = 12.3, 2.2 Hz, 2 H), 1.88 - 1.74 (m, 1 H), 1.26 (d, *J* = 6.3 Hz, 6 H), 0.94 (d, *J* = 6.7 Hz, 6 H).

### BB. 1-[3-(2-Isobutylaminopyridin-4-yl)-[2,6]naphthyridin-1-yl]-piperidine-4-carboxylic acid isobutyl amide.

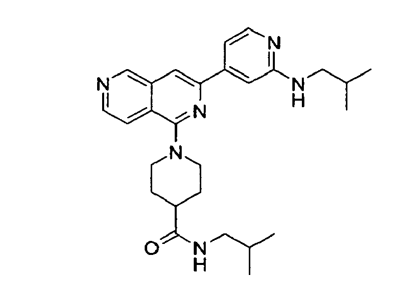

**Example 6BA** can be obtained as a side-product during the formation of **Example 6BB:** MS (ESI) *m*/*z* 461.3 (M+1); ¹H NMR (400 MHz, MeOD) δ ppm 9.26 (s, 1 H), 8.55 (d, *J* = 5.8 Hz, 1 H), 7.99 (d, *J* = 5.3 Hz, 1 H), 7.96 (s, 1 H), 7.92 (d, *J* = 5.8 Hz, 1 H), 7.39 (s, 1 H), 7.25 (dd, *J* = 5.6, 1.5 Hz, 1 H), 4.11 (br d, *J* = 12.9 Hz, 2 H), 3.16 (d, *J* = 6.8 Hz, 2 H), 3.16 - 3.07 (m, 2 H), 3.04 (d, *J* = 6.8 Hz, 2 H), 2.53 (tt, *J* = 11.6, 3.8 Hz, 1 H), 2.10 (qd, *J* = 12.4, 3.8 Hz, 2 H), 2.01 - 1.88 (m, 3 H), 1.87 - 1.74 (m, 1 H), 1.02 (d, *J* = 6.6 Hz, 6 H), 0.93 (d, *J* = 6.8 Hz, 6 H).

### BC. 1-{3-[2-Cyclopropylaminopyridin-4-yl]-[2,6]naphthyridin-1-yl}-piperidine-4-carboxylic acid isobutylamide.

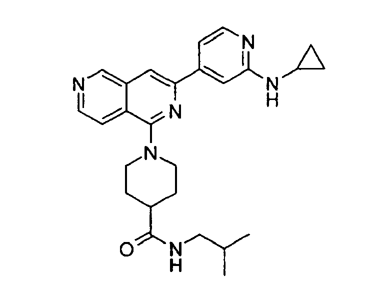

The title compound can be prepared from amide **Example 5C** by analogy to the method outlined in **Example 6AS:** MS (ESI) *m*/*z* 445.4 (M+1); ¹H NMR (400 MHz, MeOD) δ ppm 9.31 (s, 1 H), 8.58 (d, *J* = 6.1 Hz, 1 H), 8.06 (d, *J* = 5.3 Hz, 1 H), 8.04 (s, 1 H), 7.97 (d, *J* = 6.1 Hz, 1 H), 7.64 (s, 1 H), 7.39 (dd, *J* = 5.6, 1.3 Hz, 1 H), 4.15 (br d, *J* = 12.6 Hz, 2 H), 3.17 (br t, *J* = 12.8 Hz, 2 H), 3.04 (d, *J* = 6.8 Hz, 2 H), 2.67 - 2.59 (m, 1 H), 2.60 - 2.49 (m, 1 H), 2.11 (qd, *J* = 12.6, 4.2 Hz, 2 H), 1.95 (dd, *J* = 12.6, 2.5 Hz, 2 H), 1.86 - 1.74 (m, 1 H), 0.93 (d, *J* = 6.6 Hz, 6 H), 0.89 - 0.80 (m, 2 H), 0.62 - 0.53 (m, 2 H).

### BD. 1-[3-(2-Aminopyridin-4-yl)-[2,6]naphthyridin-1-yl]-piperidine-4-carboxylic acid isobutyl amide.

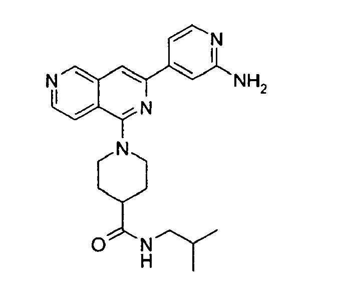

**Example 6BD** can be obtained as a side-product during the formation of **Example 6BC:** MS (ESI) *m*/*z* 405.2 (M+1); ¹H NMR (400 MHz, MeOD) δ ppm 9.28 (s, 1 H), 8.57 (d, *J* = 5.8 Hz, 1 H), 8.03 - 7.96 (m, 2 H), 7.95 (d, *J* = 5.8 Hz, 1 H), 7.44 (s, 1 H), 7.36 (dd, *J* = 5.7, 1.6 Hz, 1 H), 4.12 (d, J = 13.1 Hz, 2 H), 3.20 - 3.08 (m, 2 H), 3.04 (d, *J* = 7.1 Hz, 2 H), 2.54 (tt, *J* = 11.7, 4.0, 3.9 Hz, 1 H), 2.10 (qd, *J*= 12.4, 3.8 Hz, 2 H), 2.01 - 1.91 (m, 2 H), 1.88-1.73 (m, 1 H), 0.94 (d, *J* = 6.6 Hz, 6 H).

### BE. 1-{3-[2-((S)-1-Phenylethylamino)-pyridin-4-yl]-[2,6]naphthyridin-1-yl}-piperidine-4-carboxylic acid isobutylamide.

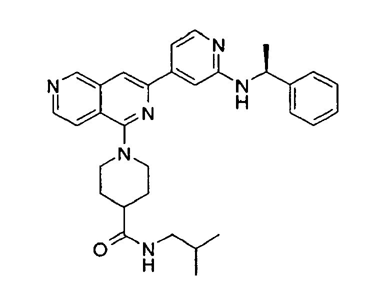

The title compound can be prepared from amide **Example 5C** by analogy to the method outlined in **Example 6AS:** MS (ESI) *m*/*z* 509.3 (M+1); ¹H NMR (400 MHz, MeOD) δ ppm 9.25 (s, 1 H), 8.55 (d, *J* = 5.8 Hz, 1 H), 8.00 (d, *J* = 5.6 Hz, 1 H), 7.96 - 7.88 (m, 2 H), 7.43 (d, *J* = 7.6 Hz, 2 H), 7.36 - 7.24 (m, 4 H), 7.20 (s, 1 H), 4.93 (q, *J* = 6.9 Hz, 1 H), 4.04 (br dd, *J* = 16.9, 13.9 Hz, 2 H), 3.13 - 2.99 (m, 4 H), 2.61 - 2.46 (m; 1 H), 2.18 - 2.00 (m, 2 H), 2.00 - 1.91 (m, 2 H), 1.89 - 1.75 (m, 1 H), 1.56 (d, *J* = 6.8 Hz, 3 H), 0.95 (d, *J* = 6.8 Hz, 6 H).

### BF. 1-{3-[2-((R)-1-Phenylethylamino)-pyridin-4-yl]-[2,6]naphthyridin-1-yl}-piperidine-4-carboxylic acid isobutylamide.

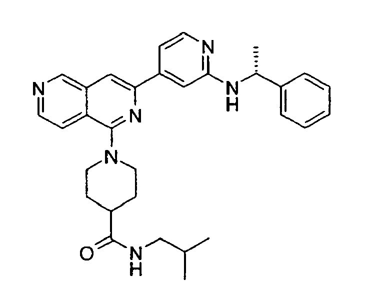

The title compound can be prepared from amide **Example 5C** by analogy to the method outlined in **Example 6AS:** MS (ESI) *m*/*z* 509.3 (M+1); ¹H NMR (400 MHz, MeOD) δ ppm 9.25 (s, 1 H), 8.55 (d, *J* = 5.8 Hz, 1 H), 8.00 (d, *J* = 5.6 Hz, 1 H), 7.96 - 7.88 (m, 2 H), 7.43 (d, *J* = 7.6 Hz, 2 H), 7.36 - 7.24 (m, 4 H), 7.20 (s, 1 H), 4.93 (q, *J* = 6.9 Hz, 1 H), 4.04 (br dd, *J* = 16.9, 13.9 Hz, 2 H), 3.13 - 2.99 (m, 4 H), 2.61 - 2.46 (m, 1 H), 2.18 - 2.00 (m, 2 H), 2.00 - 1.91 (m, 2 H), 1.89 - 1.75 (m, 1 H), 1.56 (d, *J* = 6.8 Hz, 3 H), 0.95 (d, *J* = 6.8 Hz, 6 H).

### BG. 1-[3-(2-Butylaminopyridin-4-yl)-[2,6]naphthyridin-1-yl]-piperidine-4-carboxylic acid isobutylamide.

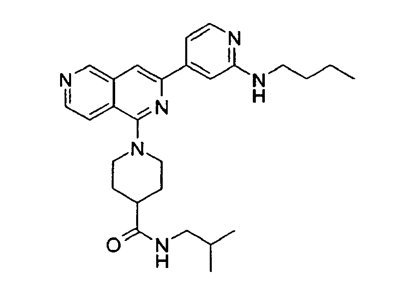

In a sealable pressure vessel, a suspension of 1-[3-(2-chloropyridin-4-yl)-[2,6]naphthyridin-1-yl]-piperidine-4-carboxylic acid isobutyl-amide **Example 5C** (140 mg, 0.32 mmol), n-butylamine (0.16 mL, 1.6 mmol), and sodium *tert*-butoxide (44 mg, 0.45 mmol) in 1.1 mL of dimethoxyethane is sparged with argon for 10 min. A separate stock solution of Josiphos (10 mg, 0.014 mmol) and palladium(II) acetate (5 mg, 0.014 mmol) in argon-degassed dimethoxyethane (1 mL) is prepared, and then 0.5 mL of the resulting orange-red catalyst solution is added to the reaction mixture. The vessel is flushed with argon, sealed, and immersed in a 100 °C oil bath for 9 h. The orange-red reaction mixture is cooled to room temperature, then diluted with water and DCM. The layers are agitated and separated. The aqueous layer is extracted twice with DCM. The combined organic layers are passed through a small plug of sodium sulfate and concentrated *in vacuo* to give a brownish orange solid. The residue is purified by preparative reverse-phase HPLC (X-Bridge RP₁₈ column, flow rate = 60 mL/min, gradient 20% → 80% acetonitrile/5 mM aqueous ammonium hydroxide over 10 min) to give the title compound as a yellowish white solid (106 mg, 71%). MS (ESI) *m*/*z* 461.3 (M+1); ¹H NMR (400 MHz, DMSO-*d*₆) δ ppm 9.36 (s, 1 H), 8.62 (d, *J* = 5.8 Hz, 1 H), 8.07 (d, *J* = 5.3 Hz, 1 H), 8.05 (s, 1 H), 7.90 - 7.79 (m, 2 H), 7.28 (s, 1 H), 7.17 (dd, *J* = 5.6, 1.5 Hz, 1 H), 6.63 (t, *J* = 5.4 Hz, 1 H), 4.03 (br d, *J* = 12.6 Hz, 2 H), 3.30 - 3.25 (m, 2 H), 3.06 (br t, *J* = 11.2 Hz, 2 H), 2.91 (t, *J* = 6.3 Hz, 2 H), 2.49 - 2.39 (m, 1 H), 2.02 - 1.80 (m, 4 H), 1.77 - 1.63 (m, 1 H), 1.60 - 1.49 (m, 2 H), 1.45 - 1.32 (m, 2 H), 0.92 (t, *J* = 7.3 Hz, 3 H), 0.85 (d, *J* = 6.8 Hz, 6 H).

### BH. 1-[3-(2-Cyclohexylaminopyridin-4-yl)-[2,6]naphthyridin-1-yl]-piperidine-4-carboxylic acid (2,2-dimethylpropyl)-amide.

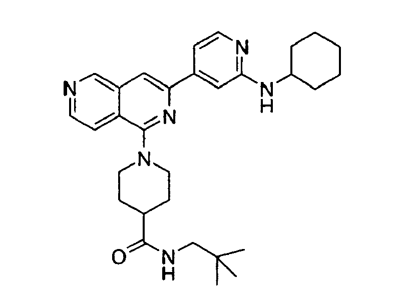

The title compound can be prepared from amide **Example 7A** by analogy to the method outlined in **Example 6AS:** MS (ESI) m/z 501.4 (M+1); ¹H NMR (400 MHz, *MeOD*) δ ppm 9.27 (s, 1 H), 8.56 (d, *J*=6.1 Hz, 1 H), 7.87 - 8.06 (m, 3 H), 7.37 (s, 1 H), 7.25 (dd, *J*=5.6, 1.5 Hz, 1 H), 4.07 - 4.19 (m, 2 H), 3.62 - 3.75 (m, 1 H), 3.09 - 3.23 (m, 2 H), 3.05 (s, 2 H), 2.52 - 2.65 (m, 1 H), 2.02 - 2.17 (m, 4 H), 1.90 - 1.99 (m, 2 H), 1.76 - 1.85 (m, 2 H), 1.63 - 1.75 (m, 1 H), 1.39 - 1.56 (m, 2 H), 1.21 - 1.35 (m, 3 H), 0.93 (s, 9 H).

### BI. 1-[3-(2-Cyclohexylamino-pyridin-4-yl)-[2,6] naphthyridin-1-yl]-piperidine-4-carboxylic acid cyclohexylamide.

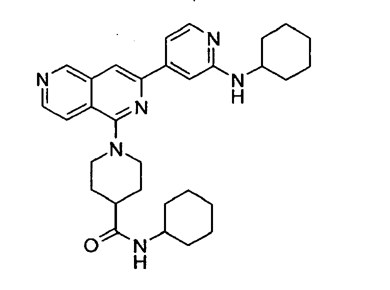

The title compound can be prepared from amide **Example 7B** by analogy to the method outlined in **Example 6AS:** MS (ESI) m/z 513.4 (M+1); ¹H NMR (400 MHz, *DMSO-d*₆) δ ppm 9.36 (s, 1 H), 8.61 (d, *J*=5.8 Hz, 1 H), 8.05 (d, *J*=5.6 Hz, 1 H), 8.03 (s, 1 H), 7.86 (d, *J*=5.8 Hz, 1 H), 7.70 (d, *J*=7.8 Hz, 1 H), 7.28 (s, 1 H), 7.14 (d, *J*=5.3 Hz, 1 H), 6.51 (d, *J*=7.6 Hz, 1 H), 4.03 (d, *J*=12.9 Hz, 2 H), 3.74 (d, *J*=13.6 Hz, 1 H), 3.54 (d, *J*=16.9 Hz, 1 H), 2.97 - 3.09 (m, 2 H), 2.35 - 2.46 (m, 1 H), 1.78 - 2.03 (m, 6 H), 1.64 - 1.78 (m, 6 H), 1.59 (m, 2 H), 1.07 - 1.42 (m, 10 H).

### BJ. 1-{3-[2-(Tetrahydropyran-4-ylamino)-pyridin-4-yl]-[2,6]naphthyridin-1-yl}-piperidine-4-carboxylic acid cyclohexylamide

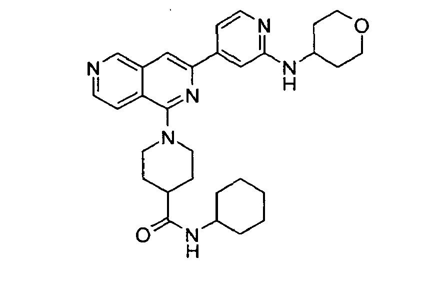

The title compound can be prepared from amide **Example 7B** by analogy to the method outlined in **Example 6AS:** MS (ESI) m/z 515.4 (M+1); ¹H NMR (400 MHz, *DMSO-d*₆) δ ppm 9.36 (s, 1 H), 8.62 (d, *J*=5.8 Hz, 1 H), 8.07 (d, *J*=5.6 Hz, 1 H), 8.04 (s, 1 H), 7.70 (d, *J*=7.8 Hz, 1 H), 7.31 (s, 1 H), 7.18 (dd, *J*=5.4, 1.4 Hz, 1 H), 6.66 (d, *J*=7.3 Hz, 1 H), 3.93 - 4.09 (m, 3 H), 3.83 - 3.92 (m, 2 H), 3.49 - 3.60 (m, 1 H), 3.36 - 3.48 (m, 2 H), 2.99 - 3.11 (m, 2 H), 2.35 - 2.46 (m, 1 H), 1.79 - 2.00 (m, 6 H), 1.71 (d, *J*=33.1 Hz, 4 H), 1.49 (d, *J*=73.0 Hz, 4 H), 1.08 -1.34 (m, 5 H).

### BK. 1-{3-[2-(1-Methyl-1H-pyrazol-3-ylamino)-pyridin-4-yl]-[2,6]naphthyridin-1-yl}-piperidine-4-carboxylic acid cyclohexylamide.

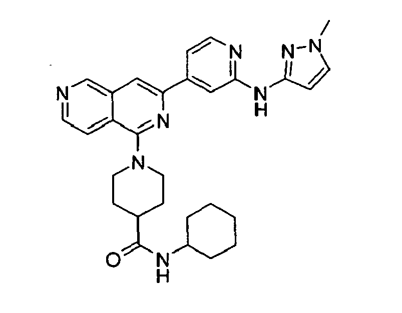

The title compound can be prepared from amide **Example 7B** by analogy to the method outlined in **Example 6AS:** MS (ESI) m/z 511.4 (M+1); ¹H NMR (400 MHz, *DMSO-d*₆) δ ppm 9.38 (s, 1 H), 9.32 (s, 1 H), 8.63 (d, *J*=5.8 Hz, 1 H), 8.18 - 8.25 (m, 2 H), 8.09 (s, 1 H), 7.87 (d, *J*=5.8 Hz, 1 H), 7.70 (d, *J*=7.8 Hz, 1 H), 7.53 (d, *J*=2.3 Hz, 1 H), 7.42 (d, *J*=6.8 Hz, 1 H), 6.30 (d, *J*=2.0 Hz, 1 H), 4.08 (d, *J*=12.9 Hz, 2 H), 3.77 (s, 3 H), 3.49 - 3.62 (m, 1 H), 2.99-3.19 (m, 2 H), 2.37 - 2.46 (m, 1 H), 1.80 - 2.05 (m, 4 H), 1.63 - 1.79 (m, 4 H), 1.51 - 1.62 (m, 1 H), 1.08 - 1.35 (m, 5 H).

### BL. 1-{3-[2-(Tetrahydropyran-4-ylamino)pyridin-4-yl]-[2,6]naphthyridin-1-yl}piperidine-4-carboxylic acid cyclopropylamide.

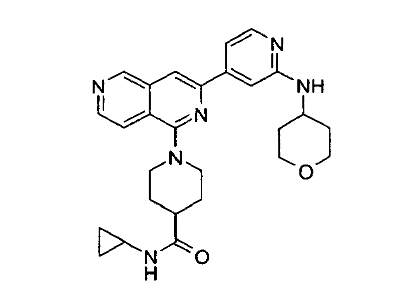

The title compound can be prepared from amide **Example 5D:** MS (ESI) *m*/*z* 473.1 (M+1); ¹H NMR (400 MHz, CDCl₃) δ ppm 9.26 (s, 1 H), 8.62 (d, *J* = 5.8 Hz, 1 H), 8.20 (d, *J* = 5.6 Hz, 1 H), 7.78 (d, *J* = 5.8 Hz, 1 H), 7.75 (s, 1 H), 7.26 - 7.21 (m, 1 H), 7.19 (s, 1H), 5.63 (br s, 1 H), 4.48 (d, *J* = 8.1 Hz, 1 H), 4.14 - 3.98 (m, 5 H), 3.67 - 3.53 (m, 2 H), 3.17 - 3.03 (m, 2 H), 2.85 - 2.71 (m, 1 H), 2.40 - 2.27 (m, 1 H), 2.19 - 1.96 (m, 6 H), 1.66 - 1.57 (m, 2 H), 0.90 - 0.74 (m, 2 H), 0.57 - 0.47 (m, 2 H).

### BM. 1-[3-(2-Cyclohexylaminopyridin-4-yl)-[2,6]naphthyridin-1-yl]-piperidine-4-carboxylic acid phenylamide.

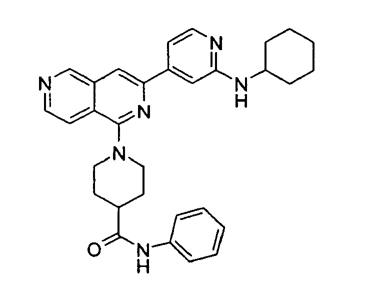

The title compound can be prepared from amide **Example 7C** by analogy to the method outlined in **Example 6AS:** MS (ESI) m/z 507.3 (M+1); ¹H NMR (400 MHz, *MeOD*) δ ppm 9.28 (s, 1 H), 8.57 (d, *J*=5.8 Hz, 1 H), 7.93 - 8.05 (m, 3 H), 7.58 (d, *J*=8.6 Hz, 2 H), 7.39 (s, 1 H), 7.31 (app t, *J*=8.0 Hz, 2 H), 7.26 (dd, *J*=5.6, 1.5 Hz, 1 H), 7.03 - 7.14 (m, 1 H), 4.10 - 4.24 (m, 2 H), 3.62 - 3.77 (m, 1 H), 3.15 - 3.25 (m, 2 H), 2.66 - 2.77 (m, 1 H), 2.12 - 2.27 (m, 2 H), 2.00 - 2.12 (m, 4 H), 1.75 - 1.85 (m, 2 H), 1.62 - 1.72 (m, 1 H), 1.39 - 1.53 (m, 2 H), 1.20 - 1.35 (m, 3 H).

### BN. 1-{3-[2-(1-Methyl-1H-pyrazol-3-ylamino)pyridin-4-yl]-[2,6]naphthyridin-1-yl}piperidine-4-carboxylic acid diethylamide.

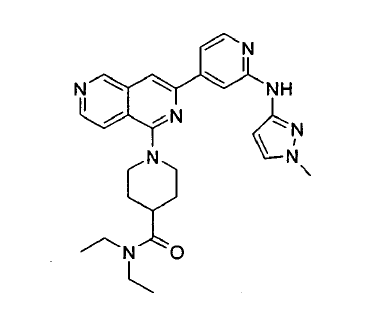

The title compound can be prepared from amide **Example 5E:** MS (ESI) *m*/*z* 485.2 (M+1); ¹H NMR (400 MHz, CDCl₃) δ ppm 9.27 (s, 1 H), 8.62 (d, *J* = 5.7 Hz, 1 H), 8.30 (d, *J* = 5.9 Hz, 1 H), 7.81 (d, *J* = 5.8 Hz, 1 H), 7.80 (s, 1 H), 7.46 (dd, *J* = 5.4, 1.5 Hz, 1 H), 7.29 (d, *J* = 2.3 Hz, 1 H), 6.89 (s, 1 H), 6.30 (d, *J* = 2.3 Hz, 1 H), 4.06 - 4.18 (m, 2 H), 3.86 (s, 3 H), 3.52 - 3.36 (m, 4 H), 3.18 - 3.07 (m, 2 H), 2.80 - 2.67 (m, 1 H), 2.33 - 2.16 (m, 2 H), 1.96 - 1.85 (m, 2 H), 1.26 (t, *J* = 7.1 Hz, 3 H), 1.16 (t, *J* = 7.1 Hz, 3 H).

### BO. (1-{3-[2-(1-Methyl-1H-pyrazol-3-ylamino)pyridin-4-yl]-[2,6]naphthyridin-1-yl}piperidin-4-yl)pyrrolidin-1-ylmethanone.

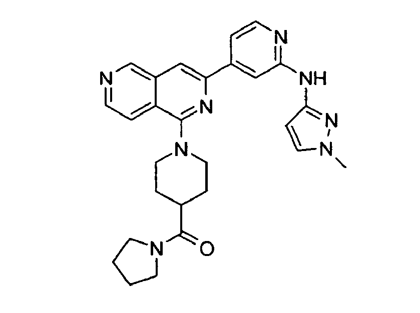

The title compound can be prepared from amide **Example 5F:** MS (ESI) *m*/*z* 483.2 (M+1); ¹H NMR (400 MHz, CDCl₃) δ ppm 9.27 (s, 1 H), 8.62 (d, *J* = 5.8 Hz, 1 H), 8.30 (d, *J* = 5.9

Hz, 1 H), 8.00 - 7.96 (m, 1 H), 7.81 (d, *J* = 6.7 Hz, 1 H), 7.79 (s, 1 H), 7.46 (dd, *J* = 5.3, 1.5 Hz, 1 H), 7.29 (d, *J* = 2.3 Hz, 1 H), 6.91 (s, 1 H), 6.30 (d, *J* = 2.3 Hz, 1 H), 4.07 - 4.17 (m, 2 H), 3.86 (s, 3 H), 3.61 - 3.48 (m, 4 H), 3.18 - 3.06 (m, 2 H), 2.72 - 2.60 (m, 1 H), 2.30 - 2.15 (m, 2 H), 2.07 - 1.85 (m, 6 H).

### BP. 1-{3-[2-(Tetrahydropyran-4-ylamino)pyridin-4-yl]-[2,6]naphthyridin-1-yl}piperidine-4-carboxylic acid (2-methoxyethyl)amide.

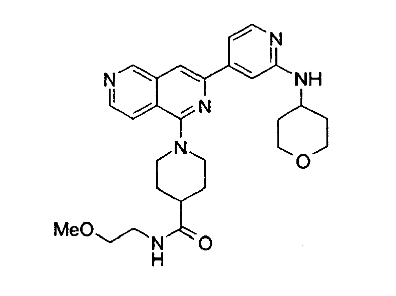

The title compound is prepared from **Example 5G:** MS (ESI) 491.1 *m*/*z* (M+1); ¹H NMR (400 MHz, MeOD) (TFA salt) δ ppm 9.37 (s, 1 H), 8.70 (d, *J* = 5.8 Hz, 1 H), 8.23 (s, 1 H), 8.03 (d, *J* = 6.1 Hz, 1 H), 8.00 (s, 1 H), 7.93 (d, *J* = 7.1 Hz, 1 H), 7.67 (d, *J* = 8.6 Hz, 1 H), 4.23 (d, *J* = 12.9 Hz, 2 H), 4.11 - 4.03 (m, 2 H), 3.97 - 3.85 (m, 1 H), 3.66 - 3.55 (m, 2 H), 3.53 - 3.47 (m, 2 H), 3.44 - 3.40 (m, 2 H), 3.39 (s, 3 H), 3.28 - 3.18 (m, 2 H), 2.66 - 2.52 (m, 1 H), 2.18 - 2.03 (m, 4 H), 2.01 - 1.89 (m, 2 H), 1.81 - 1.64 (m, 2 H).

### BQ. 1-{3-[2-(Tetrahydropyran-4-ylamino)pyridin-4-yl]-[2,6]naphthyridin-1-yl}piperidine-4-carboxylic acid (2-tert-butoxyethyl)amide.

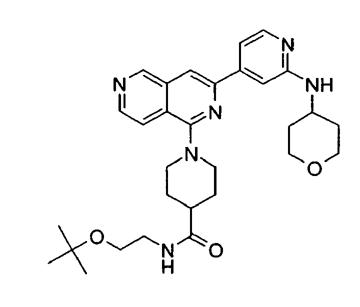

The title compound is prepared from **Example 5H:** MS (ESI) *m*/*z* 533.2 (M+1); ¹H NMR (400 MHz, CDCl₃) δ ppm 9.27 (s, 1 H), 8.63 (d, *J* = 5.8 Hz, 1 H), 8.20 (d, *J* = 5.3 Hz, 1 H), 7.79 (d, *J* = 5.8 Hz, 1 H), 7.76 (s, 1 H), 7.25 - 7.22 (m, 1 H), 7.20 (s, 1 H), 6.01 (br s, 1 H), 4.54 - 4.46 (m, 1 H), 4.15 - 3.97 (m, 5 H), 3.65 - 3.54 (m, 2 H), 3.49 - 3.44 (m, 4 H), 3.19 - 3.07 (m, 2 H), 2.49 - 2.34 (m, 1 H), 2.18 - 1.97 (m, 6 H), 1.21 (s, 9 H).

### BR. 1-{3-[2-(Tetrahydropyran-4-ylamino)pyridin-4-yl]-[2,6]naphthyridin-1-yl}piperidine-4-carboxylic acid (2-hydroxyethyl)amide.

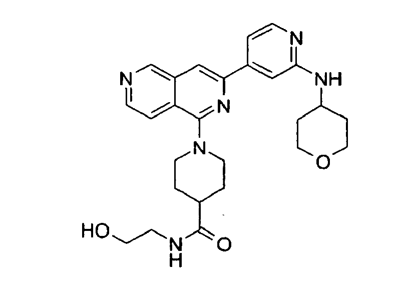

The title compound can be prepared from amide **Example 5H** by coupling to 4-aminotetrahydropyran, followed by acidic deprotection (TFA in CH₂Cl₂) of the *tert*-butyl ether to afford the title alcohol : MS (ESI) *m*/*z* 477.1 (M+1); ¹H NMR (400 MHz, CDCl₃) δ ppm 9.29 (s, 1 H), 8.65 (d, *J* = 5.6 Hz, 1 H), 8.22 - 8.15 (m, 1 H), 7.83 - 7.76 (m, 2 H), 7.27 - 7.24 (m, 2 H), 6.08 - 5.99 (m, 1 H), 4.17 - 3.98 (m, 5 H), 3.84 - 3.76 (m, 2 H), 3.67 - 3.57 (m, 2 H), 3.56 - 3.47 (m, 2 H), 3.20 - 3.09 (m, 2 H), 2.53 - 2.41 (m, 1 H), 2.39 - 2.26 (m, 1 H), 2.23 - 2.02 (m, 7 H), 1.70 - 1.60 (m, 2 H).

### BS. 1-[3-(2-Cyclohexylaminopyridin-4-yl)-[2,6]naphthyridin-1-yl]-piperidine-4-carboxylic acid (tetrahydropyran-4-yl)-amide.

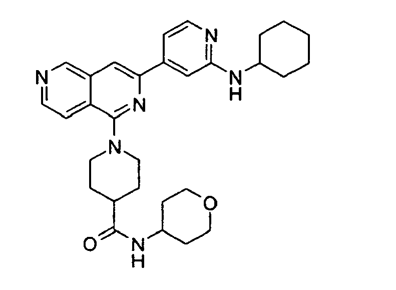

The title compound is prepared from **Example 7D:** MS (ESI) m/z 515.4 (M+1); ¹H NMR (400 MHz, *MeOD*) δ ppm 9.30 (s, 1 H), 8.59 (d, *J*=5.8 Hz, 1 H), 8.03 (d, *J*=5.6 Hz, 1 H), 8.00

(s, 1 H), 7.97 (d, *J*=5.8 Hz, 1 H), 7.40 (s, 1 H), 7.28 (dd, *J*=5.6, 1.5 Hz, 1 H), 4.10 - 4.22 (m, 2 H), 3.88 - 4.03 (m, 3 H), 3.65 - 3.78 (m, 1 H), 3.45 - 3.57 (m, 2 H), 3.11 - 3.24 (m, 2 H), 2.46 - 2.61 (m, 1 H), 2.04 - 2.20 (m, 4 H), 1.92 - 2.01 (m, 2 H), 1.79 - 1.92 (m, 4 H), 1.67 - 1.77 (m, 1 H), 1.42 - 1.64 (m, 4 H), 1.23 - 1.39 (m, 3 H).

### BT. (3'R)-1-{3-[2-(1-Methyl-1H-pyrazol-3-ylamino)pyridin-4-yl]-[2,6]naphthyridin-1-yl}piperidine-4-carboxylic acid (tetrahydrofuran-3-yl)amide.

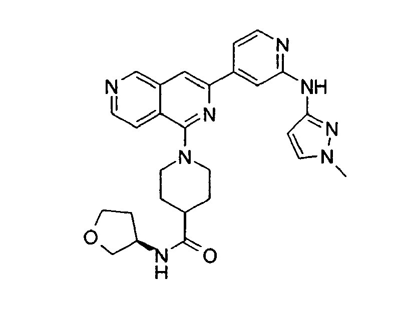

The title compound can be prepared from amide **Example 5I:** MS (ESI) *m*/*z* 499.1 (M+1); ¹H NMR (400 MHz, CDCl₃) δ ppm 9.27 (s, 1 H), 8.62 (d, *J* = 5.8 Hz, 1 H), 8.30 (d, *J* = 5.3 Hz, 1 H), 7.99 (s, 1 H), 7.81 (s, 1 H), 7.78 (d, *J* = 5.8 Hz, 1 H), 7.48 - 7.41 (m, 1 H), 7.30 (d, *J* = 2.3 Hz, 1 H), 6.89 (s, 1 H), 6.29 (d, *J* = 2.3 Hz, 1 H), 5.78 - 5.67 (m, 1 H), 4.66 - 4.52 (m, 1 H), 4.16 - 4.06 (m, 2 H), 4.04 - 3.93 (m, 1 H), 3.86 (s, 3 H), 3.86 - 3.77 (m, 2 H), 3.71 (dd, *J* = 9.6, 2.3 Hz, 1 H), 3.17 - 3.07 (m, 2 H), 2.44 - 2.26 (m, 2 H), 2.17 - 1.98 (m, 4 H), 1.88 - 1.78 (m, 1 H).

### BU. {1-[3-(2-Cyclohexylaminopyridin-4-yl)-[2,6]naphthyridin-1-yl]-piperidin-4-yl}-morpholin-4-yl-methanone.

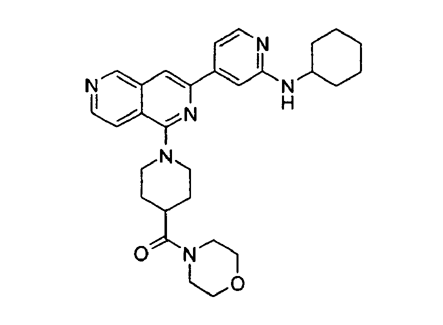

The title compound is prepared from **Example 7E:** MS (ESI) m/z 501.3 (M+1); ¹H NMR (400 MHz, *MeOD*) δ ppm 9.27 (s, 1 H), 8.56 (d, *J*=6.1 Hz, 1 H), 7.99 (d, *J*=5.6 Hz, 1 H), 7.97 (s, 1 H), 7.93 (d, *J*=5.8 Hz, 1 H), 7.36 (s, 1 H), 7.24 (dd, *J*=5.6, 1.5 Hz, 1 H), 4.06 - 4.17 (m, 2 H), 3.58 - 3.75 (m, 9 H), 3.15 - 3.25 (m, 2 H), 2.95 - 3.06 (m, 1 H), 2.02 - 2.18 (m, 4 H), 1.85 - 1.95 (m, 2 H), 1.76 - 1.85 (m, 2 H), 1.64 - 1.74 (m, 1 H), 1.40 - 1.54 (m, 2 H), 1.21 - 1.37 (m, 3 H).

### BV. {1-[3-(2-Cyclohexylaminopyridin-4-yl)-[2,6]naphthyridin-1-yl]-piperidin-4-yl}-(4-hydroxypiperidin-1-yl)-methanone.

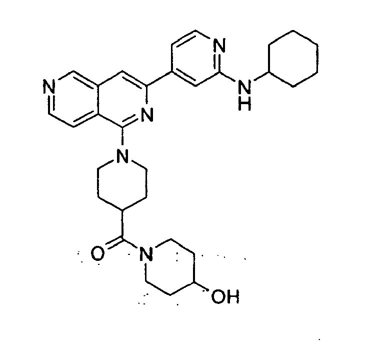

The title compound is prepared from **Example 7F:** MS (ESI) m/z 515.4 (M+1); ¹H NMR (400 MHz, *MeOD*) δ ppm 9.27 (s, 1 H), 8.56 (d, *J*=5.8 Hz, 1 H), 7.90 - 8.02 (m, 3 H), 7.37 (s, 1 H), 7.25 (d, *J*=5.6 Hz, 1 H), 4.07 - 4.18 (m, 2 H), 3.93 - 4.02 (m, 1 H), 3.83 - 3.92 (m, 1 H), 3.63 - 3.75 (m, 1 H), 3.34 - 3.45 (m, 1 H), 3.10 - 3.27 (m, 3 H), 2.98 - 3.09 (m, 1 H), 1.96 - 2.17 (m, 4 H), 1.76 - 2.00 (m, 6 H), 1.62 - 1.75 (m, 1 H), 1.38 - 1.59 (m, 4 H), 1.21 - 1.36 (m, 4 H).

### BW. 1-{3-[2-(Tetrahydropyran-4-ylamino)pyridin-4-yl]-[2,6]naphthyridin-1-yl}piperidine-4-carboxylic acid (2-aminoethyl)amide.

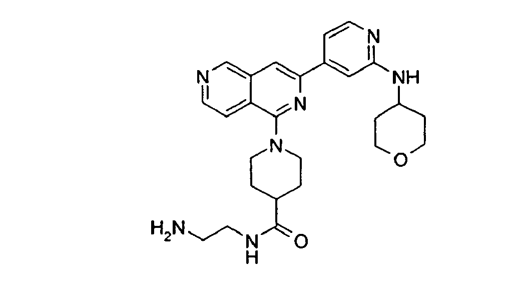

The title compound is prepared from **Example 5J:** MS (ESI) *m*/*z* (M+1); ¹H NMR (400 MHz, MeOD) (TFA salt) δ ppm 9.38 (s, 1 H), 8.69 (d, *J* = 6.1 Hz, 1 H), 8.25 (s, 1 H), 8.01 (d, *J* = 5.8 Hz, 1 H), 7.98 (s, 1 H), 7.93 (d, *J* = 6.8 Hz, 1 H), 7.67 (d, *J* = 8.3 Hz, 1 H), 4.27 - 4.17 (m, 1 H), 4.11 - 4.01 (m, 2 H), 3.97 - 3.85 (m, 1 H), 3.65 - 3.55 (m, 2 H), 3.50 (t, *J* = 6.3 Hz, 3 H), 3.26 - 3.14 (m, 2 H), 3.13 - 3.04 (m, 2 H), 2.66 - 2.51 (m, 1 H), 2.16 - 1.99 (m, 6 H), 1.82 - 1.63 (m, 2 H).

### BX. 1-{3-[2-(Tetrahydropyran-4-ylamino)pyridin-4-yl]-[2,6]naphthyridin-1-yl}piperidine-4-carboxylic acid (2-dimethylaminoethyl)amide.

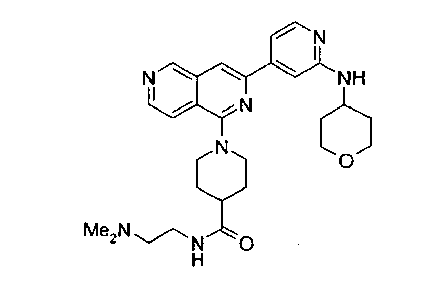

The title compound can be prepared from amide **Example 5K:** MS (ESI) *m*/*z* 504.2 (M+1); ¹H NMR (400 MHz, MeOD) (TFA salt) δ ppm 9.37 (s, 1 H), 8.68 (d, *J*=5.8 Hz, 1 H), 8.24 (s, 1 H), 8.03 - 7.98 (m, 1 H), 7.97 (s, 1 H), 7.92 (d, *J* = 6.8 Hz, 1 H), 7.66 (d, *J* = 8.6 Hz, 1 H), 4.20 (d, *J* = 12.1 Hz, 2 H), 4.05 (dd, *J* = 12.3, 3.7 Hz, 2 H), 3.95 - 3.84 (m, 1 H), 3.65 - 3.52 (m, 4 H), 3.25 - 3.10 (m, 3 H), 2.96 (s, 6 H), 2.66 - 2.47 (m, 1 H), 2.17 - 1.96 (m, 6 H), 1.80 - 1.64 (m, 2 H), 1.43 - 1.30 (m, 1 H).

### BY. 1-{3-[2-(Tetrahydropyran-4-ylamino)-pyridin-4-yl]-[2,6]naphthyridin-1-yl}-piperidine-4-carboxylic acid (2-pyrrolidin-1-yl-ethyl)-amide.

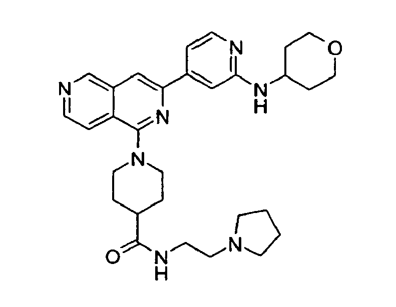

The title compound can be prepared from amide **Example 5L:** MS (ESI) m/z 530.4 (M+1); ¹H NMR (400 MHz, *DMSO-d*₆) δ ppm 9.29 (s, 1 H), 8.55 (d, *J*=5.8 Hz, 1 H), 8.00 (d, *J*=5.6 Hz, 1 H), 7.98 (s, 1 H), 7.71 - 7.84 (m, 2 H), 7.25 (s, 1 H), 7.11 (d, *J*=5.6 Hz, 1 H), 6.59 (d, *J*=7.6 Hz, 1 H), 3.87 - 4.01 (m, 3 H), 3.78 - 3.87 (m, 2 H), 3.30 - 3.41 (m, 2 H), 3.12 (d, *J*=19.2 Hz, 2 H), 2.99 (d, *J*=23.2 Hz, 2 H), 1.74 - 1.92 (m, 8 H), 1.60 (br. s., 4 H), 1.28 - 1.49 (m, 6 H).

### BZ. 1-[3-(2-Cyclohexylaminopyridin-4-yl)-[2,6]naphthyridin-1-yl]-piperidine-4-carboxylic acid (2-pyrrolidin-1-yl-ethyl)-amide.

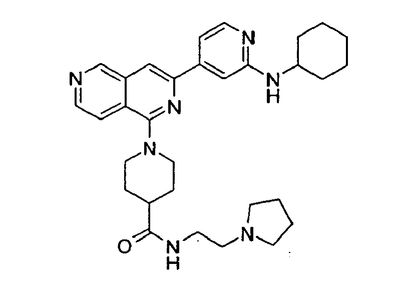

The title compound can be prepared from amide **Example 5L:** MS (ESI) m/z 528.4 (M+1); ¹H NMR (400 MHz, *DMSO-d*₆) δ ppm 9.36 (s, 1 H), 8.62 (d, *J*=5.3 Hz, 1 H), 7.99 - 8.08 (m, 2 H), 7.77 - 7.90 (m, 2 H), 7.29 (s, 1 H), 7.14 (d, *J*=5.8 Hz, 1 H), 6.51 (d, *J*=7.6 Hz, 1 H), 3.96 - 4.07 (m, 2 H), 3.74 (br. s., 1 H), 3.13 - 3.24 (m, 2 H), 2.97 - 3.13 (m, 2 H), 1.81 - 2.04 (m, 8 H), 1.56 - 1.79 (m, 8 H), 1.10 - 1.42 (m, 8 H).

**CA. 1-{3-[2-(Tetrahydropyran-4-ylamino)-pyridin-4-yl]-[2,6]naphthyridin-1-yl}-piperidine-4-carboxylic acid methyl-(2-pyrrolidin-1-yl-ethyl)-amide.** 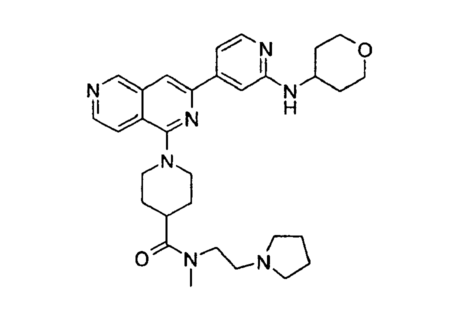 The title compound can be prepared from amide **Example 5M:** MS (ESI) m/z 544.4 (M+1); ¹H NMR (400 MHz, *DMSO-d*₆) δ ppm 9.36 (s, 1 H), 8.62 (d, *J*=5.8 Hz, 1 H), 8.07 (d, *J*=5.6 Hz, 1 H), 8.04 (s, 1 H), 7.86 (d, *J*=5.8 Hz, 1 H), 7.32 (s, 1 H), 7.18 (dd, *J*=5.6, 1.5 Hz, 1 H), 6.65 (d, *J*=7.3 Hz, 1 H), 3.93 - 4.09 (m, 3 H), 3.82 - 3.91 (m, 2 H), 3.46 - 3.55 (m, 1 H), 3.37 - 3.46 (m, 3 H), 3.11 - 3.19 (m, 2 H), 3.09 (s, 3 H), 2.89 - 2.97 (m, 1 H), 2.87 (s, 2 H), 2.57 - 2.64 (m, 1 H), 2.36 - 2.48 (m, 2 H), 1.85 - 2.03 (m, 4 H), 1.73 - 1.85 (m, 2 H), 1.60 - 1.74 (m, 4 H), 1.37 - 1.54 (m, 2 H).

### CB. 1-{3-[2-(Tetrahydropyran-4-ylamino)-pyridin-4-yl]-[2,6]naphthyridin-1-yl}-piperidine-4-carboxylic acid (2-morpholin-4-yl-ethyl)-amide.

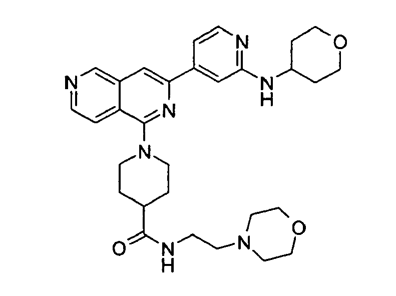

The title compound can be prepared from amide **Example 5N:** MS (ESI) m/z 546.3 (M+1); ¹H NMR (400 MHz, *DMSO-d*₆) δ ppm 9.35 (s, 1 H), 8.62 (d, *J*=5.8 Hz, 1 H), 8.07 (d, *J*=5.4 Hz, 1 H), 8.04 (s, 1 H), 7.85 (d, *J*=5.8 Hz, 2 H), 7.81 (br. s., 1 H), 7.31 (s, 1 H), 7.18 (dd, *J*=5.4, 1.4 Hz, 1 H), 6.58 - 6.75 (m, 1 H), 3.93 - 4.08 (m, 3 H), 3.83 - 3.93 (m, 2 H), 3.57 (br. s., 4 H), 3.34 - 3.48 (m, 2 H), 3.15 - 3.25 (m, 2 H), 3.01 - 3.12 (m, 2 H), 2.29 - 2.48 (m, 6 H), 1.77 - 2.01 (m, 6 H), 1.36 - 1.52 (m, 2 H).

### CC. 1-[3-(2-Cyclohexylaminopyridin-4-yl)-[2,6]naphthyridin-1-yl]-piperidine-4-carboxylic acid (2-morpholin-4-yl-ethyl)-amide.

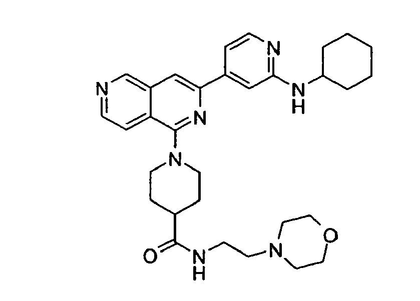

The title compound can be prepared from amide **Example 5N:** MS (ESI) m/z 544.4 (M+1); ¹H NMR (400 MHz, *DMSO-d*₆) δ ppm 9.36 (s, 1 H), 8.62 (d, *J*=5.8 Hz, 1 H), 8.06 (d, *J*=5.4 Hz, 1 H), 8.03 (s, 1 H), 7.86 (d, *J*=5.8 Hz, 1 H), 7.80 (t, *J*=5.7 Hz, 1 H), 7.29 (s, 1 H), 7.15 (dd, *J*=5.4, 1.4 Hz, 1 H), 6.51 (d, *J*=7.7 Hz, 1 H), 4.02 (d, *J*=12_{.}9 Hz, 2 H), 3.68 - 3.82 (m, 1 H), 3.53 - 3.60 (m, 5 H), 3.16 - 3.24 (m, 3 H), 3.00 - 3.14 (m, 2 H), 1.82 - 2.01 (m, 8 H), 1.69 - 1.78 (m, 3 H), 1.53 - 1.66 (m, 1 H), 1.09 - 1.41 (m, 7 H).

### CD. 1-{3-[2-(Tetrahydropyran-4-ylamino)-pyridin-4-yl]-[2,6]naphthyridin-1-yl}-piperidine-4-carboxylic acid (1,1-dimethyl-2-pyrrolidin-1-yl-ethyl)-amide.

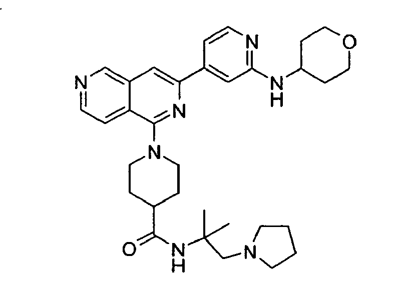

The title compound can be prepared from amide **Example 7G:** MS (ESI) m/z 558.4 (M+1); ¹H NMR (400 MHz, *DMSO-d*₆) δ ppm 9.36 (s, 1 H), 8.62 (d, *J*=5.8 Hz, 1 H), 8.07 (d, *J*=5.3 Hz, 1 H), 8.04 (s, 1 H), 7.85 (d, *J*=5.8 Hz, 1 H), 7.31 (s, 1 H), 7.26 (s, 1 H), 7.18 (dd, *J*=5.3, 1.5 Hz, 1 H), 6.65 (d, 1 H), 3.93 - 4.09 (m, 3 H), 3.84 - 3.94 (m, 2 H), 3.36 - 3.48 (m, 2 H), 2.96 - 3.09 (m, 2 H), 2.68 (s, 2 H), 2.52 - 2.59 (m, 4 H), 1.75 - 1.98 (m, 7 H), 1.60 - 1.71 (m, 4 H), 1.36 - 1.54 (m, 2 H), 1.24 (s, 6 H).

### CE. 1-{3-[2-(Tetrahydropyran-4-ylamino)pyridin-4-yl]-[2,6]naphthyridin-1-yl}-piperidine-4-carboxylic acid (2-methyl-2-piperidin-1-yl-propyl)-amide.

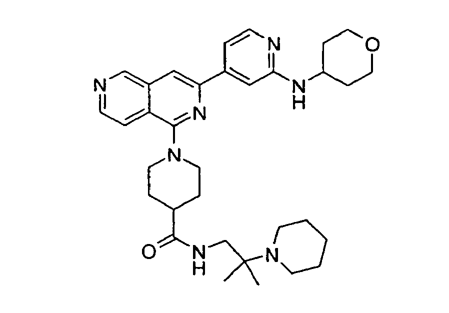

The title compound can be prepared from amide **Example 50:** MS (ESI) m/z 572.4 (M+1); ¹H NMR (400 MHz, *MeOD*) δ ppm 9.28 (s, 1 H), 8.57 (d, *J*=5.8 Hz, 1 H), 8.03 (d, *J*=5.6 Hz, 1 H), 7.99 (s, 1 H), 7.95 (d, *J*=6.1 Hz, 1 H), 7.41 (s, 1 H), 7.29 (d, *J*=5.6 Hz, 1 H), 4.10 - 4.20 (m, 2 H), 3.91 - 4.05 (m, 3 H), 3.52 - 3.63 (m, 2 H), 3.26 (s, 2 H), 3.13 - 3.23 (m, 2 H), 2.53 - 2.68 (m, 5 H), 1.91 - 2.18 (m, 6 H), 1.52 - 1.69 (m, 6 H), 1.39 - 1.51 (m, 2 H), 1.07 (s, 6 H).

### CF. (4-Pyrrolidin-1-yl-piperidin-1-yl)-(1-{3-[2-(tetrahydropyran-4-ylamino)-pyridin-4-yl]-[2,6]naphthyridin-1-yl}-piperidin-4-yl)-methanone.

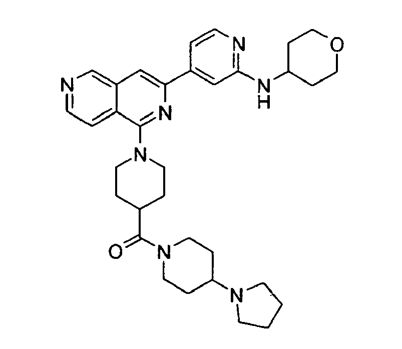

The title compound can be prepared from amide **Example 5P:** MS (ESI) m/z 570.4 (M+1); ¹H NMR (400 MHz, *DMSO-d*₆) δ ppm 9.36 (s, 1 H), 8.62 (d, *J*=5.8 Hz, 1 H), 8.07 (d, *J*=5.4 Hz, 1 H), 8.05 (s, 1 H), 7.87 (d, *J*=5.8 Hz, 1 H), 7.32 (s, 1 H), 7.18 (dd, *J*=5.4, 1.5 Hz, 1 H), 6.66 (d, *J*=7.6 Hz, 1 H), 4.16 - 4.28 (m, 1 H), 3.93 - 4.06 (m, 4 H), 3.84 - 3.92 (m, 2 H), 3.37 - 3.49 (m, 2 H), 3.08 - 3.21 (m, 4 H), 2.90 - 3.04 (m, 1 H), 2.68 - 2.84 (m, 1 H), 2.14 - 2.31 (m, 1 H), 1.75 - 2.03 (m, 9 H), 1.67 (br. s., 4 H), 1.40 - 1.55 (m, 3 H), 1.19 - 1.41 (m, 2 H).

### CG. 1-{3-[2-(Tetrahydropyran-4-ylamino)pyridin-4-yl]-[2,6]naphthyridin-1-yl}-piperidine-4-carboxylic acid (3-pyrrolidin-1-yl-propyl)-amide.

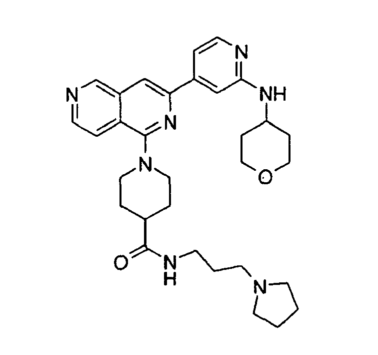

The title compound can be prepared from amide **Example 5Q:** MS (ESI) m/z 544.4 (M+1); ¹H NMR (400 MHz, *DMSO-d*₆) δ ppm 9.36 (s, 1 H), 8.62 (d, *J*=5.8 Hz, 1 H), 8.07 (d, *J*=5.3 Hz, 1 H), 8.04 (s, 1 H), 7.82 - 7.91 (m, 2 H), 7.32 (s, 1 H), 7.18 (dd, *J*=5.4, 1.4 Hz, 1 H), 6.65 (d, *J*=7.7 Hz, 1 H), 3.93 - 4.09 (m, 3 H), 3.82 - 3.92 (m, 2 H), 3.38 - 3.49 (m, 2 H), 2.99 - 3.16 (m, 4 H), 2.33 - 2.47 (m, 7 H), 1.80 - 1.99 (m, 6 H), 1.63 - 1.70 (m, 4 H), 1.53 - 1.63 (m, 2 H), 1.38 - 1.53 (m, 2 H).

### CH. 1-{3-[2-(Tetrahydropyran-4-ylamino)pyridin-4-yl]-[2,6]naphthyridin-1-yl}piperidine-4-carboxylic acid (3-imidazol-1-ylpropyl)amide.

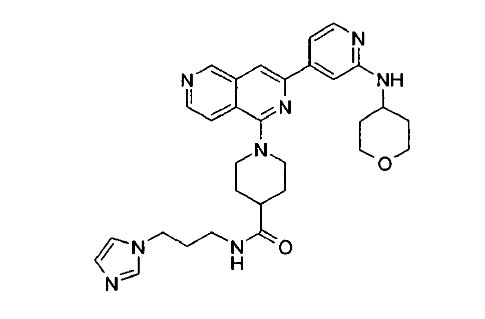

The title compound can be prepared from amide **Example 5R:** MS (ESI) *m*/*z* 541.2 (M+1); ¹H NMR (400 MHz, CDCl₃) δ ppm 9.26 (s, 1 H), 8.62 (d, *J* = 5.8 Hz, 1 H), 8.20 (d, *J* = 5.3 Hz, 1 H), 7.79 - 7.75 (m, 1 H), 7.76 (s, 1 H), 7.52 (s, 1 H), 7.25 - 7.21 (m, 1 H), 7.21 (s, 1 H), 7.10 (s, 1 H), 6.97 (s, 1 H), 5.49 - 5.41 (m, 1 H), 4.60 (d, *J* = 8.1 Hz, 1 H), 4.13 - 3.95 (m, 7 H), 3.65 - 3.52 (m, 2 H), 3.41 - 3.30 (m, 2 H), 3.18 - 3.05 (m, 2 H), 2.41 - 2.27 (m, 1 H), 2.15 - 1.93 (m, 8 H), 1.64 -1.57 (m, 2 H).

### CI. Piperazin-1-yl-(1-{3-[2-(tetrahydropyran-4-ylamino)-pyridin-4-yl]-[2,6]naphthyridin-1-yl}-piperidin-4-yl)-methanone.

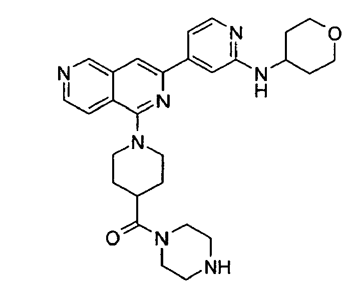

The title compound can be prepared from by coupling amide **Example 7H** to 4-aminotetrahydropyran, followed by removal of the BOC protecting group using TFA/CH₂Cl₂: MS (ESI) m/z 502.3 (M+1); ¹H NMR (400 MHz, *DMSO*-*d*₆) δ ppm 9.37 (s, 1 H), 8.62 (d, *J*=5.8 Hz, 1 H), 8.08 (d, *J*=5.3 Hz, 1 H), 8.05 (s, 1 H), 7.88 (s, 1 H), 7.31 (s, 1 H), 7.19 (dd, *J*=5.4, 1.4 Hz, 1 H), 6.65 (d, *J*=7.6 Hz, 1 H), 3.93 - 4.07 (m, 3 H), 3.83 - 3.93 (m, 2 H), 3.55 - 3.74 (m, 4 H), 3.37 - 3.48 (m, 2 H), 3.09 - 3.21 (m, 2 H), 2.90 - 3.04 (m, 5 H), 1.77 - 2.01 (m, 6 H), 1.39 - 1.53 (m, 2 H).

### CJ. (1-{3-[2-(1-Methyl-1H-pyrazol-3-ylamino)-pyridin-4-yl]-[2,6]naphthyridin-1-yl}-piperidin-4-yl)-piperazin-1-yl-methanone.

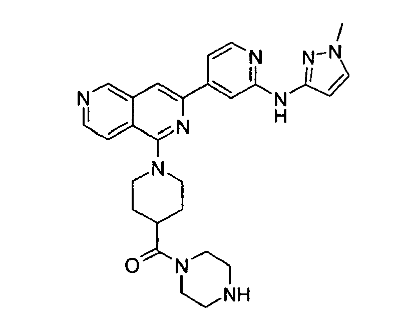

The title compound can be prepared from by coupling amide **Example 7H** to 1-methyl-1*H-*pyrazol-3-ylamine, followed by removal of the BOC protecting group using **TF**A/CH₂Cl₂: MS (ESI) m/z 498.3 (M+1); ¹H NMR (400 MHz, *DMSO-d*₆) δ 9.39 (s, 1 H), 9.32 (s, 1 H), 8.63 (d, *J*=5.6 Hz, 1 H), 8.25 (s, 1 H), 8.22 (d, *J*=5.3 Hz, 1 H), 8.10,(s, 1 H), 7.89 (d, *J*=5.8 Hz, 1 H), 7.53 (d, *J*=2.3 Hz, 1 H), 7.42 (dd, *J*=5.6, 1.5 Hz, 1 H), 6.30 (s, 1 H), 4.03 - 4.11 (m, 2 H), 3.77 (s, 3 H), 3.53 - 3.72 (m, 4 H), 3.12 - 3.23 (m, 2 H), 2.89 - 3.04 (m, 5 H), 1.77 - 2.02 (m, 4 H).

### CK. 1-{3-[2-(Tetrahydropyran-4-ylamino)-pyridin-4-yl]-[2,6]naphthyridin-1-yl}-piperidine-4-carboxylic acid (S)-pyrrolidin-3-ylamide.

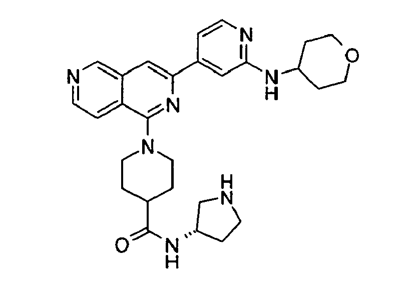

The title compound can be prepared from by coupling amide **Example 5V** to 4-aminotetrahydropyran, followed by removal of the BOC protecting group using TFA/CH₂CI₂: MS (ESI) m/z 502.3 (M+1); ¹H NMR (400 MHz, *DMSO-d*₆) δ 9.29 (s, 1 H), 8.55 (d, *J*=5.4 Hz, 1 H), 7.91 - 8.06 (m, 3 H), 7.79 (d, *J*=5.3 Hz, 1 H), 7.24 (s, 1 H), 7.12 (d, *J*=5.1 Hz, 1 H), 6.59 (d, *J*=7.3 Hz, 1 H), 4.04 - 4.18 (m, 1 H), 3.87 - 4.02 (m, 3 H), 3.76 - 3.87 (m, 2 H), 2.90 - 3.05 (m, 4 H), 2.79 - 2.88 (m, 1 H), 2.57 - 2.67 (m, 1 H), 2.30 - 2.40 (m, 2 H), 1.71 - 1.97 (m, 8 H), 1.49 - 1.62 (m, 1 H), 1.32 - 1.47 (m, 3 H).

### CL. 1-[3-(2-Cyclohexylaminopyridin-4-yl)-[2,6]naphthyridin-1-yl]-piperidine-4-carboxylic acid (S)-pyrrolidin-3-ylamide.

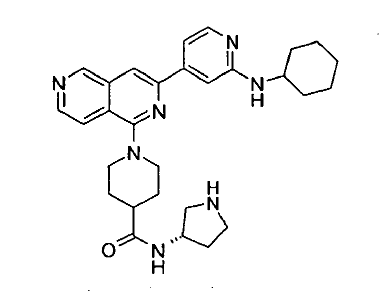

The title compound can be prepared from by coupling amide **Example 5V** to cyclohexylamine, followed by removal of the BOC protecting group using TFA/CH₂CI₂: MS (ESI) m/z 500.3 (M+1); ¹H NMR (400 MHz, *DMSO-d*₆) δ 9.35 (s, 1 H), 8.62 (d, *J*=5.8 Hz, 1 H), 8.05 (d, *J*=5.4 Hz, 1 H), 8.03 (s, 1 H), 7.85 (d, *J*=5.8 Hz, 1 H), 7.28 (s, 1 H), 7.14 (dd, *J*=5.4, 1.5 Hz, 1 H), 6.51 (d, *J*=7.7 Hz, 1 H), 4.07 - 4.27 (m, 1 H), 3.97 - 4.07 (m, 2 H), 3.66 - 3.81 (m, 1 H), 3.40 - 3.52 (m, 1 H), 2.97 - 3.13 (m, 3 H), 2.80 - 2.94 (m, 1 H), 2.64 - 2.77 (m, 1 H), 2.29 - 2.47 (m, 1 H), 1.80 - 2.08 (m, 7 H), 1.67 - 1.78 (m, 3 H), 1.57 - 1.64 (m, 1 H), 1.44 - 1.55 (m, 1 H), 1.28 - 1.41 (m, 2 H), 1.11 - 1.28 (m, 3 H).

### CM. 1-[3-(2-Cyclohexylaminopyridin-4-yl)-[2,6]naphthyridin-1-yl]-piperidine-4-carboxylic acid (R)-pyrrolidin-3-ylamide.

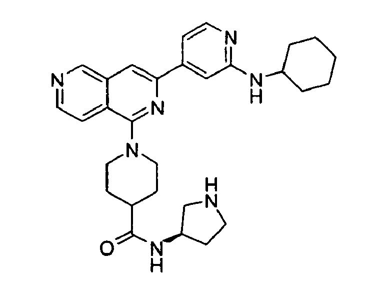

The title compound can be prepared from by coupling amide **Example 5W** to cyclohexylamine, followed by removal of the BOC protecting group using TFA/CH₂Cl₂: MS (ESI) m/z 500.3 (M+1); ¹H NMR (400 MHz, *DMSO*-*d*₆) δ ppm 9.38 (s, 1 H), 8.68 - 8.90 (m, 2 H), 8.64 (d, *J*=5.7 Hz, 1 H), 8.20 (d, *J*=6.3 Hz, 1 H), 8.10 (s, 1 H), 8.05 (d, *J*=5.7 Hz, 1 H), 7.86 (d, *J*=5.8 Hz, 1 H), 7.72 (br. s., 1 H), 7.40 (br. s., 1 H), 7.24 (d, *J*=4.7 Hz, 1 H), 4.23 - 4.35 (m, 1 H), 3.98 - 4.09 (m, 2 H), 3.63 - 3.80 (m, 1 H), 3.15 - 3.27 (m, 1 H), 3.04 - 3.14 (m, 2 H), 2.92 - 3.04 (m, 1 H), 2.32 - 2.48 (m, 1 H), 2.07 - 2.22 (m, 1 H), 1.81 - 2.03 (m, 7 H), 1.68 - 1.80 (m, 3 H), 1.54 - 1.66 (m, 1 H), 1.20 - 1.44 (m, 5 H).

### CN. 1-{3-[2-(Tetrahydropyran-4-ylamino)-pyridin-4-yl]-[2,6]naphthyridin-1-yl}-piperidine-4-carboxylic acid (R)-pyrrolidin-3-ylamide.

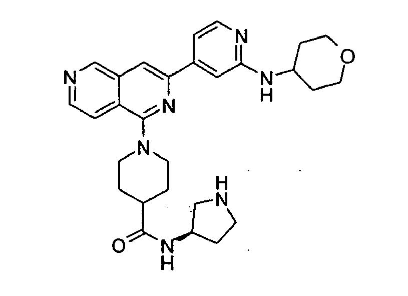

The title compound can be prepared from by coupling amide **Example 5W** to 4-aminotetrahydropyran, followed by removal of the BOC protecting group using TFA/CH₂Cl₂: MS (ESI) m/z 502.3 (M+1); ¹H NMR (400 MHz, *DMSO*-*d*₆) δ ppm 9.36 (s, 1 H), 8.62 (d, *J*=5.8 Hz, 1 H), 8.07 (d, *J*=5.4 Hz, 1 H), 8.04 (s, 1 H), 7.98 (d, *J*=7.1 Hz, 1 H), 7.86 (d, *J*=5.7 Hz, 1 H), 7.31 (s, 1 H), 7.18 (dd, *J*=5.5, 1.5 Hz, 1 H), 6.65 (d, *J*=7.6 Hz, 1 H), 4.11 - 4.26 (m, 1 H), 3.93 - 4.07 (m, 3 H), 3.82 - 3.94 (m, 2 H), 3.37 - 3.50 (m, 2 H), 2.96 - 3.13 (m, 4 H), 2.88 - 2.96 (m, 1 H), 2.75 - 2.88 (m, 1 H), 2.55 - 2.68 (m, 1 H), 2.35 - 2.47 (m, 1 H), 1.81 - 2.01 (m, 7 H), 1.52 - 1.63 (m, 1 H), 1.46 (d, *J*=39.5 Hz, 2 H).

### CO. 1-[3-(2-Cyclohexylaminopyridin-4-yl)-[2,6]naphthyridin-1-yl]-piperidine-4-carboxylic acid piperidin-4-ylamide.

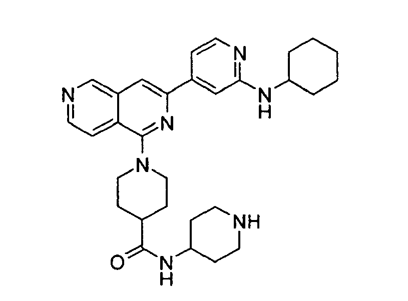

The title compound can be prepared from by coupling amide **Example 5X** to cyclohexylamine, followed by removal of the BOC protecting group using TFA/CH₂Cl₂: MS (ESI) m/z 514.3 (M+1); ¹H NMR (400 MHz, *DMSO*-*d*₆) δ ppm 9.36 (s, 1 H), 8.61 (d, *J*=5.8 Hz, 1 H), 8.05 (d, *J*=5.6 Hz, 1 H), 8.03 (s, 1 H), 7.86 (d, *J*=5.8 Hz, 1 H), 7.80 (d, *J*=7.8 Hz, 1 H), 7.28 (s, 1 H), 7.14 (dd, *J*=5.4, 1.5 Hz, 1 H), 6.50 (d, *J*=7.8 Hz, 1 H), 3.96 - 4.09 (m, 2 H), 3.66 - 3.82 (m, 1 H), 3.57 - 3.69 (m, 1 H), 2.92 - 3.11 (m, 4 H), 2.52 - 2.64 (m, 2 H), 2.34 - 2.46 (m, 1 H), 1.79 - 2.00 (m, 6 H), 1.66 - 1.78 (m, 4 H), 1.53 - 1.66 (m, 1 H), 1.12 - 1.40 (m, 8 H).

### CP. 1-{3-[2-(Tetrahydropyran-4-ylamino)-pyridin-4-yl]-[2,6]naphthyridin-1-yl}-piperidine-4-carboxylic acid piperidin-4-ylamide.

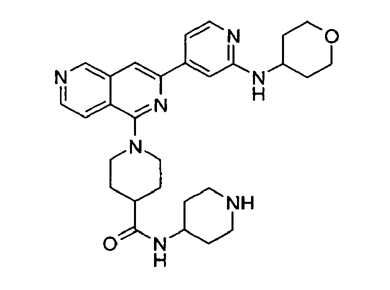

The title compound can be prepared from by coupling amide **Example 5X** to 4-aminotetrahydropyran, followed by removal of the BOC protecting group using TFA/CH₂Cl₂: MS (ESI) m/z 516.3 (M+1); ¹H NMR (400 MHz, *DMSO*-*d*₆) δ ppm 9.36 (s, 1 H), 8.62 (d, *J*=5.8 Hz, 1 H), 8.07 (d, *J*=5.4 Hz, 1 H), 8.04 (s, 1 H), 7.86 (d, *J*=5.8 Hz, 1 H), 7.74 (d, *J*=7.8 Hz, 1 H), 7.31 (s, 1 H), 7.18 (dd, *J*=5.4, 1.5 Hz, 1 H), 6.65 (d, *J*=7.6 Hz, 1 H), 3.92 - 4.09 (m, 3 H), 3.85 - 3.92 (m, 2 H), 3.53 - 3.65 (m, 1 H), 3.37 - 3.47 (m, 2 H), 2.99 - 3.12 (m, 2 H), 2.83 - 2.92 (m, 2 H), 2.33 - 2.47 (m, 3 H), 1.79 - 2.01 (m, 6 H), 1.62 - 1.70 (m, 2 H), 1.37 - 1.53 (m, 2 H), 1.17 - 1.30 (m, 2 H).

### CQ. ((S)-3-Aminopiperidin-1-yl)-(1-{3-[2-(tetrahydropyran-4-ylamino)-pyridin-4-yl]-[2,6]naphthyridin-1-yl}-piperidin-4-yl)-methanone.

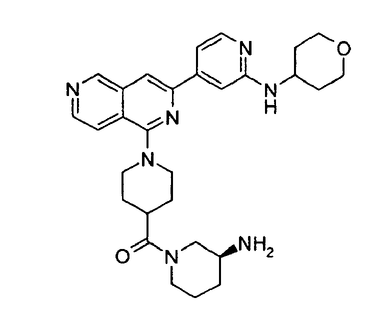

The title compound can be prepared from by coupling amide **Example 5Y** to 4-aminotetrahydropyran, followed by removal of the BOC protecting group using TFA/CH₂Cl₂: MS (ESI) m/z 516.3 (M+1); ¹H NMR (400 MHz, *DMSO-d*₆) δ ppm 9.36 (s, 1 H), 8.62 (d, *J*=5.8 Hz, 1 H), 8.07 (d, *J*=5.3 Hz, 1 H), 8.05 (s, 1 H), 7.87 (d, *J*=5.8 Hz, 1 H), 7.33 (s, 1 H), 7.19 (d, *J*=6.6 Hz, 1 H), 6.67 (d, *J*=7.6 Hz, 1 H), 3.94 - 4.14 (m, 4 H), 3.81 - 3.92 (m, 3 H), 3.39 - 3.49 (m, 2 H), 3.08 - 3.18 (m, 2 H), 2.88 - 3.04 (m, 2 H), 2.75 - 2.87 (m, 1 H), 2.59 - 2.75 (m, 1 H), 1.59 - 2.04 (m, 9 H), 1.38 - 1.57 (m, 3 H), 1.15 - 1.35 (m, 2 H).

### CR. (Z)-N-Isopropyl-2-methyl-3-{3-[2-(tetrahydropyran-4-ylamino)-pyridin-4-yl]-[2,6]naphthyridin-1-ylamino}-acrylamide.

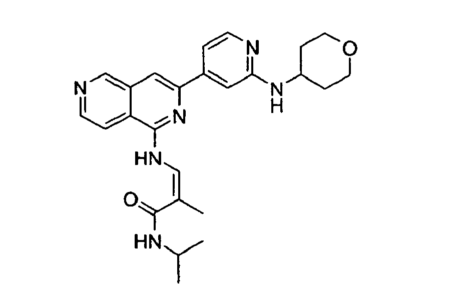

The title compound can be prepared from by coupling amide **Example 6** to 4-aminotetrahydropyran: MS (ESI) m/z 447.4 (M+1).

### CS. (4-{1-[4-(4,5-Dihydrooxazol-2-yl)piperidin-1-yl]-[2,6]naphthyridin-3-yl}pyridin-2-yl)-(1-methyl-1H-pyrazol-3-yl)amine.

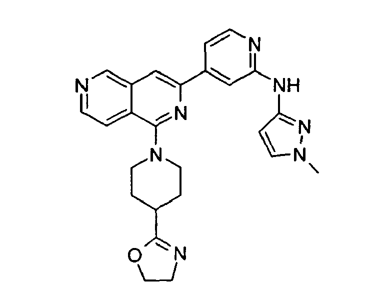

The title compound is prepared from 1-[3-(2-chloropyridin-4-yl)-[2,6]naphthyridin-1-yl]-piperidine-4-carboxylic acid (2-fluoroethyl)amide **Example 5S:** MS (ESI) *m*/*z* 455.1 (M+1); ¹H NMR (400 MHz, CDCl₃) δ ppm 9.27 (s, 1 H), 8.61 (d, *J* = 5.8 Hz, 1 H), 8.30 (d, *J* = 5.3 Hz, 1 H), 8.05 (s, 1 H), 7.80 (s, 1 H), 7.77 (d, *J* = 5.8 Hz, 1 H), 7.44 (d, *J* = 6.8 Hz, 1 H), 7.29 (d, *J* = 2.3 Hz, 1 H), 6.88 (s, 1 H), 6.28 (d, *J* = 2.3 Hz, 1 H), 4.29 (t, *J* = 9.5 Hz, 2 H), 4.12 - 4.02 (m, 2 H), 3.94 - 3.86 (m, 1 H), 3.86 (s, 3 H), 3.26 - 3.11 (m, 2 H), 2.72 - 2.57 (m, 1 H), 2.22 - 2.05 (m, 4 H).

### CT. (1-Methyl-1H-pyrazol-3-yl)-{4-[1-(4-oxazol-2-ylpiperidin-1-yl)-[2,6]naphthyridin-3-yl]-pyridin-2-yl}amine.

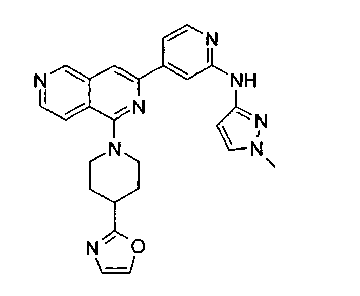

The title compound is prepared from 1-[3-(2-chloropyridin-4-yl)-[2,6]naphthyridin-1-yl]-piperidine-4-carboxylic acid (2,2-difluoroethyl)amide **Example 5T:** MS (ESI) *m*/*z* 453.1 (M+1); ¹H NMR (400 MHz, CDCl₃) δ ppm 9.28 (s, 1 H), 8.63 (d, *J* = 5.8 Hz, 1 H), 8.30 (d, *J* = 5.3 Hz, 1 H), 8.06 (s, 1 H), 7.82 (s, 1 H), 7.80 (d, *J* = 6.6 Hz, 1 H), 7.63 (s, 1 H), 7.45 (dd, *J*=5.3, 1.5 Hz, 1 H), 7.29 (d, *J* = 2.3 Hz, 1 H), 7.08 (d, *J* = 0.9 Hz, 1 H), 6.90 (s, 1 H), 6.27 (d, *J* = 2.3 Hz, 1 H), 4.15 - 4.04 (m, 2 H), 3.86 (s, 3 H), 3.34 - 3.24 (m, 2 H), 3.22 - 3.09 (m, 1 H), 2.35 - 2.17 (m, 4 H).

### Example 9

### A. 4-(1-Piperazin-1-yl-[2,6]naphthyridin-3-yl)-pyridin-2-yl]-(tetrahydropyran-4-yl)amine.

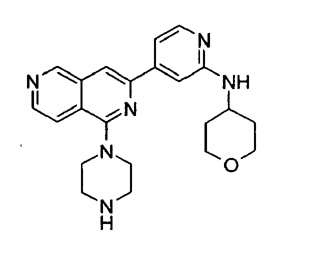

To a suspension of 4-{3-[2-(tetrahydropyran-4-ylamino)pyridin-4-yl]-[2,6]naphthyridin-1-yl}-piperazine-1-carboxylic acid *t*-butyl ester (120 mg, 0.25 mmol) in 1.80 mL of DCM at 0°C is added 0.60 mL of TFA drop by drop via pipette. The resulting brownish orange solution is warmed to rt and stirred for 1 h, after which point it is concentrated *in vacuo* to afford an amber oil. The residue is dissolved in MeOH and a small amount of water, then purified via preparative reverse-phase HPLC (X-Bridge C₁₈ column, flow rate = 40 mL/min, gradient 10% → 80% acetonitrile/5 mM aqueous ammonium hydroxide over 20 min) to give the title compound as a pale yellow solid (66 mg, 68%). MS (ESI) *m*/*z* 391.2 (M+1); ¹H NMR (400 MHz, CD₃OD) δ 9.30 (d, , *J* = 1.0 Hz, 1 H), 8.58 (d, *J* = 5.8 Hz, 1 H), 8.03 (m, 2 H), 7.98 (d, *J* = 5.8 Hz, 1 H), 7.42 (s, 1 H), 7.29 (dd, *J* = 5.7, 1.6 Hz, 1 H), 3.99 (m, 3 H), 3.59 (m, 6H), 3.14 (dd, *J* = 5.6, 4.0 Hz, 4 H), 2.03 (dd, *J* = 12, 2.8 Hz, 2 H), 1.58 (m, 2 H).

The following compounds can be prepared with similar method.

### B. (2-Methoxyethyl)-[4-(1-piperazin-1-yl-[2,6]naphthyridin-3-yl)-pyridin-2-yl]amine.

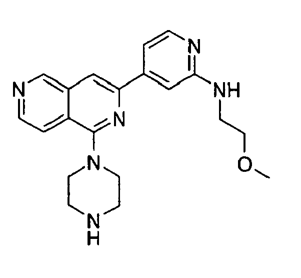

MS (ESI) *m*/*z* 365.4 (M+1); ¹H NMR (400 MHz, *DMSO*-*d*₆) δ 9.36 (d, *J* = 0.76 Hz, 1 H), 8.62 (d, *J* = 5.8 Hz, 1 H), 8.07 (d, *J* = 5.3 Hz, 1 H), 8.05 (s, 1 H), 7.88 (d, *J* = 5.8 Hz, 1 H), 7.36 (d, *J* = 0.76 Hz, 1 H), 7.20 (dd, *J* = 5.6, 1.5 Hz, 1 H), 6.71 (m, 1 H), 3.49 (m, 4 H), 3.43 (m, 4 H), 3.29 (s, 3 H), 2.98 (m, 4 H).

### C. Isopropyl-[4-(1-piperazin-1-yl-[2,6]naphthyridin-3-yl)-pyridin-2-yl]amine.

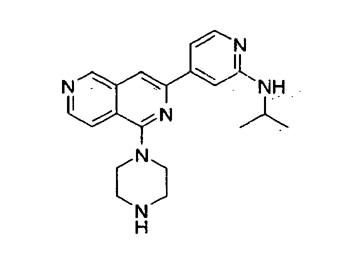

MS (ESI) *m*/*z* 349.3 (M+1); ¹H NMR (400 MHz, CD₃OD) δ 9.31 (d, 1 H), 8.59 (d, 1 H), 8.04 (d, 2 H), 7.99 (d, 1 H), 7.41 (s, 1 H), 7.29 (d, 1 H), 4.05 (m, 1 H) 3.40 (m, 4 H), 3.15 (m, 4 H), 1.30 (d, 6 H).

### D. Phenyl-[4-(1-piperazin-1-yl-[2,6]naphthyridin-3-yl)pyridin-2-yl]amine.

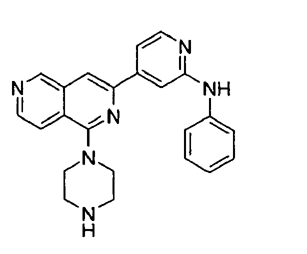

MS (ESI) *m*/*z* 383.2 (M+1); ¹H NMR (400 MHz, CD₃OD) δ 9.29 (s, 1 H), 8.57 (d, 1 H), 8.18 (d, 1 H), 8.04 (s, 1 H), 7.96 (d, 1 H), 7.77 (s, 1 H), 7.48 (m, 3 H), 7.30 (m, 2 H), 6.98 (t, 1 H), 3.55 (br d, 4 H), 3.10 (br d, 4 H).

### E. (1-Methyl-1H-pyrazol-3-yl)-[4-(1-piperazin-1-yl-[2,6]naphthyridin-3-yl)pyridin-2-yl]amine.

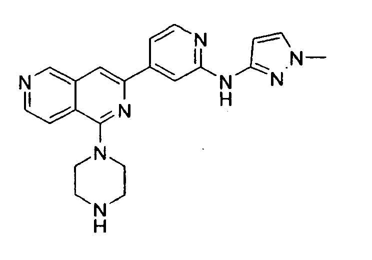

MS (ESI) *m*/*z* 387.2 (M+1); ¹H NMR (400 MHz, CDCl₃) δ ppm 9.28 (s, 1 H), 8.60 (d, 1 H), 8.25 (d, 1 H), 7.89 (d, 1 H), 7.84 (s, 1 H), 7.41 (dd, 1 H), 7.25 (d, 1 H), 6.89 (s, 1 H), 6.30 (d, 1 H), 3.84 (s, 3 H), 3.52 (m, 4 H), 3.10 (br d, 4H).

### F. Cyclopentyl-[4-(1-piperazin-1-yl-[2,6]naphthyridin-3-yl)pyridin-2-yl]amine.

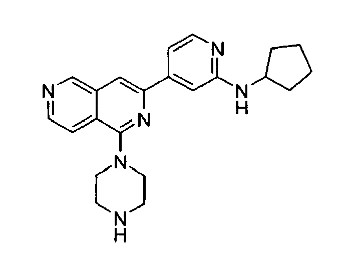

MS (ESI) *m*/*z* 375.2 (M+1); ¹H NMR (400 MHz, CD₃OD) δ 9.26 (s, 1 H), 8.55 (d, 1 H), 7.99 (d, 2 H), 7.94 (d, 1 H), 7.36 (s, 1 H), 7.22 (d, 1 H), 4.12 (m, 1 H), 3.56 (dd, 4 H), 3.13 (dd, 4 H), 2.02 (m, 2 H), 1.77 (m, 2H), 1.64 (m, 2 H), 1.52 (m, 2 H).

### G. Cyclohexyl-[4-(1-piperazin-1-yl-[2,6]naphthyridin-3-yl)pyridin-2-yl]amine.

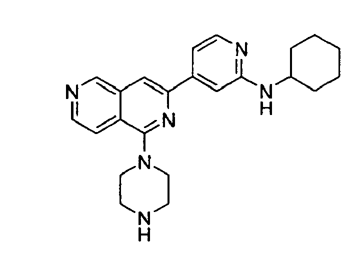

MS (ESI) m/z 389.3 (M+1); ¹H NMR (400 MHz, CD₃OD) δ 9.35 (s, 1 H), 8.62 (d, *J* = 6.1 Hz, 1 H), 8.07 (s, 1 H), 8.05 (d, *J* = 5.5 Hz, 1 H), 8.02 (d, *J* = 5.6 Hz, 1 H), 7.44 (s, 1 H), 7.30 (d, *J* = 5.6 Hz, 1 H), 3.80 - 3.71 (m, 1 H), 3.71 (s, 1 H), 3.63 (t, *J* = 4.8 Hz, 4 H), 3.20 (t, *J* = 4.8 Hz, 4 H), 2.15 - 2.08 (m, 2 H), 1.90 - 1.82 (m, 2 H), 1.78 -1.71 (m, 1 H), 1.58 - 1.46 (m, 2 H), 1.40 - 1.09 (m, 3 H).

### H. (4-Methylcyclohexyl)-[4-(1-piperazin-1-yl-[2,6]naphthyridin-3-yl)pyridin-2-yl]amine.

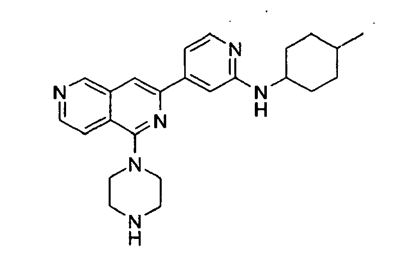

MS (ESI) *m*/*z* 403.3 (M+1); ¹H NMR (400 MHz, CD₃OD) δ 9.25 (s, 1 H), 8.55 (d, 1 H), 7.94 (m, 3 H), 7.33 (s, 1 H), 7.22 (m, 1 H), 3.60 (m, 1 H), 3.53 (dd, 4 H), 3.11 (dd, 4 H), 2.08 (d, 1 H), 1.80-1.65 (m, 3 H), 1.60 (m, 1 H), 1.38 (m, 2 H), 1.25 (m, 1 H), 1,10 (m, 1 H), 0.95 (ddd, 3 H).

### I. (2-Methylcyclohexyl)-[4-(1-piperazin-1-yl-[2,6]naphthyridin-3-yl)pyridin-2-yl]amine.

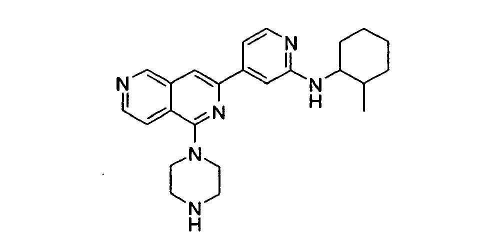

MS (ESI) *m*/*z* 403.3 (M+1); ¹H NMR (400 MHz, CD₃OD) δ 9.29 (s, 1 H), 8.57 (d, 1 H), 7.99 (m, 3 H), 7.38 (s, 1 H), 7.22 (dd, 1 H), 3.58 (dd, 4 H), 3.41 (m, 1 H), 3.12 (dd, 4 H), 2.07 (m, 1 H), 1.90-1.55 (m, 4 H), 1.45 (m, 2 H), 1.32 (m, 1 H), 1.18 (m, 1 H), 1.0 (dd, 3 H).

### J. (3-Methoxyphenyl)-[4-(1-piperazin-1-yl-[2,6]naphthyridin-3-yl)pyridin-2-yl]amine.

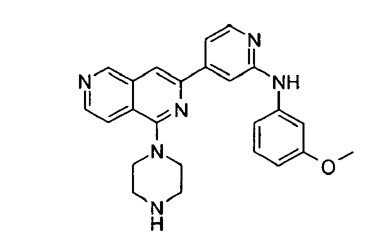

MS (ESI) m/z 413.3 (M+1).

### K. Benzyl-[4-(1-piperazin-1-yl-[2,6]naphthyridin-3-yl)pyridin-2-yl]amine.

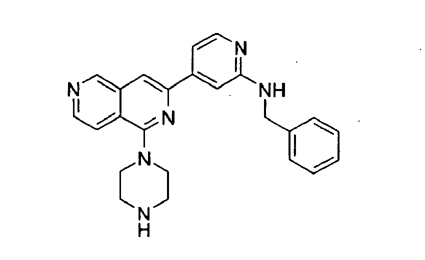

MS (ESI) *m*/*z* 396.5 (M+1).

### L. tert-Butyl-[4-(1-piperazin-1-yl-[2,6]naphthyridin-3-yl)-pyridin-2-yl]-amine.

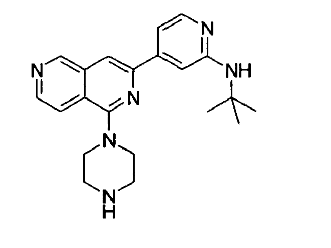

HRMS (ESI) *m*/*z* 363.2301 (M+1); ¹H NMR (400 MHz, *DMSO*-*d*₆) δ ppm 9.35 (s, 1 H), 8.61 (d, *J* = 5.6 Hz, 1 H), 8.06 (d, *J* = 5.6 Hz, 1 H), 8.00 (s, 1 H), 7.90 - 7.83 (m, 1 H), 7.36 (s, 1 H), 7.16 - 7.10 (m, 1 H), 6.36 (s, 1 H), 3.48 - 3.39 (br m, 4 H), 3.05 - 2.95 (br m, 4 H), 2.48 - 2.23 (br s, 1 H), 1.43 (s, 9 H).

### M. [3-(2-Cyclohexylaminopyridin-4-yl)-[2,6]naphthyridin-1-yl]-(S)-pyrrolidin-3-ylamine.

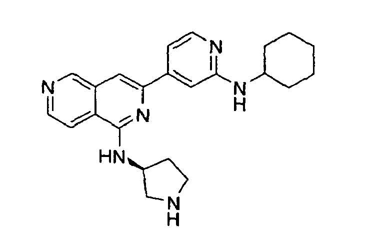

HRMS (ESI) *m*/*z* 389.2466 (M+1); ¹H NMR (400 MHz, CDCl₃) δ ppm 9.16 (s, 1 H), 8.58 (d, *J* = 5.68 Hz, 1 H), 8.17 (d, *J* = 5.31 Hz, 1 H), 7.69 (d, *J* = 5.68 Hz, 1 H), 7.49 (s, 1 H), 7.24 - 7.13 (m, 2 H), 6.07 (br s, 1 H), 4.86 (dd, *J* = 6.32, 3.16 Hz, 1 H), 4.77 - 4.62 (m, 1 H), 3.83 - 3.61 (m, 2 H), 3.37 (dd, *J* = 11.31, 6.13 Hz, 1 H), 3.30 - 3.20 (m, 1 H), 3.17 (dd, *J* = 11.37, 2.78 Hz, 1 H), 3.11 - 2.99 (m, 1 H), 2.40 - 2.26 (m, 1 H), 2.20 - 2.03 (m, 2 H), 1.99 - 1.87 (m, 1H), 1.86 - 1.53 (m, 3 H), 1.52 - 1.11 (m, 5 H).

### N. [3-(2-Cyclohexylaminopyridin-4-yl)-[2,6]naphthyridin-1-yl]-(R)-pyrrolidin-3-ylamine.

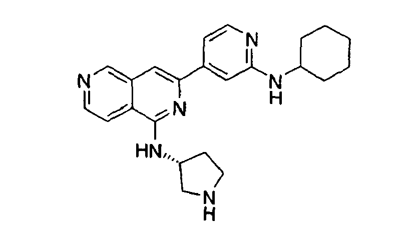

HRMS (ESI) *m*/*z* 389.2454 (M+1); ¹H NMR (400 MHz, DMSO-*d*₆) δ ppm 9.20 (s, 1 H), 8.58 (d, *J* = 5.56 Hz, 1 H), 8.28 (d, *J* = 3.79 Hz, 1 H), 8.03 (d, *J* = 5.31 Hz, 1 H), 7.64 (s, 1 H), 7.25 (s, 1 H), 7.12 (d, *J* = 6.57 Hz, 1 H), 4.76 (br s, 1 H), 3.86 - 3.62 (m, 2 H), 3.15 - 2.92 (m, 4 H), 2.37 - 2.17 (m, 1 H), 2.08 - 1.89 (m, 3 H), 1.81 - 1.53 (m, 3 H), 1.42 - 1.08 (m, 6 H).

### O. {4-[1-(4-Aminomethylpiperidin-1-yl)-[2,6]naphthyridin-3-yl]pyridin-2-yl}cyclohexylamine.

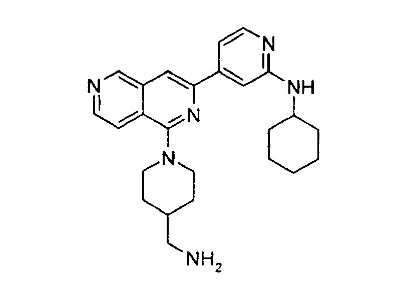

MS (ESI) *m*/*z* 417.3 (M+1); ¹H NMR (400 MHz, CD₃OD) δ 9.28 (s, 1 H), 8.56 (d, 1 H), 8.00 (d, 1 H), 7.99 (s, 1 H), 7.94 (d, 1 H), 7.37 (s, 1 H), 7.26 (d, 1 H), 4.11 (d, 2 H), 3.69 (m, 1 H), 3.13 (t, 2 H), 2.76 (d, 2 H), 2.05 (m, 2 H), 1.95 (m, 2 H), 1.80 (m, 3 H), 1.69 (m, 1H). 1.61 (m, 2 H), 1.46 (m, 2 H), 1.28 (m, 3 H).

### Example 10

### A. {4-[1-(4-Isobutylpiperazin-1-yl)-[2,6]naphthyridin-3-yl]pyridin-2-yl}-(tetrahydropyran-4-yl)amine.

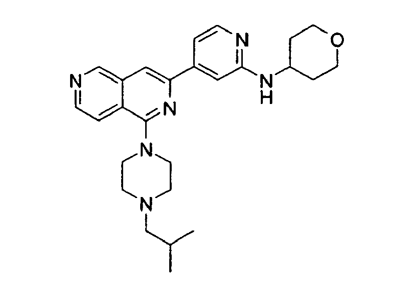

The title compound is prepared by reductive amination of 4-(1-piperazin-1-yl-[2,6]naphthyridin-3-yl)pyridin-2-yl](tetrahydropyran-4-yl)amine with commercially available 2-methylpropionaldehyde. Thus, sodium triacetoxyborohydride (339 mg, 1.59 mmol) is added to a solution of 4-(1-piperazin-1-yl-[2,6]naphthyridin-3-yl)pyridin-2-yl](tetrahydropyran-4-yl)amine (148 mg, 0.38 mmol) and 2-methylpropionaldehyde (42 µL, 0.46 mmol) in methylene chloride (8 mL) and stirred for 12 h. The reaction is concentrated on a rotary evaporator and partially purified on a 12 g (Redisep, Isco) silica gel column with a 0 to 10% methanol/methylene chloride gradient. The resulting product is further purified by reverse phase HPLC using a 30 - 95% acetonitrile/water gradient. This yields 41.2 mg (24%) after consolidation and concentration of fractions: HRMS (ESI) *m*/*z* 447.2717 (M+1); ¹H NMR (400 MHz, CDCl₃) δ ppm 9.29 (s, 1 H), 8.64 (d, *J* = 5.81 Hz, 1 H), 8.22 (d, *J* = 5.31 Hz, 1 H), 7.84 (d, *J* = 5.81 Hz, 1 H), 7.78 (s, 1 H), 7.30 - 7.28 (m, 1 H), 7.27 (s, 1 H), 4.57 (d, *J* = 7.96 Hz, 1 H), 4.15 - 3.98 (m, 3 H), 3.73 - 3.53 (m, 6 H), 2.73 (t, *J* = 4.55 Hz, 4 H), 2.25 (d, *J* = 7.33 Hz, 2 H), 2.15 (dd, *J* = 12.57, 2.08 Hz, 2 H), 1.98 - 1.77 (m, 1 H), 1.70 - 1.54 (m, 2 H), 0.99 (d, *J* = 6.57 Hz, 6 H).

### B. (4-{1-[4-(2,2-Dimethylpropyl)piperazin-1-yl]-[2,6]naphthyridin-3-yl}pyridin-2-yl)-(tetrahydropyran-4-yl)amine.

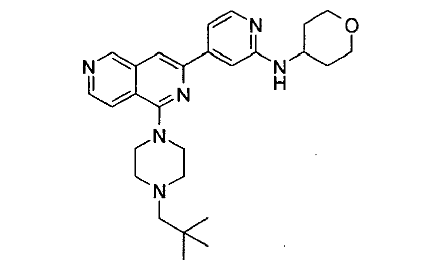

This compound is prepared by reductive amination of 4-(1-piperazin-1-yl-[2,6]naphthyridin-3-yl)-pyridin-2-yl]-(tetrahydropyran-4-yl)amine with commercially available 2,2-dimethylpropionaldehyde: HRMS (ESI) *m*/*z* 461.3041 (M+1); ¹H NMR (400 MHz, CDCl₃) δ ppm 9.27 (s, 1 H), 8.62 (d, *J* = 5.81 Hz, 1 H), 8.19 (d, *J* = 6.06 Hz, 1 H), 7.81 (d, *J* = 5.68 Hz, 1 H), 7.76 (s, 1 H), 7.27 - 7.23 (m, 2 H), 4.73 (br s, 1 H), 4.10 - 3.97 (m, 3 H), 3.66 - 3.52 (m, 6 H), 2.88 - 2.77 (m, 4 H), 2.21 (s, 2 H), 2.12 (dd, *J* = 12.63, 2.02 Hz, 2 H), 1.67 - 1.53 (m, 2 H), 0.94 (s, 9 H).

### C. Cyclohexyl-{4-[1-(4-isobutylpiperazin-1-yl)-[2,6]naphthyridin-3-yl]pyridin-2-yl}amine.

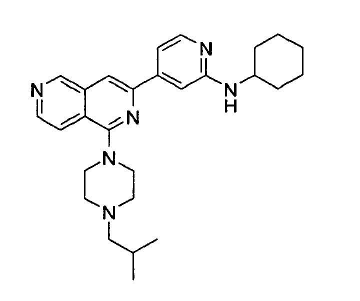

The title compound is prepared by reductive amination of cyclohexyl-[4-(1-piperazin-1-yl-[2,6]naphthyridin-3-yl)-pyridin-2-yl]amine with commercially available 2-methylpropionaldehyde: HRMS (ESI) *m*/*z* 445.3087 (M+1); ¹H NMR (400 MHz, CDCl₃)δ ppm 9.26 (s, 1 H), 8.61 (d, *J* = 5.68 Hz, 1 H), 8.20 - 8.14 (m, 1 H), 7.81 (d, *J* = 5.81 Hz, 1 H), 7.75 (s, 1 H), 7.24 - 7.18 (m, 2 H), 4.61 (d, *J* = 7.71 Hz, 1 H), 3.77 - 3.65 (m, 1 H), 3.65 - 3.57 (m, 4 H), 2.74 - 2.65 (m, 3 H), 2.22 (d, *J* = 7.33 Hz, 2 H), 2.13 (dd, *J* = 12.63, 3.16 Hz, 2 H), 1.98 - 1.74 (m, 4 H), 1.73 - 1.62 (m, 1 H), 1.54 - 1.38 (m, 2 H), 1.35 - 1.20 (m, 3 H), 0.96 (d, *J* = 6.57 Hz, 6 H).

### D. [3-(2-Cyclohexylaminopyridin-4-yl)-[2,6]naphthyridin-1-yl]-((S)-1-cyclopropylmethylpyrrolidin-3-yl)amine.

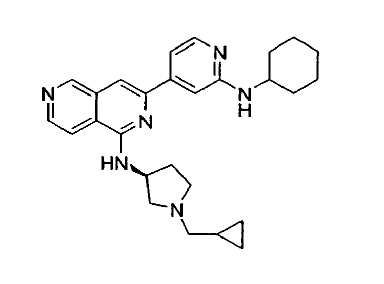

The title compound is prepared by reductive amination [3-(2-cyclohexylaminopyridin-4-yl)-[2,6]naphthyridin-1-yl]-(*S*)-pyrrolidin-3-ylamine with commercially available cyclopropanecarbaldehyde: HRMS (ESI) *m*/*z* 443.2932 (M+1); ¹H NMR (400 MHz, CDCl₃) δ ppm 9.16 (s, 1 H), 8.57 (d, *J* = 5.81 Hz, 1 H), 8.16 (d, *J* = 5.56 Hz, 1 H), 7.56 (d, *J* = 5.81 Hz, 1 H), 7.48 (s, 1 H), 5.80 (d, *J* = 6.82 Hz, 1 H), 4.99 - 4.88 (m, 1 H), 4.60 (d, *J* = 7.96 Hz, 1 H), 3.75 - 3.59 (m, 1 H), 3.11 - 3.01 (m, 1 H), 2.96 (dd, *J* = 9.98, 2.91 Hz, 1 H), 2.83 (dd, *J* = 9.98, 6.69 Hz, 1 H), 2.60 - 2.48 (m, 1 H), 2.47 - 2.32 (m, 3 H), 2.18 - 2.06 (m, 2 H), 1.93 - 1.83 (m, 1 H), 1.83 - 1.73 (m, 2 H), 1.72 - 1.61 (m, 1H), 1.51 - 1.36 (m, 2 H), 1.34 - 1.16 (m, 4 H), 1.00 - 0.86 (m, 2 H), 0.57 - 0.48 (m, 2 H), 0.15 (q, *J* = 4.88 Hz, 2 H).

### Example 11

### A. 2-Amino-2-methyl-1-(4-{3-[2-(tetrahydropyran-4-ylamino)pyridin-4-yl]-[2,6]naphthyridin-1-yl}piperazin-1-yl)propan-1-one.

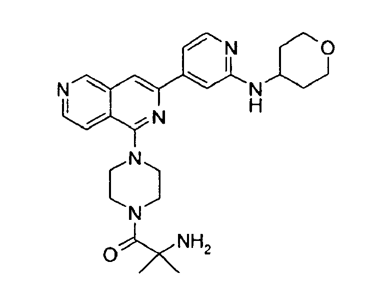

The title compound is prepared by acylation of 4-(1-piperazin-1-yl-[2,6]naphthyridin-3-yl)-pyridin-2-yl]-(tetrahydropyran-4-yl)amine with commercially available 2-*tert-*butoxycarbonylamino-2-methyl-propionic acid followed by removal of the BOC protecting group. Thus, 2-*tert-*butoxycarbonylamino-2-methylpropionic acid (108.2 mg, 0.5 mmol) and HBTU (235.9 mg, 0.67 mmol) are mixed and stirred in dimethylformamide (6 mL) for 10 min. Then a mixture of 4-(1-piperazin-1-yl-[2,6]naphthyridin-3-yl)-pyridin-2-yl]-(tetrahydropyran-4-yl)amine (161 mg, 0.42 mmol) and triethyl amine (57.8 µL, 0.42 mmol) in dimethylformamide (1 mL) is added to the reaction and stirred an additional 12 h. The reaction is concentrated on a rotary evaporator and partially purified on a 12 g (Redisep, Isco) silica gel column using a 0 to 10% methanol/methylene chloride gradient. This gives 241 mg of [1,1-dimethyl-2-oxo-2-(4-{3-[2-(tetrahydropyran-4-ylamino)-pyridin-4-yl]-[2,6]naphthyridin-1-yl}-piperazin-1-yl)-ethyl]-carbamic acid *tert-*butyl ester. This compound (238 mg, 0.42 mmol) is stirred in formic acid (5 mL) for 12 h. This mixture is concentrated on the rotary evaporator and the residue is treated with saturated sodium bicarbonate. This is extracted with methylene chloride, which is washed with brine, separated and dried over sodium sulfate. Concentration followed by purification by reverse phase HPLC using a 25 - 80% acetonitrile/water gradient. This yields 71 mg (36%) of product as a white solid after consolidation and concentration of fractions: HRMS (ESI) *m*/*z* 476.2767 (M+1); ¹H NMR (400 MHz, CDCl₃) δ ppm 9.29 (s, 1 H), 8.65 (d, *J* = 5.81 Hz, 1 H), 8.20 (d, *J* = 5.43 Hz, 1 H), 7.85 - 7.77 (m, 2 H), 7.23 (d, *J* = 5.43 Hz, 1 H), 7.19 (s, 1 H), 4.56 (d, *J* = 7.96 Hz, 1 H), 4.16 (br s, 3 H), 4.09 - 3.97 (m, 3 H), 3.66 - 3.51 (m, 6 H), 2.11 (dd, *J* = 12.57, 1.96 Hz, 2 H), 1.73 - 1.51 (m, 4 H), 1.48 (s, 6 H).

### B. (S)-Pyrrolidin-2-yl-(4-{3-[2-(tetrahydropyran-4-ylamino)pyridin-4-yl]-[2,6]naphthyridin-1-yl}piperazin-1-yl)methanone.

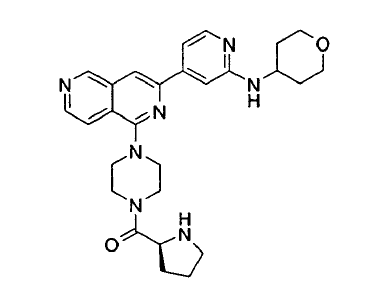

The title compound is prepared by acylation of 4-(1-piperazin-1-yl-[2,6]naphthyridin-3-yl)-pyridin-2-yl]-(tetrahydropyran-4-yl)amine with commercially available (*S*)-pyrrolidine-1,2-dicarboxylic acid 1-*tert-*butyl ester followed by removal of the BOC protecting group: HRMS (ESI) *m*/*z* 488.2752 (M+1); ¹H NMR (400 MHz, CDCl₃) δ ppm 9.31 (s, 1 H), 8.67 (d, *J* = 5.81 Hz, 1 H), 8.21 (d, *J* = 5.43 Hz, 1 H), 7.83 (s, 1 H), 7.81 (d, *J* = 5.81 Hz, 1 H), 7.23 (dd, *J* = 5.37, 1.45 Hz, 1 H), 7.17 (s, 1 H), 4.55 (d, *J* = 7.96 Hz, 1 H), 4.16 - 3.90 (m, 6 H), 3.89 - 3.71 (m, 2 H), 3.70 - 3.47 (m, 6 H), 3.29 - 3.13 (m, 1 H), 2.96 - 2.79 (m, 1 H), 2.47 (br s, 1 H), 2.24 - 2.05 (m, 3 H), 1.93 - 1.67 (m, 3 H), 1.66 - 1.48 (m, 2 H).

### C. (S)-2-Amino-3-phenyl-1-(4-{3-[2-(tetrahydropyran-4-ylamino)pyridin-4-yl]-[2,6]naphthyridin-1-yl}piperazin-1-yl)propan-1-one.

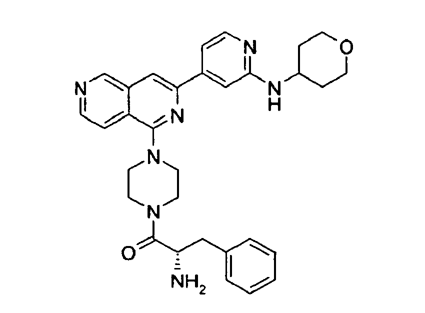

This compound is prepared by acylation of 4-(1-piperazin-1-yl-[2,6]naphthyridin-3-yl)-pyridin-2-yl]-(tetrahydropyran-4-yl)amine with commercially available (*S*)-2-*tert-*butoxycarbonylamino-3-phenylpropionic acid followed by removal of the BOC protecting group: HRMS (ESI) *m*/*z* 538.2929 (M+1); ¹H NMR (400 MHz, CDCl₃) δ ppm 9.30 (s, 1 H), 8.65 (d, *J* = 5.81 Hz, 1 H), 8.22 (d, *J* = 5.43 Hz, 1 H), 7.82 (s, 1 H), 7.73 (d, *J* = 5.81 Hz, 1 H), 7.38 - 7.30 (m, 2 H), 7.27 - 7.20 (m, 4 H), 7.15 (s, 1 H), 4.57 (d, *J* = 7.83 Hz, 1 H), 4.14 - 3.99 (m, 4 H), 3.98 - 3.79 (m, 2 H), 3.72 - 3.51 (m, 4 H), 3.50 - 3.32 (m, 3 H), 3.11 - 2.96 (m, 2 H), 2.94 - 2.84 (m, 1 H), 2.12 (dd, *J* = 12.51, 2.02 Hz, 2 H), 1.79 (br s, 2 H), 1.67 - 1.52 (m, 2 H).

### D. (S)-2-Amino-1-(4-{3-[2-(tetrahydropyran-4-ylamino)pyridin-4-yl]-[2,6]naphthyridin-1-yl}piperazin-1-yl)propan-1-one.

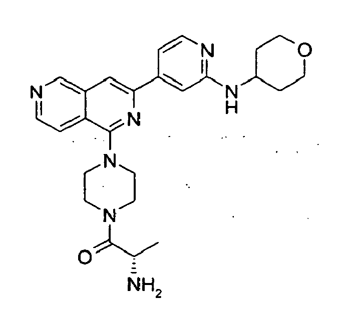

The title compound is prepared by acylation of 4-(1-piperazin-1-yl-[2,6]naphthyridin-3-yl)-pyridin-2-yl]-(tetrahydropyran-4-yl)amine with commercially available (*S*)-2-*tert-*butoxycarbonylaminopropionic acid followed by removal of the BOC protecting group: HRMS (ESI) *m*/*z* 462.2639 (M+1); ¹H NMR (400 MHz, CDCl₃) δ ppm 9.30 (s, 1 H), 8.65 (d, *J* = 5.81 Hz, 1 H), 8.20 (d, *J* = 5.43 Hz, 1 H), 7.82 (s, 1 H), 7.80 (d, *J* = 5.81 Hz, 1 H), 7.22 (d, *J* = 5.43 Hz, 1 H), 7.16 (s, 1 H), 4.60 (d, *J* = 7.96 Hz, 1 H), 4.11 - 3.98 (m, 3 H), 3.97 - 3.84 (m, 3 H), 3.79 (br s, 2 H), 3.68 - 3.49 (m, 5 H), 2.10 (dd, *J* = 12.44, 1.83 Hz, 2 H), 1.96 (br s, 3 H), 1.64 - 1.50 (m, 2 H), 1.32 (d, *J* = 6.82 Hz, 3 H).

### E. [(R)-2-Methyl-1-(4-{3-[2-(tetrahydropyran-4-ylamino)pyridin-4-yl]-[2,6]naphthyridin-1-yl}-piperazine-1-carbonyl)propyl]carbamic acid tert-butyl ester.

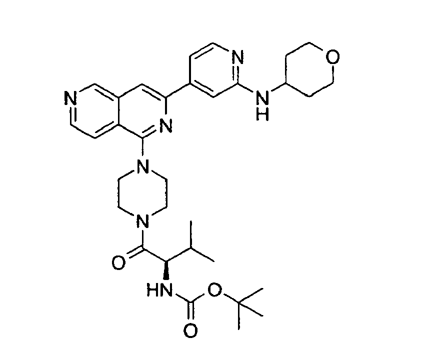

The title compound is prepared by acylation of 4-(1-Piperazin-1-yl-[2,6]naphthyridin-3-yl)-pyridin-2-yl]-(tetrahydropyran-4-yl)amine with commercially available (*R*)-2-*tert-*butoxycarbonylamino-3-methylbutyric acid. HRMS (ESI) *m*/*z* 590.3469 (M+1); ¹H NMR (400 MHz, CDCl₃) δ ppm 9.30 (s, 1 H), 8.66 (d, *J* = 5.81 Hz, 1 H), 8.21 (d, *J* = 5.31 Hz, 1 H), 7.82 (s, 1 H), 7.79 (d, *J* = 5.81 Hz, 1 H), 7.21 (d, *J* = 5.43 Hz, 1 H), 7.18 (s, 1 H), 5.42 (d, *J* = 9.22 Hz, 1 H), 4.64 (d, *J* = 7.83 Hz, 1 H), 4.55 (dd, *J* = 9.22, 5.81 Hz, 1 H), 4.14 - 3.76 (m, 6 H), 3.71 - 3.46 (m, 6 H), 2.11 (dd, *J* = 12.19, 1.58 Hz, 2 H), 2.07 - 1.96 (m, 1 H), 1.91 (br s, 1 H), 1.66 - 1.52 (m, 2 H), 1.45 (s, 9 H), 1.01 (d, *J* = 6.69 Hz, 3 H), 0.94 (d, *J* = 6.69 Hz, 3 H).

### F. N-[(S)-2-Methyl-1-(4-{3-[2-(tetrahydropyran-4-ylamino)pyridin-4-yl]-[2,6]naphthyridin-1-yl}piperazine-1-carbonyl)propyl]acetamide.

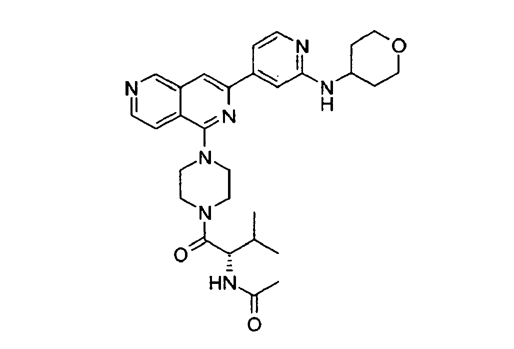

The title compound is prepared by acylation of 4-(1-piperazin-1-yl-[2,6]naphthyridin-3-yl)-pyridin-2-yl]-(tetrahydropyran-4-yl)amine with commercially available (*S*)-2-acetylamino-3-methylbutyric acid: HRMS (ESI) *m*/*z* 532.3059 (M+1); ¹H NMR (400 MHz, CDCl₃) δ ppm 9.30 (s, 1 H), 8.65 (d, *J* = 5.81 Hz, 1 H), 7.82 (s, 1 H), 7.78 (d, *J* = 5.81 Hz, 1 H), 7.21 (dd, *J* = 5.43, 1.01 Hz, 1 H), 7.17 (s, 1 H), 6.51 (d, *J* = 8.84 Hz, 1 H), 4.91 (dd, *J* = 8.91, 6.38 Hz, 1 H), 4.75 - 4.64 (m, 1 H), 4.13 - 3.92 (m, 4 H), 3.92 - 3.80 (m, 2 H), 3.67 - 3.48 (m, 6 H), 2.16 - 1.99 (m, 8 H), 1.66 - 1.52 (m, 2 H), 1.00 (d, *J* = 6.69 Hz, 3 H), 0.95 (d, *J* = 6.82 Hz, 3 H).

### G. (S)-2-Amino-3-methyl-1-(4-{3-[2-(tetrahydropyran-4-ylamino)pyridin-4-yl]-[2,6]naphthyridin-1-yl}piperazin-1-yl)butan-1-one.

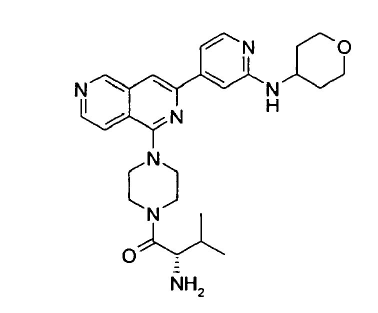

The title compound is prepared by acylation of 4-(1-piperazin-1-yl-[2,6]naphthyridin-3-yl)-pyridin-2-yl]-(tetrahydropyran-4-yl)amine with commercially available (*S*)-2-*tert-*butoxycarbonylamino-3-methylbutyric acid: HRMS (ESI) *m*/*z* 490.2910 (M+1); ¹H NMR (400 MHz, CDCl₃) δ ppm 9.29 (s, 1 H), 8.65 (d, *J* = 5.68 Hz, 1 H), 8.20 (d, *J* = 5.43 Hz, 1 H), 7.82 (s, 1 H), 7.80 (d, *J* = 5.81 Hz, 1 H), 7.22 (dd, *J* = 5.43, 1.26 Hz, 1 H), 7.16 (s, 1 H), 4.60 (d, *J* = 7.96 Hz, 1 H), 4.12 -3.88 (m, 4 H), 3.80 (t, *J* = 4.55 Hz, 2 H), 3.68 - 3.47 (m, 7 H), 2.10 (dd, *J* = 12.51, 2.02 Hz, 2 H), 2.01-1.76 (m, 4 H), 1.65 - 1.50 (m, 2 H), 1.03 (d, *J* = 6.82 Hz, 3 H), 0.94 (d, *J* = 6.82 Hz, 3 H).

### H. N-[2-Oxo-2-(4-{3-[2-(tetrahydropyran-4-ylamino)pyridin-4-yl]-[2,6]naphthyridin-1-yl}-piperazin-1-yl)ethyl]acetamide.

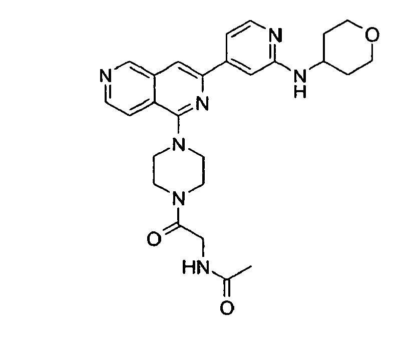

The title compound is prepared by acylation of 4-(1-piperazin-1-yl-[2,6]naphthyridin-3-yl)pyridin-2-yl]-(tetrahydropyran-4-yl)amine with commercially available acetylamino-acetic acid: HRMS (ESI) *m*/*z* 490.2543 (M+1); ¹H NMR (400 MHz, CDCl₃) δ ppm 9.31 (s, 1 H), 8.67 (d, *J* = 5.81 Hz, 1 H), 8.21 (d, *J* = 5.43 Hz, 1 H), 7.84 (s, 1 H), 7.79 (d, *J* = 5.81 Hz, 1 H), 7.22 (dd, *J* = 5.43, 1.52 Hz, 1 H), 7.16 (s, 1 H), 6.66 (br s, 1 H), 4.64 (d, *J* = 6.44 Hz, 1 H), 4.17 (d, *J* = 3.92 Hz, 2 H), 4.11 - 3.98 (m, 3 H), 3.98 - 3.89 (m, 2 H), 3.73 (dd, *J* = 6.32, 3.54 Hz, 2 H), 3.65 - 3.51 (m, 5 H), 2.16 - 2.03 (m, 6 H), 1.66 - 1.50 (m, 2 H).

### I. (R)-2-Amino-3-methyl-1-(4-{3-[2-(tetrahydropyran-4-ylamino)pyridin-4-yl]-[2,6]naphthyridin-1-yl}piperazin-1-yl)butan-1-one.

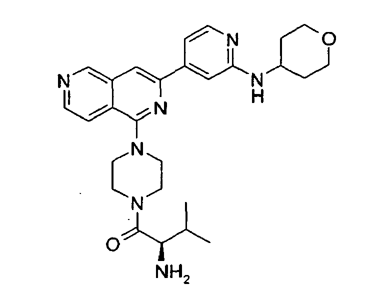

The title compound is prepared by acylation of 4-(1-piperazin-1-yl-[2,6]naphthyridin-3-yl)pyridin-2-yl]-(tetrahydropyran-4-yl)amine with commercially available (*R*)-2-*tert-*butoxycarbonylamino-3-methylbutyric acid: HRMS (ESI) *m*/*z* 490.2918 (M+1); ¹H NMR (400 MHz, CDCl₃) δ ppm 9.32 (s, 1 H), 8.67 (d, *J* = 5.68 Hz, 1 H), 8.21 (d, *J* = 5.43 Hz, 1 H), 7.84 (s, 1 H), 7.81 (d, *J* = 5.81 Hz, 1 H), 7.24 (dd, *J* = 5.43, 1.39 Hz, 1 H), 7.18 (s, 1 H), 4.75 (br s, 1 H), 4.12 - 3.89 (m, 5 H), 3.82 (t, *J* = 4.55 Hz, 2 H), 3.70 - 3.48 (m, 7 H), 2.12 (dd, *J* = 12.38, 2.02 Hz, 2 H), 1.99 - 1.87 (m, 1 H), 1.77 (br s, 2 H), 1.66 - 1.53 (m, 2 H), 1.05 (d, *J* = 6.82 Hz, 3 H), 0.96 (d, *J* = 6.82 Hz, 3 H).

### J. 2-Amino-1-(4-{3-[2-(tetrahydropyran-4-ylamino)pyridin-4-yl]-[2,6]naphthyridin-1-yl}piperazin-1-yl)ethanone.

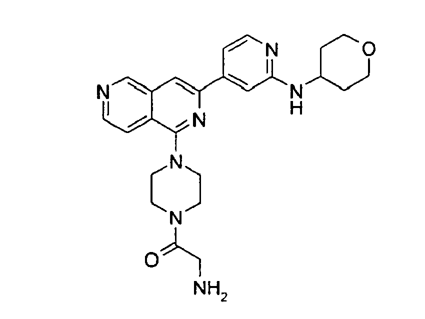

This compound is prepared by acylation of 4-(1-piperazin-1-yl-[2,6]naphthyridin-3-yl)pyridin-2-yl]-(tetrahydropyran-4-yl)amine with commercially available *tert-*butoxycarbonylamino-acetic acid, followed by acidic deprotection of the BOC group: HRMS (ESI) *m*/*z* 448.2459 (M+1); ¹H NMR (400 MHz, CDCl₃) δ ppm 9.31 (s, 1 H), 8.66 (d, *J* = 5.81 Hz, 1 H), 8.21 (d, *J* = 5.43 Hz, 1 H), 7.83 (s, 1 H), 7.80 (d, *J* = 5.81 Hz, 1 H), 7.23 (dd, *J* = 5.37, 0.95 Hz, 1 H), 7.16 (s, 1 H), 4.55 (d, *J* = 7.96 Hz, 1 H), 4.11 - 3.98 (m, 3 H), 3.98 - 3.91 (m, 2 H), 3.74 - 3.66 (m, 2 H), 3.64 - 3.53 (m, 8 H), 2.11 (dd, *J* = 12.51, 1.89 Hz, 2 H), 1.70 (br s, 2 H), 1.63 - 1.51 (m, 2H).

### K. 4-{3-[2-(1-Methyl-1H-pyrazol-3-ylamino)pyridin-4-yl]-[2,6]naphthyridin-1-yl}piperazine-1-carboxylic acid ethylamide.

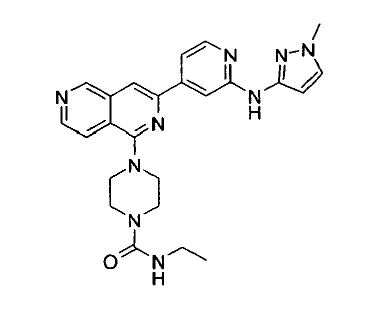

MS (ESI) *m*/*z* 486.2 (M+H); ¹H NMR (400 MHz, DMSO-d₆) δ ppm 9.20 (s, 1 H), 9.14 (s, 1 H), 8.52 (d, *J* = 5.8 Hz, 1 H), 8.16 - 8.06 (m, 2 H), 7.94 (s, 1 H), 7.85 - 7.76 (m, 1 H), 7.57 (s, 1 H), 7.45 (s, 1 H), 7.33 - 7.25 (m, 1 H), 6.34 - 6.23 (m, 2 H), 3.92 - 3.83 (m, 2 H), 3.70 (s, 3 H), 3.55 - 3.45 (m, 2 H), 3.01 - 2.86 (m, 2 H), 2.58 - 2.48 (m, 2 H), 1.93 - 1.79 (m, 1 H), 1.75 - 1.61 (m, 2 H), 1.14 - 0.99 (m, 2 H), 0.91 (t, *J* = 7.1 Hz, 3 H)

### L. {4-[1-(4-Benzenesulfonylpiperazin-1-yl)-[2,6]naphthyridin-3-yl]pyridin-2-yl}(tetrahydropyran-4-yl)amine.

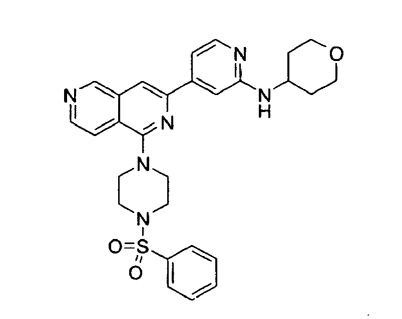

HRMS (ESI) *m*/*z* 531.2178 (M+1); ¹H NMR (400 MHz, DMSO-*d*₆) δ ppm 9.37 (s, 1 H), 8.58 (d, *J* = 5.81 Hz, 1 H), 8.11 (s, 1 H), 8.07 (d, *J* = 5.56 Hz, 1 H), 7.88 - 7.80 (m, 3 H), 7.80 - 7.73 (m, 1 H), 7.73 - 7.66 (m, 2 H), 7.30 (s, 1 H), 7.14 (d, *J* = 5.56 Hz, 1 H), 6.65 (d, *J* = 7.58 Hz, 1 H), 4.06 - 3.94 (m, 1 H), 3.94 - 3.84 (m, 2 H), 3.63 - 3.52 (m, 4 H), 3.49 - 3.38 (m, 2 H), 3.27 - 3.19 (m, 4 H), 1.92 (dd, *J* = 12.63, 2.27 Hz, 1 H), 1.41 - 1.53 (m, 2 H).

### Example 12

### A. (4-{1-[4-((S)-4-Isopropyl-4,5-dihydrooxazol-2-yl)piperidin-1-yl]-[2,6]naphthyridin-3-yl}-pyridin-2-yl)-(tetrahydropyran-4-yl)amine.

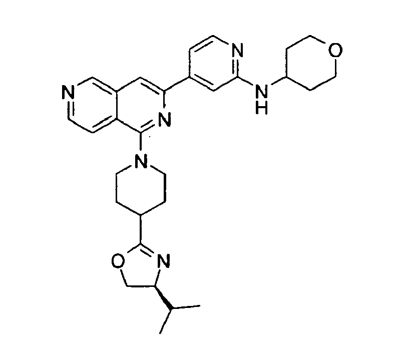

To 1-[3-(2-chloropyridin-4-yl)-[2,6]naphthyridin-1-yl]-piperidine-4-carboxylic acid prepared above (-1.93 mmol) in DMF (15 mL) is added DIEA (1.15 mL, 8.69 mmol), L-valinol (0.600 g ,5.80 mmol), PyBOP (3.00 g, 5.80 mmol), and HOBt (0.783 g, 5.80 mmol) in sequence. The mixture is stirred at rt for 1 h before it is concentrated *in vacuo.* The residue is then taken up in CH₂Cl₂ and saturated aqueous NaHCO₃. The organic layer is further washed with 10% aqueous LiCl and then brine. The organic layer is dried over Na₂SO₄, filtered and concentrated to give 1-{3-[2-(chloropyridin-4-yl]-[2,6]naphthyridin-1-yl}piperidine-4-carboxylic acid ((*S*)-1-hydroxymethyl-2-methylpropyl)amide which is used without further purification.

The crude 1-{3-[2-(chloropyridin-4-yl]-[2,6]naphthyridin-1-yl}piperidine-4-carboxylic acid ((*S*)-1-hydroxymethyl-2-methylpropyl)amide (-1.93 mmol) is taken up in CH₂Cl₂ (20 mL), Et₃N (0.81 mL, 5.79 mmol) and treated with methanesulfonyl chloride (0.22 mL, 2.89 mmol). After 2 h, additional methanesulfonyl chloride (1.5 equivalents) is added. After an additional 2 h Et₃N (3 eq) is added. Following another 2 h, the reaction is complete as judged by LCMS and the mixture is diluted with CH₂Cl₂ (40 mL) and saturated aqueous NaHCO₃ (50 mL). The aqueous layer is further extracted with CH₂Cl₂ (2 X 50 mL). The organic layers are dried over Na₂SO₄, filtered and concentrated. The residue is separated via flash chromatography (5-10% MeOH/EtOAc) to give 3-(2-chloropyridin-4-yl)-1-[4-((*S*)-4-isopropyl-4,5-dihydrooxazol-2-yl)piperidin-1-yl]-[2,6]naphthyridine. MS (ESI) *m*/*z* 436.0 (M+H); ¹H NMR (400 MHz, CDCl₃) δ ppm ¹H NMR δ ppm 9.28 (s, 1 H), 8.65 (d, *J* = 5.7 Hz, 1 H), 8.49 (d, *J* = 5.9 Hz, 1 H), 8.07 (d, *J* = 2.1 Hz, 1 H), 7.93 (dd, *J* = 5.2, 1.5 Hz, 1 H), 7.81 (s, 1 H), 7.80 (d, *J* = 6.6 Hz, 1 H), 4.30 - 4.22 (m, 1 H), 3.92 - 4.10 (m, 4 H), 3.24 - 3.12 (m, 2 H), 2.72 - 2.59 (m, 1 H), 2.22 - 1.99 (m, 4 H), 1.86 - 1.74 (m, 1 H), 0.98 (d, *J* = 6.8 Hz, 3 H), 0.90 (d, *J* = 6.7 Hz, 3 H).

The title compound is prepared from 3-(2-chloropyridin-4-yl)-1-[4-((*S*)-4-isopropyl-4,5-dihydrooxazol-2-yl)-piperidin-1-yl]-[2,6]naphthyridine as described above in Example 8A: MS (ESI) *m*/*z* 501.1 (M+H); ¹H NMR (400 MHz, DMSO-d₆) δ ppm 1H NMR 9.36 (s, 1 H), 8.62 (d, *J*=5.8 Hz, 1 H), 8.08 (d, *J*=5.3 Hz, 1 H), 8.05 (s, 1 H), 7.88 (d, *J*=5.8 Hz, 1 H), 7.32 (s, 1 H), 7.18 (dd, *J*=5.4, 1.4 Hz, 1 H), 6.67 (d, *J*=7.6 Hz, 1 H), 4.22 (dd, *J*=9.6, 8.3 Hz, 1 H), 3.81 - 4.05 (m, 7 H), 3.38 - 3.49 (m, 2 H), 3.11 - 3.22 (m, 2 H), 2.57 - 2.69 (m, 1 H), 1.86 - 2.11 (m, 6 H), 1.60 - 1.72 (m, 1 H), 1.39 - 1.53 (m, 2 H), 0.90 (d, *J*=6.8 Hz, 3 H), 0.84 (d, *J*=6.8 Hz, 3 H).

The compounds below are prepared by a similar method.

### B. (4-{1-[4-((S)-4-Isopropyl-4,5-dihydrooxazol-2-yl)piperidin-1-yl]-[2,6]naphthyridin-3-yl}-pyridin-2-yl)-(1-methyl-1H-pyrazol-3-yl)amine.

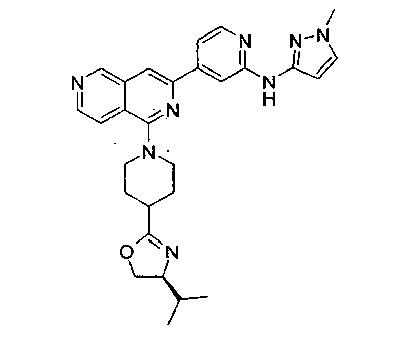

MS (ESI) *m*/*z* 497.2 (M+H); ¹H NMR (400 MHz, DMSO-*d*₆) δ ppm 9.38 (s, 1 H), 9.32 (s, 1 H), 8.63 (d, *J* = 5.8 Hz, 1 H), 8.25 (s, 1 H), 8.22 (d, *J* = 5.3 Hz, 1 H), 8.11 (s, 1 H), 7.89 (d, *J* = 5.8 Hz, 1 H), 7.53 (d, *J* = 2.3 Hz, 1 H), 7.42 (dd, *J* = 5.3, 1.5 Hz, 1 H), 6.30 (d, *J* = 2.0 Hz, 1 H), 4.26 - 4.18 (m, 1 H), 4.05 - 3.97 (m, 2 H), 3.97 - 3.91 (m, 1 H), 3.90 - 3.82 (m, 1 H), 3.77 (s, 3 H), 3.27 - 3.16 (m, 2 H), 2.69 - 2.60 (m, 1 H), 2.10 - 1.99 (m, 2 H), 2.00 - 1.89 (m, 2 H), 1.71 - 1.61 (m, 1 H), 0.89 (d, *J* = 6.8 Hz, 3 H), 0.83 (d, *J* = 6.6 Hz, 3 H).

### C. (4-{1-[4-((R)-4-Isopropyl-4,5-dihydrooxazo)-2-yl)piperidin-1-yl]-[2,6]naphthyridin-3-yl}-pyridin-2-yl)-(1-methyl-1H-pyrazol-3-yl)amine.

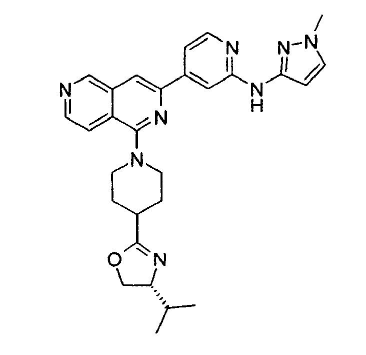

MS (ESI) *m*/*z* 497.2 (M+H); ¹H NMR (400 MHz, CDCl₃) δ ppm 9.26 (s, 1 H), 8.61 (d, *J* = 5.7 Hz, 1 H), 8.29 (d, *J* = 5.1 Hz, 1 H), 8.06 (s, 1 H), 7.80 (s, 1 H), 7.78 (d, *J* = 5.7 Hz, 1 H), 7.47 - 7.41 (m, 1 H), 7.29 (d, *J* = 2.3 Hz, 1 H), 7.02 - 6.87 (m, 1 H), 6.28 (d, *J*=2.3 Hz, 1 H), 4.31 - 4.21 (m, 1 H), 4.12 - 4.03 (m, 2 H), 4.03 - 3.90 (m, 2 H), 3.88 - 3.82 (m, 3 H); 3.23 - 3.11 (m, 2 H), 2.72 - 2.58 (m, 1 H), 2.21 - 2.02 (m, 4 H), 1.88 - 1.73 (m, 1 H), 0.98 (d, *J* = 6.8 Hz, 3 H), 0.91 (d, *J* = 6.8 Hz, 3 H).

### D. (4-{1-[4-(4,4-Dimethyl-4,5-dihydrooxazol-2-yl)-piperidin-1-yl]-[2,6]naphthyridin-3-yl}-pyridin-2-yl)-(1-methyl-1H-pyrazol-3-yl)amine.

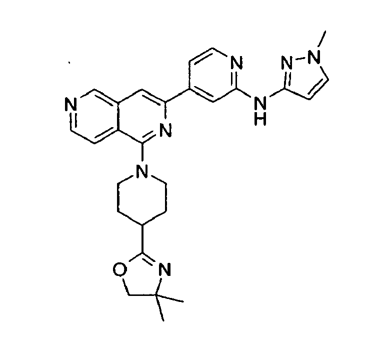

MS (ESI) *m*/*z* 483.3 (M+H); ¹H NMR (400 MHz, CD₃CN) δ ppm 9.29 (s, 1 H), 8.59 (d, *J* = 5.8 Hz, 1 H), 8.28 (s, 1 H), 8.23 (d, *J* = 6.1 Hz, 1 H), 7.96 (s, 1 H), 7.87 (d, *J* = 5.8 Hz, 1 H), 7.57 (br s, 1 H), 7.46 (dd, *J* = 5.4, 1.6 Hz, 1 H), 7.39 (d, *J* = 2.3 Hz, 1 H), 6.25 (d, *J* = 2.3 Hz, 1 H), 4.09 - 3.99 (m, 2 H), 3.91 (s, 2 H), 3.81 (s, 3 H), 3.28 - 3.16 (m, 2 H), 2.64 - 2.52 (m, 1 H), 2.11 - 2.04 (m, 2 H), 2.03 - 1.97 (m, 2 H), 1.22 (s, 6 H).

### E. (4-{1-[4-(4,4-Dimethyl-4,5-dihydrooxazol-2-yl)piperidin-1-yl]-[2,6]naphthyridin-3-yl}-pyridin-2-yl)-(tetrahydropyran-4-yl)amine.

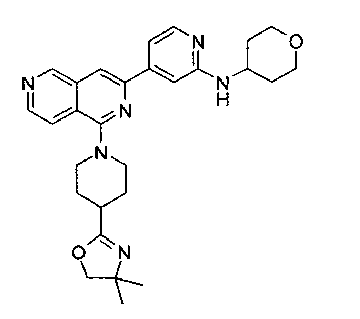

MS (ESI) *m*/*z* 487.3 (M+H); ¹H NMR (400 MHz, DMSO-*d*₆) δ ppm 9.36 (s, 1 H), 8.62 (d, *J* = 5.8 Hz, 1 H), 8.08 (d, *J* = 5.3 Hz, 1 H), 8.05 (s, 1 H), 7.87 (d, *J* = 5.8 Hz, 1 H), 7.32 (s, 1 H), 7.18 (dd, *J* = 5.6, 1.5 Hz, 1 H), 6.67 (d, *J* = 7.6 Hz, 1 H), 4.05 - 3.82 (m, 7 H), 3.48 - 3.38 (m, 2 H), 3.24 - 3.11 (m, 2 H), 2.65 - 2.55 (m, 1 H), 2.10 - 2.00 (m, 2 H), 1.98 - 1.84 (m, 4 H), 1.54 - 1.39 (m, 2 H), 1.19 (s, 6H).

### Example 13

### A. 3-[2-(Tetrahydro-pyran-4-ylamino)-pyridin-4-yl]-[2,6]naphthyridin-1-ol.

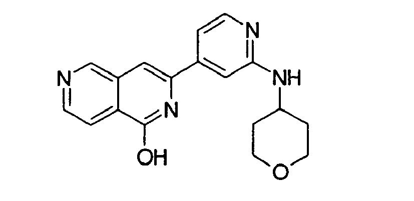

The title compound can be prepared from **1l** and 4-aminotetrahydropyran by analogy to the method outlined in **Example 6AR**: MS (ESI) *m*/*z* 323.2 (M+1).

### B. [4-(1-Chloro-[2,6]naphthyridin-3-yl)-pyridin-2-yl]-(tetrahydro-pyran-4-yl)-amine.

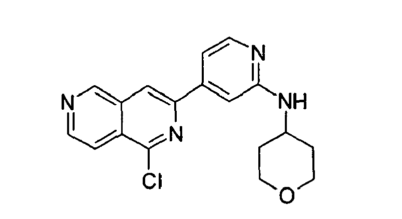

The title compound can be prepared from intermediate **13A** above, by analogy to the method outlined in **Example 3C**: MS (ESI) *m*/*z* 341.1 (M+1).

### C. 1-{3-[2-(Tetrahydropyran-4-ylamino)-pyridin-4-yl]-[2,6]naphthyridin-1-yl}-piperidine-4-carbonitrile.

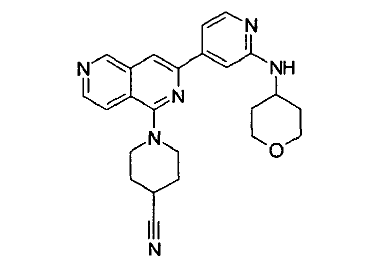

The title compound can be prepared from intermediate 2 above and 4-cyanopiperidine by analogy to the method outlined in **Example 4O**: HRMS (ESI) *m*/*z* 415.2238 (M+1); ¹H NMR (400 MHz, DMSO-*d*₆) δ ppm 9.38 (s, 1 H), 8.64 (d, *J* = 5.8 Hz, 1 H), 8.10 (s, 1 H), 8.08 (d, *J* = 5.6 Hz, 1 H), 7.89 (d, *J* = 5.8 Hz, 1 H), 7.33 (s, 1 H), 7.18 (dd, *J* = 5.6, 1.5 Hz, 1 H), 6.68 (d, *J* = 7.6 Hz, 1 H), 4.06 - 3.93 (m, 1 H), 3.92 - 3.84 (m, 2 H), 3.73 - 3.61 (m, 2 H), 3.48 - 3.36 (m, 4 H), 3.27 - 3.18 (m, 1 H), 2.24 - 2.11 (m, 2 H), 2.10 - 1.98 (m, 2 H), 1.96 - 1.86 (m, 2 H), 1.54 - 1.38 (m, 2 H).

### Example 14

### Cyclohexyl-[4-(4-methyl-1-piperazin-1-yl-[2,6]naphthyridin-3-yl)pyridin-2-yl]amine.

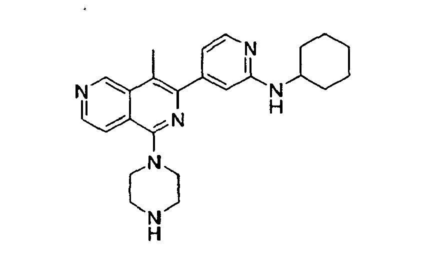

Compound of **Example 1M** can be treated by analogy to the preparation of **Example 9A** to afford the title compound: HRMS (ESI) *m*/*z* 403.2609 (M+1); ¹H NMR (400 MHz, CDCl₃) δ ppm 9.49 (d, *J* = 0.76 Hz, 1 H), 8.68 (d, *J* = 5.81 Hz, 1 H), 8.16 (dd, *J* = 5.31, 0.51 Hz, 1 H), 7.86 (dd, *J* = 5.81, 0.76 Hz, 1 H), 6.76 (dd, *J* = 5.18, 1.39 Hz, 1 H), 6.59 (s, 1 H), 4.56 (d, *J* = 7.83 Hz, 1 H), 3.67 - 3.55 (m, 1 H), 3.45 - 3.39 (m, 4 H), 3.19 - 3.11 (m, 4 H), 2.67 (s, 3 H), 2.15 - 2.05 (m, 2 H), 1.83 - 1.71 (m, 4 H), 1.70 - 1.61 (m, 1 H), 1.49 - 1.34 (m, 2 H), 1.33 - 1.22 (m, 2 H).

### Example 15

### A. 4-[4-Bromo-3-(2-chloropyridin-4-yl)-[2,6]naphthyridin-1-yl]piperazine-1-carboxylic acid tert-butyl ester.

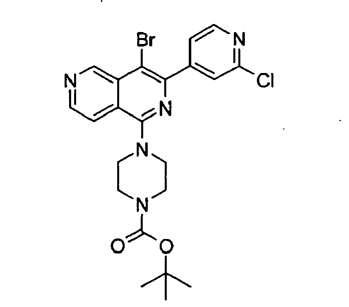

A solution of bromine (164 µL, 3.19 mmol) in methylene chloride (500 µL) is added dropwise to a stirred solution of 4-[3-(2-chloropyridin-4-yl)-[2,6]naphthyridin-1-yl]-piperazine-1-carboxylic acid *tert-*butyl ester (1.23 g, 2.9 mmol) in methylene chloride (11.6 mL) over 10 min. The reaction is judged complete within 10 min via TLC monitoring (silica gel, 80% ethyl acetate/hexanes as eluent). The reaction mixture is diluted with ethyl acetate (150 mL) and washed with saturated aqueous sodium bicarbonate, followed by saturated aqueous sodium chloride. The ethylacetate layer is dried over sodium sulfate, filtered and concentrated to give 1.43 g of product (98% yield) as a yellow foam. MS (ESI) *m*/*z* 504.12/506.14/508.20 (M+1); ¹H NMR (400 MHz, CDCl₃) δ ppm 9.72 (d, *J* = 0.51 Hz, 1 H), 8.80 (d, *J* = 5.81 Hz, 1 H), 8.52 (dd, *J* = 5.18, 0.63 Hz, 1 H), 7.80 (dd, *J* = 5.81, 0.76 Hz, 1 H), 7.75 - 7.72 (m, 1 H), 7.64 (dd, *J* = 5.18, 1.39 Hz, 1 H), 3.74 - 3.66 (m, 4 H), 3.54 - 3.45 (m, 4 H), 1.50 (s, 9 H).

### B. 4-[3-(2-Chloropyridin-4-yl)-4-phenyl-[2,6]naphthyridin-1-yl]piperazine-1-carboxylic acid tert-butyl ester.

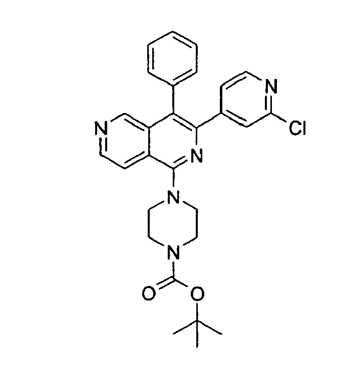

4-[4-Bromo-3-(2-chloropyridin-4-yl)-[2,6]naphthyridin-1-yl]-piperazine-1-carboxylic acid *tert-*butyl ester (94.7 mg, 0.188 mmol), phenyl boronic acid (34.38 mg, 0.282 mmol), and tetrakis triphenylphosphine palladium (21.7 mg, 0.02 mmol) are loaded as solids into a 50 mL Schlenk flask under an argon balloon. Toluene (10 mL) and ethanol (3 mL) are added and the mixture is stirred until dissolved. Sodium carbonate (136.6 mg, 1.29 mmol) is dissolved in water (5 mL) and this solution is added to the reaction mixture. The reaction mixture is degassed by evacuation and replacement of atmosphere by argon 3 times. The reaction mixture is then heated in a 100 °C oil bath. The reaction is followed by reverse phase LC/MS. After 40 min, dichloro[1,1'-bis(diphenylphosphino)ferrocene]palladium(II) dichloromethane adduct (10 mg, 0.012 mmol) is added speed. After 2 h, the reaction is removed from heat, allowed to cool, diluted with water (30 mL), and extracted with ethylacetate (2 X 30 mL). The ethylacetate layers are combined and dried over sodium sulfate, then filtered and concentrated to 172.6 mg brown oil. The residue is purified on a 12 g RediSep silica gel column using a 50% ethylacetate/hepatane to 80% gradient over 10 min. 58.9 mg of product is obtained (63% yield): MS (ESI) *m*/*z* 502.29/506.14/504.21 (M+1); ¹H NMR (400 MHz, CDCl₃) δ ppm 9.08 (s, 1 H), 8.72 (d, *J* = 5.81 Hz, 1 H), 8.17 (d, *J* = 5.31 Hz, 1 H), 7.88 (d, *J* = 5.81 Hz, 1 H), 7.52 - 7.44 (m, 3 H), 7.41 (s, 1 H), 7.31 - 7.22 (m, 2 H), 7.10 (dd, *J*=5.18, 1.39 Hz, 1 H), 3.81 - 3.69 (m, 4 H), 3.62 - 3.49 (m, 4 H), 1.52 (s, 9 H).

### C. Cyclohexyl-[4-(4-phenyl-1-piperazin-1-yl-[2,6]naphthyridin-3-yl)pyridin-2-yl]amine.

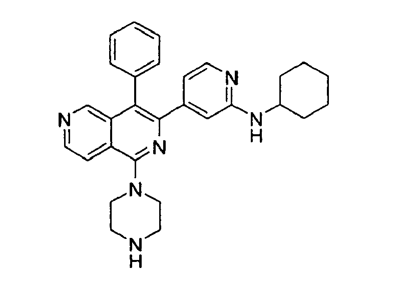

This compound is prepared from **Example 15B** above using a method analogous to that used to prepare **Example 8A**: HRMS (ESI) *m*/*z* 465.2770 (M+1); ¹H NMR (400 MHz, CDCl₃) δ ppm 9.03 (s, 1 H), 8.64 (d, *J* = 5.81 Hz, 1 H), 7.94 (d, *J* = 5.31 Hz, 1 H), 7.90 - 7.84 (m, 1 H), 7.48 - 7.37 (m, 3 H), 7.32 - 7.27 (m, 2 H), 6.67 (dd, *J* = 5.31, 1.52 Hz, 1 H), 6.33 (s, 1 H), 4.38 (d, *J* = 8.08 Hz, 1 H), 3.59 - 3.50 (m, 4 H), 3.24 - 3.15 (m, 4 H), 3.15 - 3.03 (m, 1 H), 1.88 - 1.58 (m, 6 H), 1.38 - 1.02 (m, 5 H).

The compounds below are prepared by a similar method.

### D. Cyclohexyl-{4-[4-(4-fluorophenyl)-1-piperazin-1-yl-[2,6]naphthyridin-3-yl]-pyridin-2-yl}-amine.

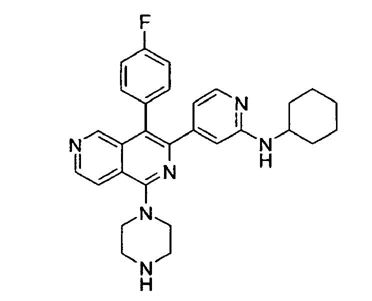

HRMS (ESI) *m*/*z* 483.2661 (M+1); ¹H NMR (400 MHz, CDCl₃) δ ppm 8.98 (s, 1 H), 8.63 (d, *J* = 5.8 Hz, 1 H), 7.93 (d, *J* = 5.3 Hz, 1 H), 7.85 (d, *J* = 5.8 Hz, 1 H), 7.24 (dd, *J* = 8.1, 5.1 Hz, 2 H), 7.12 (t, *J* = 8.6 Hz, 2 H), 6.59 (d, *J* = 5.3 Hz, 1 H), 6.28 (s, 1 H), 4.44 (br d, *J* = 8.1 Hz, 1 H), 3.59 - 3.49 (m, 4 H), 3.23 - 3.14 (m, 4 H), 3.14 - 3.05 (m, 1 H), 2.06 (br s, 1 H), 1.91 - 1.77 (m, 2 H), 1.77 - 1.55 (m, 3 H), 1.39 - 1.00 (m, 4 H).

### E. Cyclohexyl-[4-(1-piperazin-1-yl-4-p-tolyl-[2,6]naphthyridin-3-yl)-pyridin-2-yl]-amine.

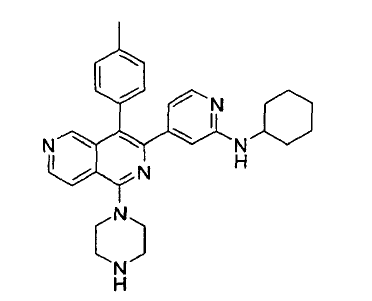

HRMS (ESI) *m*/*z* 479.2933 (M+1); ¹H NMR (400 MHz, CDCl₃) δ ppm 9.04 (d, *J*=1.0 Hz, 1 H), 8.62 (d, *J* = 5.8 Hz, 1 H), 7.94 (dd, *J* = 5.3, 0.8 Hz, 1 H), 7.85 (dd, *J* = 5.8, 1.0 Hz, 1 H), 7.25 - 7.20 (m, 2 H), 7.19 - 7.11 (m, 2 H), 6.69 (dd, *J* = 5.3, 1.5 Hz, 1 H), 6.31 (s, 1 H), 4.39 (br d, *J* = 8.1 Hz, 1 H), 3.58 - 3.48 (m, 4 H), 3.23 - 3.12 (m, 4 H), 3.12 - 2.98 (m, 1 H), 2.41 (s, 3 H), 1.89 - 1.75 (br m, 2 H), 1.75 - 1.56 (m, 3 H), 1.38 - 1.15 (m, 3 H), 1.15 - 1.00 (m, 2 H).

### F. Cyclohexyl-{4-[4-(4-trifluoromethylphenyl)-1-piperazin-1-yl-[2,6]naphthyridin-3-yl]-pyridin-2-yl}-amine.

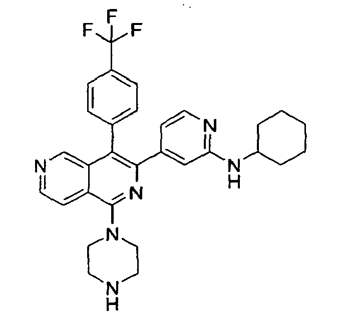

HRMS (ESI) *m*/*z* 532.6176 (M+1); ¹H NMR (400 MHz, CDCl₃) δ ppm 8.95 (d, *J* = 1.0 Hz, 1 H), 8.66 (d, *J* = 5.8 Hz, 1 H), 7.95 (d, *J* = 5.3 Hz, 1 H), 7.87 (dd, *J* = 5.7, 0.9 Hz, 1 H), 7.70 (d, *J* = 8.1 Hz, 2 H), 7.43 (d, *J* = 7.8 Hz, 2 H), 6.60 (dd, *J* = 5.3, 1.3 Hz, 1 H), 6.20 (s, 1 H), 4.43 (br d, *J* = 7.8 Hz, 1 H), 3.60 - 3.51 (m, 4 H), 3.22 - 3.14 (m, 4 H), 3.12 - 2.99 (m, 1 H), 1.84 - 1.73 (m, 2 H), 1.74 - 1.56 (m, 3 H), 1.35 - 1.15 (m, 3 H), 1.15 - 1.00 (m, 2 H).

### G. Cyclohexyl-{4-[4-(3-fluorophenyl)-1-piperazin-1-yl-[2,6]naphthyridin-3-yl]-pyridin-2-yl}-amine.

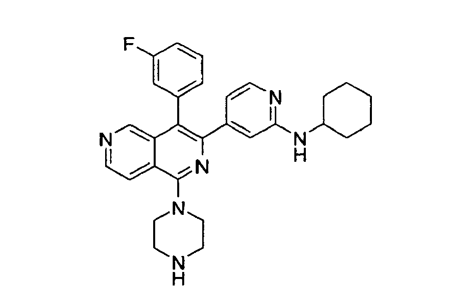

HRMS (ESI) *m*/*z* 483.2691 (M+1); ¹H NMR (400 MHz, CDCl₃) δ ppm 8.98 (d, *J* = 0.8 Hz, 1 H), 8.64 (d, *J* = 5.8 Hz, 1 H), 7.94 (dd, *J* = 5.4, 0.6 Hz, 1 H), 7.86 (dd, *J* = 5.8, 0.8 Hz, 1 H), 7.40 (dt, *J* = 8.0, 6.1 Hz, 1 H), 7.14 - 7.08 (m, 1 H), 7.08 - 7.00 (m, 2 H), 6.62 (dd, *J* = 5.3, 1.5 Hz, 1 H), 6.33 (s, 1 H), 4.43 (br d, *J* = 7.8 Hz, 1 H), 3.60 - 3.48 (m, 4 H), 3.22 - 3.15 (m, 4 H), 3.15 - 3.06 (m, 1 H), 1.94 - 1.77 (m, 2 H), 1.77 - 1.57 (m, 3 H), 1.39 - 1.15 (m, 3 H), 1.15 - 1.03 (m, 2 H).

### H. Cyclohexyl-{4-[4-(3-chlorophenyl)-1-piperazin-1-yl-[2,6]naphthyridin-3-yl]-pyridin-2-yl}-amine.

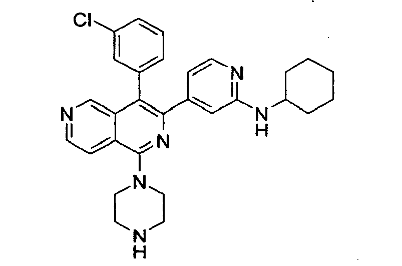

HRMS (ESI) *m*/*z* 499.2382 (M+1); ¹H NMR (400 MHz, CDCl₃) δ ppm 8.98 (d, *J* = 0.8 Hz, 1 H), 8.64 (d, *J* = 5.8 Hz, 1 H), 7.95 (d, *J* = 5.3 Hz, 1 H), 7.85 (dd, *J* = 5.8, 0.8 Hz, 1 H), 7.41 - 7.37 (m, 1 H), 7.37 - 7.31 (m, 2 H), 7.14 (dt, *J* = 7.2, 1.5, 1.4 Hz, 1 H), 6.63 (dd, *J* = 5.3, 1.5 Hz, 1 H), 6.30 (s, 1 H), 4.43 (br d, *J* = 8.1 Hz, 1 H), 3.58 - 3.50 (m, 4 H), 3.21 - 3.14 (m, 4 H), 3.15 - 3.05 (m, 1 H), 1.91 - 1.78 (m, 2 H), 1:76 - 1.57 (m, 3 H), 1.39 - 1.15 (m, 3 H), 1.15 -1.03(m,2H).

### 1. Cyclohexyl-{4-[4-(4-chlorophenyl)-1-piperazin-1-yl-[2,6]naphthyridin-3-yl]-pyridin-2-yl}-amine.

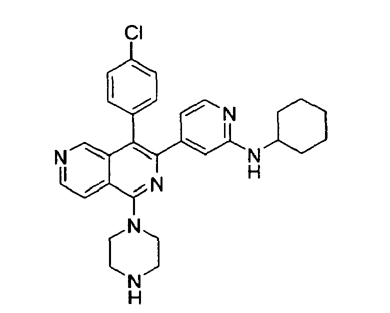

HRMS (ESI) *m*/*z*499.2382 (M+1); ¹H NMR (400 MHz, CDCl₃) δ ppm 8.99 (s, 1 H), 8.64 (d, *J* = 5.8 Hz, 1 H), 7.95 (d, *J* = 5.3 Hz, 1 H), 7.86 (dd, *J* = 5.8, 0.5 Hz, 1 H), 7.44 - 7.39 (m, 2 H), 7.25 - 7.20 (m, 2 H), 6.64 (dd, *J* = 5.3, 1.3 Hz, 1 H), 6.25 (s, 1 H), 4.48 (br d, *J* = 7.8 Hz, 1 H), 3.57 - 3.51 (m, 4 H), 3.21 - 3.14 (m, 4 H), 3.13 - 3.01 (m, 1 H), 1.86 - 1.77 (m, 2 H), 1.77 - 1.56 (m, 3 H), 1.37 - 1.16 (m, 3 H), 1.16 - 1.03 (m, 2 H).

### J. Cyclohexyl-{4-[4-(3-methoxyphenyl)-1-piperazin-1-yl-[2,6]naphthyridin-3-yl]-pyridin-2-yl}-amine.

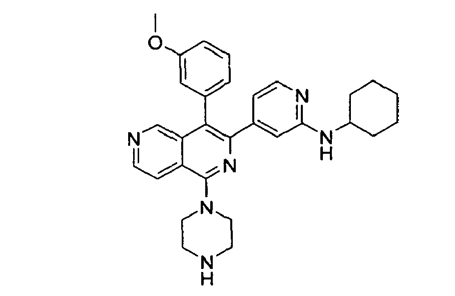

HRMS (ESI) *m*/*z* 495.2878 (M+1); ¹H NMR (400 MHz, CDCl₃) δ ppm 9.04 (d, *J* = 0.8 Hz, 1 H), 8.63 (d, *J* = 5.6 Hz, 1 H), 7.94 (d, *J* = 5.3 Hz, 1 H), 7.85 (dd, *J* = 5.7, 0.9 Hz, 1 H), 7.38 - 7.31 (m, 1 H), 6.94 (ddd, *J* = 8.3, 2.5, 0.8 Hz, 1 H), 6.88 (dt, *J* = 7.6, 1.1 Hz, 1 H), 6.82 (dd, *J* = 2.4, 1.6 Hz, 1 H), 6.69 (dd, *J* = 5.4, 1.4 Hz, 1 H), 6.38 (s, 1 H), 4.40 (br d, *J* = 7.8 Hz, 1 H), 3.76 (s, 3 H), 3.56 - 3.50 (m, 4 H), 3.22 - 3.15 (m, 4 H), 3.16 - 3.04 (m, 1 H), 1.89 - 1.58 (m, 5 H), 1.37 - 1.15 (m, 3 H), 1.15 - 1.03 (m, 2 H).

### K. [3-(2-Cyclohexylaminopyridin-4-yl)-1-piperazin-1-yl-[2,6]naphthyridin-4-yl]-phenylamine.

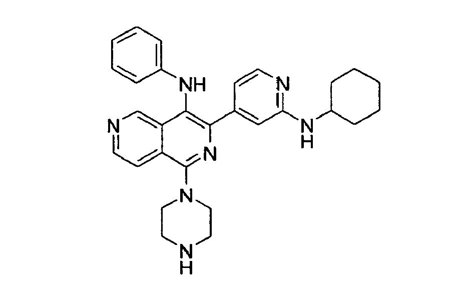

HRMS (ESI) *m*/*z* 480.2882 (M+1); ¹H NMR (400 MHz, CDCl₃) δ ppm 9.35 (d, *J* = 1.0 Hz, 1 H), 8.65 (d, *J* = 5.6 Hz, 1 H), 8.09 (d, *J* = 5.3 Hz, 1 H), 7.84 (dd, *J* = 5.8, 1.0 Hz, 1 H), 7.24 - 7.16 (m, 2 H), 6.92 - 6.82 (m, 2 H), 6.70 - 6.60 (m, 3 H), 5.53 (br s, 1 H), 4.53 (br d, *J* = 8.1 Hz, 1 H), 3.54 - 3.46 (m, 4 H), 3.23 - 3.05 (m, 5 H), 1.96 - 1.75 (m, 4 H), 1.70 - 1.50 (m, 3 H), 1.27 - 1.04 (m, 3 H).

### Example 16

### A. 1-[4-Bromo-3-(2-chloropyridin-4-yl)-[2,6]naphthyridin-1-yl]-piperidine-4-carboxylic acid methyl ester.

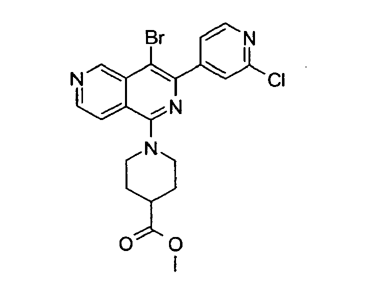

A solution of bromine (3.43 mL, 1 M CH₂Cl₂) is added to a solution of 1-[3-(2-chloropyridin-4-yl)-[2,6]naphthyridin-1-yl]-piperidine-4-carboxylic acid methyl ester (1.25 g, 3.27 mmol) and CH₂Cl₂ (20 mL) at room temperature. After 10 min the solution is quenched by the addition of a 1:1 combination of 5% aqueous Na₂S₂O₃ and 5 % aqueous NaHCO₃ (150 mL). The resulting mixture is then diluted with CH₂Cl₂ (150 mL). The layers are then separated and the aqueous phase is extracted further with CH₂Cl₂ (2 X 150 mL). The combined organic layers are then dried (Na₂SO₄), filtered and concentrated. The residue is then separated by flash chromatography (50% EtOAc/heptane) to give 1-[4-Bromo-3-(2-chloropyridin-4-yl)-[2,6]naphthyridin-1-yl]-piperidine-4-carboxylic acid methyl ester: MS (ESI) m/z 460.9, 462.8 & 464.8 (M+1).

### B. 1-[4-Bromo-3-(2-chloropyridin-4-yl)-[2,6]naphthyridin-1-yl]-piperidine-4-carboxylic acid isobutyl amide.

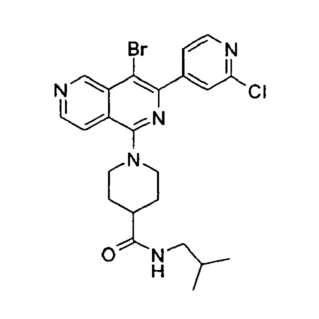

To a solution of 1-[4-bromo-3-(2-chloropyridin-4-yl)-[2,6]naphthyridin-1-yl]-piperidine-4-carboxylic acid methyl ester (1.13 g, 2.45 mmol), THF (25 mL), and water (5 mL) is added LiOH·H₂0 (0.514 g, 12.2 mmol). After 5 h, additional water (5 mL) and LiOH·H₂0 (2 equivalents) are added. After an additional 1 h, HCl in Et₂O (17.1 mL, 1 M) is added. The resulting mixture is then dried *in vacuo* to give crude 1-[4-bromo-3-(2-chloropyridin-4-yl)-[2,6]naphthyridin-1-yl]-piperidine-4-carboxylic acid.

To a suspension of 1-[4-bromo-3-(2-chloropyridin-4-yl)-[2,6]naphthyridin-1-yl]-piperidine-4-carboxylic acid (2.45 mmol) in DMF (15 mL) and CH₂Cl₂ (15 mL) is added isobutyl amine (0.73 mL, 7.34 mmol) and PyBOP® (3.82 g, 7.34 mmol). After 2 h the solvent is removed *in vacuo* and the residue separated by flash chromatography (30-100% EtOAc/heptane) to give 1-[4-bromo-3-(2-chloropyridin-4-yl)-[2,6]naphthyridin-1-yl]-piperidine-4-carboxylic acid isobutyl amide: MS (ESI) m/z 501.9, 503.9, 506.0 (M+1).

### C. 1-[3-(2-Chloropyridin-4-yl)-4-(4-fluoro-phenyl)-[2,6]naphthyridin-1-yl]-piperidine-4-carboxylic acid isobutyl amide.

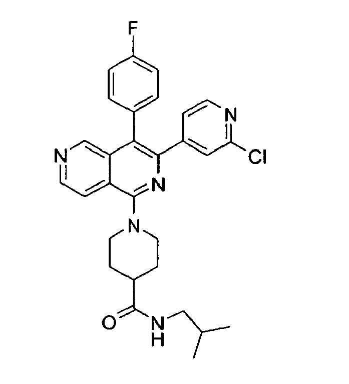

A mixture of 1-[4-bromo-3-(2-chloropyridin-4-yl)-[2,6]naphthyridin-1-yl]-piperidine-4-carboxylic acid isobutyl amide (0.390 g, 0.776 mmol), 4-fluorophenyl boronic acid (0.108 g, 0.776 mmol), Pd(dppf)Cl₂·CH₂Cl₂ (0.032g, 0.038 mmol), aqueous Na₂CO₃ (0.78 mL, 2 M), and DME (10 mL) is heated to 90 °C for 3 h. The solvents are then removed *in vacuo* and the residue separated by flash chromatography (30-100% EtOAc/heptane) to give 1-[3-(2-chloropyridin-4-yl)-4-(4-fluoro-phenyl)-[2,6]naphthyridin-1-yl]-piperidine-4-carboxylic acid isobutyl amide: MS (ESI) m/z 518.1 (M+1).

### D. 1-[3-(2-Cyclohexylaminopyridin-4-yl)-4-(4-fluorophenyl)-[2,6]naphthyridin-1-yl]-piperidine-4-carboxylic acid isobutyl-amide.

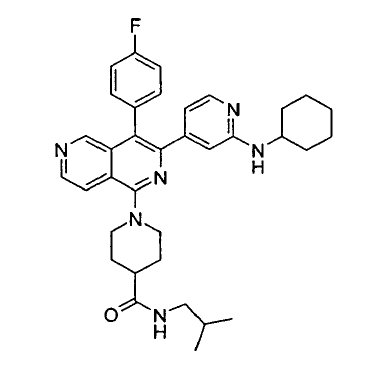

Title compound is prepared from 1-[3-(2-chloropyridin-4-yl)-4-(4-fluorophenyl)-[2,6]naphthyridin-1-yl]-piperidine-4-carboxylic acid isobutyl amide by analogy to **Example 6AR**: MS (ESI) m/z 581.2 (M+1); ¹H NMR (400 MHz, *DMSO-d*₆*)* δ ppm 8.78 (s, 1 H), 8.66 (d, *J*=5.7 Hz, 1 H), 7.94 (d, *J*=5.8 Hz, 1 H), 7.80 - 7.88 (m, 1 H), 7.75 (d, *J*=5.3 Hz, 1 H), 7.33 - 7.43 (m, 2 H), 7.25 - 7.34 (m, 2 H), 6.48 (s, 1 H), 6.21 - 6.30 (m, 2 H), 3.90 - 4.03 (m, 2 H), 3.33 - 3.48 (m, 1 H), 2.97 - 3.10 (m, 2 H), 2.91 (t, *J*=6.3 Hz, 2 H), 2.37 - 2.47 (m, 1 H), 1.75 - 2.05 (m, 6 H), 1.62 - 1.74 (m, 3 H), 1.54 - 1.62 (m, 1 H), 1.20 - 1.35 (m, 2 H), 1.04 - 1.20 (m, 3 H), 0.85 (d, 6 H).

### L. 1-[3-(2-Cyclohexylamino-pyridin-4-yl)-4-methyl-[2,6]naphthyridin-1-yl]-piperidine-4-carboxylic acid isobutyl-amide.

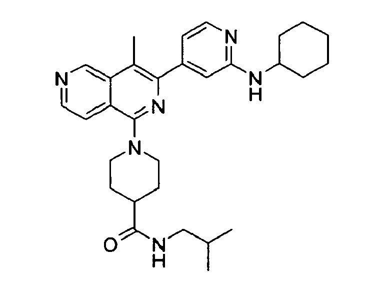

To 1-[4-bromo-3-(2-chloropyridin-4-yl)-[2,6]naphthyridin-1-yl]-piperidine-4-carboxylic acid isobutyl amide described above (320 mg, 0.636 mmol) in DMF (6.5 mL) is added K₂CO₃ (264 mg, 1.91 mmol), trimethylboroxine (80 mg, 0.64 mmol). The mixture is degassed with nitrogen before Pd(PPh₃)₄ (74 mg, 0.0636 mmol) is added. The reaction is sealed and heated at 120 °C overnight. After cooling, the reaction is partitioned between EtOAc and saturated aqueous NaHCO₃. The separated organic phase is washed with brine, dried over Na₂SO₄, and concentrated *in vacuo.* To a solution of the crude residue in dioxane (7 mL) is added cyclohexylamine (0.44 mL, 3.82 mmol) and NaO*t*Bu (122 mg, 1.28 mmol). The solution is degassed with nitrogen before the addition of Pd ((*t*Bu₃P)P)₂ (65 mg, 0.127 mmol). The reaction is heated at 130 °C for 3 h. After cooling, the reaction is filtered over Celite, using EtOAc and CH₂Cl₂ to wash the filter cake. The filtrates are diluted with CH₂Cl₂ and water. The separated organic phase is washed with brine (3x), dried over Na₂SO₄, and concentrated *in vacuo.* The residue is purified by HPLC suing an RP₁₈x-bridge 30x100 mm column and gradient solvent system from 40 to 90% CH₃CN/water to afford the title compound: MS (ESI) m/z 501.3 (M+1); ¹H NMR (400 MHz, MeOD) δ ppm 0.92 (d, *J*=6.6 Hz, 6 H), 1.16 - 1.35 (m, 4 H), 1.37 - 1.53 (m, 3 H), 1.59 - 1.72 (m, 1 H), 1.72 - 1.85 (m, 4 H), 1.84 - 1.96 (m, 3 H), 1.98 - 2.14 (m, 4 H), 2.39 - 2.54 (m, 1 H), 2.63 (s, 3 H), 2.95 - 3.08 (m, 5 H), 3.63 - 3.73 (m, 1 H), 3.90 - 3.98 (m, 2 H), 6.72 (s, 2 H), 9.46 (s, 1 H).

### Example 17

**A. [5-Bromo-4-(1-chloro-[2,6]naphthyridin-3-yl)-pyridin-2-yl]-cyclohexyl-amine.**

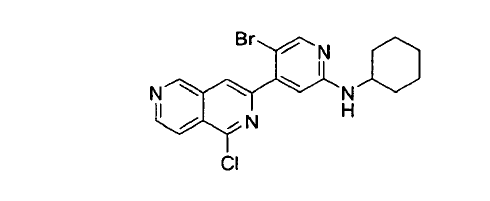

To a solution of [4-(1-chloro-[2,6]naphthyridin-3-yl)-pyridin-2-yl]-cyclohexyl-amine **Example 3C** above (230 mg, 0.678 mmol) in DMF (3.4 mL) at -10 °C, is added dropwise a solution of NBS (120 mg, 0.67 mmol) in DMF. The reaction is diluted with CH₂Cl₂ and a saturated aqueous solution of NaCl. The separated organic phase is washed with brine (3x), dried over Na₂SO₄, and concentrated under reduced pressure. The residue is purified by flash chromatography using 1 to 5% MeOH/CH₂Cl₂ to afford 300 mg of the title bromide: MS (ESI) m/z 417.1, 419.1 (M+1).

### B. Cyclohexyl-[3,5-dibromo-4-(1-chloro-[2,6]naphthyridin-3-yl)-pyridin-2-yl]-amine.

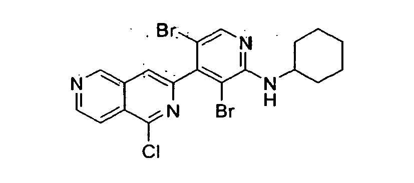

To a solution of [4-(1-chloro-[2,6]naphthyridin-3-yl)-pyridin-2-yl]-cyclohexyl-amine **Example 3C** above (100 mg, 0.295 mmol) in DMF (3.0 mL) is added NBS (108 mg, 0.6 mmol). After 18 h, the reaction is partitioned between EtOAc and a saturated aqueous solution of NaHCO₃. The separated organic layer is washed (3x) with brine, dried (Na₂SO₄), and concentrated under reduced pressure to afford the title dibromide: MS (ESI) m/z 495.0, 497.0, 499.0 (M+1).

### C. [5-Chloro-4-(1-chloro-[2,6]naphthyridin-3-yl)-pyridin-2-yl]-cyclohexyl-amine and [3-chloro-4-(1-chloro-[2,6]naphthyridin-3-yl)-pyridin-2-yl]-cyclohexyl-amine.

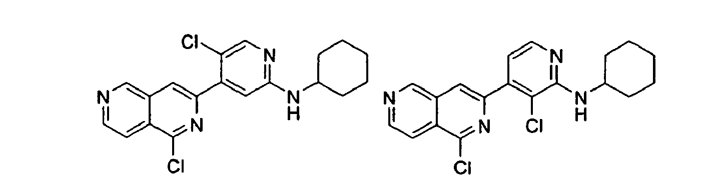

A solution of [4-(1-chloro-[2,6]naphthyridin-3-yl)-pyridin-2-yl]-cyclohexyl-amine **Example 3C** above (338 mg, 1.00 mmol) and NCS (134 mg, 1.00 mmol) in DMF (10 mL) is heated at 80 °C. After cooling, the reaction is concentrated *in vacuo* and the residue diluted with EtOAc and water. The separated organic phase is washed with brine, dried over Na₂SO₄, and concentrated under reduced pressure to afford a 4:1 mixture of the title compounds, with the 5-chloropyridine as the major regioisomers: MS (ESI) m/z 373.2 and 375.2 (M+1).

### D. 4-[3-(5-Bromo-2-cyclohexylamino-pyridin-4-yl)-[2,6]naphthyridin-1-yl]-piperazine-1-carboxylic acid tert-butyl ester.

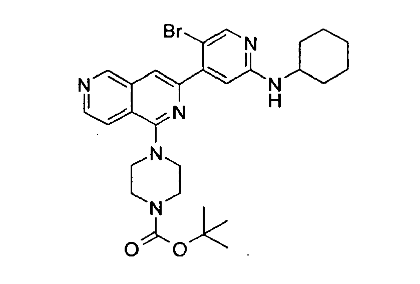

The title compound is prepared from **Example 17A** above and piperazine-1-carboxylic acid t-butyl ester by analogy to the method outlined for the preparation of **Example 4O:** MS (ESI) m/z 567.3 and 569.2 (M+1).

### E. 1-[3-(5-Bromo-2-cyclohexylamino-pyridin-4-yl)-[2,6]naphthyridin-1-yl]-piperidine-4-carboxylic acid ethyl ester.

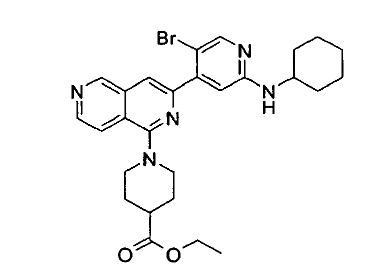

The title compound is prepared from **Example 17A** above and piperidine-4-carboxylic acid ethyl ester by analogy to the method outlined for the preparation of **Example 4S**: MS (ESI) m/z 538.4 and 540.4 (M+1).

### F. 1-[3-(5-Bromo-2-cyclohexylamino-pyridin-4-yl)-[2,6]naphthyridin-1-yl]-piperidine-4-carboxylic acid isopropylamide.

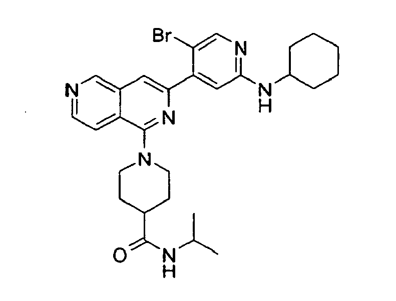

The title compound is prepared from **Example 17E** by hydrolysis of the ethyl ester using LiOH·H₂O and HATU coupling of the resultant carboxylic acid to isopropyl amine yielded the *N*-isopropyl amide. The amide is purified using RP HPLC on an X-Bridge RP₁₈ 30x100 mm column using a gradient of 30 to 100% CH₃CN/H₂O: MS (ESI) m/z 551.2139 (M+1); ¹H NMR (400 MHz, *DMSO-d*₆) δ ppm 1.05 (d, *J*=6.6 Hz, 6 H), 1.12 - 1.40 (m, 5 H), 1.54 - 1.65 (m, 1 H), 1.67 - 1.77 (m, 2 H), 1.78 - 1.99 (m, 6 H), 2.29 - 2.40 (m, 1 H), 2.91 - 3.04 (m, 2 H), 3.64 - 3.76 (m, 1 H), 3.80 - 3.91 (m, 1 H), 3.93 - 4.02 (m, 2 H), 6.70 - 6.80 (m, 2 H), 7.68 (d, *J*=7.6 Hz, 1 H), 7.72 (s, 1 H), 7.89 (d, *J*=5.8 Hz, 1 H), 8.16 (s, 1 H), 8.65 (d, *J*=5.8 Hz, 1 H), 9.36 (s, 1 H).

### G. [5-Bromo-4-(1-piperazin-1-yl-[2,6]naphthyridin-3-yl)-pyridin-2-yl]-cyclohexyl-amine.

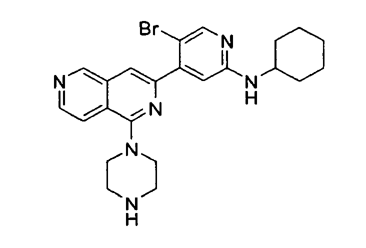

The title compound is prepared from **Example 17D** (125 mg, 0.22 mmol) by removal of the BOC-protecting group using 4N HCl in dioxane at room temperature. The volatiles are removed under reduced pressure and the residue partitioned between CH₂Cl₂ and a saturated aqueous solution of NaHCO₃. The separated organic phase is washed with brine, dried (Na₂SO₄), and concentrated under reduced pressure. The residue is purified by RP HPLC using 5 to 100% CH₃CN/H₂O to afford the title compound: MS (ESI) m/z 469.1505 (M+1); ¹H NMR (400 MHz, MeOD) δ ppm 1.27 - 1.53 (m, 5 H), 1.68 - 1.76 (m, 1 H), 1.82 - 1.90 (m, 2 H), 2.02 - 2.11 (m, 2 H), 3.50 - 3.56 (m, 4 H), 3.59 - 3.69 (m, 1 H), 3.78 - 3.86 (m, 4 H), 7.27 (s, 1 H), 8.02 (s, 1 H), 8.12 (d, *J*=6.1 Hz, 1 H), 8.16 (s, 1 H), 8.75 (d, *J*=5.9 Hz, 1 H), 9.43 (s, 1 H).

### H. [5-Chloro-4-(1-piperazin-1-yl-[2,6]naphthyridin-3-yl)-pyridin-2-yl]-cyclohexyl-amine.

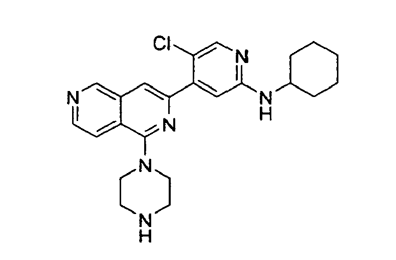

The title compound is prepared from the mixture of chlorides from **Example 17C** and piperazine-1-carboxylic acid *t*-butyl ester by analogy to the method outlined for the preparation of **Example 40**, followed by removal of the BOC-protecting group as in **Example 17G:** MS (ESI) m/z 423.2061 (M+1); ¹H NMR (400 MHz, MeOD) δ ppm 1.25 - 1.58 (m, 5 H), 1.65 - 1.77 (m, 1 H), 1.82 - 1.92 (m, 2H), 2.03 - 2.13 (m, 2 H), 3.48 - 3.60 (m, 4 H), 3.66 - 3.75 (m, 1 H), 3.92 - 4.02 (m, 4 H), 7.65 (s, 1 H), 8.11 (s, 1 H), 8.41 (s, 1 H), 8.76 (d, *J*=6.6 Hz, 1 H), 8.86 (d, *J*=6.6 Hz, 1 H), 9.97 (s, 1 H).

### I. 1-[3-(5-Chloro-2-cyclohexylamino-pyridin-4-yl)-[2,6]naphthyridin-1-yl]-piperidine-4-carboxylic acid isopropylamide.

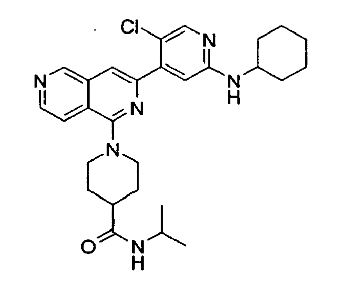

The title compound is prepared from the mixture of chlorides as **Example 17C** above and piperidine-4-carboxylic acid ethyl ester by analogy to the method outlined for the preparation of **Example 17F.** Hydrolysis of the methyl ester using LiOH·H₂O and coupling to isopropyl amine yielded the *N*-isopropyl amide. The amides are separated by RP HPLC using an X-Bridge C₁₈ 30 x 50 mm column and 25-85% CH₃CN/H₂O: MS (ESI) m/z 507.264 and 509.264 (M+1); ¹H NMR (400 MHz, MeOD) δ ppm 1.15 (d, *J*=6.8 Hz, 6 H), 1.19 - 1.35 (m, 3 H), 1.36 - 1.50 (m, 2 H), 1.61 - 1.72 (m, 1 H), 1.73 - 1.84 (m, 2 H), 1.85 - 1.94 (m, 2 H), 1.98 - 2.14 (m, 4 H), 2.37 - 2.52 (m, 1 H), 3.01 - 3.14 (m, 2 H), 3.61 - 3.72 (m, 1 H), 3.94 - 4.03 (m, 1 H), 4.04 - 4.16 (m, 2 H), 6.88 (s, 1 H), 7.81 (s, 1 H), 7.96 - 8.00 (m, 2 H), 8.61 (d, *J*=6.1 Hz, 1 H), 9.26 (s, 1 H).

### J. 1-[3-(3-Chloro-2-cyclohexylamino-pyridin-4-yl)-[2,6]naphthyridin-1-yl]-piperidine-4-carboxylic acid isopropylamide.

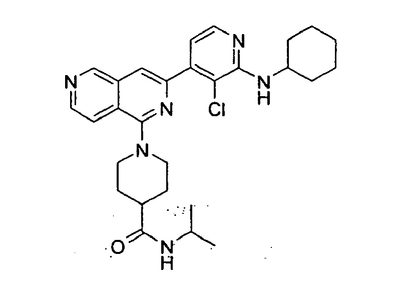

The title compound is prepared from the mixture of chlorides as **Example 17C** above and piperidine-4-carboxylic acid ethyl ester by analogy to the method outlined for the preparation of **Example 17F.** Hydrolysis of the methyl ester using LiOH·H₂O and coupling to isopropyl amine yields the *N*-isopropyl amide. The title compound is isolated by RP HPLC as the minor component: MS (ESI) m/z 507.26 and 509.26 (M+1); ¹H NMR (400 MHz, MeOD) δ ppm 1.15 (d, *J*=6.8 Hz, 6 H), 1.24 - 1.54 (m, 5 H), 1.64 - 1.74 (m, 1 H), 1.76 - 1.93 (m, 4 H), 1.98 - 2.14 (m, 4 H), 2.37 - 2.50 (m, 1 H), 2.98 - 3.14 (m, 2 H), 3.87 - 4.03 (m, 2 H), 4.02 - 4.12 (m, 2 H), 6.85 (d, *J*=5.3 Hz, 1 H), 7.71 (s, 1 H), 7.92 - 8.04 (m, 2 H), 8.60 (d, *J*=5.8 Hz, 1 H), 9.27 (s, 1 H).

### K. [3,5-Dibromo-4-(1-piperazin-1-yl-[2,6]naphthyridin-3-yl)-pyridin-2-yl]-cyclohexylamine.

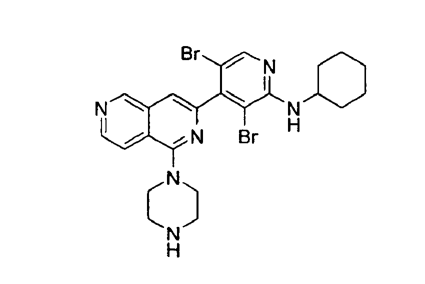

The title compound is prepared from **Example 17B** above and piperazine-1-carboxylic acid t-butyl ester by analogy to the method outlined for the preparation of **Example 17G:** MS (ESI) m/z 545.0649, 547.0550, and 549.0590 (M+1); ¹H NMR (400 MHz, CDCl₃) δ ppm 1.11 I - 1.37 (m, 4 H), 1.37 - 1.53 (m, 2 H), 1.58 - 1.82 (m, 2 H), 1.97 - 2.14 (m, 2 H), 3.00 - 3.20 (m, 4 H), 3.45 - 3.60 (m, 4 H), 3.88 - 4.04 (m, 1 H), 5.06 - 5.24 (m, 1 H), 7.29 (s, 1 H), 7.83 (d, *J*=5.8 Hz, 1 H), 8.23 (s, 1 H), 8.65 (d, *J*=5.8 Hz, 1 H), 9.23 (s, 1 H).

### L. [5-Methyl-4-(1-piperazin-1-yl-[2,6]naphthyridin-3-yl)-pyridin-2-yl]-cyclohexyl-amine.

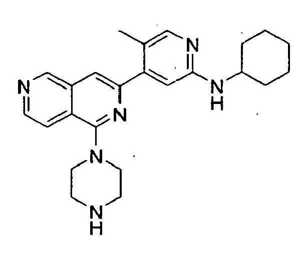

4-[3-(5-Bromo-2-cyclohexylamino-pyridin-4-yl)-[2,6]naphthyridin-1-yl]-piperazine-1-carboxylic acid *tert-*butyl ester **Example 17D** above is reacted with trimethylboroxine by analogy to **Example 16L.** Removal of the BOC group using the protocol outlined in **Example 17G** yields the title compound: MS (ESI) m/z 403.2629 (M+1); ¹H NMR (400 MHz, *DMSO-d*₆) δ ppm 1.09 - 1.42 (m, 5 H), 1.53 - 1.65 (m, 1 H), 1.66 - 1.77 (m, 2 H), 1.89 - 1.97 (m, 2 H), 2.18 (s, 3 H), 2.91 - 3.03 (m, 4 H), 3.33 - 3.40 (m, 4 H), 3.61 - 3.78 (m, 1 H), 6.20 (d, *J*=8.1 Hz, 1 H), 6.66 (s, 1 H), 7.64 (s, 1 H), 7.79 - 7.96 (m, 2 H), 8.63 (d, *J*=5.8 Hz, 2 H), 9.30 (s, 1 H).

### M. [5-Phenyl-4-(1-piperazin-1-yl-[2,6]naphthyridin-3-yl)-pyridin-2-yl]-cyclohexyl-amine.

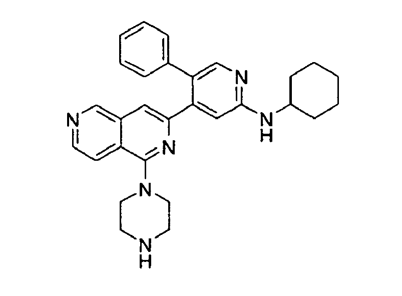

A mixture of the 4-[3-(5-bromo-2-cyclohexylamino-pyridin-4-yl)-[2,6]naphthyridin-1-yl]-piperazine-1-carboxylic acid *tert-*butyl ester from **Example 17D** above (200 mg, 0.352 mmol), Na₂CO₃ (254 mg, 2.40 mmol), phenyl boronic acid (64 mg, 0.52 mmol) in toluene (14 mL), EtOH (4.5 mL), and water (7.3 mL) is degassed with nitrogen. The precatalyst PdCl₂(dppf)₂ (26 mg, 0.035 mmol) is added and the reaction is heated at 100 °C for 3 h. After cooling, the reaction is filtered through Celite and the filtrate is diluted with CH₂Cl₂ and a saturated aqueous solution of NaHCO₃. The separated organic phase is washed with brine, dried over Na₂SO₄ and concentrated *in vacuo.* The residue is purified by flash chromatography using 2.5 to 5% MeOH/CH₂Cl₂ to afford 170 mg of the BOC-protected title compound. Removal of the BOC group is effected in 4 N HCl in dioxane (10 mL) and CH₂Cl₂ (2.0 mL). After complete reaction, the volatiles are removed *in vacuo,* and the residue is purified by HPLC to afford the title compound: MS (ESI) m/z 465.2776 (M+1); ¹H NMR (400 MHz, CDCl₃) δ ppm 1.18 - 1.37 (m, 3 H), 1.35 - 1.51 (m, 2 H), 1.57 - 1.71 (m, 1 H), 1.71 - 1.90 (m, 3 H), 2.06 - 2.20 (m, 2 H), 2.86 - 3.01 (m, 4 H), 3.06 - 3.19 (m, 4 H), 3.59 - 3.75 (m, 1 H), 4.61 (d, *J*=8.1 Hz, 1 H), 6.77 (s, 1 H), 7.05 - 7.13 (m, 2 H), 7.15 - 7.21 (m, 3 H), 7.27 (d, *J*=0.6 Hz, 1 H), 7.72 (d, *J*=5.8 Hz, 1 H), 8.14 (s, 1 H), 8.55 (d, *J*=5.8 Hz, 1 H), 9.05 (s, 1 H).

### N. [5-Amino-4-(1-piperazin-1-yl-[2,6]naphthyridin-3-yl)-pyridin-2-yl]-cyclohexyl-amine.

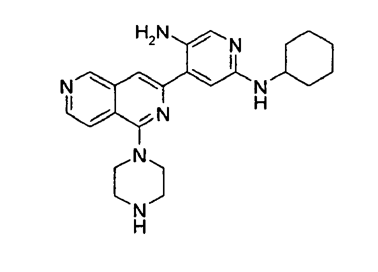

The title compound is prepared from bromide **Example 17D** by analogy to the procedure outlined in **Example 17R,** followed by deprotection of the BOC-group to afford the title piperidine: MS (ESI) m/z 404.2560 (M+1); ¹H NMR (400 MHz, MeOD) δ ppm 1.08 - 1.38 (m, 5 H), 1.52 - 1.62 (m, 1 H), 1.64 - 1.74 (m, 2 H), 1.86 - 1.96 (m, 2 H), 2.96 - 3.06 (m, 4 H), 3.24 - 3.47 (m, 4 H), 3.48 - 3.61 (m, 1 H), 5.36 - 5.49 (m, 1 H), 6.70 (s, 1 H), 7.66 (s, 1 H), 7.84 (s, 1 H), 7.90 (d, *J*=5.8 Hz, 1 H), 8.62 (d, *J*=5.8 Hz, 1 H), 9.36 (s, 1 H)

### O. 1-[3-(5-Methyl-2-cyclohexylamino-pyridin-4-yl)-[2,6]naphthyridin-1-yl]-piperidine-4-carboxylic acid isopropylamide.

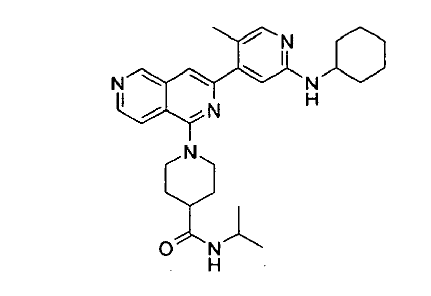

1-[3-(5-Bromo-2-cyclohexylamino-pyridin-4-yl)-[2,6]naphthyridin-1-yl]-piperidine-4-carboxylic acid ethyl ester **Example 17E** is reacted with trimethylboroxine according to procedure outlined in **Example 16L** to yield 1-[3-(5-methyl-2-cyclohexylamino-pyridin-4-yl)-[2,6]naphthyridin-1-yl]-piperidine-4-carboxylic acid ethyl ester, which is purified by column chromatography using 30 to 50% EtOAc/hexanes. Conversion of the ethyl ester by analogy to the preparation of amide **Example 17F** affords the title compound, which is purified by RP-HPLC on an X-Bridge 30x100 mm column using 20 to 100% CH₃CN/H₂O: MS (ESI) m/z 487.3 (M+1); ¹H NMR (400 MHz, MeOD) δ ppm 1.15 (d, *J*=6.6 Hz, 6 H), 1.18 - 1.35 (m, 3 H), 1.35 - 1.52 (m, 2 H), 1.60 - 1.72 (m, 1 H), 1.72 - 1.83 (m, 2 H), 1.85 - 1.95 (m, 2 H), 1.97 - 2.14 (m, 4 H), 2.24 (s, 3 H), 2.37 - 2.50 (m, 1 H), 3.00 - 3.11 (m, 2 H), 3.58 - 3.69 (m, 1 H), 3.92 - 4.08 (m, 3 H), 6.72 (s, 1 H), 7.60 (s, 1 H), 7.82 (s, 1 H), 7.97 (d, *J*=5.8 Hz, 1 H), 8.58 (d, *J*=5.8 Hz, 1 H), 9.25 (s, 1 H).

### P. 1-[3-(5-Cyclopropyl-2-cyclohexylamino-pyridin-4-yl)-[2,6]naphthyridin-1-yl]-piperidine-4-carboxylic acid isopropylamide.

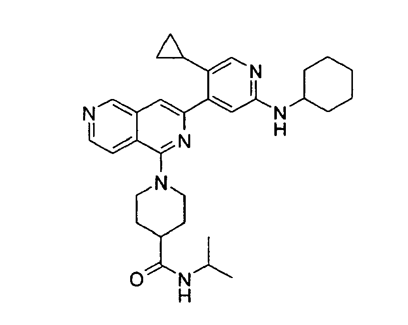

1 -[3-(5-Bromo-2-cydohexylamino-pyridin-4-yl)-[2,6]naphthyridin-1-yl]-piperidine-4-carboxylic acid ethyl ester **Example 17E** (200 mg, 0.371 mmol), cyclopropyl boronic acid (96 mg, 1.11 mmol), K₃PO₄ (315 mg, 1.49 mmol), and tricyclohexylphosphine (25 mg, 0.089 mmol) in toluene (5 mL) are degassed with nitrogen before Pd(OAc)₂ (10 mg, 0.0445 mmol) is added. The reaction is heate at 100 °C for 6 h. After cooling, the reaction is partitioned between CH₂Cl₂ and a saturated aqeous solution of NaHCO₃. The separated organic phase is washed with brine, dried (Na₂SO₄), and concentrated *in vacuo.* The residue is purified by flash chromatography (30 to 60% EtOAc/hexanes) to yield 1-[3-(5-cyclopropyl-2-cyclohexylamino-pyridin-4-yl)-[2,6]naphthyridin-1-yl]-piperidine-4-carboxylic acid ethyl ester.

The above ethyl ester is saponified and coupled to isopropylamine using HATU by analogy to above amides. The amide is purified by RP-HPLC using an X-Bridge 30x100 mm column and 25 to 100% CH₃CN/water to afford the title amide: MS (ESI) m/z 513.33 (M+1); ¹H NMR (400 MHz, MeOD) δ ppm 0.37 - 0.48 (m, 2 H), 0.64 - 0.77 (m, 2 H), 1.14 (d, *J*=6.6 Hz, 6 H), 1.18 - 1.32 (m, 3 H), 1.36 - 1.50 (m, 2 H), 1.56 - 1.72 (m, 1 H), 1.73 - 1.83 (m, 2 H), 1.84 - 1.93 (m, 2 H), 1.96 - 2.13 (m, 5 H), 2.34 - 2.50 (m, 1 H), 2.97 - 3.15 (m, 2 H), 3.56 - 3.69 (m, 1 H), 3.90 - 4.10 (m, 3 H), 6.72 (s, 1 H), 7.73 (s, 1 H), 7.79 (s, 1 H), 7.97 (d, *J*=6.1 Hz, 1 H), 8.56 (d, *J*=5.8 Hz, 1 H), 9.24 (s, 1 H).

### Q. 1-[3-(5-Cyano-2-cyclohexylamino-pyridin-4-yl)-[2,6]naphthyridin-1-yl]-piperidine-4-carboxylic acid isopropylamide.

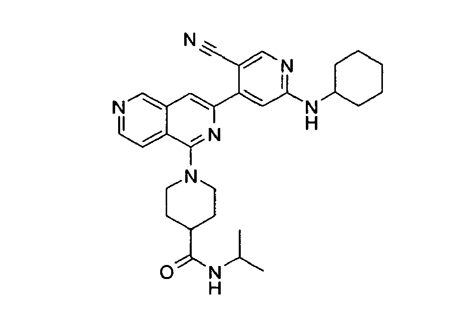

1-[3-(5-Bromo-2-cyclohexylamino-pyridin-4-yl)-[2,6]naphthyridin-1-yl]-piperidine-4-carboxylic acid ethyl ester **Example 17E** (200 mg, 0.371 mmol), Zn(CN)₂ (26 mg, 0.222 mmol), and dppf (25 mg, 0.0445 mmol) in DMF (4.0 mL) and water (0.1 mL) is degassed with nitrogen for 10 min. Pd₂(dba)₃ (17 mg, 0.0185 mmol) is added and the sealed reaction is heated to 120 °C for 20 h. After cooling, the reaction is diluted with CH₂Cl₂ and water. The separated organic phase is washed with brine, dried over Na₂SO₄, and concentrated *in vacuo.* The residue is purified by flash chromatography (30 to 60% EtOAc/hexanes) to afford 1-[3-(5-cyano-2-cyclohexylamino-pyridin-4-yl)-[2,6]naphthyridin-1-yl]-piperidine-4-carboxylic acid ethyl ester. The ethyl ester is saponified and coupled to isopropylamine using HATU by analogy to **Example 17F** above. The title compound is purified using RP-HPLC (X-Bridge 30x100 mm column and 25-100% CH₃CN/H₂O) to an off-white powder: MS (ESI) m/z 498.2986 (M+1); ¹H NMR (400 MHz, MeOD) δ ppm 1.14 (d, *J*=6.6 Hz, 6 H), 1.23 - 1.37 (m, 3 H), 1.37 - 1.52 (m, 2 H), 1.64 -1.73 (m, 1 H), 1.75 - 1.85 (m, 2 H), 1.85 - 1.94 (m, 2 H), 1.98 - 2.13 (m, 4 H), 2.37 - 2.52 (m, 1 H), 3.08 - 3.19 (m, 2 H), 3.79 - 3.92 (m, 1 H), 3.92 - 4.05 (m, 1 H), 4.13 - 4.24 (m, 2 H), 6.96 (s, 1 H), 7.88 (s, 1 H), 7.96 (d, *J*=5.8 Hz, 1 H), 8.39 (s, 1 H), 8.61 (d, *J*=6.1 Hz, 1 H), 9.29 (s, 1 H).

### R. 1-[3-(5-Amino-2-cyclohexylamino-pyridin-4-yl)-[2,6]naphthyridin-1-yl]-piperidine-4-carboxylic acid isopropylamide.

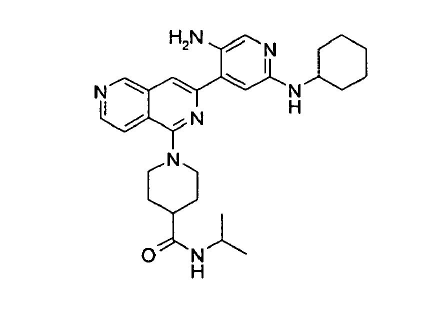

1-[3-(5-Bromo-2-cyclohexylamino-pyridin-4-yl)-[2,6]naphthyridin-1-yl]-piperidine-4-carboxylic acid isopropylamide **Example 17F** (270 mg, 0.49 mmol), benzophenone imine (337 mg, 1.85 mmol), BINAP (229 mg, 0.367 mmol), and NaOtBu (146 mg, 1.52 mmol) are suspended in toluene (5.0 mL). After the mixture is degassed with nitrogen, Pd₂(dba)₃ (112 mg, 0.123 mmol) is added. The reaction is heated at 100 °C for 16 h. After cooling, the reaction is partitioned between CH₂Cl₂ and a saturated aqueous solution of NaHCO₃. The separated organic phase is washed with brine, dried with Na₂SO₄, and concentrated under reduced pressure. The residue is purified by flash chromatography (20 to 50% EtOAc/hexanes) to afford the imine. Hydrolysis of the imine (110 mg, 0.168 mmol) in 4N HCl in dioxane (5.0 mL), THF (5.0 mL), and water (0.1 mL) is effected at room temperature overnight. The reaction is concentrated under reduced pressure. The residue is diluted with CH₂Cl₂ and a saturated aqueous solution of NaHCO₃ The separated organic phase is washed with brine, dried with Na₂SO₄, and concentrated in vacuo. The residue is purified by RP-HPLC on a C₁₈ X-Bridge 30x100 mm column using 25 to 100% CH₃CN/water to afford the title compound: MS (ESI) m/z 488.3147 (M+1); ¹H NMR (400 MHz, MeOD) δ ppm 1.16 (d, *J*=6.6 Hz, 6 H), 1.18 - 1.35 (m, 3 H), 1.36-1.51 (m, 2 H), 1.59 - 1.71 (m, 1 H), 1.72 - 1.84 (m, 2 H), 1.87 - 1.97 (m, 2 H), 1.98 - 2.15 (m, 4 H), 2.40 - 2.53 (m, 1 H), 3.01 - 3.14 (m, 2 H), 3.50 - 3.63 (m, 1 H), 3.92 - 4.09 (m, 3 H), 6.85 (s, 1 H), 7.72 (s, 1 H), 7.85 (s, 1 H), 7.95 (d, *J*=6.1 Hz, 1 H), 8.56 (d, *J*=5.8 Hz, 1 H), 9.28 (s, 1 H).

### Example 18

### A. 3-[2-(2-chloropyridin-4-yl)-2-oxoethyl]isonicotinonitrile and 3-[2-(2-methoxypyridin-4-yl)-2-oxoethyl]isonicotinonitrile.

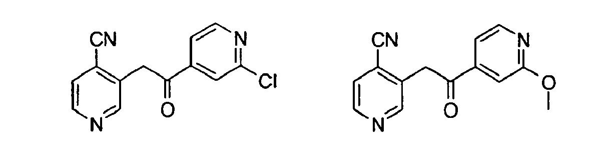

In a three-necked round-bottomed flask equipped with a reflux cooler, 60 % NaH in mineral oil (1.35 g, 33.9 mmol) is added to DME (70 mL). The suspension is heated to 95 °C and a solution of 3-methylisonicotinonitrile (1.00 g, 8.47 mmol) and 2-chloroisonicotinic acid methyl ester (2.18 g, 12.71 mmol) in DME (15 mL) is added. The resulting reaction mixture is stirred overnight at 95 °C. After cooling down to room temperature the reaction mixture is partitioned between EtOAc and water. The aqueous phase is extracted with EtOAc and the combined organic layers are dried over Na₂SO₄, filtered and concentrated *in vacuo* to afford a 1 : 2 mixture of 3-[2-(2-chloropyridin-4-yl)-2-oxoethyl]isonicotinonitrile and 3-[2-(2-methoxypyridin-4-yl)-2-oxoethyl]isonicotinonitrile as a brown solid (2.25 g, 8.47 mmol, 100%). 3-[2-(2-chloropyridin-4-yl)-2-oxoethyl]isonicotinonitrile: MS (ES⁺): 258 (M(C₁₃H₈CIN₃O)+H)⁺; 3-[2-(2-methoxy-pyridin-4-yl)-2-oxo-ethyl]-isonicotinonitrile: MS (ES⁺): 254 (M(C₁₄H₁₁N₃O₂)+H)⁺.

### B. N*1*-[3-(2-Chloropyridin-4-yl)-[2,6]naphthyridin-1-yl]-2-methylpropane-1,2-diamine.

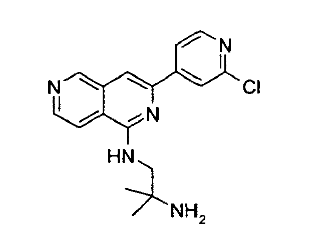

To a solution of a 1:2 mixture of 3-[2-(2-chloro-pyridin-4-yl)-2-oxo-ethyl]-isonicotinonitrile and 3-[2-(2-methoxypyridin-4-yl)-2-oxoethyl]isonicotinonitrile (1.5 g, 5.82 mmol) in a mixture of EtOAc acetic acid 7 : 3 (58 mL) is added 2-methylpropane-1,2-diamine (3.08 g, 34.9 mmol). Silica gel 60 (6.98 g) is added and the reaction mixture is stirred for 2 h at rt. The reaction mixture is filtered and the residue washed with EtOAc. The filtrate is concentrated by rotary evaporation and the residue is partitioned between EtOAc and an aqueous 1 M NaOH solution. The organic phase is dried over Na₂SO₄, filtered and concentrated at reduced pressure. Purification by preparative reverse phase HPLC affords the title compound as a yellow powder (256 mg, 0.780 mmol, 13%, TFA salt). ¹H NMR (400 MHz, DMSO-d₆): δ ppm 9.27 (s, 1 H), 8.72 (d, *J* = 5.4 Hz, 1 H), 8.54 (d, *J* = 5.4 Hz, 1 H), 8.27 (d, *J* = 5.9 Hz, 1 H), 8.22 (s, 1 H), 8.21 (d, *J* = 5.9 Hz, 1 H), 8.05 (s, 1 H), 8.01 (t, *J* = 6.1 Hz, 1 H), 7.97 (bs, 2 H), 3.91 (d, *J* = 6.1 Hz, 2 H), 1.37 (s, 6H). MS (ES⁺): 328 (M(C₁₇H₁₈CIN₅)+H)⁺.

### C. N*1*-[3.(2-Isopropylaminopyridin-4-yl)-[2,6]naphthyridin-1-yl]-2-methylpropane-1,2-diamine.

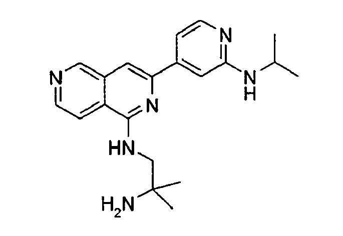

A suspension of *N**1*-[3-(2-chloropyridin-4-yl)-[2,6]naphthyridin-1-yl]-2-methylpropane-1,2-diamine (80.0 mg, 0.244 mmol) in toluene (10 mL) is purged with argon for 10 min. Subsequently, isopropylamine (36.1 mg, 0.610 mmol), Pd₂(dba)₃ (22.3 mg, 0.0244 mmol), BINAP (15.2 mg, 0.0244 mmol) and NaOtBu (117 mg, 1.22 mmol) are added and the resulting reaction mixture is heated for 18 h at 90°C under an argon atmosphere. The reaction mixture is filtered over hyflo and the filtrate concentrated *in vacuo.* The residue is purified by preparative reverse phase HPLC to afford the title compound as a yellow solid (24.0 mg, 0.0347 mmol, 14%, TFA salt). ¹H NMR (400 MHz, DMSO-*d*₆,): δ ppm 9.31 (s, 1 H), 8.76 (d, *J* = 5.7 Hz, 1 H), 8.25 (d, *J* = 5.7 Hz, 1 H), 8.08 - 7.97 (m, 3 H), 7.92 (s, 1 H), 7.78 (s, 1 H), 7.56 (d, *J* = 6.9 Hz, 1 H), 4.07-3.98 (m, 1 H), 3.89 (d, *J* = 5.9 Hz, 2 H), 1.35 (s, 6 H), 1.29 (d, *J* = 6.4 Hz, 6 H). MS (ES⁺): 351 (M(C₂₀H₂₆N₆)+H)⁺.

## Claims

1. A compound of formula (I): 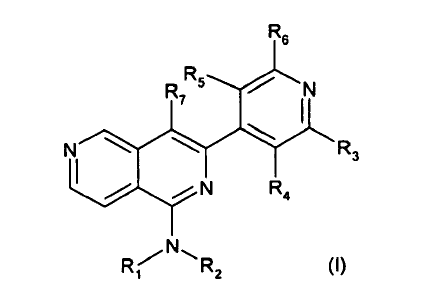 wherein
R₁ and R₂ are independently hydrogen, alkyl, cycloalkyl, heterocyclyl, each of which is optionally substituted by one to two R₈, wherein R₈ is hydrogen, halogen, alkyl, R₉-O--, (R₁₀)(R₁₁)N--, (R₁₂)(R₁₃)N-C(O)--, aryl, or heterocyclyl or heteroaryl, said heterocyclyl and heteroaryl are optionally substituted by one or two alkyl groups;
R₁ and R₂ taken together with the nitrogen atom to which they are attached to optionally form a 4-7 membered ring;
R₃ is (R₁₄)(R₁₅)N--, or halogen;
R₄, R₅, R₆ and R₇ are independently hydrogen, halogen, alkyl, (C₃-C₇) cycloalkyl, aryl-alkyl, aryl, or alkoxy;
R₉, R₁₀, R₁₁, R₁₂ and R₁₃ are independently hydrogen, alkyl-O-C(O)--, alkyl-NH-C(O)- -, alkyl-C(O)-NH-C(O)--, cycloalkyl, cycloalkyl-alkyl--, R₁₆-SO₂--, R₁₇-C(O)--, heterocyclyl or alkyl, said heterocyclyl is further optionally substituted by one or two cycloalkyl-alkylgroups, and said alkyl is further optionally substituted by one or two groups selected from hydroxy, alkoxy, alkylamine, dialkylamine, or heteroaryl;
R₁₀ and R₁₁ taken together with the nitrogen atom to which they are attached to optionally form a 5-7 membered ring;
R₁₂ and R₁₃ taken together with the nitrogen atom to which they are attached to optionally form a 5-7 membered ring;
R₁₄ and R₁₅ are independently hydrogen, alkyl, aryl, cycloalkyl, aryl-alkyl--, heterocyclyl or heteroaryl, said alkyl, cycloalkyl, aryl and heteroaryl are further optionally substituted by one or two groups selected from alkyl, alkoxy, hydroxy, halogen, haloalkyl, cyano, or R₁₈-NH-C(O)--;
R₁₆ is aryl or heteroaryl;
R₁₇ is heterocyclyl, or alkyl optionally substituted by one or two groups selected from H₂N--, aryl-alkyl--, or alkyl-C(O)-NH--;
R₁₈ is heterocyclyl-alkyl--; or
a pharmaceutically acceptable salt thereof; or an optical isomer thereof; or a mixture of optical isomers.

2. The compound of claim 1, wherein R₁ and R₂ are independently hydrogen, (C₁-C₇) alkyl, (C₃-C₇) cycloalkyl, (4-7 membered)-heterocyclyl, each of which is optionally substituted by one to two R₈, wherein R₈ is hydrogen, (C₁-C₇) alkyl, R₉-O--, (R₁₀)(R₁₁)N--, (R₁₂)(R₁₃)N-C(O)--, (C₆-C₁₀) aryl, (5-7 membered)-heteroaryl, or (4-7 membered)-heterocyclyl;
R₃ is (R₁₄)(R₁₅)N--, or halogen;
R₄ and R₅ are independently hydrogen, halogen, (C₁-C₇) alkyl, (C₃-C₇) cycloalkyl, or (C₁-C₇) alkoxy;
R₉, R₁₀, R₁₁, R₁₂ and R₁₃ are independently hydrogen, (C₁-C₇) alkyl-O-C(O)--, (C₁-C₇) alkyl-NH-C(O)--, (C₁-C₇) alkyl-C(O)-NH-C(O)--, (C₃-C₇) cycloalkyl, (C₃-C₇) cycloalkyl-(C₁-C₇) alkyl, R₁₆-SO₂--, R₁₇-C(O)--, (4-7 membered)-heterocyclyl or (C₁-C₇) alkyl, said (4-7 membered)-heterocyclyl is further optionally substituted by one or two (C₃-C₇) cycloalkyl-(C₁-C₇) alkyl groups, and said (C₁-C₇) alkyl is further optionally substituted by one or two groups selected from hydroxy, (C₁-C₇) alkoxy, (C₁-C₇) dialkylamine, or (5-7 membered)-heteroaryl;
R₁₂ and R₁₃ taken together with the nitrogen atom to which they are attached to optionally form a 5-7 membered ring;
R₁₄ and R₁₅ are independently hydrogen, (C₁-C₇) alkyl, (C₆-C₁₀) aryl, (C₃-C₇) cycloalkyl, (C₆-C₁₀) aryl-(C₁-C₇) alkyl--, (4-7 membered)-heterocyclyl or (5-7 membered)-heteroaryl, said (C₁-C₇) alkyl, (C₃-C₇) cycloalkyl, (C₆-C₁₀) aryl and (5-7 membered)-heteroaryl are further optionally substituted by one or two groups selected from (C₁-C₇)alkyl, (C₁-C₇) alkoxy, hydroxy, halogen, (C₁-C₇)haloalkyl, or R₁₄-NH-C(O)--;
R₁₆ is (C₆-C₁₀) aryl or (5-7 membered)-heteroaryl;
R₁₇ is (4-7 membered)-heterocyclyl, or (C₁-C₇)alkyl optionally substituted by one or two groups selected from H₂N--, (C₆-C₁₀) aryl-(C₁-C₇)alkyl--, or (C₁-C₇)alkyl-C(O)-NH--;
R₁₈ is (4-7 membered)-heterocyclyl-(C₁-C₇)alkyl--; or a pharmaceutically acceptable salt thereof; or an optical isomer thereof; or a mixture of optical isomers.

3. A pharmaceutical composition comprising a therapeutically effective amount of a compound of claim 1 and one or more pharmaceutically acceptable carriers.

4. A pharmaceutical composition comprising a therapeutically effective amount of the compound according to claim 1 and one or more therapeutically active agents selected from (i) HMG-Co-A reductase inhibitor or a pharmaceutically acceptable salt thereof; (ii) angiotensin II receptor antagonist or a pharmaceutically acceptable salt thereof; (iii) angiotensin converting enzyme (ACE) Inhibitor or a pharmaceutically acceptable salt thereof; (iv) calcium channel blocker (CCB) or a pharmaceutically acceptable salt thereof; (v) dual angiotensin converting enzyme/neutral endopeptidase (ACE/NEP) inhibitor or a pharmaceutically acceptable salt thereof; (vi) endothelin antagonist or a pharmaceutically acceptable salt thereof; (vii) renin inhibitor or a pharmaceutically acceptable salt thereof; (viii) diuretic or a pharmaceutically acceptable salt thereof; (ix) an ApoA-I mimic; (x) an antidiabetic agent; (xi) an obesity-reducing agent; (xii) an aldosterone receptor blocker; (xiii) an endothelin receptor blocker; and (xiv) CETP inhibitor.

5. A compound of formula I of claim 1 for use as a medicament.

6. A compound of formula I according to claim 1 for inhibiting PKD activity in a subject.

7. A compound of formula I according to claim 1 for the treatment of a disorder or a disease in a subject mediated by PKD.

8. A compound of formula I according to claim 1 for the treatment of a disorder or a disease in a subject **characterized by** an abnormal activity of PKD.

9. A compound of formula I according to claim 1 for the treatment of a disorder or a disease **characterized by** an abnormal expression of PKD.

10. A compound of formula I according to claim 1 for the treatment of a disorder or a disease in a subject wherein the disorder or the disease is selected from heart failure, colorectal cancer, regulation of cell growth, autoimmune disorders, or hyperproliferative skin disorders.

11. Use of a compound of formula I according to claim 1, for the preparation of a pharmaceutical composition for the treatment of a disorder or disease in a subject mediated by PKD.

12. Use of a compound of formula I according to claim 1, for the preparation of a pharmaceutical composition for the treatment of a disorder or disease in a subject **characterized by** an abnormal activity of PKD.

13. Use of a compound of formula I according to claim 1, for the preparation of a pharmaceutical composition for the treatment of a disorder or disease in a subject **characterized by** an abnormal expression of PKD.

14. Use of a pharmaceutical composition according to claim 8 or 9 for the preparation of a medicament for the treatment of a disorder or disease in a subject mediated by PKD.

15. Use of a pharmaceutical composition according to claim 8 or 9 for the preparation of a medicament for the treatment of a disorder or disease in a subject **characterized by** an abnormal activity of PKD.

16. Use of a pharmaceutical composition according to claim 8 or 9 for the preparation of a medicament for the treatment of a disorder or disease in a subject **characterized by** an abnormal expression of PKD.

17. The use of claim 11, wherein the disorder or disease is selected from heart failure, colorectal cancer, regulation of cell growth, autoimmune disorders, or hyperproliferative skin disorders.

## Patentansprüche

1. Verbindungen der Formel (I) : 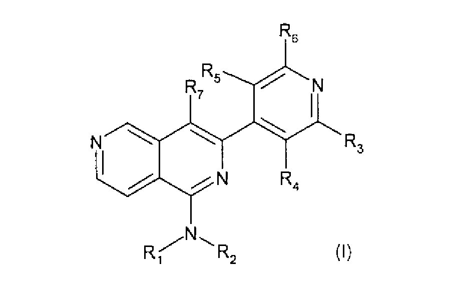 wobei
R₁ und R₂ unabhängig voneinander für Wasserstoff, Alkyl, Cycloalkyl oder Heterocyclyl stehen, die jeweils gegebenenfalls durch einen oder zwei Reste R₈ substituiert sind, wobei R₈ für Wasserstoff, Halogen, Alkyl, R₉-O--, (R₁₀)(R₁₁)N--, (R₁₂)(R₁₃)N-C(O)--, Aryl oder Heterocyclyl oder Heteroaryl steht, wobei Heterocyclyl und Heteroaryl gegebenenfalls durch eine oder zwei Alkylgruppen substituiert sind;
R₁ und R₂ zusammen mit dem Stickstoffatom, an das sie gebunden sind, gegebenenfalls einen 4- bis 7- gliedrigen Ring bilden;
R₃ für (R₁₄) (R₁₅)N-- oder Halogen steht;
R₄, R₅, R₆ und R₇ unabhängig voneinander für Wasserstoff, Halogen, Alkyl, (C₃-C₇)-Cycloalkyl, Arylalkyl, Aryl oder Alkoxy stehen;
R₉, R₁₀, R₁₁, R₁₂ und R₁₃ unabhängig voneinander für Wasserstoff, Alkyl-O-C(O)--, Alkyl-NH-C(O)--, Alkyl-C(O)-NH-C(O)--, Cycloalkyl, Cycloalkylalkyl--, R₁₆-SO₂--, R₁₇-C(O)--, Heterocyclyl oder Alkyl stehen, wobei Heterocyclyl gegebenenfalls weiter durch eine oder zwei Cycloalkylalkyl-Gruppen substituiert ist und wobei Alkyl gegebenenfalls weiter durch eine oder zwei Gruppen ausgewählt aus Hydroxy, Alkoxy, Alkylamin, Dialkylamin und Heteroaryl substituiert ist;
R₁₀ und R₁₁ zusammen mit dem Stickstoffatom, an das sie gebunden sind, gegebenenfalls einen 5- bis 7-gliedrigen Ring bilden;
R₁₂ und R₁₃ zusammen mit dem Stickstoffatom, an das sie gebunden sind, gegebenenfalls einen 5- bis 7-gliedrigen Ring bilden;
R₁₄ und R₁₅ unabhängig voneinander für Wasserstoff, Alkyl, Aryl, Cycloalkyl, Arylalkyl--, Heterocyclyl oder Heteroaryl stehen, wobei Alkyl, Cycloalkyl, Aryl und Heteroaryl gegebenenfalls weiter substituiert sind durch eine oder zwei Gruppen ausgewählt aus Alkyl, Alkoxy, Hydroxy, Halogen, Halogenalkyl, Cyano und R₁₈-NH-C(O)--;
R₁₆ für Aryl oder Heteroaryl steht;
R₁₇ für Heterocyclyl oder Alkyl, gegebenenfalls substituiert durch eine oder zwei Gruppen ausgewählt aus H₂N--, Arylalkyl-- und Alkyl-C(O)-NH--, steht;
R₁₈ für Heterocyclylalkyl-- steht und
deren pharmazeutisch unbedenkliche Salze und deren optische Isomere und Mischungen optischer Isomere.

2. Verbindungen nach Anspruch 1, wobei R₁ und R₂ unabhängig voneinander für Wasserstoff, (C₁-C₇)-Alkyl, (C₃-C₇)-Cycloalkyl, (4- bis 7-gliedriges) Heterocyclyl stehen, die jeweils gegebenenfalls durch einen oder zwei Reste R₈ substituiert sind, wobei R₈ für Wasserstoff, (C₁-C₇)-Alkyl, Rg-O--, (R₁₀)(R₁₁)N--, (R₁₂) (R₁₃)N-C(O)--, (C₆-C₁₀)-Aryl, (5- bis 7-gliedriges) Heteroaryl oder (4- bis 7-gliedriges) Heterocyclyl steht;
R₃ für (R₁₄)(R₁₅) N-- oder Halogen steht;
R₄ und R₅ unabhängig voneinander für Wasserstoff, Halogen, (C₁-C₇) -Alkyl, (C₃-C₇) -Cycloalkyl oder (C₁-C₇)-Alkoxy stehen;
R₉, R₁₀, R₁₁, R₁₂ und R₁₃ unabhängig voneinander für Wasserstoff, (C₁-C₇)-Alkyl-O-C(O)--, (C₁-C₇)-Alkyl-NH-C(O)--, (C₁-C-₇)-Alkyl-C(O)-NH-C(O)--, (C₃-C₇)-Cycloalkyl, (C₃-C₇)-Cycloalkyl-(C₁-C₇)-alkyl, R₁₆-SO₂--, R₁₇-C(O)--, (4- bis 7-gliedriges) Heterocyclyl oder (C₁-C₇)-Alkyl stehen, wobei (4- bis 7-gliedriges) Heterocyclyl gegebenenfalls weiter substituiert ist durch eine oder zwei (C₃-C-₇)-Cycloalkyl-(C₁-C₇)-alkylgruppen und (C₁-C₇)-Alkyl gegebenenfalls weiter substituiert ist durch eine oder zwei Gruppen ausgewählt aus Hydroxy, (C₁-C₇)-Alkoxy, (C₁-C-₇)-Dialkylamin und (5- bis 7-gliedriges) Heteroaryl;
R₁₂ und R₁₃ zusammen mit dem Stickstoffatom, an das sie gebunden sind, gegebenenfalls einen 5- bis 7-gliedrigen Ring bilden;
R₁₄ und R₁₅ unabhängig voneinander für Wasserstoff, (C₁-C-₇)-Alkyl, (C₆-C₁₀)-Aryl, (C₃-C₇)-Cycloalkyl, (C₆-C₁₀)-Aryl-(C₁-C₇)-alkyl--, (4- bis 7-gliedriges) Heterocyclyl oder (5- bis 7-gliedriges) Heteroaryl stehen, wobei (C₁-C₇)-Alkyl, (C₃-C₇)-Cycloalkyl, (C₆-C₁₀)-Aryl und (5- bis 7-gliedriges) Heteroaryl gegebenenfalls weiter substituiert sind durch eine oder zwei Gruppen ausgewählt aus (C₁-C₇) -alkyl, (C₁-C₇)-Alkoxy, Hydroxy, Halogen, (C₁-C-₇)-Halogenalkyl und R₁₄-NH-C(O)--;
R₁₆ für (C₆-C₁₀)-Aryl oder (5- bis 7-gliedriges) Heteroaryl steht;
R₁₇ für (4- bis 7-gliedriges) Heterocyclyl oder (C₁-C₇)-Alkyl, gegebenenfalls substituiert durch eine oder zwei Gruppen ausgewählt aus H₂N--, (C₆-C₁₀)-Aryl-(C₁-C₇)-alkyl-- und (C₁-C₇)-Alkyl-C(O)-NH--, steht;
R₁₈ für (4- bis 7-gliedriges) Heterocyclyl-(C₁-C₇)-alkyl-- steht und deren pharmazeutisch unbedenkliche Salze und deren optische Isomere und Mischungen optischer Isomere.

3. Pharmazeutische Zusammensetzung, enthaltend eine therapeutisch wirksame Menge einer Verbindung nach Anspruch 1 und einen oder mehrere pharmazeutisch unbedenkliche Träger.

4. Pharmazeutische Zusammensetzung, enthaltend eine therapeutisch wirksame Menge der Verbindung nach Anspruch 1 und ein oder mehrere therapeutisch wirksame Mittel, ausgewählt aus (i) HMG-Co-A-Reduktaseinhibitoren und deren pharmazeutisch unbedenklichen Salzen; (ii) Angiotensin-II-Rezeptorantagonisten und deren pharmazeutisch unbedenklichen Salzen; (iii) Inhibitoren des Angiotensin Converting Enzyme (ACE) und deren pharmazeutisch unbedenklichen Salzen; (iv) Calciumkanalblockern (CCB) und deren pharmazeutisch unbedenklichen Salzen; (v) dualen Inhibitoren von Angiotensin Converting Enzyme-/Neutral Endopeptidase-(ACE/NEP-) Inhibitoren und deren pharmazeutisch unbedenklichen Salzen; (vi) Endothelinantagonisten und deren pharmazeutisch unbedenklichen Salzen; (vii) Renininhibitoren und deren pharmazeutisch unbedenklichen Salzen; (viii) Diuretika und deren pharmazeutisch unbedenklichen Salzen; (ix) ApoA-I-Mimetika; (x) Antidiabetika; (xi) abesitasreduzierenden Mitteln; (xii) Aldosteronrezeptorblockern; (xiii) Endothelinrezeptorblockern und (xiv) CETP-Inhibitoren.

5. Verbindungen der Formel I nach Anspruch 1 zur Verwendung als Medikament.

6. Verbindungen der Formel I nach Anspruch 1 zur Inhibierung der PKD-Aktivität in einem Patienten.

7. Verbindungen der Formel I nach Anspruch 1 zur Behandlung einer durch PKD vermittelten Erkrankung oder Krankheit in einem Patienten.

8. Verbindungen der Formel I nach Anspruch 1 zur Behandlung einer durch eine anormale Aktivität von PKD charakterisierten Erkrankung oder Krankheit in einem Patienten.

9. Verbindungen der Formel I nach Anspruch 1 zur Behandlung einer durch eine anormale Expression von PKD charakterisierten Erkrankung oder Krankheit.

10. Verbindungen der Formel I nach Anspruch 1 zur Behandlung einer Erkrankung oder Krankheit in einem Patienten, wobei die Erkrankung oder Krankheit aus Herzinsuffizienz, Kolorektalkrebs, Zellwachstumsregulierung, 1 Autoimmunerkrankungen und hyperproliferativen Hauterkrankungen ausgewählt ist.

11. Verwendung einer Verbindung der Formel I nach Anspruch 1 zur Herstellung einer pharmazeutischen Zusammensetzung zur Behandlung einer durch PKD vermittelten Erkrankung oder Krankheit in einem Patienten.

12. Verwendung einer Verbindung der Formel I nach Anspruch I zur Herstellung einer pharmazeutischen Zusammensetzung zur Behandlung einer durch eine anormale Aktivität von PKD charakterisierten Erkrankung oder Krankheit in einem Patienten.

13. Verwendung einer Verbindung der Formel I nach Anspruch 1 zur Herstellung einer pharmazeutischen Zusammensetzung zur Behandlung einer durch eine anormale Expression von PKD charakterisierten Erkrankung oder Krankheit in einem Patienten.

14. Verwendung einer pharmazeutischen Zusammensetzung nach Anspruch 8 oder 9 zur Herstellung eines Medikaments zur Behandlung einer durch PKD vermittelten Erkrankung oder Krankheit in einem Patienten.

15. Verwendung einer pharmazeutischen Zusammensetzung nach Anspruch 8 oder 9 zur Herstellung eines Medikaments zur Behandlung einer durch eine anormale Aktivität von PKD charakterisierten Erkrankung oder Krankheit in einem Patienten.

16. Verwendung einer pharmazeutischen Zusammensetzung nach Anspruch 8 oder 9 zur Herstellung eines Medikaments zur Behandlung einer durch eine anormale Expression von PKD charakterisierten Erkrankung oder Krankheit in einem Patienten.

17. Verwendung nach Anspruch 11, wobei die Erkrankung oder Krankheit aus Herzinsuffizienz, Kolorektalkrebs, Zellwachstumsregulierung, Autoimmunerkrankungen und hyperproliferativen Hauterkrankungen ausgewählt ist.

## Revendications

1. Composé de formule (I) : 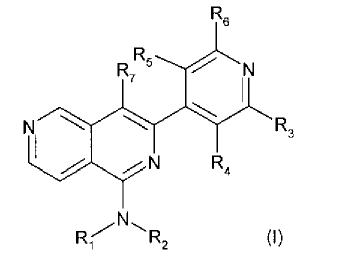 dans laquelle
R₁ et R₂ sont indépendamment un hydrogène, un alkyle, un cycloalkyle, un hétérocyclyle, dont chacun est facultativement substitué par un à deux R₈, où R₈ est un hydrogène, un halogène, un alkyle, R₉-O-, (R₁₀)(R₁₁)N-, (R₁₂)(R₁₃)N-C (O) -, un aryle, ou un hétérocyclyle ou un hétéroaryle, lesdits hétérocyclyle et hétéroaryle sont facultativement substitués par un ou deux groupes alkyle ;
R₁ et R₂ conjointement avec l'atome d'azote auquel ils sont liés forment facultativement un cycle de 4 à 7 chaînons ;
R₃ est (R₁₄)(R₁₅)N-, ou un halogène ;
R₄, R₅, R₆ et R₇ sont indépendamment un hydrogène, un halogène, un alkyle, un cycloalkyle en C₃-C₇, un aryl-alkyle, un aryle, ou un alcoxy ;
R₉, R₁₀, R₁₁, R₁₂ et R₁₃ sont indépendamment un hydrogène, un alkyl-O-C(O)-, un alkyl-NH-C(O)-, un alkyl-C(O)-NH-C(O)-, un cycloalkyle, un cycloalkyl-alkyl-, R₁₆-SO₂-, R₁₇-C(O)-, un hétérocyclyle ou un alkyle, ledit hétérocyclyle est en outre facultativement substitué par un ou deux groupes cycloalkyl-alkyl-, et ledit alkyle est en outre facultativement substitué par un ou deux groupes choisis parmi un hydroxy, un alcoxy, une alkylamine, une dialkylamine, ou un hétéroaryle ;
R₁₀ et R₁₁ conjointement avec l'atome d'azote auquel ils sont liés forment facultativement un cycle de 5 à 7 chaînons ;
R₁₂ et R₁₃ conjointement avec l'atome d'azote auquel ils sont liés forment facultativement un cycle de 5 à 7 chaînons ;
R₁₄ et R₁₅ sont indépendamment un hydrogène, un alkyl, un aryle, un cycloalkyle, un aryl-alkyl-, un hétérocyclyle ou un hétéroaryle, lesdits alkyle, cycloalkyle, aryle et hétéroaryle sont en outre facultativement substitués par un ou deux groupes choisis parmi un alkyle, un alcoxy, un hydroxyl, un halogène, un halogénoalkyle, un cyano, ou R₁₅-NH-C(O)- ;
R₁₆ est un aryle ou un hétéroaryle ;
R₁₇ est un hétérocyclyle, ou un alkyle facultativement substitué par un ou deux groupes choisis parmi H₂N-, un aryl-alkyl-, ou alkyl-C(O)-NH-
R₁₈ est un hétérocyclyl-alkyl- ; ou
un sel pharmaceutiquement acceptable de celui-ci ; ou un isomère optique de celui-ci ; ou un mélange d'isomères optiques.

2. Composé de la revendication 1, dans lequel R₁ et R₂ sont indépendamment un hydrogène, un alkyle en C₁-C₇, un cycloalkyle en C₃-C₇, un hétérocyclyle de 4 à 7 chaînons, dont chacun est facultativement substitué par un à deux R₈, où R₈ est un hydrogène, un alkyle en C₁-C₇, R₉-O-, (R₁₀)(R₁₁)N-, (R₁₂)(R₁₃)N-C(O)-, un aryle en C₆-C₁₀, un hétéroaryle de 5 à 7 chaînons, ou un hétérocyclyle de à 7 chaînons ;
R₃ est (R₁₄) (R₁₅) N-, ou un halogène ;
R₄ et R₅ sont indépendamment un hydrogène, un halogène, un alkyle en C₁-C₇, un cycloalkyle en C₃-C₇, ou un alcoxy en C₁-C₇ ;
R₉, R₁₀, R₁₁, R₁₂ et R₁₃ sont indépendamment un hydrogène, (alkyle en C₁-C-₇)-C-C(C)-, (alkyle en C₁-C₇)-NH-C(O)-, (alkyle en C₁-C₇)-C(O)-NH-C(O)-, un cycloalkyle en C₃-C₇, (cycloalkyle en C₃-C₇)-(alkyle en C₁-C₇), R₁₆-SO₂-, R₁₇-C(O)-, un hétérocyclyle de 4 à 7 chaînons ou un alkyle en C₁-C₇, ledit hétérocyclyle de 4 à 7 chaînons est en outre facultativement substitué par un ou deux groupes (cycloalkyle en C₃-C₇)-(alkyle en C₁-C₇), et ledit alkyle en C₁-C₇ est en outre facultativement substitué par un ou deux groupes choisis parmi un hydroxy, un alcoxy en C₁-C₇, une dialkylamine en C₁-C₇, ou un hétéroaryle de 5 à 7 chaînons ;
R₁₂ et R₁₃ conjointement avec l'atome d'azote auquel ils sont liés forment facultativement un cycle de 5 à 7 chaînons ;
R₁₄ et R₁₅ sont indépendamment un hydrogène, un alkyle en C₁-C₇, un aryle en C₆-C₁₀, un cycloalkyle en C₃-C₇, un (aryle en C₆-C₁₀)-(alkyle en C₁-C₇)-, un hétérocyclyle de 4 à 7 chaînons ou un hétéroaryle de 5 à 7 chaînons, lesdits alkyle en C₁-C₇, cycloalkyl, en C₃-C₇, aryle en C₆-C₁₀ et hétéroaryle de 5 à 7 chaînons sont en outre facultativement substitués par un ou deux groupes choisis parmi un alkyle en C₁-C₇, un alcoxy en C₁-C₇, un hydroxy, un halogène, un halogénoalkyle en C₁-C₇, ou R₁₉-NH-C(O)- ;
R₁₆ est un aryle en C₆-C₁₀ ou un hétéroaryle de 5 à 7 chaînons ;
R₁₇ est un hétérocyclyle de 4 à 7 chaînons, ou un alkyle en C₁-C₇ facultativement substitué par un ou deux groupes choisis parmi H₂N-, (aryle en C₆-C₁₀) - (alkyle en C₁-C₇)-, ou (alkyle en C₁-C₇)-C(O)-NH- ;
R₁₈ est un hétérocyclyle de 4 à 7 chaînons- (alkyle en C₁-C₇)- ; ou un sel pharmaceutiquement acceptable de celui-ci ; ou un isomère optique de celui-ci ; ou un mélange d'isomères optiques.

3. Composition pharmaceutique comprenant une quantité thérapeutiquement efficace d'un composé de la revendication 1 et un ou plusieurs véhicules pharmaceutiquement acceptables.

4. Composition pharmaceutique comprenant une quantité thérapeutiquement efficace du composé selon la revendication 1 et un ou plusieurs agents thérapeutiquement actifs choisis parmi (i) un inhibiteur de HMG-Co-A réductase ou un sel pharmaceutiquement acceptable de celui-ci ; (ii) un antagoniste de récepteur d'angiotensine II ou un sel pharmaceutiquement acceptable de celui-ci ; (iii) un inhibiteur d'enzyme de conversion d'angiotensine (ACE) ou un sel pharmaceutiquement acceptable de celui-ci ; (iv) un inhibiteur calcique (CCB) ou un sel pharmaceutiquement acceptable de celui-ci ; (v) un double inhibiteur d'enzyme de conversion d'angiotensine/endopeptidase neutre (ACE/NEP) ou un sel pharmaceutiquement acceptable de celui-ci ; (vi) un antagoniste d'endothéline ou un sel pharmaceutiquement acceptable de celui-ci ; (vii) un inhibiteur de rénine ou un sel pharmaceutiquement acceptable de celui-ci ; (viii) un diurétique ou un sel pharmaceutiquement acceptable de celui-ci ; (ix) un mimétique d'ApoA-I ; (x) un agent antidiabétique ; (xi) un agent de réduction de l'obésité ; (xii) un inhibiteur de récepteur d'aldostérone ; (xiii) un inhibiteur de récepteur d'endothéline ; et (xiv) un inhibiteur de CETP.

5. Composé de formule I de la revendication 1 pour utilisation en tant que médicament.

6. Composé de formule I selon la revendication 1 pour inhiber l'activité PKD chez un sujet.

7. Composé de formule I selon la revendication 1 pour le traitement d'un trouble ou une maladie chez un sujet médié par PKD.

8. Composé de formule I selon la revendication 1 pour le traitement d'un trouble ou une maladie chez un sujet **caractérisé par** une activité anormale de PKD.

9. Composé de formule I selon la revendication 1 pour le traitement d'un trouble ou une maladie chez un sujet **caractérisé par** une expression anormale de PKD.

10. Composé de formule I selon la revendication 1 pour le traitement d'un trouble ou une maladie chez un sujet dans lequel le trouble ou la maladie est choisi parmi l'insuffisance cardiaque, le cancer colorectal, la régulation de la croissance cellulaire, des troubles auto-immuns, ou des troubles cutanés hyperprolifératifs.

11. Utilisation d'un composé de formule I selon la revendication 1, pour la préparation d'une composition pharmaceutique pour le traitement d'un trouble ou une maladie chez un sujet médié par PKD.

12. Utilisation d'un composé de formule I selon la revendication 1, pour la préparation d'une composition pharmaceutique pour le traitement d'un trouble ou une maladie chez un sujet **caractérisé par** une activité anormale de PKD.

13. Utilisation d'un composé de formule I selon la revendication 1, pour la préparation d'une composition pharmaceutique pour le traitement d'un trouble ou une maladie chez un sujet **caractérisé par** une expression anormale de PKD.

14. Utilisation d'une composition pharmaceutique selon la revendication 8 ou 9 pour la préparation d'un médicament pour le traitement d'un trouble ou une maladie chez un sujet médié par PKD.

15. Utilisation d'une composition pharmaceutique selon la revendication 8 ou 9 pour la préparation d'un médicament pour le traitement d'un trouble ou une maladie chez un sujet **caractérisé par** une activité anormale de PKD.

16. Utilisation d'une composition pharmaceutique selon la revendication 8 ou 9 pour la préparation d'un médicament pour le traitement d'un trouble ou une maladie chez un sujet **caractérisé par** une expression anormale de PKD.

17. Utilisation de la revendication 11, dans laquelle le trouble ou la maladie est choisi parmi l'insuffisance cardiaque, le cancer colorectal, la régulation de la croissance cellulaire, des troubles auto--immuns, ou des troubles cutanés hyperprolifératifs.
